(19) Europäisches Patentamt
European Patent Office
Office européen des brevets

(11) **EP 3 875 609 A1**

(12) **EUROPEAN PATENT APPLICATION**

(43) Date of publication:
**08.09.2021 Bulletin 2021/36**

(51) Int Cl.:
***C12Q 1/6886*** *(2018.01)*

(21) Application number: **20161181.1**

(22) Date of filing: **05.03.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **HOFFMANN, Ralf Dieter**
  **5656 AE Eindhoven (NL)**
- **ORSEL, Joukje Garrelina**
  **5656 AE Eindhoven (NL)**
- **DE KLERK - STARMANS, Maud**
  **5656 AE Eindhoven (NL)**
- **VAN LIESHOUT, Ron Martinus Laurentius**
  **5656 AE Eindhoven (NL)**

(74) Representative: **Philips Intellectual Property & Standards**
**High Tech Campus 5**
**5656 AE Eindhoven (NL)**

# (54) PREDICTION OF RADIOTHERAPY RESPONSE FOR PROSTATE CANCER SUBJECT BASED ON T-CELL RECEPTOR SIGNALING GENES

(57) The invention relates to a method of predicting a response of a prostate cancer subject to radiotherapy, comprising determining or receiving the result of a determination of a gene expression profile for each of one or more T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said gene expression profile(s) being determined in a biological sample obtained from the subject, and determining the prediction of the radiotherapy response based on the gene expression profile(s) for the one or more T-Cell receptor signaling genes.

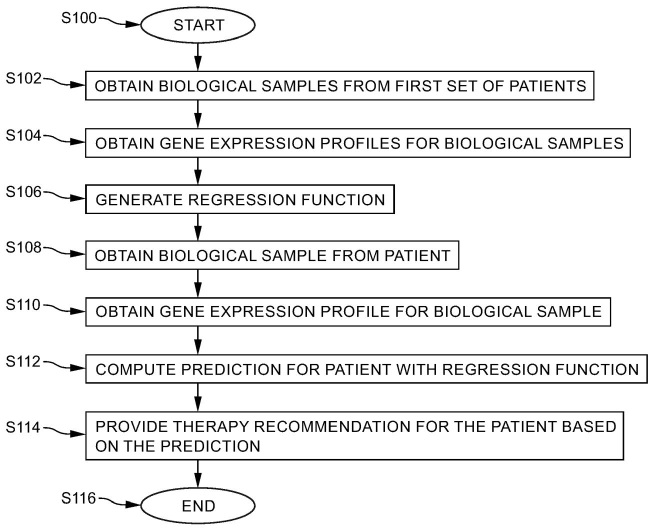


FIG. 1

EP 3 875 609 A1

SEQUENCE LISTING

<110> Koninklijke Philips N.V.

<120> Prediction of radiotherapy response for prostate cancer subject based on T-Cell Receptor signaling genes

<130> 2019PF00712

<160> 86

<170> PatentIn version 3.5

<210> 1
<211> 1565
<212> DNA
<213> Homo sapiens

<400> 1
agtctcactt cagttccttt tgcatgaaga gctcagaatc aaaagaggaa accaacccct 60

aagatgagct ttccatgtaa atttgtagcc agcttccttc tgattttcaa tgtttcttcc 120

aaaggtgcag tctccaaaga gattacgaat gccttggaaa cctggggtgc cttgggtcag 180

gacatcaact tggacattcc tagttttcaa atgagtgatg atattgacga tataaaatgg 240

gaaaaaactt cagacaagaa aaagattgca caattcagaa aagagaaaga gactttcaag 300

gaaaaagata catataagct atttaaaaat ggaactctga aaattaagca tctgaagacc 360

gatgatcagg atatctacaa ggtatcaata tatgatacaa aaggaaaaaa tgtgttggaa 420

aaaatatttg atttgaagat tcaagagagg gtctcaaaac caaagatctc ctggacttgt 480

atcaacacaa ccctgacctg tgaggtaatg aatggaactg accccgaatt aaacctgtat 540

caagatggga aacatctaaa actttctcag agggtcatca cacacaagtg gaccaccagc 600

ctgagtgcaa aattcaagtg cacagcaggg aacaaagtca gcaaggaatc cagtgtcgag 660

cctgtcagct gtccagagaa aggtctggac atctatctca tcattggcat atgtggagga 720

ggcagcctct tgatggtctt tgtggcactg ctcgttttct atatcaccaa aaggaaaaaa 780

cagaggagtc ggagaaatga tgaggagctg gagacaagag cccacagagt agctactgaa 840

gaaaggggcc ggaagcccca ccaaattcca gcttcaaccc ctcagaatcc agcaacttcc 900

caacatcctc ctccaccacc tggtcatcgt tcccaggcac ctagtcatcg tcccccgcct 960

cctggacacc gtgttcagca ccagcctcag aagaggcctc ctgctccgtc gggcacacaa 1020

gttcaccagc agaaaggccc gcccctcccc agacctcgag ttcagccaaa acctccccat 1080

ggggcagcag aaaactcatt gtccccttcc tctaattaaa aaagatagaa actgtctttt 1140

tcaataaaaa gcactgtgga tttctgccct cctgatgtgc atatccgtac ttccatgagg 1200

tgttttctgt gtgcagaaca ttgtcacctc ctgaggctgt gggccacagc cacctctgca 1260

tcttcgaact cagccatgtg gtcaacatct ggagtttttg gtctcctcag agagctccat 1320

```
cacaccagta aggagaagca atataagtgt gattgcaaga atggtagagg accgagcaca      1380

gaaatcttag agatttcttg tcccctctca ggtcatgtgt agatgcgata aatcaagtga      1440

ttggtgtgcc tgggtctcac tacaagcagc ctatctgctt aagagactct ggagtttctt      1500

atgtgccctg gtggacactt gcccaccatc ctgtgagtaa aagtgaaata aaagctttga      1560

ctaga                                                                  1565


<210>  2
<211>  351
<212>  PRT
<213>  Homo sapiens

<400>  2

Met Ser Phe Pro Cys Lys Phe Val Ala Ser Phe Leu Leu Ile Phe Asn
1               5                   10                  15

Val Ser Ser Lys Gly Ala Val Ser Lys Glu Ile Thr Asn Ala Leu Glu
            20                  25                  30

Thr Trp Gly Ala Leu Gly Gln Asp Ile Asn Leu Asp Ile Pro Ser Phe
        35                  40                  45

Gln Met Ser Asp Asp Ile Asp Asp Ile Lys Trp Glu Lys Thr Ser Asp
    50                  55                  60

Lys Lys Lys Ile Ala Gln Phe Arg Lys Glu Lys Glu Thr Phe Lys Glu
65                  70                  75                  80

Lys Asp Thr Tyr Lys Leu Phe Lys Asn Gly Thr Leu Lys Ile Lys His
                85                  90                  95

Leu Lys Thr Asp Asp Gln Asp Ile Tyr Lys Val Ser Ile Tyr Asp Thr
            100                 105                 110

Lys Gly Lys Asn Val Leu Glu Lys Ile Phe Asp Leu Lys Ile Gln Glu
        115                 120                 125

Arg Val Ser Lys Pro Lys Ile Ser Trp Thr Cys Ile Asn Thr Thr Leu
    130                 135                 140

Thr Cys Glu Val Met Asn Gly Thr Asp Pro Glu Leu Asn Leu Tyr Gln
145                 150                 155                 160

Asp Gly Lys His Leu Lys Leu Ser Gln Arg Val Ile Thr His Lys Trp
                165                 170                 175

Thr Thr Ser Leu Ser Ala Lys Phe Lys Cys Thr Ala Gly Asn Lys Val
```

180 185 190

Ser Lys Glu Ser Ser Val Glu Pro Val Ser Cys Pro Glu Lys Gly Leu
195 200 205

Asp Ile Tyr Leu Ile Ile Gly Ile Cys Gly Gly Gly Ser Leu Leu Met
210 215 220

Val Phe Val Ala Leu Leu Val Phe Tyr Ile Thr Lys Arg Lys Lys Gln
225 230 235 240

Arg Ser Arg Arg Asn Asp Glu Glu Leu Glu Thr Arg Ala His Arg Val
245 250 255

Ala Thr Glu Glu Arg Gly Arg Lys Pro His Gln Ile Pro Ala Ser Thr
260 265 270

Pro Gln Asn Pro Ala Thr Ser Gln His Pro Pro Pro Pro Pro Gly His
275 280 285

Arg Ser Gln Ala Pro Ser His Arg Pro Pro Pro Pro Gly His Arg Val
290 295 300

Gln His Gln Pro Gln Lys Arg Pro Pro Ala Pro Ser Gly Thr Gln Val
305 310 315 320

His Gln Gln Lys Gly Pro Pro Leu Pro Arg Pro Arg Val Gln Pro Lys
325 330 335

Pro Pro His Gly Ala Ala Glu Asn Ser Leu Ser Pro Ser Ser Asn
340 345 350

<210> 3
<211> 1674
<212> DNA
<213> Homo sapiens

<400> 3
gtcctccact tcctggggag gtagctgcag aataaaacca gcagagactc cttttctcct 60

aaccgtcccg gccaccgctg cctcagcctc tgcctcccag cctctttctg agggaaagga 120

caagatgaag tggaaggcgc ttttcaccgc ggccatcctg caggcacagt tgccgattac 180

agaggcacag agctttggcc tgctggatcc caaactctgc tacctgctgg atggaatcct 240

cttcatctat ggtgtcattc tcactgcctt gttcctgaga gtgaagttca gcaggagcgc 300

agacgccccc gcgtaccagc agggccagaa ccagctctat aacgagctca atctaggacg 360

aagagaggag tacgatgttt tggacaagag acgtggccgg gaccctgaga tggggggaaa 420

```
gccgagaagg aagaaccctc aggaaggcct gtacaatgaa ctgcagaaag ataagatggc      480
ggaggcctac agtgagattg ggatgaaagg cgagcgccgg aggggcaagg ggcacgatgg      540
cctttaccag ggtctcagta cagccaccaa ggacacctac gacgcccttc acatgcaggc      600
cctgccccct cgctaacagc caggggattt caccactcaa aggccagacc tgcagacgcc      660
cagattatga gacacaggat gaagcattta caacccggtt cactcttctc agccactgaa      720
gtattcccct ttatgtacag gatgctttgg ttatatttag ctccaaacct tcacacacag      780
actgttgtcc ctgcactctt taagggagtg tactcccagg gcttacggcc ctggccttgg      840
gccctctggt ttgccggtgg tgcaggtaga cctgtctcct ggcggttcct cgttctccct      900
gggaggcggg cgcactgcct ctcacagctg agttgttgag tctgttttgt aaagtcccca      960
gagaaagcgc agatgctagc acatgcccta atgtctgtat cactctgtgt ctgagtggct     1020
tcactcctgc tgtaaatttg gcttctgttg tcaccttcac ctcctttcaa ggtaactgta     1080
ctgggccatg ttgtgcctcc ctggtgagag ggccgggcag aggggcagat ggaaaggagc     1140
ctaggccagg tgcaaccagg gagctgcagg ggcatgggaa ggtgggcggg caggggaggg     1200
tcagccaggg cctgcgaggg cagcgggagc ctccctgcct caggcctctg tgccgcacca     1260
ttgaactgta ccatgtgcta caggggccag aagatgaaca gactgacctt gatgagctgt     1320
gcacaaagtg gcataaaaaa catgtggtta cacagtgtga ataaagtgct gcggagcaag     1380
aggaggccgt tgattcactt cacgctttca gcgaatgaca aaatcatctt tgtgaaggcc     1440
tcgcaggaag acccaacaca tgggacctat aactgcccag cggacagtgg caggacagga     1500
aaaacccgtc aatgtactag gatactgctg cgtcattaca gggcacaggc catggatgga     1560
aaacgctctc tgctctgctt tttttctact gttttaattt atactggcat gctaaagcct     1620
tcctattttg cataataaat gcttcagtga aaaaaaaaaa aaaaaaaaaa aaaa           1674
```

```
<210>  4
<211>  1690
<212>  DNA
<213>  Homo sapiens

<400>  4
tgctttctca aaggccccac agtcctccac ttcctgggga ggtagctgca gaataaaacc       60
agcagagact ccttttctcc taaccgtccc ggccaccgct gcctcagcct ctgcctccca      120
gcctctttct gagggaaagg acaagatgaa gtggaaggcg cttttcaccg cggccatcct      180
gcaggcacag ttgccgatta cagaggcaca gagctttggc ctgctggatc ccaaactctg      240
ctacctgctg gatggaatcc tcttcatcta tggtgtcatt ctcactgcct tgttcctgag      300
agtgaagttc agcaggagcg cagacgcccc cgcgtaccag cagggccaga accagctcta      360
taacgagctc aatctaggac gaagagagga gtacgatgtt ttggacaaga gacgtggccg      420
```

```
ggaccctgag atggggggaa agccgcagag aaggaagaac cctcaggaag gcctgtacaa      480
tgaactgcag aaagataaga tggcggaggc ctacagtgag attgggatga aaggcgagcg      540
ccggaggggc aaggggcacg atggccttta ccagggtctc agtacagcca ccaaggacac      600
ctacgacgcc cttcacatgc aggccctgcc ccctcgctaa cagccagggg atttcaccac      660
tcaaaggcca gacctgcaga cgcccagatt atgagacaca ggatgaagca tttacaaccc      720
ggttcactct tctcagccac tgaagtattc ccctttatgt acaggatgct ttggttatat      780
ttagctccaa accttcacac acagactgtt gtccctgcac tctttaaggg agtgtactcc      840
cagggcttac ggccctggcc ttgggccctc tggtttgccg gtggtgcagg tagacctgtc      900
tcctggcggt tcctcgttct ccctgggagg cgggcgcact gcctctcaca gctgagttgt      960
tgagtctgtt ttgtaaagtc cccagagaaa gcgcagatgc tagcacatgc cctaatgtct     1020
gtatcactct gtgtctgagt ggcttcactc ctgctgtaaa tttggcttct gttgtcacct     1080
tcacctcctt tcaaggtaac tgtactgggc catgttgtgc ctccctggtg agagggccgg     1140
gcagaggggc agatggaaag gagcctaggc caggtgcaac cagggagctg caggggcatg     1200
ggaaggtggg cgggcagggg agggtcagcc agggcctgcg agggcagcgg gagcctccct     1260
gcctcaggcc tctgtgccgc accattgaac tgtaccatgt gctacagggg ccagaagatg     1320
aacagactga ccttgatgag ctgtgcacaa agtggcataa aaaacatgtg gttacacagt     1380
gtgaataaag tgctgcggag caagaggagg ccgttgattc acttcacgct ttcagcgaat     1440
gacaaaatca tctttgtgaa ggcctcgcag gaagacccaa cacatgggac ctataactgc     1500
ccagcggaca gtggcaggac aggaaaaacc cgtcaatgta ctaggatact gctgcgtcat     1560
tacagggcac aggccatgga tggaaaacgc tctctgctct gctttttttc tactgtttta     1620
atttatactg gcatgctaaa gccttcctat tttgcataat aaatgcttca gtgaaaatgc     1680
aaaaaaaaaa                                                            1690
```

<210> 5
<211> 163
<212> PRT
<213> Homo sapiens

<400> 5

```
Met Lys Trp Lys Ala Leu Phe Thr Ala Ala Ile Leu Gln Ala Gln Leu
1               5                   10                  15

Pro Ile Thr Glu Ala Gln Ser Phe Gly Leu Leu Asp Pro Lys Leu Cys
            20                  25                  30

Tyr Leu Leu Asp Gly Ile Leu Phe Ile Tyr Gly Val Ile Leu Thr Ala
        35                  40                  45
```

```
Leu Phe Leu Arg Val Lys Phe Ser Arg Ser Ala Asp Ala Pro Ala Tyr
    50                  55                  60

Gln Gln Gly Gln Asn Gln Leu Tyr Asn Glu Leu Asn Leu Gly Arg Arg
65                  70                  75                  80

Glu Glu Tyr Asp Val Leu Asp Lys Arg Arg Gly Arg Asp Pro Glu Met
                85                  90                  95

Gly Gly Lys Pro Arg Arg Lys Asn Pro Gln Glu Gly Leu Tyr Asn Glu
            100                 105                 110

Leu Gln Lys Asp Lys Met Ala Glu Ala Tyr Ser Glu Ile Gly Met Lys
        115                 120                 125

Gly Glu Arg Arg Arg Gly Lys Gly His Asp Gly Leu Tyr Gln Gly Leu
    130                 135                 140

Ser Thr Ala Thr Lys Asp Thr Tyr Asp Ala Leu His Met Gln Ala Leu
145                 150                 155                 160

Pro Pro Arg


<210>  6
<211>  164
<212>  PRT
<213>  Homo sapiens

<400>  6

Met Lys Trp Lys Ala Leu Phe Thr Ala Ala Ile Leu Gln Ala Gln Leu
1               5                   10                  15

Pro Ile Thr Glu Ala Gln Ser Phe Gly Leu Leu Asp Pro Lys Leu Cys
            20                  25                  30

Tyr Leu Leu Asp Gly Ile Leu Phe Ile Tyr Gly Val Ile Leu Thr Ala
        35                  40                  45

Leu Phe Leu Arg Val Lys Phe Ser Arg Ser Ala Asp Ala Pro Ala Tyr
    50                  55                  60

Gln Gln Gly Gln Asn Gln Leu Tyr Asn Glu Leu Asn Leu Gly Arg Arg
65                  70                  75                  80

Glu Glu Tyr Asp Val Leu Asp Lys Arg Arg Gly Arg Asp Pro Glu Met
                85                  90                  95
```

```
Gly Gly Lys Pro Gln Arg Arg Lys Asn Pro Gln Glu Gly Leu Tyr Asn
            100                 105                 110

Glu Leu Gln Lys Asp Lys Met Ala Glu Ala Tyr Ser Glu Ile Gly Met
        115                 120                 125

Lys Gly Glu Arg Arg Arg Gly Lys Gly His Asp Gly Leu Tyr Gln Gly
    130                 135                 140

Leu Ser Thr Ala Thr Lys Asp Thr Tyr Asp Ala Leu His Met Gln Ala
145                 150                 155                 160

Leu Pro Pro Arg
```

<210> 7
<211> 4721
<212> DNA
<213> Homo sapiens

<400> 7

```
acacttcggg ttcctcgggg aggaggggct ggaaccctag cccatcgtca ggacaaagat      60
gctcaggctg ctcttggctc tcaacttatt cccttcaatt caagtaacag gaaacaagat     120
tttggtgaag cagtcgccca tgcttgtagc gtacgacaat gcggtcaacc ttagctgcaa     180
gtattcctac aatctcttct caagggagtt ccgggcatcc cttcacaaag gactggatag     240
tgctgtggaa gtctgtgttg tatatgggaa ttactcccag cagcttcagg tttactcaaa     300
aacggggttc aactgtgatg ggaaattggg caatgaatca gtgacattct acctccagaa     360
tttgtatgtt aaccaaacag atatttactt ctgcaaaatt gaagttatgt atcctcctcc     420
ttacctagac aatgagaaga gcaatggaac cattatccat gtgaaaggga aacacctttg     480
tccaagtccc ctatttcccg gaccttctaa gcccttttgg gtgctggtgg tggttggtgg     540
agtcctggct tgctatagct tgctagtaac agtggccttt attattttct gggtgaggag     600
taagaggagc aggctcctgc acagtgacta catgaacatg actccccgcc gccccgggcc     660
cacccgcaag cattaccagc cctatgcccc accacgcgac ttcgcagcct atcgctcctg     720
acacggacgc ctatccagaa gccagccggc tggcagcccc catctgctca atatcactgc     780
tctggatagg aaatgaccgc catctccagc cggccacctc aggcccctgt tgggccacca     840
atgccaattt ttctcgagtg actagaccaa atatcaagat cattttgaga ctctgaaatg     900
aagtaaaaga gatttcctgt gacaggccaa gtcttacagt gccatggccc acattccaac     960
ttaccatgta cttagtgact tgactgagaa gttagggtag aaaacaaaaa gggagtggat    1020
tctgggagcc tcttcccttt ctcactcacc tgcacatctc agtcaagcaa agtgtggtat    1080
```

ccacagacat tttagttgca gaagaaaggc taggaaatca ttccttttgg ttaaatgggt 1140
gtttaatctt ttggttagtg ggttaaacgg ggtaagttag agtaggggga gggataggaa 1200
gacatattta aaaaccatta aaacactgtc tcccactcat gaaatgagcc acgtagttcc 1260
tatttaatgc tgttttcctt tagtttagaa atacatagac attgtctttt atgaattctg 1320
atcatattta gtcattttga ccaaatgagg gatttggtca aatgagggat tccctcaaag 1380
caatatcagg taaaccaagt tgctttcctc actccctgtc atgagacttc agtgttaatg 1440
ttcacaatat actttcgaaa gaataaaata gttctcctac atgaagaaag aatatgtcag 1500
gaaataaggt cactttatgt caaaattatt tgagtactat gggacctggc gcagtggctc 1560
atgcttgtaa tcccagcact ttgggaggcc gaggtgggca gatcacttga gatcaggacc 1620
agcctggtca agatggtgaa actccgtctg tactaaaaat acaaaattta gcttggcctg 1680
gtggcaggca cctgtaatcc cagctgccca agaggctgag gcatgagaat cgcttgaacc 1740
tggcaggcgg aggttgcagt gagccgagat agtgccacag ctctccagcc tgggcgacag 1800
agtgagactc catctcaaac aacaacaaca acaacaacaa caacaacaaa ccacaaaatt 1860
atttgagtac tgtgaaggat tatttgtcta acagttcatt ccaatcagac caggtaggag 1920
ctttcctgtt tcatatgttt cagggttgca cagttggtct ctttaatgtc ggtgtggaga 1980
tccaaagtgg gttgtggaaa gagcgtccat aggagaagtg agaatactgt gaaaaaggga 2040
tgttagcatt cattagagta tgaggatgag tcccaagaag gttctttgga aggaggacga 2100
atagaatgga gtaatgaaat tcttgccatg tgctgaggag atagccagca ttaggtgaca 2160
atcttccaga agtggtcagg cagaaggtgc cctggtgaga gctcctttac agggacttta 2220
tgtggtttag ggctcagagc tccaaaactc tgggctcagc tgctcctgta ccttggaggt 2280
ccattcacat gggaaagtat tttggaatgt gtcttttgaa gagagcatca gagttcttaa 2340
gggactgggt aaggcctgac cctgaaatga ccatggatat ttttctacct acagtttgag 2400
tcaactagaa tatgcctggg gaccttgaag aatggccctt cagtggccct caccatttgt 2460
tcatgcttca gttaattcag gtgttgaagg agcttaggtt ttagaggcac gtagacttgg 2520
ttcaagtctc gttagtagtt gaatagcctc aggcaagtca ctgcccacct aagatgatgg 2580
ttcttcaact ataaaatgga gataatggtt acaaatgtct cttcctatag tataatctcc 2640
ataagggcat ggcccaagtc tgtctttgac tctgcctatc cctgacattt agtagcatgc 2700
ccgacataca atgttagcta ttggtattat tgccatatag ataaattatg tataaaaatt 2760
aaactgggca atagcctaag aagggggaa tattgtaaca caaatttaaa cccactacgc 2820
agggatgagg tgctataata tgaggacctt ttaacttcca tcattttcct gtttcttgaa 2880
atagtttatc ttgtaatgaa atataaggca cctcccactt ttatgtatag aaagaggtct 2940
tttaattttt ttttaatgtg agaaggaagg gaggagtagg aatcttgaga ttccagatcg 3000

```
aaaatactgt actttggttg atttttaagt gggcttccat tccatggatt taatcagtcc     3060
caagaagatc aaactcagca gtacttgggt gctgaagaac tgttggattt accctggcac     3120
gtgtgccact tgccagcttc ttgggcacac agagttcttc aatccaagtt atcagattgt     3180
atttgaaaat gacagagctg gagagttttt tgaaatggca gtggcaaata aataaatact     3240
ttttttttaaa tggaaagact tgatctatgg taataaatga ttttgttttc tgactggaaa    3300
aataggccta ctaaagatga atcacacttg agatgtttct tactcactct gcacagaaac     3360
aaagaagaaa tgttatacag ggaagtccgt tttcactatt agtatgaacc aagaaatggt     3420
tcaaaaacag tggtaggagc aatgctttca tagtttcaga tatggtagtt atgaagaaaa     3480
caatgtcatt tgctgctatt attgtaagag tcttataatt aatggtactc ctataatttt     3540
tgattgtgag ctcacctatt tgggttaagc atgccaattt aaagagacca agtgtatgta     3600
cattatgttc tacatattca gtgataaaat tactaaacta ctatatgtct gctttaaatt     3660
tgtactttaa tattgtcttt tggtattaag aaagatatgc tttcagaata gatatgcttc     3720
gctttggcaa ggaatttgga tagaacttgc tatttaaaag aggtgtgggg taaatccttg     3780
tataaatctc cagtttagcc ttttttgaaa aagctagact ttcaaatact aatttcactt     3840
caagcagggt acgtttctgg tttgtttgct tgacttcagt cacaatttct tatcagacca     3900
atggctgacc tctttgagat gtcaggctag gcttacctat gtgttctgtg tcatgtgaat     3960
gctgagaagt ttgacagaga tccaacttca gccttgaccc catcagtccc tcgggttaac     4020
taactgagcc accggtcctc atggctattt taatgagggt attgatggtt aaatgcatgt     4080
ctgatccctt atcccagcca tttgcactgc cagctgggaa ctataccaga cctggatact     4140
gatcccaaag tgttaaattc aactacatgc tggagattag agatggtgcc aataaaggac     4200
ccagaaccag gatcttgatt gctatagact tattaataat ccaggtcaaa gagagtgaca     4260
cacactctct caagacctgg ggtgagggag tctgtgttat ctgcaaggcc atttgaggct     4320
cagaaagtct ctctttccta tagatatatg catactttct gacatatagg aatgtatcag     4380
gaatactcaa ccatcacagg catgttccta cctcagggcc tttacatgtc ctgtttactc     4440
tgtctagaat gtccttctgt agatgacctg gcttgcctcg tcacccttca ggtccttgct     4500
caagtgtcat cttctcccct agttaaacta ccccacaccc tgtctgcttt ccttgcttat     4560
ttttctccat agcattttac catctcttac attagacatt tttcttattt atttgtagtt     4620
tataagcttc atgaggcaag taactttgct ttgtttcttg ctgtatctcc agtgcccaga     4680
gcagtgcctg gtatataata aatatttatt gactgagtga a                         4721

<210>  8
<211>  4543
<212>  DNA
```

<213> Homo sapiens

<400> 8
taaagtcatc aaaacaacgt tatatcctgt gtgaaatgct gcagtcagga tgccttgtgg 60

tttgagtgcc ttgatcatgt gccctaaggg gatggtggcg gtggtggtgg ccgtggatga 120

cggagactct caggccttgg caggtgcgtc tttcagttcc cctcacactt cgggttcctc 180

ggggaggagg ggctggaacc ctagcccatc gtcaggacaa agatgctcag gctgctcttg 240

gctctcaact tattcccttc aattcaagta acagggaaac acctttgtcc aagtccccta 300

tttcccggac cttctaagcc cttttgggtg ctggtggtgg ttggtggagt cctggcttgc 360

tatagcttgc tagtaacagt ggcctttatt attttctggg tgaggagtaa gaggagcagg 420

ctcctgcaca gtgactacat gaacatgact ccccgccgcc ccgggcccac ccgcaagcat 480

taccagccct atgccccacc acgcgacttc gcagcctatc gctcctgaca cggacgccta 540

tccagaagcc agccggctgg cagcccccat ctgctcaata tcactgctct ggataggaaa 600

tgaccgccat ctccagccgg ccacctcagg cccctgttgg gccaccaatg ccaatttttc 660

tcgagtgact agaccaaata tcaagatcat tttgagactc tgaaatgaag taaaagagat 720

ttcctgtgac aggccaagtc ttacagtgcc atggcccaca ttccaactta ccatgtactt 780

agtgacttga ctgagaagtt agggtagaaa acaaaaaggg agtggattct gggagcctct 840

tccctttctc actcacctgc acatctcagt caagcaaagt gtggtatcca cagacatttt 900

agttgcagaa gaaaggctag gaaatcattc cttttggtta aatgggtgtt taatcttttg 960

gttagtgggt taaacggggt aagttagagt agggggaggg ataggaagac atatttaaaa 1020

accattaaaa cactgtctcc cactcatgaa atgagccacg tagttcctat ttaatgctgt 1080

tttcctttag tttagaaata catagacatt gtcttttatg aattctgatc atatttagtc 1140

attttgacca aatgagggat ttggtcaaat gagggattcc ctcaaagcaa tatcaggtaa 1200

accaagttgc tttcctcact ccctgtcatg agacttcagt gttaatgttc acaatatact 1260

ttcgaaagaa taaaatagtt ctcctacatg aagaaagaat atgtcaggaa ataaggtcac 1320

tttatgtcaa aattatttga gtactatggg acctggcgca gtggctcatg cttgtaatcc 1380

cagcactttg ggaggccgag gtgggcagat cacttgagat caggaccagc ctggtcaaga 1440

tggtgaaact ccgtctgtac taaaaataca aaatttagct tggcctggtg gcaggcacct 1500

gtaatcccag ctgcccaaga ggctgaggca tgagaatcgc ttgaacctgg caggcggagg 1560

ttgcagtgag ccgagatagt gccacagctc tccagcctgg gcgacagagt gagactccat 1620

ctcaaacaac aacaacaaca acaacaacaa caacaaacca caaaattatt tgagtactgt 1680

gaaggattat ttgtctaaca gttcattcca atcagaccag gtaggagctt tcctgtttca 1740

tatgtttcag ggttgcacag ttggtctctt taatgtcggt gtggagatcc aaagtgggtt 1800

gtggaaagag cgtccatagg agaagtgaga atactgtgaa aaagggatgt tagcattcat 1860

tagagtatga ggatgagtcc caagaaggtt ctttggaagg aggacgaata gaatggagta 1920

atgaaattct tgccatgtgc tgaggagata gccagcatta ggtgacaatc ttccagaagt 1980

ggtcaggcag aaggtgccct ggtgagagct cctttacagg gactttatgt ggtttagggc 2040

tcagagctcc aaaactctgg gctcagctgc tcctgtacct tggaggtcca ttcacatggg 2100

aaagtatttt ggaatgtgtc ttttgaagag agcatcagag ttcttaaggg actgggtaag 2160

gcctgaccct gaaatgacca tggatatttt tctacctaca gtttgagtca actagaatat 2220

gcctggggac cttgaagaat ggcccttcag tggccctcac catttgttca tgcttcagtt 2280

aattcaggtg ttgaaggagc ttaggtttta gaggcacgta gacttggttc aagtctcgtt 2340

agtagttgaa tagcctcagg caagtcactg cccacctaag atgatggttc ttcaactata 2400

aaatggagat aatggttaca aatgtctctt cctatagtat aatctccata agggcatggc 2460

ccaagtctgt ctttgactct gcctatccct gacatttagt agcatgcccg acatacaatg 2520

ttagctattg gtattattgc catatagata aattatgtat aaaaattaaa ctgggcaata 2580

gcctaagaag gggggaatat tgtaacacaa atttaaaccc actacgcagg gatgaggtgc 2640

tataatatga ggacctttta acttccatca ttttcctgtt tcttgaaata gtttatcttg 2700

taatgaaata taaggcacct cccactttta tgtatagaaa gaggtctttt aatttttttt 2760

taatgtgaga aggaagggag gagtaggaat cttgagattc cagatcgaaa atactgtact 2820

ttggttgatt tttaagtggg cttccattcc atggatttaa tcagtcccaa gaagatcaaa 2880

ctcagcagta cttgggtgct gaagaactgt tggatttacc ctggcacgtg tgccacttgc 2940

cagcttcttg ggcacacaga gttcttcaat ccaagttatc agattgtatt tgaaaatgac 3000

agagctggag agtttttga aatggcagtg gcaaataaat aaatactttt ttttaaatgg 3060

aaagacttga tctatggtaa taaatgattt tgttttctga ctggaaaaat aggcctacta 3120

aagatgaatc acacttgaga tgtttcttac tcactctgca cagaaacaaa gaagaaatgt 3180

tatacaggga agtccgtttt cactattagt atgaaccaag aaatggttca aaaacagtgg 3240

taggagcaat gctttcatag tttcagatat ggtagttatg aagaaaacaa tgtcatttgc 3300

tgctattatt gtaagagtct tataattaat ggtactccta taatttttga ttgtgagctc 3360

acctatttgg gttaagcatg ccaatttaaa gagaccaagt gtatgtacat tatgttctac 3420

atattcagtg ataaaattac taaactacta tatgtctgct ttaaatttgt actttaatat 3480

tgtcttttgg tattaagaaa gatatgcttt cagaatagat atgcttcgct ttggcaagga 3540

atttggatag aacttgctat ttaaaagagg tgtggggtaa atccttgtat aaatctccag 3600

tttagccttt tttgaaaaag ctagactttc aaatactaat ttcacttcaa gcagggtacg 3660

tttctggttt gtttgcttga cttcagtcac aatttcttat cagaccaatg gctgacctct 3720

ttgagatgtc aggctaggct tacctatgtg ttctgtgtca tgtgaatgct gagaagtttg 3780

acagagatcc aacttcagcc ttgaccccat cagtccctcg ggttaactaa ctgagccacc 3840

ggtcctcatg gctattttaa tgagggtatt gatggttaaa tgcatgtctg atcccttatc 3900

ccagccattt gcactgccag ctgggaacta taccagacct ggatactgat cccaaagtgt 3960

taaattcaac tacatgctgg agattagaga tggtgccaat aaaggaccca gaaccaggat 4020

cttgattgct atagacttat taataatcca ggtcaaagag agtgacacac actctctcaa 4080

gacctggggt gagggagtct gtgttatctg caaggccatt tgaggctcag aaagtctctc 4140

tttcctatag atatatgcat actttctgac atataggaat gtatcaggaa tactcaacca 4200

tcacaggcat gttcctacct cagggccttt acatgtcctg tttactctgt ctagaatgtc 4260

cttctgtaga tgacctggct tgcctcgtca cccttcaggt ccttgctcaa gtgtcatctt 4320

ctcccctagt taaactaccc cacaccctgt ctgctttcct tgcttatttt tctccatagc 4380

attttaccat ctcttacatt agacattttt cttatttatt tgtagtttat aagcttcatg 4440

aggcaagtaa ctttgctttg tttcttgctg tatctccagt gcccagagca gtgcctggta 4500

tataataaat atttattgac tgagtgaaaa aaaaaaaaaa aaa 4543

<210> 9
<211> 220
<212> PRT
<213> Homo sapiens

<400> 9

Met Leu Arg Leu Leu Leu Ala Leu Asn Leu Phe Pro Ser Ile Gln Val
1 5 10 15

Thr Gly Asn Lys Ile Leu Val Lys Gln Ser Pro Met Leu Val Ala Tyr
20 25 30

Asp Asn Ala Val Asn Leu Ser Cys Lys Tyr Ser Tyr Asn Leu Phe Ser
35 40 45

Arg Glu Phe Arg Ala Ser Leu His Lys Gly Leu Asp Ser Ala Val Glu
50 55 60

Val Cys Val Val Tyr Gly Asn Tyr Ser Gln Gln Leu Gln Val Tyr Ser
65 70 75 80

Lys Thr Gly Phe Asn Cys Asp Gly Lys Leu Gly Asn Glu Ser Val Thr
85 90 95

Phe Tyr Leu Gln Asn Leu Tyr Val Asn Gln Thr Asp Ile Tyr Phe Cys
100 105 110

Lys Ile Glu Val Met Tyr Pro Pro Pro Tyr Leu Asp Asn Glu Lys Ser
115 120 125

Asn Gly Thr Ile Ile His Val Lys Gly Lys His Leu Cys Pro Ser Pro
130 135 140

Leu Phe Pro Gly Pro Ser Lys Pro Phe Trp Val Leu Val Val Val Gly
145 150 155 160

Gly Val Leu Ala Cys Tyr Ser Leu Leu Val Thr Val Ala Phe Ile Ile
165 170 175

Phe Trp Val Arg Ser Lys Arg Ser Arg Leu Leu His Ser Asp Tyr Met
180 185 190

Asn Met Thr Pro Arg Arg Pro Gly Pro Thr Arg Lys His Tyr Gln Pro
195 200 205

Tyr Ala Pro Pro Arg Asp Phe Ala Ala Tyr Arg Ser
210 215 220

<210> 10
<211> 101
<212> PRT
<213> Homo sapiens

<400> 10

Met Leu Arg Leu Leu Leu Ala Leu Asn Leu Phe Pro Ser Ile Gln Val
1 5 10 15

Thr Gly Lys His Leu Cys Pro Ser Pro Leu Phe Pro Gly Pro Ser Lys
20 25 30

Pro Phe Trp Val Leu Val Val Val Gly Gly Val Leu Ala Cys Tyr Ser
35 40 45

Leu Leu Val Thr Val Ala Phe Ile Ile Phe Trp Val Arg Ser Lys Arg
50 55 60

Ser Arg Leu Leu His Ser Asp Tyr Met Asn Met Thr Pro Arg Arg Pro
65 70 75 80

Gly Pro Thr Arg Lys His Tyr Gln Pro Tyr Ala Pro Pro Arg Asp Phe
85 90 95

Ala Ala Tyr Arg Ser
100

<210> 11
<211> 1534
<212> DNA
<213> Homo sapiens

<400> 11
tattgtcaga gtcctcttgt ttggccttct aggaaggctg tgggacccag ctttcttcaa 60

ccagtccagg tggaggcctc tgccttgaac gtttccaagt gaggtaaaac ccgcaggccc 120

agaggcctct ctacttcctg tgtggggttc agaaaccctc ctcccctccc agcctcaggt 180

gcctgcttca gaaaatgaag tagtaagtct gctggcctcc gccatcttag taaagtaaca 240

gtcccatgaa acaaagatgc agtcgggcac tcactggaga gttctgggcc tctgcctctt 300

atcagttggc gtttgggggc aagatggtaa tgaagaaatg ggtggtatta cacagacacc 360

atataaagtc tccatctctg gaaccacagt aatattgaca tgccctcagt atcctggatc 420

tgaaatacta tggcaacaca atgataaaaa cataggcggt gatgaggatg ataaaaacat 480

aggcagtgat gaggatcacc tgtcactgaa ggaattttca gaattggagc aaagtggtta 540

ttatgtctgc taccccagag gaagcaaacc agaagatgcg aacttttatc tctacctgag 600

ggcaagagtg tgtgagaact gcatggagat ggatgtgatg tcggtggcca caattgtcat 660

agtggacatc tgcatcactg gggggcttgct gctgctggtt tactactgga gcaagaatag 720

aaaggccaag gccaagcctg tgacacgagg agcgggtgct ggcggcaggc aaaggggaca 780

aaacaaggag aggccaccac ctgttcccaa cccagactat gagcccatcc ggaaaggcca 840

gcgggacctg tattctggcc tgaatcagag acgcatctga ccctctggag aacactgcct 900

cccgctggcc caggtctcct ctccagtccc cctgcgactc cctgtttcct gggctagtct 960

tggaccccac gagagagaat cgttcctcag cctcatggtg aactcgcgcc ctccagcctg 1020

atcccccgct ccctcctccc tgccttctct gctggtaccc agtcctaaaa tattgctgct 1080

tcctcttcct ttgaagcatc atcagtagtc acaccctcac agctggcctg ccctcttgcc 1140

aggatattta tttgtgctat tcactccctt ccctttggat gtaacttctc cgttcagttc 1200

cctccttttc ttgcatgtaa gttgtccccc atcccaaagt attccatcta cttttctatc 1260

gccgtcccct tttgcagccc tctctgggga tggactgggt aaatgttgac agaggccctg 1320

ccccgttcac agatcctggc cctgagccag ccctgtgctc ctccctcccc caacactccc 1380

taccaacccc ctaatcccct actccctcca cccccctcc actgtaggcc actggatggt 1440

catttgcatc tccgtaaatg tgctctgctc ctcagctgag agagaaaaaa ataaactgta 1500

tttggctgca agaaaaaaaa aaaaaaaaaa aaaa 1534

<210> 12
<211> 207

<212> PRT
<213> Homo sapiens

<400> 12

```
Met Gln Ser Gly Thr His Trp Arg Val Leu Gly Leu Cys Leu Leu Ser
1               5                   10                  15

Val Gly Val Trp Gly Gln Asp Gly Asn Glu Glu Met Gly Gly Ile Thr
            20                  25                  30

Gln Thr Pro Tyr Lys Val Ser Ile Ser Gly Thr Thr Val Ile Leu Thr
        35                  40                  45

Cys Pro Gln Tyr Pro Gly Ser Glu Ile Leu Trp Gln His Asn Asp Lys
    50                  55                  60

Asn Ile Gly Gly Asp Glu Asp Asp Lys Asn Ile Gly Ser Asp Glu Asp
65                  70                  75                  80

His Leu Ser Leu Lys Glu Phe Ser Glu Leu Glu Gln Ser Gly Tyr Tyr
                85                  90                  95

Val Cys Tyr Pro Arg Gly Ser Lys Pro Glu Asp Ala Asn Phe Tyr Leu
            100                 105                 110

Tyr Leu Arg Ala Arg Val Cys Glu Asn Cys Met Glu Met Asp Val Met
        115                 120                 125

Ser Val Ala Thr Ile Val Ile Val Asp Ile Cys Ile Thr Gly Gly Leu
    130                 135                 140

Leu Leu Leu Val Tyr Tyr Trp Ser Lys Asn Arg Lys Ala Lys Ala Lys
145                 150                 155                 160

Pro Val Thr Arg Gly Ala Gly Ala Gly Gly Arg Gln Arg Gly Gln Asn
                165                 170                 175

Lys Glu Arg Pro Pro Pro Val Pro Asn Pro Asp Tyr Glu Pro Ile Arg
            180                 185                 190

Lys Gly Gln Arg Asp Leu Tyr Ser Gly Leu Asn Gln Arg Arg Ile
        195                 200                 205
```

<210> 13
<211> 2690
<212> DNA
<213> Homo sapiens

<400> 13

```
agtctagctg ctgcacaggc tggctggctg gctggctgct aagggctgct ccacgctttt      60
gccggaggac agagactgac atggaacagg ggaagggcct ggctgtcctc atcctggcta     120
tcattcttct tcaaggtact ttggcccagt caatcaaagg aaaccacttg gttaaggtgt     180
atgactatca agaagatggt tcggtacttc tgacttgtga tgcagaagcc aaaaatatca     240
catggtttaa agatgggaag atgatcggct tcctaactga agataaaaaa aaatggaatc     300
tgggaagtaa tgccaaggac cctcgaggga tgtatcagtg taaaggatca cagaacaagt     360
caaaaccact ccaagtgtat tacagaatgt gtcagaactg cattgaacta aatgcagcca     420
ccatatctgg ctttctcttt gctgaaatcg tcagcatttt cgtccttgct gttggggtct     480
acttcattgc tggacaggat ggagttcgcc agtcgagagc ttcagacaag cagactctgt     540
tgcccaatga ccagctctac cagcccctca aggatcgaga agatgaccag tacagccacc     600
ttcaaggaaa ccagttgagg aggaattgaa ctcaggactc agagtagtcc aggtgttctc     660
ctcctattca gttcccagaa tcaaagcaat gcattttgga aagctcctag cagagagact     720
ttcagcccta aatctagact caaggttccc agagatgaca aatggagaag aaaggccatc     780
agagcaaatt tgggggtttc tcaaataaaa taaaaataaa aacaaatact gtgtttcaga     840
agcgccacct attggggaaa attgtaaaag aaaaatgaaa agatcaaata accccctgga     900
tttgaatata atttttgtg ttgtaatttt tatttcgttt ttgtataggt tataattcac     960
atggctcaaa tattcagtga aagctctccc tccaccgcca tcccctgcta cccagtgacc    1020
ctgttgccct cttcagagac aaattagttt ctcttttttt ttttttttt tttttttttg    1080
agacagtctg gctctgtcac ccaggctgaa atgcagtggc accatctcgg ctcactgcaa    1140
cctctgcctc ctgggttcaa gcgattctcc tgcctcagcc tcccgggcag ctgggattac    1200
aggcacacac taccacacct ggctaatttt tgtattttta gtagagacag ggttttgctc    1260
tgttggccaa gctggtctcg aactcctgac ctcaagtgat ccgcccgcct cagcctccca    1320
aagtgctggg attacaggtg tgagccacca tgcctggtct taaaaccagt ttcttatata    1380
tctctctgga ggtattctag gcatatatga gcacattctc aagtacatat tatcctccct    1440
tcccctatct tttagacaaa tgatatcaaa ctatacatct tgtgagatta ttgcatacca    1500
ttatatgaag ataccattat atccttttta atgcaaccat attgtacaaa tagactatga    1560
tttatttaac ctgttatcta tcagtggata tttaagttgg tagttggttc caatcttttg    1620
ctcttacaac aattctgcaa tgactaacat tgtataaata tcatttttaa aaataattgc    1680
attgaagcat aatgtacatg ccataaaatc cacccatctt aagtgatttc acctgttctc    1740
agaaattttt agtaaattta actaattgta cagccattac cataatccag ctttaggaca    1800
ttttcttttt tttcttttct tttcttttt ttcttttttt tttttttttg aagtggaatc    1860
ttgctctgtg gcccaggctg gagtgcagtg gcgcgatctc agctcactgc aacctccacc    1920
```

tcctgggttc aagcgattct cttgccttgg cctcccgagt agctgagact acaggcacat 1980

gccaccacgc ccagctcatt ttttgtgtat ttagtatttg tgtatctagt atttgtgtac 2040

ttagtagaga cagggtttca ccatgttggc caggctggtc tccaattcct gacctcaggc 2100

gatccacccg ccttgacctc ccaaagtgct gggattacag gtgtgagcca ccgcgccagg 2160

cccgtaactg tattttaata tagccattct atggatttaa tatggtattt tattatggcc 2220

ttaatttgca tttccctaga tactaaccat gctgagtgtc ctgtcttgtg tttattaacc 2280

attcatatat ttttagtgaa atgtgtatca aatcttttgc ccatttttaa gttgacttat 2340

ttgtttgtct tcttactatt gggttgcata tgtttttgat ataagtcctt tatcagatat 2400

atgatttgga aatattttct accaatctgt ggtttgtttt tcttaatggt gtcttttgaa 2460

gtgcaaaagg tttgaatttt gaagtacatt ttattgattt tttcttctat atattgtgct 2520

tttggtatca tgtctaataa atctttacca aacccacagt tacaaagatt ttctcctgtc 2580

ttctttttat actttttaca gctttatggt tttagctcta acaataaatg tgattttgaa 2640

catacataag actatttgta acaaacacaa ataaattgaa ttgttgggca 2690

<210> 14
<211> 182
<212> PRT
<213> Homo sapiens

<400> 14

Met Glu Gln Gly Lys Gly Leu Ala Val Leu Ile Leu Ala Ile Ile Leu
1 5 10 15

Leu Gln Gly Thr Leu Ala Gln Ser Ile Lys Gly Asn His Leu Val Lys
20 25 30

Val Tyr Asp Tyr Gln Glu Asp Gly Ser Val Leu Leu Thr Cys Asp Ala
35 40 45

Glu Ala Lys Asn Ile Thr Trp Phe Lys Asp Gly Lys Met Ile Gly Phe
50 55 60

Leu Thr Glu Asp Lys Lys Lys Trp Asn Leu Gly Ser Asn Ala Lys Asp
65 70 75 80

Pro Arg Gly Met Tyr Gln Cys Lys Gly Ser Gln Asn Lys Ser Lys Pro
85 90 95

Leu Gln Val Tyr Tyr Arg Met Cys Gln Asn Cys Ile Glu Leu Asn Ala
100 105 110

Ala Thr Ile Ser Gly Phe Leu Phe Ala Glu Ile Val Ser Ile Phe Val
115 120 125

Leu Ala Val Gly Val Tyr Phe Ile Ala Gly Gln Asp Gly Val Arg Gln
130 135 140

Ser Arg Ala Ser Asp Lys Gln Thr Leu Leu Pro Asn Asp Gln Leu Tyr
145 150 155 160

Gln Pro Leu Lys Asp Arg Glu Asp Asp Gln Tyr Ser His Leu Gln Gly
165 170 175

Asn Gln Leu Arg Arg Asn
180

<210> 15
<211> 3049
<212> DNA
<213> Homo sapiens

<400> 15
ctctcttcat ttaagcacga ctctgcagaa ggaacaaagc accctcccca ctgggctcct 60

ggttgcagag ctccaagtcc tcacacagat acgcctgttt gagaagcagc gggcaagaaa 120

gacgcaagcc cagaggccct gccatttctg tgggctcagg tccctactgg ctcaggcccc 180

tgcctccctc ggcaaggcca caatgaaccg gggagtccct tttaggcact tgcttctggt 240

gctgcaactg gcgctcctcc cagcagccac tcagggaaag aaagtggtgc tgggcaaaaa 300

aggggataca gtggaactga cctgtacagc ttcccagaag aagagcatac aattccactg 360

gaaaaactcc aaccagataa agattctggg aaatcagggc tccttcttaa ctaaaggtcc 420

atccaagctg aatgatcgcg ctgactcaag aagaagcctt tgggaccaag gaaactttcc 480

cctgatcatc aagaatctta agatagaaga ctcagatact tacatctgtg aagtggagga 540

ccagaaggag gaggtgcaat tgctagtgtt cggattgact gccaactctg acacccacct 600

gcttcagggg cagagcctga ccctgacctt ggagagcccc cctggtagta gcccctcagt 660

gcaatgtagg agtccaaggg gtaaaaacat acaggggggg aagaccctct ccgtgtctca 720

gctggagctc caggatagtg gcacctggac atgcactgtc ttgcagaacc agaagaaggt 780

ggagttcaaa atagacatcg tggtgctagc tttccagaag gcctccagca tagtctataa 840

gaaagagggg gaacaggtgg agttctcctt cccactcgcc tttacagttg aaaagctgac 900

gggcagtggc gagctgtggt ggcaggcgga gagggcttcc tcctccaagt cttggatcac 960

ctttgacctg aagaacaagg aagtgtctgt aaaacgggtt acccaggacc ctaagctcca 1020

gatgggcaag aagctcccgc tccacctcac cctgccccag gccttgcctc agtatgctgg 1080

ctctggaaac ctcaccctgg cccttgaagc gaaaacagga aagttgcatc aggaagtgaa 1140

cctggtggtg atgagagcca ctcagctcca gaaaaatttg acctgtgagg tgtggggacc 1200
cacctcccct aagctgatgc tgagtttgaa actggagaac aaggaggcaa aggtctcgaa 1260
gcgggagaag gcggtgtggg tgctgaaccc tgaggcgggg atgtggcagt gtctgctgag 1320
tgactcggga caggtcctgc tggaatccaa catcaaggtt ctgcccacat ggtccacccc 1380
ggtgcagcca atggccctga ttgtgctggg gggcgtcgcc ggcctcctgc ttttcattgg 1440
gctaggcatc ttcttctgtg tcaggtgccg gcaccgaagg cgccaagcag agcggatgtc 1500
tcagatcaag agactcctca gtgagaagaa gacctgccag tgtcctcacc ggtttcagaa 1560
gacatgtagc cccatttgag gcacgaggcc aggcagatcc cacttgcagc ctccccaggt 1620
gtctgccccg cgtttcctgc ctgcggacca gatgaatgta gcagatcccc agcctctggc 1680
ctcctgttcg cctcctctac aatttgccat tgtttctcct gggttaggcc ccggcttcac 1740
tggttgagtg ttgctctcta gtttccagag gcttaatcac accgtcctcc acgccatttc 1800
cttttccttc aagcctagcc cttctctcat tatttctctc tgaccctctc cccactgctc 1860
atttggatcc caggggagtg ttcagggcca gccctggctg gcatggaggg tgaggctggg 1920
tgtctggaag catggagcat gggactgttc ttttacaaga caggaccctg ggaccacaga 1980
gggcaggaac ttgcacaaaa tcacacagcc aagccagtca aggatggatg cagatccaga 2040
ggtttctggc agccagtacc tcctgcccca tgctgcccgc ttctcaccct atgtgggtgg 2100
gaccacagac tcacatcctg accttgcaca aacagcccct ctggacacag ccccatgtac 2160
acggcctcaa gggatgtctc acatcctctg tctatttgag acttagaaaa atcctacaag 2220
gctggcagtg acagaactaa gatgatcatc tccagtttat agaccagaac cagagctcag 2280
agaggctaga tgattgatta ccaagtgccg gactagcaag tgctggagtc gggactaacc 2340
caggtccctt gtcccaagtt ccactgctgc ctcttgaatg cagggacaaa tgccacacgg 2400
ctctcaccag tggctagtgg tgggtactca atgtgtactt ttgggttcac agaagcacag 2460
cacccatggg aagggtccat ctcagagaat ttacgagcag ggatgaaggc ctccctgtct 2520
aaaatccctc cttcatcccc cgctggtggc agaatctgtt accagaggac aaagcctttg 2580
gctcttctaa tcagagcgca agctgggagc acaggcactg caggagagaa tgcccagtga 2640
ccagtcactg accctgtgca gaacctcctg gaagcgagct ttgctgggag aggggggtagc 2700
tagcctgaga gggaaccctc taagggacct caaaggtgat tgtgccaggc tctgcgcctg 2760
ccccacaccc tcccttaccc tcctccagac cattcaggac acagggaaat cagggttaca 2820
aatcttcttg atccacttct ctcaggatcc cctctcttcc taccctttcct caccacttcc 2880
ctcagtccca actccttttc cctatttcct tctcctcctg tctttaaagc ctgcctcttc 2940
caggaagacc cccctattgc tgctggggct ccccatttgc ttactttgca tttgtgccca 3000

ctctccaccc ctgctcccct gagctgaaat aaaaatacaa taaacttac 3049

<210> 16
<211> 458
<212> PRT
<213> Homo sapiens

<400> 16

```
Met Asn Arg Gly Val Pro Phe Arg His Leu Leu Leu Val Leu Gln Leu
1               5                   10                  15

Ala Leu Leu Pro Ala Ala Thr Gln Gly Lys Lys Val Val Leu Gly Lys
            20                  25                  30

Lys Gly Asp Thr Val Glu Leu Thr Cys Thr Ala Ser Gln Lys Lys Ser
        35                  40                  45

Ile Gln Phe His Trp Lys Asn Ser Asn Gln Ile Lys Ile Leu Gly Asn
    50                  55                  60

Gln Gly Ser Phe Leu Thr Lys Gly Pro Ser Lys Leu Asn Asp Arg Ala
65                  70                  75                  80

Asp Ser Arg Arg Ser Leu Trp Asp Gln Gly Asn Phe Pro Leu Ile Ile
                85                  90                  95

Lys Asn Leu Lys Ile Glu Asp Ser Asp Thr Tyr Ile Cys Glu Val Glu
            100                 105                 110

Asp Gln Lys Glu Glu Val Gln Leu Leu Val Phe Gly Leu Thr Ala Asn
        115                 120                 125

Ser Asp Thr His Leu Leu Gln Gly Gln Ser Leu Thr Leu Thr Leu Glu
    130                 135                 140

Ser Pro Pro Gly Ser Ser Pro Ser Val Gln Cys Arg Ser Pro Arg Gly
145                 150                 155                 160

Lys Asn Ile Gln Gly Gly Lys Thr Leu Ser Val Ser Gln Leu Glu Leu
                165                 170                 175

Gln Asp Ser Gly Thr Trp Thr Cys Thr Val Leu Gln Asn Gln Lys Lys
            180                 185                 190

Val Glu Phe Lys Ile Asp Ile Val Val Leu Ala Phe Gln Lys Ala Ser
        195                 200                 205

Ser Ile Val Tyr Lys Lys Glu Gly Glu Gln Val Glu Phe Ser Phe Pro
```

```
    210                 215                 220

Leu Ala Phe Thr Val Glu Lys Leu Thr Gly Ser Gly Glu Leu Trp Trp
225                 230                 235                 240

Gln Ala Glu Arg Ala Ser Ser Ser Lys Ser Trp Ile Thr Phe Asp Leu
                245                 250                 255

Lys Asn Lys Glu Val Ser Val Lys Arg Val Thr Gln Asp Pro Lys Leu
            260                 265                 270

Gln Met Gly Lys Lys Leu Pro Leu His Leu Thr Leu Pro Gln Ala Leu
        275                 280                 285

Pro Gln Tyr Ala Gly Ser Gly Asn Leu Thr Leu Ala Leu Glu Ala Lys
    290                 295                 300

Thr Gly Lys Leu His Gln Glu Val Asn Leu Val Val Met Arg Ala Thr
305                 310                 315                 320

Gln Leu Gln Lys Asn Leu Thr Cys Glu Val Trp Gly Pro Thr Ser Pro
                325                 330                 335

Lys Leu Met Leu Ser Leu Lys Leu Glu Asn Lys Glu Ala Lys Val Ser
            340                 345                 350

Lys Arg Glu Lys Ala Val Trp Val Leu Asn Pro Glu Ala Gly Met Trp
        355                 360                 365

Gln Cys Leu Leu Ser Asp Ser Gly Gln Val Leu Leu Glu Ser Asn Ile
    370                 375                 380

Lys Val Leu Pro Thr Trp Ser Thr Pro Val Gln Pro Met Ala Leu Ile
385                 390                 395                 400

Val Leu Gly Gly Val Ala Gly Leu Leu Leu Phe Ile Gly Leu Gly Ile
                405                 410                 415

Phe Phe Cys Val Arg Cys Arg His Arg Arg Arg Gln Ala Glu Arg Met
            420                 425                 430

Ser Gln Ile Lys Arg Leu Leu Ser Glu Lys Lys Thr Cys Gln Cys Pro
        435                 440                 445

His Arg Phe Gln Lys Thr Cys Ser Pro Ile
    450                 455
```

<210> 17
<211> 2755
<212> DNA
<213> Homo sapiens

<400> 17
ccgggccgcg cttcctctcg ccaggcctgc gagcttcctc ccagcggagc cctgggcgag 60

ccgaggttgg ccgccgccgc cgccgagccc gctgccgccc tcccgctcct gccccacccg 120

cgccttgccc gggggcttct gccggggtgg ggtccgagcc gggcgaccgc ccggctgcgc 180

cgccgtcggg gccgtaaccc ggcccgccgt ccctcccgcc ccagccagcc tctggccgcc 240

ggagcccgcg gggcgtggag cgcgaggagc cccgcggccc cgatcgagcg tccggggcgg 300

cccccggcag ccagcgcgac gttccaaaat cgaacctcag tggcggcgct cggaagcgga 360

actctgccgg ggccgcgccg gctacattgt ttcctccccc cgactccctc ccgccccctt 420

cccccgcctt tcttccctcc gcgacccggg ccgtgcgtcc gtccccctgc ctctgcctgg 480

cggtccctcc tcccctctcc ttgcacccat acctctttgt accgcacccc ctggggaccc 540

ctgcgcccct cccctccccc ctgaccgcat ggaccgtccc gcaggccgct gatgccgccc 600

gcggcgaggt ggcccggacc gcagtgcccc aagagagctc taatggtacc aagtgacagg 660

ttggctttac tgtgactcgg ggacgccaga gctcctgaga agatgtcagc aatacaggcc 720

gcctggccat ccggtacaga atgtattgcc aagtacaact tccacggcac tgccgagcag 780

gacctgccct tctgcaaagg agacgtgctc accattgtgg ccgtcaccaa ggaccccaac 840

tggtacaaag ccaaaaacaa ggtgggccgt gagggcatca tcccagccaa ctacgtccag 900

aagcgggagg gcgtgaaggc gggtaccaaa ctcagcctca tgccttggtt ccacggcaag 960

atcacacggg agcaggctga gcggcttctg tacccgccgg agacaggcct gttcctggtg 1020

cgggagagca ccaactaccc cggagactac acgctgtgcg tgagctgcga cggcaaggtg 1080

gagcactacc gcatcatgta ccatgccagc aagctcagca tcgacgagga ggtgtacttt 1140

gagaacctca tgcagctggt ggagcactac acctcagacg cagatggact ctgtacgcgc 1200

ctcattaaac caaaggtcat ggagggcaca gtggcggccc aggatgagtt ctaccgcagc 1260

ggctgggccc tgaacatgaa ggagctgaag ctgctgcaga ccatcgggaa gggggagttc 1320

ggagacgtga tgctgggcga ttaccgaggg aacaaagtcg ccgtcaagtg cattaagaac 1380

gacgccactg cccaggcctt cctggctgaa gcctcagtca tgacgcaact gcggcatagc 1440

aacctggtgc agctcctggg cgtgatcgtg gaggagaagg gcgggctcta catcgtcact 1500

gagtacatgg ccaaggggag ccttgtggac tacctgcggt ctaggggtcg gtcagtgctg 1560

ggcggagact gtctcctcaa gttctcgcta gatgtctgcg aggccatgga atacctggag 1620

ggcaacaatt tcgtgcatcg agacctggct gcccgcaatg tgctggtgtc tgaggacaac 1680

gtggccaagg tcagcgactt tggtctcacc aaggaggcgt ccagcaccca ggacacgggc 1740

aagctgccag tcaagtggac agcccctgag gccctgagag agaagaaatt ctccactaag 1800

tctgacgtgt ggagtttcgg aatccttctc tgggaaatct actcctttgg gcgagtgcct 1860

tatccaagaa ttcccctgaa ggacgtcgtc cctcgggtgg agaagggcta caagatggat 1920

gcccccgacg gctgcccgcc cgcagtctat gaagtcatga agaactgctg gcacctggac 1980

gccgccatgc ggccctcctt cctacagctc cgagagcagc ttgagcacat caaaacccac 2040

gagctgcacc tgtgacggct ggcctccgcc tgggtcatgg gcctgtgggg actgaacctg 2100

gaagatcatg gacctggtgc ccctgctcac tgggcccgag cctgaactga gccccagcgg 2160

gctggcgggc ctttttcctg cgtcccagcc tgcacccctc cggccccgtc tctcttggac 2220

ccacctgtgg ggcctgggga gcccactgag gggccaggga ggaaggaggc cacggagcgg 2280

gaggcagcgc ccccaccacgt cgggcttccc tggcctcccg ccactcgcct tcttagagtt 2340

ttattccttt cctttttga gatttttttt ccgtgtgttt atttttatt atttttcaag 2400

ataaggagaa agaaagtacc cagcaaatgg gcattttaca agaagtacga atcttatttt 2460

tcctgtcctg cccgtgaggg tgggggggac cgggcccctc tctagggacc cctcgcccca 2520

gcctcattcc ccattctgtg tcccatgtcc cgtgtctcct cggtcgcccc gtgtttgcgc 2580

ttgaccatgt tgcactgttt gcatgcgccc gaggcagacg tctgtcaggg gcttggattt 2640

cgtgtgccgc tgccacccgc ccacccgcct tgtgagatgg aattgtaata aaccacgcca 2700

tgaggacacc gccgcccgcc tcggcgcttc ctccaccgag aaaaaaaaaa aaaaa 2755

<210> 18
<211> 450
<212> PRT
<213> Homo sapiens

<400> 18

Met Ser Ala Ile Gln Ala Ala Trp Pro Ser Gly Thr Glu Cys Ile Ala
1 5 10 15

Lys Tyr Asn Phe His Gly Thr Ala Glu Gln Asp Leu Pro Phe Cys Lys
20 25 30

Gly Asp Val Leu Thr Ile Val Ala Val Thr Lys Asp Pro Asn Trp Tyr
35 40 45

Lys Ala Lys Asn Lys Val Gly Arg Glu Gly Ile Ile Pro Ala Asn Tyr
50 55 60

Val Gln Lys Arg Glu Gly Val Lys Ala Gly Thr Lys Leu Ser Leu Met
65 70 75 80

```
Pro Trp Phe His Gly Lys Ile Thr Arg Glu Gln Ala Glu Arg Leu Leu
                85                  90                  95

Tyr Pro Pro Glu Thr Gly Leu Phe Leu Val Arg Glu Ser Thr Asn Tyr
            100                 105                 110

Pro Gly Asp Tyr Thr Leu Cys Val Ser Cys Asp Gly Lys Val Glu His
        115                 120                 125

Tyr Arg Ile Met Tyr His Ala Ser Lys Leu Ser Ile Asp Glu Glu Val
    130                 135                 140

Tyr Phe Glu Asn Leu Met Gln Leu Val Glu His Tyr Thr Ser Asp Ala
145                 150                 155                 160

Asp Gly Leu Cys Thr Arg Leu Ile Lys Pro Lys Val Met Glu Gly Thr
                165                 170                 175

Val Ala Ala Gln Asp Glu Phe Tyr Arg Ser Gly Trp Ala Leu Asn Met
            180                 185                 190

Lys Glu Leu Lys Leu Leu Gln Thr Ile Gly Lys Gly Glu Phe Gly Asp
        195                 200                 205

Val Met Leu Gly Asp Tyr Arg Gly Asn Lys Val Ala Val Lys Cys Ile
    210                 215                 220

Lys Asn Asp Ala Thr Ala Gln Ala Phe Leu Ala Glu Ala Ser Val Met
225                 230                 235                 240

Thr Gln Leu Arg His Ser Asn Leu Val Gln Leu Leu Gly Val Ile Val
                245                 250                 255

Glu Glu Lys Gly Gly Leu Tyr Ile Val Thr Glu Tyr Met Ala Lys Gly
            260                 265                 270

Ser Leu Val Asp Tyr Leu Arg Ser Arg Gly Arg Ser Val Leu Gly Gly
        275                 280                 285

Asp Cys Leu Leu Lys Phe Ser Leu Asp Val Cys Glu Ala Met Glu Tyr
    290                 295                 300

Leu Glu Gly Asn Asn Phe Val His Arg Asp Leu Ala Ala Arg Asn Val
305                 310                 315                 320

Leu Val Ser Glu Asp Asn Val Ala Lys Val Ser Asp Phe Gly Leu Thr
                325                 330                 335
```

Lys Glu Ala Ser Ser Thr Gln Asp Thr Gly Lys Leu Pro Val Lys Trp
340 345 350

Thr Ala Pro Glu Ala Leu Arg Glu Lys Lys Phe Ser Thr Lys Ser Asp
355 360 365

Val Trp Ser Phe Gly Ile Leu Leu Trp Glu Ile Tyr Ser Phe Gly Arg
370 375 380

Val Pro Tyr Pro Arg Ile Pro Leu Lys Asp Val Val Pro Arg Val Glu
385 390 395 400

Lys Gly Tyr Lys Met Asp Ala Pro Asp Gly Cys Pro Pro Ala Val Tyr
405 410 415

Glu Val Met Lys Asn Cys Trp His Leu Asp Ala Ala Met Arg Pro Ser
420 425 430

Phe Leu Gln Leu Arg Glu Gln Leu Glu His Ile Lys Thr His Glu Leu
435 440 445

His Leu
450

<210> 19
<211> 3069
<212> DNA
<213> Homo sapiens

<400> 19
gagtgtcggg cgcggcagga ggacgaggca gggcgggcgg gcgctctaag ggttctgctc 60

tgactccagg ttgggacagc gtcttcgctg ctgctggata gtcgtgtttt cggggatcga 120

ggatactcac cagaaaccga aaatgccgaa accaatcaat gtccgagtta ccaccatgga 180

tgcagagctg gagtttgcaa tccagccaaa tacaactgga aaacagcttt ttgatcaggt 240

ggtaaagact atcggcctcc gggaagtgtg gtactttggc ctccactatg tggataataa 300

aggatttcct acctggctga agctggataa gaaggtgtct gcccaggagg tcaggaagga 360

gaatcccctc cagttcaagt tccgggccaa gttctaccct gaagatgtgg ctgaggagct 420

catccaggac atcacccaga aacttttctt cctccaagtg aaggaaggaa tccttagcga 480

tgagatctac tgcccccctg agactgccgt gctcttgggg tcctacgctg tgcaggccaa 540

gtttggggac tacaacaaag aagtgcacaa gtctgggtac ctcagctctg agcggctgat 600

ccctcaaaga gtgatggacc agcacaaact taccagggac cagtgggagg accggatcca 660

ggtgtggcat gcggaacacc gtgggatgct caaagataat gctatgttgg aatacctgaa 720

gattgctcag gacctggaaa tgtatggaat caactatttc gagataaaaa acaagaaagg 780
aacagacctt tggcttggag ttgatgccct tggactgaat atttatgaga aagatgataa 840
gttaacccca aagattggct ttccttggag tgaaatcagg aacatctctt tcaatgacaa 900
aaagtttgtc attaaaccca tcgacaagaa ggcacctgac tttgtgtttt atgccccacg 960
tctgagaatc aacaagcgga tcctgcagct ctgcatgggc aaccatgagt tgtatatgcg 1020
ccgcaggaag cctgacacca tcgaggtgca gcagatgaag gcccaggccc gggaggagaa 1080
gcatcagaag cagctggagc ggcaacagct ggaaacagag aagaaaagga gagaaaccgt 1140
ggagagagag aaagagcaga tgatgcgcga gaaggaggag ttgatgctgc ggctgcagga 1200
ctatgaggag aagacaaaga aggcagagag agagctctcg gagcagattc agagggccct 1260
gcagctggag gaggagagga agcgggcaca ggaggaggcc gagcgcctag aggctgaccg 1320
tatggctgca ctgcgggcta aggaggagct ggagagacag gcggtggatc agataaagag 1380
ccaggagcag ctggctgcgg agcttgcaga atacactgcc aagattgccc tcctggaaga 1440
ggcgcggagg cgcaaggagg atgaagttga agagtggcag cacagggcca aagaagccca 1500
ggatgacctg gtgaagacca aggaggagct gcacctggtg atgacagcac ccccgccccc 1560
accacccccc gtgtacgagc cggtgagcta ccatgtccag gagagcttgc aggatgaggg 1620
cgcagagccc acgggctaca gcgcggagct gtctagtgag ggcatccggg atgaccgcaa 1680
tgaggagaag cgcatcactg aggcagagaa gaacgagcgt gtgcagcggc agctgctgac 1740
gctgagcagc gagctgtccc aggcccgaga tgagaataag aggacccaca atgacatcat 1800
ccacaacgag aacatgaggc aaggccggga caagtacaag acgctgcggc agatccggca 1860
gggcaacacc aagcagcgca tcgacgagtt cgaggccctg taacagccag gccaggacca 1920
agggcagagg ggtgctcata gcgggcgctg ccagccccgc cacgcttgtg tctttagtgc 1980
tccaagtcta ggaactccct cagatcccag ttcctttaga aagcagttac ccaacagaaa 2040
cattctgggc tgggaaccag ggaggcgccc tggtttgttt tccccagttg taatagtgcc 2100
aagcaggcct gattctcgcg attattctcg aatcacctcc tgtgttgtgc tgggagcagg 2160
actgattgaa ttacggaaaa tgcctgtaaa gtctgagtaa gaaacttcat gctggcctgt 2220
gtgatacaag agtcagcatc attaaaggaa acgtggcagg acttccatct gtgccatact 2280
tgttctgtat tcgaaatgag ctcaaattga ttttttaatt tctatgaagg atccatcttt 2340
gtatatttac atgcttagag gggtgaaaat tattttggaa attgagtctg aagcactctc 2400
gcacacacag tgattccctc ctcccgtcac tccacgcagc tggcagagag cacagtgatc 2460
accagcgtga gtggtggagg aggacacttg gatttttttt tttgtttttt ttttttttgc 2520
ttaacagttt tagaatacat tgtacttata caccttatta atgatcagct atatactatt 2580
tatatacaag tgataataca gatttgtaac attagtttta aaaagggaaa gttttgttct 2640

gtatattttg ttacctttta cagaataaaa gaattacata tgaaaaaccc tctaaaccat 2700

ggcacttgat gtgatgtggc aggagggcag tggtggagct ggacctgcct gctgcagtca 2760

cgtgtaaaca ggattattat tagtgtttta tgcatgtaat ggactatgca cacttttaat 2820

tttgtcagat tcacacatgc cactatgagc tttcagactc cagctgtgaa gagactctgt 2880

ttgcttgtgt ttgtttgttt gcagtctctc tctgccatgg ccttggcagg ctgctggaag 2940

gcagcttgtg gaggccgttg gttccgccca ctcattcctt ctcgtgcact gctttctcct 3000

tcacagctaa gatgccatgt gcaggtggat tccatgccgc agacatgaaa taaaagcttt 3060

gcaaaggca 3069

<210> 20
<211> 586
<212> PRT
<213> Homo sapiens

<400> 20

Met Pro Lys Pro Ile Asn Val Arg Val Thr Thr Met Asp Ala Glu Leu
1               5                   10                  15

Glu Phe Ala Ile Gln Pro Asn Thr Thr Gly Lys Gln Leu Phe Asp Gln
            20                  25                  30

Val Val Lys Thr Ile Gly Leu Arg Glu Val Trp Tyr Phe Gly Leu His
        35                  40                  45

Tyr Val Asp Asn Lys Gly Phe Pro Thr Trp Leu Lys Leu Asp Lys Lys
    50                  55                  60

Val Ser Ala Gln Glu Val Arg Lys Glu Asn Pro Leu Gln Phe Lys Phe
65                  70                  75                  80

Arg Ala Lys Phe Tyr Pro Glu Asp Val Ala Glu Glu Leu Ile Gln Asp
                85                  90                  95

Ile Thr Gln Lys Leu Phe Phe Leu Gln Val Lys Glu Gly Ile Leu Ser
            100                 105                 110

Asp Glu Ile Tyr Cys Pro Pro Glu Thr Ala Val Leu Leu Gly Ser Tyr
        115                 120                 125

Ala Val Gln Ala Lys Phe Gly Asp Tyr Asn Lys Glu Val His Lys Ser
    130                 135                 140

Gly Tyr Leu Ser Ser Glu Arg Leu Ile Pro Gln Arg Val Met Asp Gln
145                 150                 155                 160

His Lys Leu Thr Arg Asp Gln Trp Glu Asp Arg Ile Gln Val Trp His
165 170 175

Ala Glu His Arg Gly Met Leu Lys Asp Asn Ala Met Leu Glu Tyr Leu
180 185 190

Lys Ile Ala Gln Asp Leu Glu Met Tyr Gly Ile Asn Tyr Phe Glu Ile
195 200 205

Lys Asn Lys Lys Gly Thr Asp Leu Trp Leu Gly Val Asp Ala Leu Gly
210 215 220

Leu Asn Ile Tyr Glu Lys Asp Asp Lys Leu Thr Pro Lys Ile Gly Phe
225 230 235 240

Pro Trp Ser Glu Ile Arg Asn Ile Ser Phe Asn Asp Lys Lys Phe Val
245 250 255

Ile Lys Pro Ile Asp Lys Lys Ala Pro Asp Phe Val Phe Tyr Ala Pro
260 265 270

Arg Leu Arg Ile Asn Lys Arg Ile Leu Gln Leu Cys Met Gly Asn His
275 280 285

Glu Leu Tyr Met Arg Arg Arg Lys Pro Asp Thr Ile Glu Val Gln Gln
290 295 300

Met Lys Ala Gln Ala Arg Glu Glu Lys His Gln Lys Gln Leu Glu Arg
305 310 315 320

Gln Gln Leu Glu Thr Glu Lys Lys Arg Arg Glu Thr Val Glu Arg Glu
325 330 335

Lys Glu Gln Met Met Arg Glu Lys Glu Glu Leu Met Leu Arg Leu Gln
340 345 350

Asp Tyr Glu Glu Lys Thr Lys Lys Ala Glu Arg Glu Leu Ser Glu Gln
355 360 365

Ile Gln Arg Ala Leu Gln Leu Glu Glu Glu Arg Lys Arg Ala Gln Glu
370 375 380

Glu Ala Glu Arg Leu Glu Ala Asp Arg Met Ala Ala Leu Arg Ala Lys
385 390 395 400

Glu Glu Leu Glu Arg Gln Ala Val Asp Gln Ile Lys Ser Gln Glu Gln

405 410 415

Leu Ala Ala Glu Leu Ala Glu Tyr Thr Ala Lys Ile Ala Leu Leu Glu
420 425 430

Glu Ala Arg Arg Arg Lys Glu Asp Glu Val Glu Glu Trp Gln His Arg
435 440 445

Ala Lys Glu Ala Gln Asp Asp Leu Val Lys Thr Lys Glu Glu Leu His
450 455 460

Leu Val Met Thr Ala Pro Pro Pro Pro Pro Pro Pro Val Tyr Glu Pro
465 470 475 480

Val Ser Tyr His Val Gln Glu Ser Leu Gln Asp Glu Gly Ala Glu Pro
485 490 495

Thr Gly Tyr Ser Ala Glu Leu Ser Ser Glu Gly Ile Arg Asp Asp Arg
500 505 510

Asn Glu Glu Lys Arg Ile Thr Glu Ala Glu Lys Asn Glu Arg Val Gln
515 520 525

Arg Gln Leu Leu Thr Leu Ser Ser Glu Leu Ser Gln Ala Arg Asp Glu
530 535 540

Asn Lys Arg Thr His Asn Asp Ile Ile His Asn Glu Asn Met Arg Gln
545 550 555 560

Gly Arg Asp Lys Tyr Lys Thr Leu Arg Gln Ile Arg Gln Gly Asn Thr
565 570 575

Lys Gln Arg Ile Asp Glu Phe Glu Ala Leu
580 585

<210> 21
<211> 3628
<212> DNA
<213> Homo sapiens

<400> 21
agagcatcag caagagtagc agcgagcagc cgcgctggtg gcggcggcgc gtcgttgcag 60

ttgcgccatc tgtcaggagc ggagccggcg aggagggggc tgccgcgggc gaggaggagg 120

ggtcgccgcg agccgaaggc cttcgagacc cgcccgccgc ccggcggcga gagtagaggc 180

gaggttgttg tgcgagcggc gcgtcctctc ccgcccgggc gcgccgcgct tctcccagcg 240

caccgaggac cgcccgggcg cacacaaagc cgccgcccgc gccgcaccgc ccggcggccg 300

ccgcccgcgc cagggaggga ttcggccgcc gggccgggga caccccggcg ccgccccctc 360
ggtgctctcg gaaggcccac cggctcccgg gcccgccggg gacccccccgg agccgcctcg 420
gccgcgccgg aggagggcgg ggagaggacc atgtgagtgg gctccggagc ctcagcgccg 480
cgcagttttt ttgaagaagc aggatgctga tctaaacgtg gaaaaagacc agtcctgcct 540
ctgttgtaga agacatgtgg tgtatataaa gtttgtgatc gttggcggac attttggaat 600
ttagataatg ggctgtgtgc aatgtaagga taaagaagca acaaaactga cggaggagag 660
ggacggcagc ctgaaccaga gctctgggta ccgctatggc acagacccca cccctcagca 720
ctaccccagc ttcggtgtga cctccatccc caactacaac aacttccacg cagccggggg 780
ccaaggactc accgtctttg gaggtgtgaa ctcttcgtct catacgggga ccttgcgtac 840
gagaggagga acaggagtga cactctttgt ggccctttat gactatgaag cacggacaga 900
agatgacctg agttttcaca aaggagaaaa atttcaaata ttgaacagct cggaaggaga 960
ttggtgggaa gcccgctcct tgacaactgg agagacaggt tacattccca gcaattatgt 1020
ggctccagtt gactctatcc aggcagaaga gtggtacttt ggaaaacttg gccgaaaaga 1080
tgctgagcga cagctattgt cctttggaaa cccaagaggt acctttctta tccgcgagag 1140
tgaaaccacc aaaggtgcct attcactttc tatccgtgat tgggatgata tgaaaggaga 1200
ccatgtcaaa cattataaaa ttcgcaaact tgacaatggt ggatactaca ttaccacccg 1260
ggcccagttt gaaacacttc agcagcttgt acaacattac tcagagagag ctgcaggtct 1320
ctgctgccgc ctagtagttc cctgtcacaa agggatgcca aggcttaccg atctgtctgt 1380
caaaaccaaa gatgtctggg aaatccctcg agaatccctg cagttgatca agagactggg 1440
aaatgggcag tttggggaag tatggatggg tacctggaat ggaaacacaa aagtagccat 1500
aaagactctt aaaccaggca caatgtcccc cgaatcattc cttgaggaag cgcagatcat 1560
gaagaagctg aagcacgaca agctggtcca gctctatgca gtggtgtctg aggagcccat 1620
ctacatcgtc accgagtata tgaacaaagg aagtttactg gatttcttaa aagatggaga 1680
aggaagagct ctgaaattac caaatcttgt ggacatggca gcacaggtgg ctgcaggaat 1740
ggcttacatc gagcgcatga attatatcca tagagatctg cgatcagcaa acattctagt 1800
ggggaatgga ctcatatgca agattgctga cttcggattg gcccgattga tagaagacaa 1860
tgagtacaca gcaagacaag gtgcaaagtt ccccatcaag tggacggccc ccgaggcagc 1920
cctgtacggg aggttcacaa tcaagtctga cgtgtggtct tttggaatct tactcacaga 1980
gctggtcacc aaaggaagag tgccataccc aggcatgaac aaccgggagg tgctggagca 2040
ggtggagcga ggctacagga tgccctgccc gcaggactgc cccatctctc tgcatgagct 2100
catgatccac tgctggaaaa aggaccctga agaacgcccc acttttgagt acttgcagag 2160
cttcctggaa gactacttta ccgcgacaga gccccagtac caacctggtg aaaacctgta 2220

aggcccgggt ctgcggagag aggccttgtc ccagaggctg ccccacccct ccccattagc 2280

tttcaattcc gtagccagct gctccccagc agcggaaccg cccaggatca gattgcatgt 2340

gactctgaag ctgacgaact tccatggccc tcattaatga cacttgtccc caaatccgaa 2400

cctcctctgt gaagcattcg agacagaacc ttgttatttc tcagactttg gaaaatgcat 2460

tgtatcgatg ttatgtaaaa ggccaaacct ctgttcagtg taaatagtta ctccagtgcc 2520

aacaatccta gtgctttcct tttttaaaaa tgcaaatcct atgtgatttt aactctgtct 2580

tcacctgatt caactaaaaa aaaaaaagta ttattttcca aaagtggcct ctttgtctaa 2640

aacaataaaa tttttttca tgttttaaca aaaaccaatc aggacaggtg tttgtttttg 2700

ttttcttttt tataaatatg aatatatata atatatatgt ccctgtacat atacaatgtg 2760

ggtgctaatg tggagactgt ggccggcctg agccaccaag ctgcgggacc cagagggagg 2820

attttactgc aagtcagcat caaagcaccg gtgttattct gaaaacacca gtggcctcat 2880

ttttggcttt tgcaaagcat gaatttttc atttggattg cactttcctg gttcatgact 2940

gtacctgtag gtggttgtta ctttgactct tttcaggaac caccccccaa gctgaattta 3000

caagttctgt tagcactatt tgcttcaact tactgcgatt tgttctcaaa acttaaaaat 3060

aagcaagcaa atggctgata ctaccaagag aactggaaga tggataccac acaaacttct 3120

tgtataaaaa tatgaatgct gaaatgtttc agacattttt aatttaataa acctgtaacc 3180

acatttaagt gatctaaaac ccatagcatt gtagtcatgg caacccgcta aactttctca 3240

tgcaactaaa atttctgggg gaaatgaggg tgggggttgt acatttccca ttgtaaaata 3300

agtgttttaa atgtcctgta ctgctaacga atgactttct atatgtccag gagttctcca 3360

gtggaataac tatgcactac tttacatttc atggggatgc acaaaaacaa aaaagtatta 3420

catttttagt tgctgtttgt accaacctta aattacatat gtttaacaac aacaaatcaa 3480

aaatcctatt tctattgagt ttttaatact gactagcaac tctgaagtct taattccttt 3540

tttgttatga tttatttgtg agtttacatt tttaaattgt ttaactttct taatttagta 3600

attaaaaaga gagcatttta catttgaa 3628

<210> 22
<211> 3238
<212> DNA
<213> Homo sapiens

<400> 22
ggctcccggg cccgccgggg accccccgga gccgcctcgg ccgcgccgga ggagggcggg 60

gagaggacca tgtgagtggg ctccggagcc tcagcgccgc gcagtttttt tgaagaagca 120

ggatgctgat ctaaacgtgg aaaaagacca gtcctgcctc tgttgtagaa gacatgtggt 180

gtatataaag tttgtgatcg ttggcggaca ttttggaatt tagataatgg gctgtgtgca 240

atgtaaggat aaagaagcaa caaaactgac ggaggagagg gacggcagcc tgaaccagag 300
ctctgggtac cgctatggca cagaccccac ccctcagcac taccccagct tcggtgtgac 360
ctccatcccc aactacaaca acttccacgc agccgggggc caaggactca ccgtctttgg 420
aggtgtgaac tcttcgtctc atacggggac cttgcgtacg agaggaggaa caggagtgac 480
actctttgtg gccctttatg actatgaagc acggacagaa gatgacctga gttttcacaa 540
aggagaaaaa tttcaaatat tgaacagctc ggaaggagat tggtgggaag cccgctcctt 600
gacaactgga gagacaggtt acattcccag caattatgtg gctccagttg actctatcca 660
ggcagaagag tggtactttg gaaaacttgg ccgaaaagat gctgagcgac agctattgtc 720
ctttggaaac ccaagaggta cctttcttat ccgcgagagt gaaaccacca aaggtgccta 780
ttcactttct atccgtgatt gggatgatat gaaaggagac catgtcaaac attataaaat 840
tcgcaaactt gacaatggtg gatactacat taccacccgg gcccagtttg aaacacttca 900
gcagcttgta caacattact cagagaaagc tgatggtttg tgttttaact taactgtgat 960
tgcatcgagt tgtaccccac aaacttctgg attggctaaa gatgcttggg aagttgcacg 1020
tcgttcgttg tgtctggaga agaagctggg tcaggggtgt ttcgctgaag tgtggcttgg 1080
tacctggaat ggaaacacaa aagtagccat aaagactctt aaaccaggca caatgtcccc 1140
cgaatcattc cttgaggaag cgcagatcat gaagaagctg aagcacgaca agctggtcca 1200
gctctatgca gtggtgtctg aggagcccat ctacatcgtc accgagtata tgaacaaagg 1260
aagtttactg gatttcttaa aagatggaga aggaagagct ctgaaattac caaatcttgt 1320
ggacatggca gcacaggtgg ctgcaggaat ggcttacatc gagcgcatga attatatcca 1380
tagagatctg cgatcagcaa acattctagt ggggaatgga ctcatatgca agattgctga 1440
cttcggattg gcccgattga tagaagacaa tgagtacaca gcaagacaag gtgcaaagtt 1500
ccccatcaag tggacggccc ccgaggcagc cctgtacggg aggttcacaa tcaagtctga 1560
cgtgtggtct tttggaatct tactcacaga gctggtcacc aaaggaagag tgccataccc 1620
aggcatgaac aaccgggagg tgctggagca ggtggagcga ggctacagga tgccctgccc 1680
gcaggactgc cccatctctc tgcatgagct catgatccac tgctggaaaa aggaccctga 1740
agaacgcccc acttttgagt acttgcagag cttcctggaa gactacttta ccgcgacaga 1800
gccccagtac caacctggtg aaaacctgta aggcccgggt ctgcggagag aggccttgtc 1860
ccagaggctg ccccacccct ccccattagc tttcaattcc gtagccagct gctccccagc 1920
agcggaaccg cccaggatca gattgcatgt gactctgaag ctgacgaact tccatggccc 1980
tcattaatga cacttgtccc caaatccgaa cctcctctgt gaagcattcg agacagaacc 2040
ttgttatttc tcagactttg gaaaatgcat tgtatcgatg ttatgtaaaa ggccaaacct 2100

ctgttcagtg taaatagtta ctccagtgcc aacaatccta gtgctttcct tttttaaaaa 2160

tgcaaatcct atgtgatttt aactctgtct tcacctgatt caactaaaaa aaaaaaagta 2220

ttattttcca aaagtggcct ctttgtctaa aacaataaaa tttttttca tgttttaaca 2280

aaaaccaatc aggacaggtg tttgtttttg ttttcttttt tataaatatg aatatatata 2340

atatatatgt ccctgtacat atacaatgtg ggtgctaatg tggagactgt ggccggcctg 2400

agccaccaag ctgcgggacc cagagggagg attttactgc aagtcagcat caaagcaccg 2460

gtgttattct gaaaacacca gtggcctcat ttttggcttt tgcaaagcat gaatttttttc 2520

atttggattg cactttcctg gttcatgact gtacctgtag gtggttgtta ctttgactct 2580

tttcaggaac caccccccaa gctgaattta caagttctgt tagcactatt tgcttcaact 2640

tactgcgatt tgttctcaaa acttaaaaat aagcaagcaa atggctgata ctaccaagag 2700

aactggaaga tggataccac acaaacttct tgtataaaaa tatgaatgct gaaatgtttc 2760

agacattttt aatttaataa acctgtaacc acatttaagt gatctaaaac ccatagcatt 2820

gtagtcatgg caacccgcta aactttctca tgcaactaaa atttctgggg gaaatgaggg 2880

tgggggttgt acatttccca ttgtaaaata agtgttttaa atgtcctgta ctgctaacga 2940

atgactttct atatgtccag gagttctcca gtggaataac tatgcactac tttacatttc 3000

atggggatgc acaaaaacaa aaaagtatta catttttagt tgctgtttgt accaacctta 3060

aattacatat gtttaacaac aacaaatcaa aaatcctatt tctattgagt ttttaatact 3120

gactagcaac tctgaagtct taattccttt tttgttatga tttatttgtg agtttacatt 3180

tttaaattgt ttaactttct taatttagta attaaaaaga gagcatttta catttgaa 3238

<210> 23
<211> 2959
<212> DNA
<213> Homo sapiens

<400> 23
aaatgatcaa gtgttgggta taagccaagg agctgagaga gggggagacc agcgcaggtc 60

tgaggagctg agaagggagg cttacgtgaa gggaatttag ataatgggct gtgtgcaatg 120

taaggataaa gaagcaacaa aactgacgga ggagagggac ggcagcctga accagagctc 180

tgggtaccgc tatggcacag accccacccc tcagcactac cccagcttcg gtgtgacctc 240

catccccaac tacaacaact tccacgcagc cgggggccaa ggactcaccg tctttggagg 300

tgtgaactct tcgtctcata cggggacctt gcgtacgaga ggaggaacag gagtgacact 360

ctttgtggcc ctttatgact atgaagcacg gacagaagat gacctgagtt ttcacaaagg 420

agaaaaattt caaatattga acagctcgga aggagattgg tgggaagccc gctccttgac 480

aactggagag acaggttaca ttcccagcaa ttatgtggct ccagttgact ctatccaggc 540

agaagagtgg tactttggaa aacttggccg aaaagatgct gagcgacagc tattgtcctt 600
tggaaaccca agaggtacct ttcttatccg cgagagtgaa accaccaaag gtgcctattc 660
actttctatc cgtgattggg atgatatgaa aggagaccat gtcaaacatt ataaaattcg 720
caaacttgac aatggtggat actacattac cacccgggcc cagtttgaaa cacttcagca 780
gcttgtacaa cattactcag gtacctggaa tggaaacaca aaagtagcca taaagactct 840
taaaccaggc acaatgtccc ccgaatcatt ccttgaggaa gcgcagatca tgaagaagct 900
gaagcacgac aagctggtcc agctctatgc agtggtgtct gaggagccca tctacatcgt 960
caccgagtat atgaacaaag gaagtttact ggatttctta aaagatggag aaggaagagc 1020
tctgaaatta ccaaatcttg tggacatggc agcacaggtg gctgcaggaa tggcttacat 1080
cgagcgcatg aattatatcc atagagatct gcgatcagca aacattctag tggggaatgg 1140
actcatatgc aagattgctg acttcggatt ggcccgattg atagaagaca atgagtacac 1200
agcaagacaa ggtgcaaagt tccccatcaa gtggacggcc cccgaggcag ccctgtacgg 1260
gaggttcaca atcaagtctg acgtgtggtc ttttggaatc ttactcacag agctggtcac 1320
caaaggaaga gtgccatacc caggcatgaa caaccgggag gtgctggagc aggtggagcg 1380
aggctacagg atgccctgcc cgcaggactg ccccatctct ctgcatgagc tcatgatcca 1440
ctgctggaaa aaggaccctg aagaacgccc cacttttgag tacttgcaga gcttcctgga 1500
agactacttt accgcgacag agccccagta ccaacctggt gaaaacctgt aaggcccggg 1560
tctgcggaga gaggccttgt cccagaggct gccccacccc tccccattag ctttcaattc 1620
cgtagccagc tgctccccag cagcggaacc gcccaggatc agattgcatg tgactctgaa 1680
gctgacgaac ttccatggcc ctcattaatg acacttgtcc ccaaatccga acctcctctg 1740
tgaagcattc gagacagaac cttgttattt ctcagacttt ggaaaatgca ttgtatcgat 1800
gttatgtaaa aggccaaacc tctgttcagt gtaaatagtt actccagtgc caacaatcct 1860
agtgctttcc tttttaaaa atgcaaatcc tatgtgattt taactctgtc ttcacctgat 1920
tcaactaaaa aaaaaaaagt attattttcc aaaagtggcc tctttgtcta aaacaataaa 1980
attttttttc atgttttaac aaaaaccaat caggacaggt gtttgttttt gttttctttt 2040
ttataaatat gaatatatat aatatatatg tccctgtaca tatacaatgt gggtgctaat 2100
gtggagactg tggccggcct gagccaccaa gctgcgggac ccagagggag gattttactg 2160
caagtcagca tcaaagcacc ggtgttattc tgaaaacacc agtggcctca tttttggctt 2220
ttgcaaagca tgaatttttt catttggatt gcactttcct ggttcatgac tgtacctgta 2280
ggtggttgtt actttgactc ttttcaggaa ccacccccca agctgaattt acaagttctg 2340
ttagcactat ttgcttcaac ttactgcgat ttgttctcaa aacttaaaaa taagcaagca 2400
aatggctgat actaccaaga gaactggaag atggatacca cacaaacttc ttgtataaaa 2460

atatgaatgc tgaaatgttt cagacatttt taatttaata aacctgtaac cacatttaag 2520

tgatctaaaa cccatagcat tgtagtcatg gcaacccgct aaactttctc atgcaactaa 2580

aatttctggg ggaaatgagg gtgggggttg tacatttccc attgtaaaat aagtgtttta 2640

aatgtcctgt actgctaacg aatgactttc tatatgtcca ggagttctcc agtggaataa 2700

ctatgcacta ctttacattt catggggatg cacaaaaaca aaaaagtatt acatttttag 2760

ttgctgtttg taccaacctt aaattacata tgtttaacaa caacaaatca aaaatcctat 2820

ttctattgag tttttaatac tgactagcaa ctctgaagtc ttaattcctt ttttgttatg 2880

atttatttgt gagtttacat ttttaaattg tttaactttc ttaatttagt aattaaaaag 2940

agagcatttt acatttgaa 2959

<210> 24
<211> 537
<212> PRT
<213> Homo sapiens

<400> 24

Met Gly Cys Val Gln Cys Lys Asp Lys Glu Ala Thr Lys Leu Thr Glu
1 5 10 15

Glu Arg Asp Gly Ser Leu Asn Gln Ser Ser Gly Tyr Arg Tyr Gly Thr
20 25 30

Asp Pro Thr Pro Gln His Tyr Pro Ser Phe Gly Val Thr Ser Ile Pro
35 40 45

Asn Tyr Asn Asn Phe His Ala Ala Gly Gly Gln Gly Leu Thr Val Phe
50 55 60

Gly Gly Val Asn Ser Ser Ser His Thr Gly Thr Leu Arg Thr Arg Gly
65 70 75 80

Gly Thr Gly Val Thr Leu Phe Val Ala Leu Tyr Asp Tyr Glu Ala Arg
85 90 95

Thr Glu Asp Asp Leu Ser Phe His Lys Gly Glu Lys Phe Gln Ile Leu
100 105 110

Asn Ser Ser Glu Gly Asp Trp Trp Glu Ala Arg Ser Leu Thr Thr Gly
115 120 125

Glu Thr Gly Tyr Ile Pro Ser Asn Tyr Val Ala Pro Val Asp Ser Ile
130 135 140

Gln Ala Glu Glu Trp Tyr Phe Gly Lys Leu Gly Arg Lys Asp Ala Glu
145 150 155 160

Arg Gln Leu Leu Ser Phe Gly Asn Pro Arg Gly Thr Phe Leu Ile Arg
165 170 175

Glu Ser Glu Thr Thr Lys Gly Ala Tyr Ser Leu Ser Ile Arg Asp Trp
180 185 190

Asp Asp Met Lys Gly Asp His Val Lys His Tyr Lys Ile Arg Lys Leu
195 200 205

Asp Asn Gly Gly Tyr Tyr Ile Thr Thr Arg Ala Gln Phe Glu Thr Leu
210 215 220

Gln Gln Leu Val Gln His Tyr Ser Glu Arg Ala Ala Gly Leu Cys Cys
225 230 235 240

Arg Leu Val Val Pro Cys His Lys Gly Met Pro Arg Leu Thr Asp Leu
245 250 255

Ser Val Lys Thr Lys Asp Val Trp Glu Ile Pro Arg Glu Ser Leu Gln
260 265 270

Leu Ile Lys Arg Leu Gly Asn Gly Gln Phe Gly Glu Val Trp Met Gly
275 280 285

Thr Trp Asn Gly Asn Thr Lys Val Ala Ile Lys Thr Leu Lys Pro Gly
290 295 300

Thr Met Ser Pro Glu Ser Phe Leu Glu Glu Ala Gln Ile Met Lys Lys
305 310 315 320

Leu Lys His Asp Lys Leu Val Gln Leu Tyr Ala Val Val Ser Glu Glu
325 330 335

Pro Ile Tyr Ile Val Thr Glu Tyr Met Asn Lys Gly Ser Leu Leu Asp
340 345 350

Phe Leu Lys Asp Gly Glu Gly Arg Ala Leu Lys Leu Pro Asn Leu Val
355 360 365

Asp Met Ala Ala Gln Val Ala Ala Gly Met Ala Tyr Ile Glu Arg Met
370 375 380

Asn Tyr Ile His Arg Asp Leu Arg Ser Ala Asn Ile Leu Val Gly Asn
385 390 395 400

```
Gly Leu Ile Cys Lys Ile Ala Asp Phe Gly Leu Ala Arg Leu Ile Glu
                405                 410                 415

Asp Asn Glu Tyr Thr Ala Arg Gln Gly Ala Lys Phe Pro Ile Lys Trp
            420                 425                 430

Thr Ala Pro Glu Ala Ala Leu Tyr Gly Arg Phe Thr Ile Lys Ser Asp
        435                 440                 445

Val Trp Ser Phe Gly Ile Leu Leu Thr Glu Leu Val Thr Lys Gly Arg
    450                 455                 460

Val Pro Tyr Pro Gly Met Asn Asn Arg Glu Val Leu Glu Gln Val Glu
465                 470                 475                 480

Arg Gly Tyr Arg Met Pro Cys Pro Gln Asp Cys Pro Ile Ser Leu His
                485                 490                 495

Glu Leu Met Ile His Cys Trp Lys Lys Asp Pro Glu Glu Arg Pro Thr
            500                 505                 510

Phe Glu Tyr Leu Gln Ser Phe Leu Glu Asp Tyr Phe Thr Ala Thr Glu
        515                 520                 525

Pro Gln Tyr Gln Pro Gly Glu Asn Leu
    530                 535


<210>  25
<211>  534
<212>  PRT
<213>  Homo sapiens

<400>  25

Met Gly Cys Val Gln Cys Lys Asp Lys Glu Ala Thr Lys Leu Thr Glu
1               5                   10                  15

Glu Arg Asp Gly Ser Leu Asn Gln Ser Ser Gly Tyr Arg Tyr Gly Thr
            20                  25                  30

Asp Pro Thr Pro Gln His Tyr Pro Ser Phe Gly Val Thr Ser Ile Pro
        35                  40                  45

Asn Tyr Asn Asn Phe His Ala Ala Gly Gly Gln Gly Leu Thr Val Phe
    50                  55                  60

Gly Gly Val Asn Ser Ser Ser His Thr Gly Thr Leu Arg Thr Arg Gly
65                  70                  75                  80
```

```
Gly Thr Gly Val Thr Leu Phe Val Ala Leu Tyr Asp Tyr Glu Ala Arg
                85                  90                  95

Thr Glu Asp Asp Leu Ser Phe His Lys Gly Glu Lys Phe Gln Ile Leu
            100                 105                 110

Asn Ser Ser Glu Gly Asp Trp Trp Glu Ala Arg Ser Leu Thr Thr Gly
        115                 120                 125

Glu Thr Gly Tyr Ile Pro Ser Asn Tyr Val Ala Pro Val Asp Ser Ile
    130                 135                 140

Gln Ala Glu Glu Trp Tyr Phe Gly Lys Leu Gly Arg Lys Asp Ala Glu
145                 150                 155                 160

Arg Gln Leu Leu Ser Phe Gly Asn Pro Arg Gly Thr Phe Leu Ile Arg
                165                 170                 175

Glu Ser Glu Thr Thr Lys Gly Ala Tyr Ser Leu Ser Ile Arg Asp Trp
            180                 185                 190

Asp Asp Met Lys Gly Asp His Val Lys His Tyr Lys Ile Arg Lys Leu
        195                 200                 205

Asp Asn Gly Gly Tyr Tyr Ile Thr Thr Arg Ala Gln Phe Glu Thr Leu
    210                 215                 220

Gln Gln Leu Val Gln His Tyr Ser Glu Lys Ala Asp Gly Leu Cys Phe
225                 230                 235                 240

Asn Leu Thr Val Ile Ala Ser Ser Cys Thr Pro Gln Thr Ser Gly Leu
                245                 250                 255

Ala Lys Asp Ala Trp Glu Val Ala Arg Arg Ser Leu Cys Leu Glu Lys
            260                 265                 270

Lys Leu Gly Gln Gly Cys Phe Ala Glu Val Trp Leu Gly Thr Trp Asn
        275                 280                 285

Gly Asn Thr Lys Val Ala Ile Lys Thr Leu Lys Pro Gly Thr Met Ser
    290                 295                 300

Pro Glu Ser Phe Leu Glu Glu Ala Gln Ile Met Lys Lys Leu Lys His
305                 310                 315                 320

Asp Lys Leu Val Gln Leu Tyr Ala Val Val Ser Glu Glu Pro Ile Tyr
                325                 330                 335
```

Ile Val Thr Glu Tyr Met Asn Lys Gly Ser Leu Leu Asp Phe Leu Lys
340 345 350

Asp Gly Glu Gly Arg Ala Leu Lys Leu Pro Asn Leu Val Asp Met Ala
355 360 365

Ala Gln Val Ala Ala Gly Met Ala Tyr Ile Glu Arg Met Asn Tyr Ile
370 375 380

His Arg Asp Leu Arg Ser Ala Asn Ile Leu Val Gly Asn Gly Leu Ile
385 390 395 400

Cys Lys Ile Ala Asp Phe Gly Leu Ala Arg Leu Ile Glu Asp Asn Glu
405 410 415

Tyr Thr Ala Arg Gln Gly Ala Lys Phe Pro Ile Lys Trp Thr Ala Pro
420 425 430

Glu Ala Ala Leu Tyr Gly Arg Phe Thr Ile Lys Ser Asp Val Trp Ser
435 440 445

Phe Gly Ile Leu Leu Thr Glu Leu Val Thr Lys Gly Arg Val Pro Tyr
450 455 460

Pro Gly Met Asn Asn Arg Glu Val Leu Glu Gln Val Glu Arg Gly Tyr
465 470 475 480

Arg Met Pro Cys Pro Gln Asp Cys Pro Ile Ser Leu His Glu Leu Met
485 490 495

Ile His Cys Trp Lys Lys Asp Pro Glu Glu Arg Pro Thr Phe Glu Tyr
500 505 510

Leu Gln Ser Phe Leu Glu Asp Tyr Phe Thr Ala Thr Glu Pro Gln Tyr
515 520 525

Gln Pro Gly Glu Asn Leu
530

<210> 26
<211> 482
<212> PRT
<213> Homo sapiens

<400> 26

Met Gly Cys Val Gln Cys Lys Asp Lys Glu Ala Thr Lys Leu Thr Glu
1 5 10 15

```
Glu Arg Asp Gly Ser Leu Asn Gln Ser Ser Gly Tyr Arg Tyr Gly Thr
            20                  25                  30

Asp Pro Thr Pro Gln His Tyr Pro Ser Phe Gly Val Thr Ser Ile Pro
        35                  40                  45

Asn Tyr Asn Asn Phe His Ala Ala Gly Gly Gln Gly Leu Thr Val Phe
    50                  55                  60

Gly Gly Val Asn Ser Ser Ser His Thr Gly Thr Leu Arg Thr Arg Gly
65                  70                  75                  80

Gly Thr Gly Val Thr Leu Phe Val Ala Leu Tyr Asp Tyr Glu Ala Arg
                85                  90                  95

Thr Glu Asp Asp Leu Ser Phe His Lys Gly Glu Lys Phe Gln Ile Leu
            100                 105                 110

Asn Ser Ser Glu Gly Asp Trp Trp Glu Ala Arg Ser Leu Thr Thr Gly
        115                 120                 125

Glu Thr Gly Tyr Ile Pro Ser Asn Tyr Val Ala Pro Val Asp Ser Ile
    130                 135                 140

Gln Ala Glu Glu Trp Tyr Phe Gly Lys Leu Gly Arg Lys Asp Ala Glu
145                 150                 155                 160

Arg Gln Leu Leu Ser Phe Gly Asn Pro Arg Gly Thr Phe Leu Ile Arg
                165                 170                 175

Glu Ser Glu Thr Thr Lys Gly Ala Tyr Ser Leu Ser Ile Arg Asp Trp
            180                 185                 190

Asp Asp Met Lys Gly Asp His Val Lys His Tyr Lys Ile Arg Lys Leu
        195                 200                 205

Asp Asn Gly Gly Tyr Tyr Ile Thr Thr Arg Ala Gln Phe Glu Thr Leu
    210                 215                 220

Gln Gln Leu Val Gln His Tyr Ser Gly Thr Trp Asn Gly Asn Thr Lys
225                 230                 235                 240

Val Ala Ile Lys Thr Leu Lys Pro Gly Thr Met Ser Pro Glu Ser Phe
                245                 250                 255

Leu Glu Glu Ala Gln Ile Met Lys Lys Leu Lys His Asp Lys Leu Val
```

```
            260                 265                 270

Gln Leu Tyr Ala Val Val Ser Glu Glu Pro Ile Tyr Ile Val Thr Glu
        275                 280                 285

Tyr Met Asn Lys Gly Ser Leu Leu Asp Phe Leu Lys Asp Gly Glu Gly
    290                 295                 300

Arg Ala Leu Lys Leu Pro Asn Leu Val Asp Met Ala Ala Gln Val Ala
305                 310                 315                 320

Ala Gly Met Ala Tyr Ile Glu Arg Met Asn Tyr Ile His Arg Asp Leu
                325                 330                 335

Arg Ser Ala Asn Ile Leu Val Gly Asn Gly Leu Ile Cys Lys Ile Ala
            340                 345                 350

Asp Phe Gly Leu Ala Arg Leu Ile Glu Asp Asn Glu Tyr Thr Ala Arg
        355                 360                 365

Gln Gly Ala Lys Phe Pro Ile Lys Trp Thr Ala Pro Glu Ala Ala Leu
    370                 375                 380

Tyr Gly Arg Phe Thr Ile Lys Ser Asp Val Trp Ser Phe Gly Ile Leu
385                 390                 395                 400

Leu Thr Glu Leu Val Thr Lys Gly Arg Val Pro Tyr Pro Gly Met Asn
                405                 410                 415

Asn Arg Glu Val Leu Glu Gln Val Glu Arg Gly Tyr Arg Met Pro Cys
            420                 425                 430

Pro Gln Asp Cys Pro Ile Ser Leu His Glu Leu Met Ile His Cys Trp
        435                 440                 445

Lys Lys Asp Pro Glu Glu Arg Pro Thr Phe Glu Tyr Leu Gln Ser Phe
    450                 455                 460

Leu Glu Asp Tyr Phe Thr Ala Thr Glu Pro Gln Tyr Gln Pro Gly Glu
465                 470                 475                 480

Asn Leu
```

<210> 27
<211> 1671
<212> DNA
<213> Homo sapiens

<400> 27
acagcttcct gccgcaggcg ggcgggaggg cgggcacgga gaggcgggcg ccgaggaggg 60

gcaggtaggg ctgggacgca ggggtaactg gatcccccga cttcagccca ggccctggtc 120

tgaccaccct gggagcaggg actttccaca gtcagctgga cgcacactca gcccagtaaa 180

agaggggacc catcccggga gccccgggga gggcacagct gcctcctccc gggctcccct 240

gccacctggt gcctacctgc cccctgctcc ctgccgggtc cggtcctcac cccatcttca 300

tctggccttg actctgccct tgaggggcct aggggtgcag ccagcctgct ccgagctccc 360

ctgcagatgg aggaggccat cctggtcccc tgcgtgctgg ggctcctgct gctgcccatc 420

ctggccatgt tgatggcact gtgtgtgcac tgccacagac tgccaggctc ctacgacagc 480

acatcctcag atagtttgta tccaaggggc atccagttca aacggcctca cacggttgcc 540

ccctggccac ctgcctaccc acctgtcacc tcctacccac ccctgagcca gccagacctg 600

ctccccatcc caagatcccc gcagcccctt gggggctccc accggacgcc atcttcccgg 660

cgggattctg atggtgccaa cagtgtggcg agctacgaga acgaggaacc agcctgtgag 720

gatgcggatg aggatgagga cgactatcac aacccaggct acctggtggt gcttcctgac 780

agcaccccgg ccactagcac tgctgcccca tcagctcctg cactcagcac ccctggcatc 840

cgagacagtg ccttctccat ggagtccatt gatgattacg tgaacgttcc ggagagcggg 900

gagagcgcag aagcgtctct ggatggcagc cgggagtatg tgaatgtgtc ccaggaactg 960

catcctggag cggctaagac tgagcctgcc gccctgagtt cccaggaggc agaggaagtg 1020

gaggaagagg gggctccaga ttacgagaat ctgcaggagc tgaactgagg gcctgtggag 1080

gccgagtctg tcctggaacc aggcttgcct gggacggctg agctgggcag ctggaagtgg 1140

ctctggggtc ctcacatggc gtcctgccct tgctccagcc tgacaacagc ctgagaaatc 1200

cccccgtaac ttattatcac tttggggttc ggcctgtgtc cccgaacgc tctgcacctt 1260

ctgacgcagc ctgagaatga cctgccctgg ccccagccct actctgtgta atagaataaa 1320

ggcctgcgtg tgtctgtgtt gagcgtgcgt ctgtgtgtgc ctgtgtgcga gtctgagtca 1380

gagatttgga gatgtctctg tgtgtttgtg tgtatctgtg ggtctccatc ctccatgggg 1440

gctcagccag gtgctgtgac accccccttc tgaatgaagc cttctgacct gggctggcac 1500

tgctgggggt gaggacacat tgccccatga gacagtccca gaacacggca gctgctggct 1560

gtgacaatgg tttcaccatc cttagaccaa gggatgggac ctgatgacct gggaggactc 1620

tcttagttct taccttttgt ggttctcaat aaaacagaac ttaaaaaatt g 1671

<210> 28
<211> 1758
<212> DNA
<213> Homo sapiens

<400> 28
acagcttcct gccgcaggcg ggcgggaggg cgggcacgga gaggcgggcg ccgaggaggg 60
gcaggtaggg ctgggacgca ggggtaactg gatcccccga cttcagccca ggccctggtc 120
tgaccaccct gggagcaggg actttccaca gtcagctgga cgcacactca gcccagtaaa 180
agaggggacc catcccggga gccccgggga gggcacagct gcctcctccc gggctcccct 240
gccacctggt gcctacctgc cccctgctcc ctgccgggtc cggtcctcac cccatcttca 300
tctggccttg actctgccct tgaggggcct aggggtgcag ccagcctgct ccgagctccc 360
ctgcagatgg aggaggccat cctggtcccc tgcgtgctgg ggctcctgct gctgcccatc 420
ctggccatgt tgatggcact gtgtgtgcac tgccacagac tgccaggctc ctacgacagc 480
acatcctcag atagtttgta tccaaggggc atccagttca aacggcctca cacggttgcc 540
ccctggccac ctgcctaccc acctgtcacc tcctacccac ccctgagcca gccagacctg 600
ctccccatcc caagatcccc gcagcccctt gggggctccc accggacgcc atcttcccgg 660
cgggattctg atggtgccaa cagtgtggcg agctacgaga acgagggtgc gtctgggatc 720
cgaggtgccc aggctgggtg gggagtctgg ggtccgtcct ggactaggct gacccctgtg 780
tcgttacccc cagaaccagc ctgtgaggat gcggatgagg atgaggacga ctatcacaac 840
ccaggctacc tggtggtgct tcctgacagc accccggcca ctagcactgc tgccccatca 900
gctcctgcac tcagcacccc tggcatccga gacagtgcct tctccatgga gtccattgat 960
gattacgtga acgttccgga gagcggggag agcgcagaag cgtctctgga tggcagccgg 1020
gagtatgtga atgtgtccca ggaactgcat cctggagcgg ctaagactga gcctgccgcc 1080
ctgagttccc aggaggcaga ggaagtggag gaagaggggg ctccagatta cgagaatctg 1140
caggagctga actgagggcc tgtggaggcc gagtctgtcc tggaaccagg cttgcctggg 1200
acggctgagc tgggcagctg gaagtggctc tggggtcctc acatggcgtc ctgcccttgc 1260
tccagcctga caacagcctg agaaatcccc ccgtaactta ttatcacttt ggggttcggc 1320
ctgtgtcccc cgaacgctct gcaccttctg acgcagcctg agaatgacct gccctggccc 1380
cagccctact ctgtgtaata gaataaaggc ctgcgtgtgt ctgtgttgag cgtgcgtctg 1440
tgtgtgcctg tgtgcgagtc tgagtcagag atttggagat gtctctgtgt gtttgtgtgt 1500
atctgtgggt ctccatcctc catgggggct cagccaggtg ctgtgacacc ccccttctga 1560
atgaagcctt ctgacctggg ctggcactgc tgggggtgag gacacattgc cccatgagac 1620
agtcccagaa cacggcagct gctggctgtg acaatggttt caccatcctt agaccaaggg 1680
atgggacctg atgacctggg aggactctct tagttcttac cttttgtggt tctcaataaa 1740
acagaactta aaaaattg 1758

<210> 29
<211> 233
<212> PRT
<213> Homo sapiens

<400> 29

```
Met Glu Glu Ala Ile Leu Val Pro Cys Val Leu Gly Leu Leu Leu Leu
1               5                   10                  15

Pro Ile Leu Ala Met Leu Met Ala Leu Cys Val His Cys His Arg Leu
            20                  25                  30

Pro Gly Ser Tyr Asp Ser Thr Ser Ser Asp Ser Leu Tyr Pro Arg Gly
        35                  40                  45

Ile Gln Phe Lys Arg Pro His Thr Val Ala Pro Trp Pro Pro Ala Tyr
    50                  55                  60

Pro Pro Val Thr Ser Tyr Pro Pro Leu Ser Gln Pro Asp Leu Leu Pro
65                  70                  75                  80

Ile Pro Arg Ser Pro Gln Pro Leu Gly Gly Ser His Arg Thr Pro Ser
                85                  90                  95

Ser Arg Arg Asp Ser Asp Gly Ala Asn Ser Val Ala Ser Tyr Glu Asn
            100                 105                 110

Glu Glu Pro Ala Cys Glu Asp Ala Asp Glu Asp Glu Asp Asp Tyr His
        115                 120                 125

Asn Pro Gly Tyr Leu Val Val Leu Pro Asp Ser Thr Pro Ala Thr Ser
    130                 135                 140

Thr Ala Ala Pro Ser Ala Pro Ala Leu Ser Thr Pro Gly Ile Arg Asp
145                 150                 155                 160

Ser Ala Phe Ser Met Glu Ser Ile Asp Asp Tyr Val Asn Val Pro Glu
                165                 170                 175

Ser Gly Glu Ser Ala Glu Ala Ser Leu Asp Gly Ser Arg Glu Tyr Val
            180                 185                 190

Asn Val Ser Gln Glu Leu His Pro Gly Ala Ala Lys Thr Glu Pro Ala
        195                 200                 205

Ala Leu Ser Ser Gln Glu Ala Glu Glu Val Glu Glu Glu Gly Ala Pro
    210                 215                 220
```

Asp Tyr Glu Asn Leu Gln Glu Leu Asn
225 230

<210> 30
<211> 262
<212> PRT
<213> Homo sapiens

<400> 30

Met Glu Glu Ala Ile Leu Val Pro Cys Val Leu Gly Leu Leu Leu Leu
1 5 10 15

Pro Ile Leu Ala Met Leu Met Ala Leu Cys Val His Cys His Arg Leu
20 25 30

Pro Gly Ser Tyr Asp Ser Thr Ser Ser Asp Ser Leu Tyr Pro Arg Gly
35 40 45

Ile Gln Phe Lys Arg Pro His Thr Val Ala Pro Trp Pro Pro Ala Tyr
50 55 60

Pro Pro Val Thr Ser Tyr Pro Pro Leu Ser Gln Pro Asp Leu Leu Pro
65 70 75 80

Ile Pro Arg Ser Pro Gln Pro Leu Gly Gly Ser His Arg Thr Pro Ser
85 90 95

Ser Arg Arg Asp Ser Asp Gly Ala Asn Ser Val Ala Ser Tyr Glu Asn
100 105 110

Glu Gly Ala Ser Gly Ile Arg Gly Ala Gln Ala Gly Trp Gly Val Trp
115 120 125

Gly Pro Ser Trp Thr Arg Leu Thr Pro Val Ser Leu Pro Pro Glu Pro
130 135 140

Ala Cys Glu Asp Ala Asp Glu Asp Glu Asp Asp Tyr His Asn Pro Gly
145 150 155 160

Tyr Leu Val Val Leu Pro Asp Ser Thr Pro Ala Thr Ser Thr Ala Ala
165 170 175

Pro Ser Ala Pro Ala Leu Ser Thr Pro Gly Ile Arg Asp Ser Ala Phe
180 185 190

Ser Met Glu Ser Ile Asp Asp Tyr Val Asn Val Pro Glu Ser Gly Glu
195 200 205

Ser Ala Glu Ala Ser Leu Asp Gly Ser Arg Glu Tyr Val Asn Val Ser
    210                 215                 220

Gln Glu Leu His Pro Gly Ala Ala Lys Thr Glu Pro Ala Ala Leu Ser
225                 230                 235                 240

Ser Gln Glu Ala Glu Glu Val Glu Glu Glu Gly Ala Pro Asp Tyr Glu
                245                 250                 255

Asn Leu Gln Glu Leu Asn
            260

<210> 31
<211> 2091
<212> DNA
<213> Homo sapiens

<400> 31
gagacaggtg gtggctacga cggcgaaggg agctgagact gtccaggcag ccaggttagg 60

ccaggaggac catgtgaatg gggccagagg gctcccgggc tgggcaggga ccatgggctg 120

tggctgcagc tcacacccgg aagatgactg gatggaaaac atcgatgtgt gtgagaactg 180

ccattatccc atagtcccac tggatggcaa gggcacgctg ctcatccgaa atggctctga 240

ggtgcgggac ccactggtta cctacgaagg ctccaatccg ccggcttccc cactgcaaga 300

caacctggtt atcgctctgc acagctatga gccctctcac gacggagatc tgggctttga 360

gaagggggaa cagctccgca tcctggagca gagcggcgag tggtggaagg cgcagtccct 420

gaccacgggc caggaaggct tcatcccctt caattttgtg gccaaagcga acagcctgga 480

gcccgaaccc tggttcttca agaacctgag ccgcaaggac gcggagcggc agctcctggc 540

gcccgggaac actcacggct ccttcctcat ccgggagagc gagagcaccg cgggatcgtt 600

ttcactgtcg gtccgggact tcgaccagaa ccagggagag gtggtgaaac attacaagat 660

ccgtaatctg gacaacggtg gcttctacat ctcccctcga atcacttttc ccggcctgca 720

tgaactggtc cgccattaca ccaatgcttc agatgggctg tgcacacggt tgagccgccc 780

ctgccagacc cagaagcccc agaagccgtg gtgggaggac gagtgggagg ttcccaggga 840

gacgctgaag ctggtggagc ggctgggggc tggacagttc ggggaggtgt ggatggggta 900

ctacaacggg cacacgaagg tggcggtgaa gagcctgaag cagggcagca tgtccccgga 960

cgccttcctg gccgaggcca acctcatgaa gcagctgcaa caccagcggc tggttcggct 1020

ctacgctgtg gtcacccagg agcccatcta catcatcact gaatacatgg agaatgggag 1080

tctagtggat ttttctcaaga ccccttcagg catcaagttg accatcaaca aactcctgga 1140

catggcagcc caaattgcag aaggcatggc attcattgaa gagcggaatt atattcatcg 1200

tgaccttcgg gctgccaaca ttctggtgtc tgacaccctg agctgcaaga ttgcagactt 1260

tggcctagca cgcctcattg aggacaacga gtacacagcc agggaggggg ccaagtttcc 1320

cattaagtgg acagcgccag aagccattaa ctacgggaca ttcaccatca agtcagatgt 1380

gtggtctttt gggatcctgc tgacggaaat tgtcacccac ggccgcatcc cttacccagg 1440

gatgaccaac ccggaggtga ttcagaacct ggagcgaggc taccgcatgg tgcgccctga 1500

caactgtcca gaggagctgt accaactcat gaggctgtgc tggaaggagc gcccagagga 1560

ccggcccacc tttgactacc tgcgcagtgt gctggaggac ttcttcacgg ccacagaggg 1620

ccagtaccag cctcagcctt gagaggcctt gagaggccct ggggttctcc ccctttctct 1680

ccagcctgac ttggggagat ggagttcttg tgccatagtc acatggccta tgcacatatg 1740

gactctgcac atgaatccca cccacatgtg acacatatgc accttgtgtc tgtacacgtg 1800

tcctgtagtt gcgtggactc tgcacatgtc ttgtacatgt gtagcctgtg catgtatgtc 1860

ttggacactg tacaaggtac ccctttctgg ctctcccatt tcctgagacc acagagagag 1920

gggagaagcc tgggattgac agaagcttct gcccacctac ttttctttcc tcagatcatc 1980

cagaagttcc tcaagggcca ggactttatc taatacctct gtgtgctcct ccttggtgcc 2040

tggcctggca cacatcagga gttcaataaa tgtctgttga tgactgttgt a 2091

<210> 32
<211> 509
<212> PRT
<213> Homo sapiens

<400> 32

Met Gly Cys Gly Cys Ser Ser His Pro Glu Asp Asp Trp Met Glu Asn
1 5 10 15

Ile Asp Val Cys Glu Asn Cys His Tyr Pro Ile Val Pro Leu Asp Gly
20 25 30

Lys Gly Thr Leu Leu Ile Arg Asn Gly Ser Glu Val Arg Asp Pro Leu
35 40 45

Val Thr Tyr Glu Gly Ser Asn Pro Pro Ala Ser Pro Leu Gln Asp Asn
50 55 60

Leu Val Ile Ala Leu His Ser Tyr Glu Pro Ser His Asp Gly Asp Leu
65 70 75 80

Gly Phe Glu Lys Gly Glu Gln Leu Arg Ile Leu Glu Gln Ser Gly Glu
85 90 95

Trp Trp Lys Ala Gln Ser Leu Thr Thr Gly Gln Glu Gly Phe Ile Pro
100 105 110

Phe Asn Phe Val Ala Lys Ala Asn Ser Leu Glu Pro Glu Pro Trp Phe
115 120 125

Phe Lys Asn Leu Ser Arg Lys Asp Ala Glu Arg Gln Leu Leu Ala Pro
130 135 140

Gly Asn Thr His Gly Ser Phe Leu Ile Arg Glu Ser Glu Ser Thr Ala
145 150 155 160

Gly Ser Phe Ser Leu Ser Val Arg Asp Phe Asp Gln Asn Gln Gly Glu
165 170 175

Val Val Lys His Tyr Lys Ile Arg Asn Leu Asp Asn Gly Gly Phe Tyr
180 185 190

Ile Ser Pro Arg Ile Thr Phe Pro Gly Leu His Glu Leu Val Arg His
195 200 205

Tyr Thr Asn Ala Ser Asp Gly Leu Cys Thr Arg Leu Ser Arg Pro Cys
210 215 220

Gln Thr Gln Lys Pro Gln Lys Pro Trp Trp Glu Asp Glu Trp Glu Val
225 230 235 240

Pro Arg Glu Thr Leu Lys Leu Val Glu Arg Leu Gly Ala Gly Gln Phe
245 250 255

Gly Glu Val Trp Met Gly Tyr Tyr Asn Gly His Thr Lys Val Ala Val
260 265 270

Lys Ser Leu Lys Gln Gly Ser Met Ser Pro Asp Ala Phe Leu Ala Glu
275 280 285

Ala Asn Leu Met Lys Gln Leu Gln His Gln Arg Leu Val Arg Leu Tyr
290 295 300

Ala Val Val Thr Gln Glu Pro Ile Tyr Ile Ile Thr Glu Tyr Met Glu
305 310 315 320

Asn Gly Ser Leu Val Asp Phe Leu Lys Thr Pro Ser Gly Ile Lys Leu
325 330 335

Thr Ile Asn Lys Leu Leu Asp Met Ala Ala Gln Ile Ala Glu Gly Met
340 345 350

Ala Phe Ile Glu Glu Arg Asn Tyr Ile His Arg Asp Leu Arg Ala Ala

355 360 365

Asn Ile Leu Val Ser Asp Thr Leu Ser Cys Lys Ile Ala Asp Phe Gly
370 375 380

Leu Ala Arg Leu Ile Glu Asp Asn Glu Tyr Thr Ala Arg Glu Gly Ala
385 390 395 400

Lys Phe Pro Ile Lys Trp Thr Ala Pro Glu Ala Ile Asn Tyr Gly Thr
405 410 415

Phe Thr Ile Lys Ser Asp Val Trp Ser Phe Gly Ile Leu Leu Thr Glu
420 425 430

Ile Val Thr His Gly Arg Ile Pro Tyr Pro Gly Met Thr Asn Pro Glu
435 440 445

Val Ile Gln Asn Leu Glu Arg Gly Tyr Arg Met Val Arg Pro Asp Asn
450 455 460

Cys Pro Glu Glu Leu Tyr Gln Leu Met Arg Leu Cys Trp Lys Glu Arg
465 470 475 480

Pro Glu Asp Arg Pro Thr Phe Asp Tyr Leu Arg Ser Val Leu Glu Asp
485 490 495

Phe Phe Thr Ala Thr Glu Gly Gln Tyr Gln Pro Gln Pro
500 505

<210> 33
<211> 10744
<212> DNA
<213> Homo sapiens

<400> 33
attctccgcg gcgcggcggc gagtgctggc tgcggtaccc tcccgctcgc ttgtccgtct 60

gcgcgcccgc gatgtgacca gccgactggg ggcgggctgg cggctctgcc tgcggcagcg 120

catcggtttt tctcctggca ggtccctaat tgagccgtga ttcccttgga gccagagaca 180

cccaccaggg gctcggagcg ccgcgctccg gggctggagg cagctgcagc gctcgggtcc 240

gcgcggccgg gtgcagtagc ctcagtccca gccgccgcct cgctgagtcg tgtgccctgg 300

cgaatgcccg gcgcccggca cagctgagcc aaggcgactg gtgcagagcg gcggcgcctg 360

gtcctgcact tgccgctgct gccgggctgt gccggggcgg cggcgctgcg cgccggagtt 420

tgcagaggct gcctccgagc gccgtccgcc aggaagactc cctgcctcct gagtcccaaa 480

aacacgagat tttctcccta tttcagcagt tggccacaac ttcattgctg ggaaaaggcc 540

aagaaagaag agagaactct caccacaaca tttcaaaggg aatacattgc cagaacttgg 600
aatactctac tggacaaaca tttcctccaa ggacacagct ctctgcctcc atgtcaccac 660
ctttgaagga ctgactgatt ccctcggctg gtgccagtgc ctgctcctgc catggggccc 720
gcggggagcc tgctgggcag cggacagatg cagatcaccc tgtggggaag tctggctgct 780
gtcgccattt tcttcgtcat caccttcctc atcttcctgt gctctagttg tgacagggaa 840
aagaagccgc gacagcatag tggggaccat gagaacctga tgaacgtgcc ttcagacaag 900
gagatgttca gccgttcagt tactagcctg gcaacagatg ctcctgccag cagtgagcag 960
aatggggcac tcaccaatgg ggacattctt tcagaggaca gtactctgac ctgcatgcag 1020
cattacgagg aagtccagac atcggcctcg gatctgctgg attcccagga cagcacaggg 1080
aaaccaaaat gtcatcagag tcgggagctg cccagaatcc ctcccgagag cgcagtggat 1140
accatgctca cggcgagaag tgtggacggg gaccaggggc tggggatgga agggccctat 1200
gaagtgctca aggacagctc ctcccaagaa aacatggtgg aggactgctt gtatgaaact 1260
gtgaaagaga tcaaggaggt ggctgcagct gcacacctgg agaaaggcca cagtggcaag 1320
gcaaaatcta cttctgcctc gaaagagctc ccagggcccc agactgaagg caaagctgag 1380
tttgctgaat atgcctcggt ggacagaaac aaaaaatgtc gtcaaagtgt taatgtagag 1440
agtatccttg gaaattcatg tgatccagaa gaggaggccc caccacctgt ccctgttaag 1500
cttctggacg agaatgaaaa ccttcaggag aaggaagggg gagaggcgga agagagtgcc 1560
acagacacga ccagtgaaac taacaagaga tttagctcat tgtcatacaa gtctcgggaa 1620
gaagacccca ctctcacaga agaagagatc tcagctatgt actcatcagt aaataaacct 1680
ggacagttag tgaataaatc ggggcagtcg cttacagttc cggagtccac ctacacctcc 1740
attcaagggg acccacagag gtcaccctcc tcctgtaatg atctctatgc tactgttaaa 1800
gacttcgaaa aaactccaaa cagcacactt ccaccagcag ggaggcccag cgaggagcca 1860
gagcctgatt atgaagcgat acagactctc aacagagagg aagaaaaggc caccctgggg 1920
accaatggcc accacggtct cgtcccaaag gagaacgact acgagagcat aagtgacttg 1980
cagcaaggca gagatattac caggctctag caacccagaa gacaaccctg ggtagcctgt 2040
gatcagtgtc tggagacgtt tcttctgtgg aagagaagaa gtgacacaaa cctatacttc 2100
atatgctgct ttagtcacct gaagatggtt ggagaggccc tgtcgactgt tctcccagtt 2160
gttcagtttc tgagacagag aggtacggac taggctgcac ctgagtgtgc ccctgcctgc 2220
cagatggaca ggtacaccca agcacatctc cctgctgcac cctcaccacc cacaaaagat 2280
cccagctgtc agtggtctca tctcattagt gaggaaagcc aagctgtatg gaaaagctgc 2340
actcaccaag gaccacaatg cccccggcct aaagtactgc cattcagaaa agcaggtttt 2400
tcttcctctc tttccttttc tctgtctgct actgacttta aggctttttc cccttgaaa 2460

tgtccagatt cctgtggttc atcccaagga aattttcaca caaagcttgg cctttgccct 2520
caatataggt gttttaggat ggtgacaaac catggctgct gctttctgcc cagctcgcca 2580
gtcctcccca aagagttgcg catcagcacc tggggatctg gaccctgcgg gtgaagggat 2640
ggggagggac gtccctggag tctcttctgt ctttgttcct tcttattttg gcattcgata 2700
tcagcagcct ctccccaaag tacttgaagt cagttttaga tgctttattt tatttttcta 2760
gtcaaaaacg tgtttccccc agtgtttgaa aactcgtccg aatcttttca gtattttcca 2820
tgagtattgt ggtacttcta gacttgttta agcccagaac tcattccttc aaaacagaga 2880
gccttaatct ttatgttggg acacagacca catatttgga cggcagccat gcatccatcg 2940
ctgaagggct gtggacatga atgtgtattt cccatggtct ccgctgccca caccaacagt 3000
gtggcatctc ataagttaac tgctacccta aggtaatcta agattaaaat gtaaacattt 3060
atttttgtta tgtaagttta taagatgttt tatgttcaat gcctaatttc tcaaaagtgc 3120
cagaaaaaaa tgtatattag ctattttgat tttatgtaca atgatttata ctctcctttt 3180
gaaaagatac cataaagcac ataagctaga tcactacaag gagctgttat ctttttttcta 3240
atcaagtgtt taaaacactg atggttttta aagactcacc tttttaaatg gtacttggag 3300
ctcctgattc aaattaccta gaccccctag agaaataaat ggaatataca taaataatca 3360
ttttcagtgg tttatggtgg gcaatattgc aatatttgaa atggtaaaaa tggaaagaag 3420
aacaaaatat gatgagaggt ggctgtgaat tataaacctc ataaaagtgt cataattcca 3480
ttaaggttta attatatttt ttcagaaaac agtgatgaat tctgtagtcc agtgcttgcc 3540
aatgcaaatt gcctattgga atcttcttcc tatattttac aaacatcagt ggctgaaata 3600
gctcagagta agagctcagc ctggtttgaa tttaatcatc tctttagatc ttataaggcc 3660
agcattagga aacttgttca cttttcattt tcaaaggagc ctagttgaag tgctattatg 3720
agtgtgggct atggaaagac agcttttcct acactgataa agaaaaaaaa tgaggaaatt 3780
atttcatccc cttgtgacat ctgtgacttt ttggatttaa taatcttgct gtttttcctc 3840
tttatgacaa agaatataat tgggaggatg aagtgtctta aaaattgtag agaccagctc 3900
actggaatgt ttttccatcc ctgtattcat ggcttgactt tgtgactgct ctacactgca 3960
tgtctgacat tgcagagtga gctatgttga ggtaaactgg ttggttgtca ttattttgca 4020
atcagcctgg tctctcccat gaagatgtcg tgtgcataag cacaatcatc actgattaga 4080
agatcacagc agaataccct tggattagag agaagttcgt accttgcatt tctctgaatt 4140
ctagtctctc ataagcactg ctttgctgga tgattttcac tgctttgtgt taatgacttt 4200
gagcgatctc tcacatgatg gggttcttta gtacatggta acagccatgt catcttacac 4260
acctagcatt gtgaatgctg tagtgacatc ctttataggc accttacagc tcaaaacttt 4320

tgtttcattt catgccttac ttatcaaaaa ggcaggaaag taggtatgat ctctaaagta 4380
aaaaaaaaaa aaaaaaaaaa actttttata gaaagctcat aaataatcat gtcattttgc 4440
aattttgtta ccaaaatttc ccccaagagt tttcaaatat tagttctgca atgtggctat 4500
gaaatatgca ctgaaatata ccttttaatt tgagaaccag tggttagaat aagctgtgat 4560
ataaagtatt ttcagtgtac ttttaaagga actataaggc cctccagcat aaacgctaaa 4620
agaatagatg gtagcacagg ccatgagggc tgggggagag aagcagagtg aaccttagaa 4680
agatggctca gctatttgga gcactggata ttttactgaa gttatttact gaggcaccat 4740
cactgttttg actgtacagt atagtttttc ataaatttca tcacatttac tttgttcaga 4800
atctgggctt gaatctttga gttggacaaa agcctatggt ttcttttaaa aagtttcatc 4860
ttgagctaat gctacagttt aaataaaatg tatgaaaggt agttatcaaa aacaatttgt 4920
atatttaaaa ttctattttg acatccattt tttacagcga attatagcag gttatgtgaa 4980
atttaaaaat aaattttagg aaattttttt atcttgtaag taagaattat aaatctatct 5040
cagttaaaag taggatcttt gtttttattc agatattttg aaagtttaga agcattttgt 5100
atgctacttt ggtatttctt aagtatagtc agcaggaaag ggtaacctgt tttatgcaaa 5160
atttttttaa tttgtcaaat gtttttagtg tgtctacttt ctgaaatagt ctcaatatcc 5220
tgtctgtatc ctaggcagat aactacacaa gaacaaaacg ctatatagaa aagaaaatat 5280
ttgaggaaat cttaaattcc ataagaaagt aattcttgca agaatgttgg ctacatattt 5340
ttttttctgt cctgaataaa ttacatcatt aggtctgttg aagcttttga agctaccact 5400
attctttctg ggataaaggc taattactga tttatctgcc ctcaaaatcc cagagttaat 5460
tctaattgag tactaaatta acagtaagta gttaacacag agaactaaaa tttaaccgca 5520
ttatatcact gtatatattt ctcatgattt ttgctaatta gagcatttac ttaaccagta 5580
ataaactaca cacacacaca cacacacaca cacgtatacg tgtgtgtgtg tatatacttg 5640
gctctgacac aactctggtg cttaattagg atatgttcta ttatgtattt tgaatgttta 5700
tcctgctagt gtgatgaact tttgttggaa aagcagaatt agaaggaaca gtttttcaat 5760
cagtttcatt tggtaattac aggaaaacct tgagttgctc taatttaatt tttccaatta 5820
atacatttag actttaaatt gcatccgtaa ttctcttggc tgaaaaacag tggtatttta 5880
agacagtttt gttatacatg tgacttactt ttgactcagc ctgagaggag gaaatgtaaa 5940
gagataagag atttggccca aagcagcaag agactagtaa ccttgagcca ggacgaagcc 6000
aagacaacgt gactcccaag tagcaggagc agaggtcggt cacctgtggc attctgtttc 6060
attccctttt aaaattatga gactttatag gcagtgtaaa acatcagcag gcagcgatgt 6120
gataggatgt gcgaaaaagc tggtctgata ccatggctat aattacagag ctttagttca 6180
attaggattt tgtaaatgag caaatgacct ttttttccag tgccccttgt aatagttaat 6240

atgagtccat gcaatcttgt gatgccattc tcctgaaggt gttgatttct ggtacccagc 6300

cagcttagct ttggctgctg gaagcacagt aggtactgat gattacttcc tggaaatctt 6360

gacaggctta ttgtactgtg taatggggaa aagttaaagt ataatgtttg gtgttaataa 6420

atgactgatg tgaaaatagg aacatgtgtc tttaatatca aatatggctt tatttgcagt 6480

gaaatctaaa ttccttattc tacagtagta ttttcttgcc ctgtgttgta tattttccta 6540

aatacatttt tcctgacagt ggctttaggc cagttgaaca cacttagact gaaagtgttt 6600

aacttaaaga gatcagtcca gaaaccatga taaattagaa tctttatctg gaattaccaa 6660

attaaaattt aaaatcatgg tccagaagag aacaaacatt catggttttt tttatcctac 6720

tgctcatttt taggtctgtg tttacatcta gtctctacta tttgtgaagt atgtcattat 6780

ttttttcaag ttcttctttg acttaactga aaaataatat tccaaattct taatgtaaca 6840

tgaaaagaga aatacaattt ttgcttaaaa gacatttttt aaaaagcgtt caattcagta 6900

atcatttctg ttaatacagg aagttttttt ggcttgccag ttatatactg tgggtatttt 6960

ttaaaatgtg ctacccttgg gttgtctcat atcaccttgc gtaatcatgt attacaaagg 7020

ttgataattg tcatttctca gatgtctgta tgttacattg gcagcagtaa aatgttttaa 7080

tgttgttacc ttttaaataa ttgtgttgta ttaacatgcc tcatattttc tggggaaact 7140

tgaaatatat ctaaacaaaa aaggtctgtt ataaatagga attggcattt cattgttaat 7200

gtttttttcct cataaaaata gttacaccaa aagtgacata ttgtctatta catgggccca 7260

atcagtatta aagtactccc cttactcaaa aatattaaaa aataattcat gacacttagt 7320

agcattttca ttgattgttt caaaagatct tcataatggt caaattgtcc aattcacaaa 7380

agagtgagaa agttgttttc agtactggga atttttaaac cttagtttta agaccaaaaa 7440

tatctcttat tagcttgaac attttgtgat tacttttccc tccccggatc tagtcctttt 7500

aaggagtaag tctaaagaat gaggccatga agtcactatt tcctcccacc tcagtttgtc 7560

tcctggtact tagctggcct atcgctttgt gtggcaatac ccctgggtcg cttccccact 7620

cagccttctg tatctgctgt tcacaggaca tgccattatg tctttccagt acggatcaca 7680

gtgctgccat atcacagacc ctgccagccg gcacagaacc atgcctcgtc acagctctgc 7740

ccaactcaca ggctgccaag atgaggccac aggtccacct agggcggcag atgctggtgc 7800

tatttgtcac gacttttgcc aaaacatgta cactgttgct cacatctttc gtaagaatgg 7860

ttgacactaa ggggccatgg aaaagcactt ttaaaaaatt atgtggtgga atcaaactca 7920

cagaggcaga aatgttagtg ccgtgctcta accaagtaag cttagtgttc cccacagtga 7980

tctgtatctg gcctgagtct cctcaagcag caaccccccac tgtccagcat gtggaaggta 8040

gatccttatg acaacaccag acccatagtt accaggaaag aaaaaggaag ctgagtgttg 8100

tgaaggggaa gaaatccttt ttataagaag taatcatttt taggccgggt gcagtggctc 8160
atgcctgtaa tcccagcact ttgggaggct gagacaggtg gatcacttga ggtcaggagt 8220
ttgagacctg cctggccaac atgatgaaac cctgtctcta ctaaaaatgc aaaaaaaatt 8280
agccaggcat ggtggcatgt gcctgtagtc ccaactactc aggaggctga ggcaggagaa 8340
tcacttgaac ctgggaggca gaggttgcaa tgagctgaga tcgcaacact gcactccagc 8400
ctgggcgaca gagcaagact ccatctcaaa aacaaaaaca aaaaaaaaac ctttttaaag 8460
taaattgagt gtaaaatgtt ttcaccatga agccattgca ccctctcccc caggaaacag 8520
tgcctaagga tgatgtcaag ttacagccag tttgctctga tcccccagga cctaagaaaa 8580
ctgtgagtgc ttcagcatgc aaaggtagag ctaccagata aggagaataa cttggccatt 8640
tatagcaaac tgcctgactt tggagtgaga aaccaagcac cttctaggtc ctaggatagg 8700
tgaattggag gtgcagctgc tgaagttccc attttctaca tacttcagga gccagggtgt 8760
ttcatttggt tttgtaaaac tttgctgtaa gtcatcacag ttaattcttt gaatggacat 8820
tttagagtgt aacttttttt caaaattagc agaacagata tctctgctca ttctctttat 8880
acatttagtt ttttaaagtt cccctagtat acctgttagg cactctaaat aggacacagt 8940
taaagctaaa agaaggacca gttgctccct agagcctaca gttcagaact atcttgttgt 9000
catgtcctgg tttggtttgg tttgttttta actcaacaag ttggaaacca tgagtttaaa 9060
gcaaactatt tccttttctt aagcttaaag agaatttttt ttttaatctt tagctcctca 9120
tttaggtagt gaaggcttcc ctgaaagaca gcagtgccct tctctggaag aagaggctgg 9180
gaccaaaagc ctgagtctat gggttgagga ttttgagcat tcctttgaca agcttctatg 9240
tatttttaat ataagactca taaatgaacc tgatgtggtg ttcatgaccc atcccttaag 9300
catgcttgct ttttttaact ttactttttg agtatagtac ttgtcttgtt tttcctggag 9360
ccatgctagg atttaattcc tatgtgccac tacccgccca tgtcctggaa gatggccatg 9420
cctgcagcaa tgctcatgtc ttgaggagaa gcaatgtgaa aagcatcagg aaagctgaac 9480
cttacgttca agtcagcaaa catttgtgag cattctagta tgcttgcgac cttgacactt 9540
tttaaaaatt atctttttat ttgcttcgag tatcaaccgt atagttgact ctagtggggc 9600
gaagggagca gagatgagag caaaattcag acttgctgcc cctgtgtgtc tcagttctca 9660
tgtctctctg agaaatgtgt tggcccaatt ccctatctgc cactctgagt ctcttgagta 9720
aatgtgtgta aatgagaaaa attatctcaa agatttaact tattatttag atattttctt 9780
ctttttaaac tcaggaataa acctggtttg tagataatat aatatggcta ctagagctat 9840
aattgagtag gtgactagaa gcatttttttc aaatatattt cttgtaaaga catctacttt 9900
gttttaacta ggaaaagaaa ctcctcatat taaaataaac atttccataa gcactttcta 9960
gaagagtgta gcatccatct accatccatc ccagaatgtt tgtatcttgc tcatagtatt 10020

tcaaaaattt ttatgggtga aaaaaatgca ctttttttatt tattggaaaa gtgtagagtt 10080

attgaattaa attctcaagt gggacaattt agtgtaaagg gaaacatggg ttttggagac 10140

aagagcacag ggctgtggtc cctgttccag cattgacttt actatgtgac ctcgagcaaa 10200

tgatttaact tctctgtgcc tcagtttctc catatacaaa atggggataa tgatactaac 10260

cattgcctac ctcatagaga tgttatgggg acaaacgaaa taatagagat aagcacgaat 10320

gctttcaact cttcggaaga aaggtgctat ataaattgaa gttgtgtttt taatgatcca 10380

attattaatt atgtttaggg tttaaataac aatactgtta gtcatgttaa gaataagttt 10440

tgttttgatg catatttcca cttctcttct gtggccagat cttgatcatt caaccagtaa 10500

tggtacctga ggaactgaaa tgggtatttg ttttcgtatg tttctgccag tagtatttca 10560

attgaatatc cagaaaagac tggagtttaa cttgtaatat cttatagttt acatgtaata 10620

aaacttattc aagctatata taaaagtatg attacatgta aatagcaggt atgttgtaat 10680

taaaggcact tatcaaattg tctttgttct tttttaattg taataaaagt cagttttaca 10740

taaa 10744

<210> 34
<211> 432
<212> PRT
<213> Homo sapiens

<400> 34

Met Gly Pro Ala Gly Ser Leu Leu Gly Ser Gly Gln Met Gln Ile Thr
1 5 10 15

Leu Trp Gly Ser Leu Ala Ala Val Ala Ile Phe Phe Val Ile Thr Phe
20 25 30

Leu Ile Phe Leu Cys Ser Ser Cys Asp Arg Glu Lys Lys Pro Arg Gln
35 40 45

His Ser Gly Asp His Glu Asn Leu Met Asn Val Pro Ser Asp Lys Glu
50 55 60

Met Phe Ser Arg Ser Val Thr Ser Leu Ala Thr Asp Ala Pro Ala Ser
65 70 75 80

Ser Glu Gln Asn Gly Ala Leu Thr Asn Gly Asp Ile Leu Ser Glu Asp
85 90 95

Ser Thr Leu Thr Cys Met Gln His Tyr Glu Glu Val Gln Thr Ser Ala
100 105 110

Ser Asp Leu Leu Asp Ser Gln Asp Ser Thr Gly Lys Pro Lys Cys His
115 120 125

Gln Ser Arg Glu Leu Pro Arg Ile Pro Pro Glu Ser Ala Val Asp Thr
130 135 140

Met Leu Thr Ala Arg Ser Val Asp Gly Asp Gln Gly Leu Gly Met Glu
145 150 155 160

Gly Pro Tyr Glu Val Leu Lys Asp Ser Ser Ser Gln Glu Asn Met Val
165 170 175

Glu Asp Cys Leu Tyr Glu Thr Val Lys Glu Ile Lys Glu Val Ala Ala
180 185 190

Ala Ala His Leu Glu Lys Gly His Ser Gly Lys Ala Lys Ser Thr Ser
195 200 205

Ala Ser Lys Glu Leu Pro Gly Pro Gln Thr Glu Gly Lys Ala Glu Phe
210 215 220

Ala Glu Tyr Ala Ser Val Asp Arg Asn Lys Lys Cys Arg Gln Ser Val
225 230 235 240

Asn Val Glu Ser Ile Leu Gly Asn Ser Cys Asp Pro Glu Glu Glu Ala
245 250 255

Pro Pro Pro Val Pro Val Lys Leu Leu Asp Glu Asn Glu Asn Leu Gln
260 265 270

Glu Lys Glu Gly Gly Glu Ala Glu Glu Ser Ala Thr Asp Thr Thr Ser
275 280 285

Glu Thr Asn Lys Arg Phe Ser Ser Leu Ser Tyr Lys Ser Arg Glu Glu
290 295 300

Asp Pro Thr Leu Thr Glu Glu Glu Ile Ser Ala Met Tyr Ser Ser Val
305 310 315 320

Asn Lys Pro Gly Gln Leu Val Asn Lys Ser Gly Gln Ser Leu Thr Val
325 330 335

Pro Glu Ser Thr Tyr Thr Ser Ile Gln Gly Asp Pro Gln Arg Ser Pro
340 345 350

Ser Ser Cys Asn Asp Leu Tyr Ala Thr Val Lys Asp Phe Glu Lys Thr
355 360 365

```
Pro Asn Ser Thr Leu Pro Pro Ala Gly Arg Pro Ser Glu Glu Pro Glu
    370                 375                 380

Pro Asp Tyr Glu Ala Ile Gln Thr Leu Asn Arg Glu Glu Glu Lys Ala
385                 390                 395                 400

Thr Leu Gly Thr Asn Gly His His Gly Leu Val Pro Lys Glu Asn Asp
                405                 410                 415

Tyr Glu Ser Ile Ser Asp Leu Gln Gln Gly Arg Asp Ile Thr Arg Leu
            420                 425                 430


<210>  35
<211>  8240
<212>  DNA
<213>  Homo sapiens

<400>  35
cccctctcgg tagccctgag gctctggcgc cttcaagtga gaagctaagc accagcctct      60

gctgggctgc agaagcggcg gcggcggcag cagcagcagc agcatcagga aggcgctcgg     120

gccagcgcgg tgaacccggg ctgggcagca ggtcgcggag ccgcgagcca ggatggaggc     180

agagggcagc agcgcgccgg cccgggcggg cagcggagag ggcagcgaca gcgccggcgg     240

ggccacgctc aaagccccca agcatctctg gaggcacgag cagcaccacc agtacccgct     300

ccggcagccc cagttccgcc tcctgcatcc ccatcaccac ctgccccccgc cgccgccacc     360

ctcgccccag ccccagcccc agtgtccgct acagccgccg ccgccgcccc ccctgccgcc     420

gcccccgccg ccgcccgggg ctgcccgcgg ccgctacgcc tcgagcgggg ccaccggccg     480

cgtccggcat cgcggctact cggacaccga gcgctacctg tactgtcgcg ccatggaccg     540

cacctcctac gcggtggaga ccggccaccg gcccggcctg aagaaatcca ggatgtcctg     600

gccctcctcg ttccagggac tcaggcgttt tgatgtggac aatggcacat ctgcgggacg     660

gagtcccttg gatcccatga ccagcccagg atccgggcta attctccaag caaattttgt     720

ccacagtcaa cgacgggagt ccttcctgta tcgatccgac agcgattatg acctctctcc     780

aaagtctatg tcccggaact cctccattgc cagtgatata cacggagatg acttgattgt     840

gactccattt gctcaggtct tggccagtct gcgaactgta cgaaacaact ttgctgcatt     900

aactaatttg caagatcgag cacctagcaa aagatcaccc atgtgcaacc aaccatccat     960

caacaaagcc accataacag aggaggccta ccagaaactg gccagcgaga ccctggagga    1020

gctggactgg tgtctggacc agctagagac cctacagacc aggcactccg tcagtgagat    1080

ggcctccaac aagtttaaaa ggatgcttaa tcgggagctc acccatctct ctgaaatgag    1140

tcggtctgga aatcaagtgt cagagtttat atcaaacaca ttcttagata agcaacatga    1200
```

agtggaaatt ccttctccaa ctcagaagga aaaggagaaa aagaaaagac caatgtctca 1260
gatcagtgga gtcaagaaat tgatgcacag ctctagtctg actaattcaa gtatcccaag 1320
gtttggagtt aaaactgaac aagaagatgt ccttgccaag gaactagaag atgtgaacaa 1380
atggggtctt catgttttca gaatagcaga gttgtctggt aaccggccct tgactgttat 1440
catgcacacc atttttcagg aacgggattt attaaaaaca tttaaaattc cagtagatac 1500
tttaattaca tatcttatga ctctcgaaga ccattaccat gctgatgtgg cctatcacaa 1560
caatatccat gctgcagatg ttgtccagtc tactcatgtg ctattatcta cacctgcttt 1620
ggaggctgtg tttacagatt tggagattct tgcagcaatt tttgccagtg caatacatga 1680
tgtagatcat cctggtgtgt ccaatcaatt tctgatcaat acaaactctg aacttgcctt 1740
gatgtacaat gattcctcag tcttagagaa ccatcatttg gctgtgggct ttaaattgct 1800
tcaggaagaa aactgtgaca ttttccagaa tttgaccaaa aaacaaagac aatctttaag 1860
gaaaatggtc attgacatcg tacttgcaac agatatgtca aaacacatga atctactggc 1920
tgatttgaag actatggttg aaactaagaa agtgacaagc tctggagttc ttcttcttga 1980
taattattcc gataggattc aggttcttca gaatatggtg cactgtgcag atctgagcaa 2040
cccaacaaag cctctccagc tgtaccgcca gtggacggac cggataatgg aggagttctt 2100
ccgccaagga gaccgagaga gggaacgtgg catggagata agccccatgt gtgacaagca 2160
caatgcttcc gtggaaaaat cacaggtggg cttcatagac tatattgttc atcccctctg 2220
ggagacatgg gcagacctcg tccaccctga cgcccaggat attttggaca ctttggagga 2280
caatcgtgaa tggtaccaga gcacaatccc tcagagcccc tctcctgcac ctgatgaccc 2340
agaggagggc cggcagggtc aaactgagaa attccagttt gaactaactt tagaggaaga 2400
tggtgagtca gacacggaaa aggacagtgg cagtcaagtg gaagaagaca ctagctgcag 2460
tgactccaag actctttgta ctcaagactc agagtctact gaaattcccc ttgatgaaca 2520
ggttgaagag gaggcagtag gggaagaaga ggaaagccag cctgaagcct gtgtcataga 2580
tgatcgttct cctgacacgt aacagtgcaa aaactttcat gccttttttt tttttaagta 2640
gaaaaattgt ttccaaagtg catgtcacat gccacaacca cggtcacacc tcactgtcat 2700
ctgccaggac gtttgttgaa caaaactgac cttgactact cagtccagcg ctcaggaata 2760
tcgtaaccag tttttttcacc tccatgtcat ccgagcaagg tggacatctt cacgaacagc 2820
gtttttaaca agatttcagc ttggtagagc tgacaaagca gataaaatct actccaaatt 2880
attttcaaga gagtgtgact catcaggcag cccaaaagtt tattggactt ggggtttcta 2940
ttcctttttta tttgtttgca atattttcag aagaaaggca ttgcacagag tgaacttaat 3000
ggacgaagca acaaatatgt caagaacagg acatagcacg aatctgttac cagtaggagg 3060
aggatgagcc acagaaattg cataattttc taatttcaag tcttcctgat acatgactga 3120

atagtgtggt tcagtgagct gcactgacct ctacattttg tatgatatgt aaaacagatt 3180

ttttgtagag cttactttta ttattaaatg tattgaggta ttatatttaa aaaaaactat 3240

gttcagaact tcatctgcca ctggttattt ttttctaagg agtaacttgc aagttttcag 3300

tacaaatctg tgctacactg gataaaaatc taatttatga attttacttg caccttatag 3360

ttcatagcaa ttaactgatt tgtagtgatt cattgtttgt tttatatacc aatgacttcc 3420

atattttaaa agagaaaaac aactttatgt tgcaggaaac ccttttttgta agtctttatt 3480

atttactttg cattttgttt cactctttcc agataagcag agttgctctt caccagtgtt 3540

tttcttcatg tgcaaagtga ctatttgttc tataatactt ttatgtgtgt tatatcaaat 3600

gtgtcttaag cttcatgcaa actcagtcat cagttcgtgt tgtctgaagc aagtgggaga 3660

tatataaata cccagtagct aaaatggtca gtctttttta gatgttttcc tacttagtat 3720

ctcctaataa cgttttgctg tgtcactaga tgttcatttc acaagtgcat gtctttctaa 3780

taatccacac atttcatgct ctaataatcc acacatttca tgctcatttt tattgttttt 3840

acagccagtt atagtaagaa aaaggttttt ccccttgtgc tgctttataa tttagcgtgt 3900

gtctgaacct tatccatgtt tgctagatga ggtcttgtca aatatatcac taccattgtc 3960

accggtgaaa agaaacaggt agttaagtta gggttaacat tcatttcaac cacgaggttg 4020

tatatcatga ctagctttta ctcttggttt acagagaaaa gttaaacagc caactaggca 4080

gtttttaaga atattaacaa tatattaaca aacaccaata caactaatcc tatttggttt 4140

taatgatttc accatgggat taagaactat atcaggaaca tccctgagaa acggttttaa 4200

gtgtagcaac tactcttcct taatggacag ccacataacg tgtaggaagt cctttatcac 4260

ttatcctcga tccataagca tatcttgcag aggggaacta cttctttaaa cacatggagg 4320

gaaagaagat gatgccactg gcaccagagg gttagtactg tgatgcatcc taaaatattt 4380

attatattgg taaaaattct ggttaaataa aaaattagag atcactcttg gctgatttca 4440

gcaccaggaa ctgtattaca gttttagaga ttaattccta gtgtttacct gattatagca 4500

gttggcatca tggggcattt aattctgact ttatccccac gtcagcctta ataaagtctt 4560

ctttaccttc tctatgaaga ctttaaagcc caaataatca tttttcacat tgatattcaa 4620

gaattgagat agatagaagc caaagtgggt atctgacaag tggaaaatca aacgtttaag 4680

aagaattaca actctgaaaa gcatttatat gtggaacttc tcaaggagcc tcctggggac 4740

tggaaagtaa gtcatcagcc aggcaaatga ctcatgctga agagagtccc catttcagtc 4800

ccctgagatc tagctgatgc ttagatcctt tgaaataaaa attatgtctt tataactctg 4860

atcttttaca taaagcagaa gaggaatcaa ctagttaatt gcaaggtttc tactctgttt 4920

cctctgtaaa gatcagatgg taatctttca aataagaaaa aaataaagac gtatgtttga 4980

ccaagtagtt tcacaagaat atttgggaac ttgtttcttt taattttatt tgtccctgag 5040

tgaagtctag aaagaaaggt aaagagtcta gagtttattc ctctttccaa aacattctca 5100

ttcctctcct ccctacactt agtatttccc ccacagagtg cctagaatct taataatgaa 5160

taaaataaaa agcagcaata tgtcattaac aaatccagac ctgaaagggt aaagggttta 5220

taactgcact aataaagaga ggctcttttt ttttcttcca gtttgttggt ttttaatggt 5280

accgtgttgt aaagataccc actaatggac aatcaaattg cagaaaaggc tcaatatcca 5340

agagacaggg actaatgcac tgtacaatct gcttatcctt gcccttctct cttgccaaag 5400

tgtgcttcag aaatatatac tgctttaaaa aagaataaaa gaatatcctt ttacaagtgg 5460

ctttacattt cctaaaatgc cataagaaaa tgcaatatct gggtactgta tggggaaaaa 5520

aatgtccaag tttgtgtaaa accagtgcat ttcagcttgc aagttactga acacaataat 5580

gctgttttaa ttttgtttta tatcagttaa aattcacaat aatgtagata gaacaaatta 5640

cagacaagga aagaaaaaac ttgaatgaaa tggattttac agaaagcttt atgataattt 5700

ttgaatgcat tatttatttt ttgtgccatg catttttttt ctcaccaaat gaccttacct 5760

gtaatacagt cttgtttgtc tgtttacaac catgtattta ttgcaatgta catactgtaa 5820

tgttaattgt aaattatctg ttcttattaa aacatcatcc catgatggga tggtgttgat 5880

atatttggaa actcttggtg agagaatgaa tggtgtgtat acatactctg tacatttttc 5940

ttttctcctg taatatagtc ttgtcacctt agagcttgtt tatggaagat tcaagaaaac 6000

tataaaatac ttaaagatat ataaatttaa aaaaacatag ctgcaggtct ttggtcccag 6060

ggctgtgcct taactttaac caatattttc ttctgttttg ctgcatttga aaggtaacag 6120

tggagctagg gctgggcatt ttacatccag gcttttaatt gattagaatt ctgccaatag 6180

gtggatttta caaaaccaca gacaacctct gaaagattct gagacccttt tgagacagaa 6240

gctcttaagt acttcttgcc agggagcagc actgcatgtg tgatggttgt ttgccatctg 6300

ttgatcagga actacttcag ctacttgcat ttgattattt cctttttttt tttttttaac 6360

tcggaaacac aactggggaa atatattctt tcccagtgat tataaacaat ctttttcttt 6420

tttttaagtc cttttggctt ctagagctca taggaaaatg gacttgattt gaaattggag 6480

ccagagttta ctcgtgttgg ttatctattc atcagcttcc tgacatgtta agagaataca 6540

ttaaagagaa aatactgttt tttaatccta aaatttttct tccactaaga taaaccaaat 6600

gtccttacat atatgtaaac ccatctattt aaacgcaaag gtgggttgat gtcagtttac 6660

atagcagaaa gcattcacta tcctctaaga tttgtttctg caaaactttc attgctttag 6720

aattttaaaa tttcaccttg tacaatggcc agcccctaaa gcaggaaaca tttataatgg 6780

attatatgga aacatcctcc cagtacttgc ccagcccttg aatcatgtgg cttttcagtg 6840

aaaggaaaga ttctttttct aggaaaaatg agcctatttt attttatttt attttatttt 6900

ttgacacaaa ctgtagattt tagcagccct ggcccaaagg aatttgatta cttttgtttt 6960

aaacagtaca aaggggacac tataattaca aaaacatcct taactgattt gagttgtttt 7020

tatttctttg gatatatttt cagagtggta aattgtgtgt gagaattaca aatgattatt 7080

cttttagtgg tttcttagcc tctcttacag cccacgggga tagtactgta catcaatacc 7140

ttcatatgaa atttttatat gcaatgaaaa taaaagcatg ggttgattct gcctatttat 7200

gactcaatct tttacaaata aaagattatt cattttaaat tatagttcaa tcagcatgtc 7260

tcttaggata ctgaacgtgg ttgaaatgaa aggatagtga catcataagt tagtactgat 7320

attcataacc aaataaagcc aacttgagta attttgctac attaaaaatt accaaaatta 7380

cttagatggc ctataagatt aagcatggtg ttttctaagc aagctttgaa aggggccttc 7440

catacttact taattgaata ttctgggata ttgaaaatta ttcagatact tgacaattat 7500

ttttggttac ctactccgca aactacaaag ttttaaggac tcaacaataa gttaatgaga 7560

cacagtgttt gctttcatgg agcttacagt ctggagggga caaaggctta aacaatactc 7620

atataattat atatgtgatc agtacaatga aggagctcag tggggtaaat aagcaggaac 7680

ctgaacttga tctgttccgg agggccacag aaggcttcct tgaggccttg agaaagtgat 7740

ttgcatctga gttctgaagg attgtaagag gtaactaggg aaaaagttga caggaagagg 7800

aaggggatcc agacaagaaa catttgcaaa gatcttgagg cataaatgag cttgagacat 7860

ctggagaaac tgaggaaaag tgagagagta ggcagggcct ggagccgcag agccattgct 7920

aaccatcctg tgtgagatat cccccattct gtagctttat tctcataacc ctgctcaatt 7980

ttctttataa cacttctcac agatttatat acgtgtttgt ttttgttatc tgtctctccc 8040

accagaccac agctccatga gagcaaggtc tttgcttacc aatatatcac tagcacttaa 8100

aactatgcct ggtacacagt aggttcttaa tatgtgttga atatagccat caaattgata 8160

ttggatataa ttcaatctga taagatattt tgagatatta aagagttttt aacttgatac 8220

cataaaaaaa aaaaaaaaaa 8240

<210> 36
<211> 8153
<212> DNA
<213> Homo sapiens

<400> 36
aatacttgtt gcaataattg cccacgatag ctgctcaaac aagagagttg gaattcatct 60

gtaaaaatca ctacatgtaa cgtaggagac aagaaaaata ttaatgacag aagatctgcg 120

aacatgatgc acgtgaataa ttttcccttt agaaggcatt cctggatatg ctgatggtaa 180

cagcagaaaa atgtgaactc tgaataaaga ggtgggagtt tttcagcaca taaagaatat 240

ttaaagccaa ttcattggat gcattgacca gtaagtgtag agatcaaaat caagaacaac 300

tccaagaatt gagagcagat gcaaacccca atttttgtgg gtctccagtc cagccttccc 360
aggaatgctg gtctgactac tcagatttgc ttttacttct tgccttttgg atataatgag 420
tttgccaagc agctgtgagt acctgactct ggggaaggtg gctagattcc gaagcgctta 480
tgttcatgga tcaccatacg cgatcaacat gccaattgat attaagccac agaggagacg 540
ttttgatgtg gacaatggca catctgcggg acggagtccc ttggatccca tgaccagccc 600
aggatccggg ctaattctcc aagcaaattt tgtccacagt caacgacggg agtccttcct 660
gtatcgatcc gacagcgatt atgacctctc tccaaagtct atgtcccgga actcctccat 720
tgccagtgat atacacggag atgacttgat tgtgactcca tttgctcagg tcttggccag 780
tctgcgaact gtacgaaaca actttgctgc attaactaat ttgcaagatc gagcacctag 840
caaaagatca cccatgtgca accaaccatc catcaacaaa gccaccataa cagaggaggc 900
ctaccagaaa ctggccagcg agaccctgga ggagctggac tggtgtctgg accagctaga 960
gaccctacag accaggcact ccgtcagtga gatggcctcc aacaagttta aaaggatgct 1020
taatcgggag ctcacccatc tctctgaaat gagtcggtct ggaaatcaag tgtcagagtt 1080
tatatcaaac acattcttag ataagcaaca tgaagtggaa attccttctc caactcagaa 1140
ggaaaaggag aaaaagaaaa gaccaatgtc tcagatcagt ggagtcaaga aattgatgca 1200
cagctctagt ctgactaatt caagtatccc aaggtttgga gttaaaactg aacaagaaga 1260
tgtccttgcc aaggaactag aagatgtgaa caaatggggt cttcatgttt tcagaatagc 1320
agagttgtct ggtaaccggc ccttgactgt tatcatgcac accatttttc aggaacggga 1380
tttattaaaa acatttaaaa ttccagtaga tactttaatt acatatctta tgactctcga 1440
agaccattac catgctgatg tggcctatca caacaatatc catgctgcag atgttgtcca 1500
gtctactcat gtgctattat ctacacctgc tttggaggct gtgtttacag atttggagat 1560
tcttgcagca atttttgcca gtgcaataca tgatgtagat catcctggtg tgtccaatca 1620
atttctgatc aatacaaact ctgaacttgc cttgatgtac aatgattcct cagtcttaga 1680
gaaccatcat ttggctgtgg gctttaaatt gcttcaggaa gaaaactgtg acattttcca 1740
gaatttgacc aaaaaacaaa gacaatcttt aaggaaaatg gtcattgaca tcgtacttgc 1800
aacagatatg tcaaaacaca tgaatctact ggctgatttg aagactatgg ttgaaactaa 1860
gaaagtgaca agctctggag ttcttcttct tgataattat tccgatagga ttcaggttct 1920
tcagaatatg gtgcactgtg cagatctgag caacccaaca aagcctctcc agctgtaccg 1980
ccagtggacg gaccggataa tggaggagtt cttccgccaa ggagaccgag agagggaacg 2040
tggcatggag ataagcccca tgtgtgacaa gcacaatgct tccgtggaaa aatcacaggt 2100
gggcttcata gactatattg ttcatcccct ctgggagaca tgggcagacc tcgtccaccc 2160

tgacgcccag gatattttgg acactttgga ggacaatcgt gaatggtacc agagcacaat 2220
ccctcagagc ccctctcctg cacctgatga cccagaggag ggccggcagg gtcaaactga 2280
gaaattccag tttgaactaa ctttagagga agatggtgag tcagacacgg aaaaggacag 2340
tggcagtcaa gtggaagaag acactagctg cagtgactcc aagactcttt gtactcaaga 2400
ctcagagtct actgaaattc cccttgatga acaggttgaa gaggaggcag taggggaaga 2460
agaggaaagc cagcctgaag cctgtgtcat agatgatcgt tctcctgaca cgtaacagtg 2520
caaaaacttt catgcctttt tttttttaa gtagaaaaat tgtttccaaa gtgcatgtca 2580
catgccacaa ccacggtcac acctcactgt catctgccag gacgtttgtt gaacaaaact 2640
gaccttgact actcagtcca gcgctcagga atatcgtaac cagtttttc acctccatgt 2700
catccgagca aggtggacat cttcacgaac agcgttttta acaagatttc agcttggtag 2760
agctgacaaa gcagataaaa tctactccaa attattttca agagagtgtg actcatcagg 2820
cagcccaaaa gtttattgga cttggggttt ctattccttt ttatttgttt gcaatatttt 2880
cagaagaaag gcattgcaca gagtgaactt aatggacgaa gcaacaaata tgtcaagaac 2940
aggacatagc acgaatctgt taccagtagg aggaggatga gccacagaaa ttgcataatt 3000
ttctaatttc aagtcttcct gatacatgac tgaatagtgt ggttcagtga gctgcactga 3060
cctctacatt ttgtatgata tgtaaaacag atttttgta gagcttactt ttattattaa 3120
atgtattgag gtattatatt taaaaaaaac tatgttcaga acttcatctg ccactggtta 3180
ttttttcta aggagtaact tgcaagtttt cagtacaaat ctgtgctaca ctggataaaa 3240
atctaattta tgaattttac ttgcacctta tagttcatag caattaactg atttgtagtg 3300
attcattgtt tgttttatat accaatgact tccatatttt aaaagagaaa aacaacttta 3360
tgttgcagga aaccctttt gtaagtcttt attatttact ttgcattttg tttcactctt 3420
tccagataag cagagttgct cttcaccagt gtttttcttc atgtgcaaag tgactatttg 3480
ttctataata cttttatgtg tgttatatca aatgtgtctt aagcttcatg caaactcagt 3540
catcagttcg tgttgtctga agcaagtggg agatatataa atacccagta gctaaaatgg 3600
tcagtctttt ttagatgttt tcctacttag tatctcctaa taacgttttg ctgtgtcact 3660
agatgttcat ttcacaagtg catgtctttc taataatcca cacatttcat gctctaataa 3720
tccacacatt tcatgctcat ttttattgtt tttacagcca gttatagtaa gaaaaaggtt 3780
tttcccttg tgctgcttta taatttagcg tgtgtctgaa ccttatccat gtttgctaga 3840
tgaggtcttg tcaaatatat cactaccatt gtcaccggtg aaaagaaaca ggtagttaag 3900
ttagggttaa cattcatttc aaccacgagg ttgtatatca tgactagctt ttactcttgg 3960
tttacagaga aaagttaaac agccaactag gcagttttta agaatattaa caatatatta 4020
acaaacacca atacaactaa tcctatttgg ttttaatgat ttcaccatgg gattaagaac 4080

tatatcagga acatccctga gaaacggttt taagtgtagc aactactctt ccttaatgga 4140
cagccacata acgtgtagga agtcctttat cacttatcct cgatccataa gcatatcttg 4200
cagaggggaa ctacttcttt aaacacatgg agggaaagaa gatgatgcca ctggcaccag 4260
agggttagta ctgtgatgca tcctaaaata tttattatat tggtaaaaat tctggttaaa 4320
taaaaaatta gagatcactc ttggctgatt tcagcaccag gaactgtatt acagttttag 4380
agattaattc ctagtgttta cctgattata gcagttggca tcatggggca tttaattctg 4440
actttatccc cacgtcagcc ttaataaagt cttctttacc ttctctatga agactttaaa 4500
gcccaaataa tcatttttca cattgatatt caagaattga gatagataga agccaaagtg 4560
ggtatctgac aagtggaaaa tcaaacgttt aagaagaatt acaactctga aaagcattta 4620
tatgtggaac ttctcaagga gcctcctggg gactggaaag taagtcatca gccaggcaaa 4680
tgactcatgc tgaagagagt ccccatttca gtcccctgag atctagctga tgcttagatc 4740
ctttgaaata aaaattatgt ctttataact ctgatctttt acataaagca gaagaggaat 4800
caactagtta attgcaaggt ttctactctg tttcctctgt aaagatcaga tggtaatctt 4860
tcaaataaga aaaaaataaa gacgtatgtt tgaccaagta gtttcacaag aatatttggg 4920
aacttgtttc ttttaatttt atttgtccct gagtgaagtc tagaaagaaa ggtaaagagt 4980
ctagagttta ttcctctttc caaaacattc tcattcctct cctccctaca cttagtattt 5040
cccccacaga gtgcctagaa tcttaataat gaataaaata aaaagcagca atatgtcatt 5100
aacaaatcca gacctgaaag ggtaaagggt ttataactgc actaataaag agaggctctt 5160
tttttttctt ccagtttgtt ggtttttaat ggtaccgtgt tgtaaagata cccactaatg 5220
gacaatcaaa ttgcagaaaa ggctcaatat ccaagagaca gggactaatg cactgtacaa 5280
tctgcttatc cttgcccttc tctcttgcca aagtgtgctt cagaaatata tactgcttta 5340
aaaaagaata aaagaatatc cttttacaag tggctttaca tttcctaaaa tgccataaga 5400
aaatgcaata tctgggtact gtatggggaa aaaaatgtcc aagtttgtgt aaaaccagtg 5460
catttcagct tgcaagttac tgaacacaat aatgctgttt taattttgtt ttatatcagt 5520
taaaattcac aataatgtag atagaacaaa ttacagacaa ggaaagaaaa aacttgaatg 5580
aaatggattt tacagaaagc tttatgataa tttttgaatg cattatttat tttttgtgcc 5640
atgcattttt tttctcacca aatgacctta cctgtaatac agtcttgttt gtctgtttac 5700
aaccatgtat ttattgcaat gtacatactg taatgttaat tgtaaattat ctgttcttat 5760
taaaacatca tcccatgatg ggatggtgtt gatatatttg gaaactcttg gtgagagaat 5820
gaatggtgtg tatacatact ctgtacattt ttcttttctc ctgtaatata gtcttgtcac 5880
cttagagctt gtttatggaa gattcaagaa aactataaaa tacttaaaga tatataaatt 5940

taaaaaaaca tagctgcagg tctttggtcc cagggctgtg ccttaacttt aaccaatatt 6000
ttcttctgtt ttgctgcatt tgaaaggtaa cagtggagct agggctgggc attttacatc 6060
caggctttta attgattaga attctgccaa taggtggatt ttacaaaacc acagacaacc 6120
tctgaaagat tctgagaccc ttttgagaca gaagctctta agtacttctt gccagggagc 6180
agcactgcat gtgtgatggt tgtttgccat ctgttgatca ggaactactt cagctacttg 6240
catttgatta tttccttttt tttttttttt aactcggaaa cacaactggg gaaatatatt 6300
ctttcccagt gattataaac aatctttttc ttttttttaa gtccttttgg cttctagagc 6360
tcataggaaa atggacttga tttgaaattg gagccagagt ttactcgtgt tggttatcta 6420
ttcatcagct tcctgacatg ttaagagaat acattaaaga gaaaatactg ttttttaatc 6480
ctaaaatttt tcttccacta agataaacca aatgtcctta catatatgta aacccatcta 6540
tttaaacgca aaggtgggtt gatgtcagtt tacatagcag aaagcattca ctatcctcta 6600
agatttgttt ctgcaaaact ttcattgctt tagaatttta aaatttcacc ttgtacaatg 6660
gccagcccct aaagcaggaa acatttataa tggattatat ggaaacatcc tcccagtact 6720
tgcccagccc ttgaatcatg tggcttttca gtgaaaggaa agattctttt tctaggaaaa 6780
atgagcctat tttattttat tttattttat tttttgacac aaactgtaga ttttagcagc 6840
cctggcccaa aggaatttga ttacttttgt tttaaacagt acaaagggga cactataatt 6900
acaaaaacat ccttaactga tttgagttgt ttttatttct ttggatatat tttcagagtg 6960
gtaaattgtg tgtgagaatt acaaatgatt attcttttag tggtttctta gcctctctta 7020
cagcccacgg ggatagtact gtacatcaat accttcatat gaaattttta tatgcaatga 7080
aaataaaagc atgggttgat tctgcctatt tatgactcaa tcttttacaa ataaaagatt 7140
attcatttta aattatagtt caatcagcat gtctcttagg atactgaacg tggttgaaat 7200
gaaaggatag tgacatcata agttagtact gatattcata accaaataaa gccaacttga 7260
gtaattttgc tacattaaaa attaccaaaa ttacttagat ggcctataag attaagcatg 7320
gtgttttcta agcaagcttt gaaaggggcc ttccatactt acttaattga atattctggg 7380
atattgaaaa ttattcagat acttgacaat tatttttggt tacctactcc gcaaactaca 7440
aagttttaag gactcaacaa taagttaatg agacacagtg tttgctttca tggagcttac 7500
agtctggagg ggacaaaggc ttaaacaata ctcatataat tatatatgtg atcagtacaa 7560
tgaaggagct cagtggggta aataagcagg aacctgaact tgatctgttc cggagggcca 7620
cagaaggctt ccttgaggcc ttgagaaagt gatttgcatc tgagttctga aggattgtaa 7680
gaggtaacta gggaaaaagt tgacaggaag aggaagggga tccagacaag aaacatttgc 7740
aaagatcttg aggcataaat gagcttgaga catctggaga aactgaggaa aagtgagaga 7800
gtaggcaggg cctggagccg cagagccatt gctaaccatc ctgtgtgaga tatcccccat 7860

tctgtagctt tattctcata accctgctca atttcttta taacacttct cacagattta 7920

tatacgtgtt tgtttttgtt atctgtctct cccaccagac cacagctcca tgagagcaag 7980

gtctttgctt accaatatat cactagcact taaaactatg cctggtacac agtaggttct 8040

taatatgtgt tgaatatagc catcaaattg atattggata taattcaatc tgataagata 8100

ttttgagata ttaaagagtt tttaacttga taccataaaa aaaaaaaaaa aaa 8153

<210> 37
<211> 8203
<212> DNA
<213> Homo sapiens

<400> 37
gcggcgcatt agttccaata gtttaacagg ctgctttgta gcacggacaa agccgtgcga 60

gcgcggtgct ttccctcctt gttcatttgc tatgcgagct tgtcagtgat ctttggcagg 120

ggcttgggag aggaggggtg acgtttaata cgcttccgcg gaggagtgcg ctcgcctcct 180

ctgcacccag ccccaggctc tacagagaga ctgaggcagg cgactgaatg cactaacagc 240

agcaggctca gacctgcttc cctggacatt tccgggaccg tgagcgaggg aaccacgttg 300

ccctggattc ttgccagctg tacaaagttg accaggaaaa tggctcagca gacaagcccg 360

gacactttaa cagtacctga agtggataat ccgcattgtc caaacccgtg gctgaacgaa 420

gaccttgtga aatccttgcg agaaaacctg ttgcagcatg agaagtccaa gacagcgagg 480

aaatcggttt ctcccaagct ctctccagtg atctctccga gaaattcccc caggcttctg 540

cgcagaatgc ttctcagcag caacatcccc aaacagcggc gtttcacggt ggcacataca 600

tgttttgatg tggacaatgg cacatctgcg ggacggagtc ccttggatcc catgaccagc 660

ccaggatccg ggctaattct ccaagcaaat tttgtccaca gtcaacgacg ggagtccttc 720

ctgtatcgat ccgacagcga ttatgacctc tctccaaagt ctatgtcccg gaactcctcc 780

attgccagtg atatacacgg agatgacttg attgtgactc catttgctca ggtcttggcc 840

agtctgcgaa ctgtacgaaa caactttgct gcattaacta atttgcaaga tcgagcacct 900

agcaaaagat cacccatgtg caaccaacca tccatcaaca aagccaccat aacagaggag 960

gcctaccaga aactggccag cgagaccctg gaggagctgg actggtgtct ggaccagcta 1020

gagaccctac agaccaggca ctccgtcagt gagatggcct ccaacaagtt taaaaggatg 1080

cttaatcggg agctcaccca tctctctgaa atgagtcggt ctggaaatca agtgtcagag 1140

tttatatcaa acacattctt agataagcaa catgaagtgg aaattccttc tccaactcag 1200

aaggaaaagg agaaaaagaa aagaccaatg tctcagatca gtggagtcaa gaaattgatg 1260

cacagctcta gtctgactaa ttcaagtatc ccaaggtttg gagttaaaac tgaacaagaa 1320

gatgtccttg ccaaggaact agaagatgtg aacaaatggg gtcttcatgt tttcagaata 1380

gcagagttgt ctggtaaccg gcccttgact gttatcatgc acaccatttt tcaggaacgg 1440
gatttattaa aaacatttaa aattccagta gatactttaa ttacatatct tatgactctc 1500
gaagaccatt accatgctga tgtggcctat cacaacaata tccatgctgc agatgttgtc 1560
cagtctactc atgtgctatt atctacacct gctttggagg ctgtgtttac agatttggag 1620
attcttgcag caatttttgc cagtgcaata catgatgtag atcatcctgg tgtgtccaat 1680
caatttctga tcaatacaaa ctctgaactt gccttgatgt acaatgattc ctcagtctta 1740
gagaaccatc atttggctgt gggctttaaa ttgcttcagg aagaaaactg tgacattttc 1800
cagaatttga ccaaaaaaca aagacaatct ttaaggaaaa tggtcattga catcgtactt 1860
gcaacagata tgtcaaaaca catgaatcta ctggctgatt tgaagactat ggttgaaact 1920
aagaaagtga caagctctgg agttcttctt cttgataatt attccgatag gattcaggtt 1980
cttcagaata tggtgcactg tgcagatctg agcaacccaa caaagcctct ccagctgtac 2040
cgccagtgga cggaccggat aatggaggag ttcttccgcc aaggagaccg agagagggaa 2100
cgtggcatgg agataagccc catgtgtgac aagcacaatg cttccgtgga aaaatcacag 2160
gtgggcttca tagactatat tgttcatccc ctctgggaga catgggcaga cctcgtccac 2220
cctgacgccc aggatatttt ggacactttg gaggacaatc gtgaatggta ccagagcaca 2280
atccctcaga gcccctctcc tgcacctgat gacccagagg agggccggca gggtcaaact 2340
gagaaattcc agtttgaact aactttagag gaagatggtg agtcagacac ggaaaaggac 2400
agtggcagtc aagtggaaga agacactagc tgcagtgact ccaagactct ttgtactcaa 2460
gactcagagt ctactgaaat tccccttgat gaacaggttg aagaggaggc agtaggggaa 2520
gaagaggaaa gccagcctga agcctgtgtc atagatgatc gttctcctga cacgtaacag 2580
tgcaaaaact ttcatgcctt tttttttttt aagtagaaaa attgtttcca aagtgcatgt 2640
cacatgccac aaccacggtc acacctcact gtcatctgcc aggacgtttg ttgaacaaaa 2700
ctgaccttga ctactcagtc cagcgctcag gaatatcgta accagttttt tcacctccat 2760
gtcatccgag caaggtggac atcttcacga acagcgtttt taacaagatt tcagcttggt 2820
agagctgaca aagcagataa aatctactcc aaattatttt caagagagtg tgactcatca 2880
ggcagcccaa aagtttattg gacttggggt ttctattcct ttttatttgt ttgcaatatt 2940
ttcagaagaa aggcattgca cagagtgaac ttaatggacg aagcaacaaa tatgtcaaga 3000
acaggacata gcacgaatct gttaccagta ggaggaggat gagccacaga aattgcataa 3060
ttttctaatt tcaagtcttc ctgatacatg actgaatagt gtggttcagt gagctgcact 3120
gacctctaca ttttgtatga tatgtaaaac agattttttg tagagcttac ttttattatt 3180
aaatgtattg aggtattata tttaaaaaaa actatgttca gaacttcatc tgccactggt 3240

tatttttttc taaggagtaa cttgcaagtt ttcagtacaa atctgtgcta cactggataa 3300

aaatctaatt tatgaatttt acttgcacct tatagttcat agcaattaac tgatttgtag 3360

tgattcattg tttgttttat ataccaatga cttccatatt ttaaaagaga aaaacaactt 3420

tatgttgcag gaaacccttt ttgtaagtct ttattattta ctttgcattt tgtttcactc 3480

tttccagata agcagagttg ctcttcacca gtgtttttct tcatgtgcaa agtgactatt 3540

tgttctataa tacttttatg tgtgttatat caaatgtgtc ttaagcttca tgcaaactca 3600

gtcatcagtt cgtgttgtct gaagcaagtg ggagatatat aaatacccag tagctaaaat 3660

ggtcagtctt ttttagatgt tttcctactt agtatctcct aataacgttt tgctgtgtca 3720

ctagatgttc atttcacaag tgcatgtctt tctaataatc cacacatttc atgctctaat 3780

aatccacaca tttcatgctc atttttattg tttttacagc cagttatagt aagaaaaagg 3840

tttttcccct tgtgctgctt tataatttag cgtgtgtctg aaccttatcc atgtttgcta 3900

gatgaggtct tgtcaaatat atcactacca ttgtcaccgg tgaaaagaaa caggtagtta 3960

agttagggtt aacattcatt tcaaccacga ggttgtatat catgactagc ttttactctt 4020

ggtttacaga gaaaagttaa acagccaact aggcagtttt taagaatatt aacaatatat 4080

taacaaacac caatacaact aatcctattt ggttttaatg atttcaccat gggattaaga 4140

actatatcag gaacatccct gagaaacggt tttaagtgta gcaactactc ttccttaatg 4200

gacagccaca taacgtgtag gaagtccttt atcacttatc ctcgatccat aagcatatct 4260

tgcagagggg aactacttct ttaaacacat ggagggaaag aagatgatgc cactggcacc 4320

agagggttag tactgtgatg catcctaaaa tatttattat attggtaaaa attctggtta 4380

aataaaaaat tagagatcac tcttggctga tttcagcacc aggaactgta ttacagtttt 4440

agagattaat tcctagtgtt tacctgatta tagcagttgg catcatgggg catttaattc 4500

tgactttatc cccacgtcag ccttaataaa gtcttcttta ccttctctat gaagacttta 4560

aagcccaaat aatcattttt cacattgata ttcaagaatt gagatagata gaagccaaag 4620

tgggtatctg acaagtggaa aatcaaacgt ttaagaagaa ttacaactct gaaaagcatt 4680

tatatgtgga acttctcaag gagcctcctg gggactggaa agtaagtcat cagccaggca 4740

aatgactcat gctgaagaga gtccccattt cagtcccctg agatctagct gatgcttaga 4800

tcctttgaaa taaaaattat gtctttataa ctctgatctt ttacataaag cagaagagga 4860

atcaactagt taattgcaag gtttctactc tgtttcctct gtaaagatca gatggtaatc 4920

tttcaaataa gaaaaaaata aagacgtatg tttgaccaag tagtttcaca agaatatttg 4980

ggaacttgtt tcttttaatt ttatttgtcc ctgagtgaag tctagaaaga aaggtaaaga 5040

gtctagagtt tattcctctt tccaaaacat tctcattcct ctcctcccta cacttagtat 5100

ttcccccaca gagtgcctag aatcttaata atgaataaaa taaaaagcag caatatgtca 5160

ttaacaaatc cagacctgaa agggtaaagg gtttataact gcactaataa agagaggctc 5220
ttttttttttc ttccagtttg ttggttttta atggtaccgt gttgtaaaga tacccactaa 5280
tggacaatca aattgcagaa aaggctcaat atccaagaga cagggactaa tgcactgtac 5340
aatctgctta tccttgccct tctctcttgc caaagtgtgc ttcagaaata tatactgctt 5400
taaaaaagaa taaaagaata tccttttaca agtggcttta catttcctaa aatgccataa 5460
gaaaatgcaa tatctgggta ctgtatgggg aaaaaaatgt ccaagtttgt gtaaaaccag 5520
tgcatttcag cttgcaagtt actgaacaca ataatgctgt tttaattttg ttttatatca 5580
gttaaaattc acaataatgt agatagaaca aattacagac aaggaaagaa aaaacttgaa 5640
tgaaatggat tttacagaaa gctttatgat aatttttgaa tgcattattt attttttgtg 5700
ccatgcattt tttttctcac caaatgacct tacctgtaat acagtcttgt ttgtctgttt 5760
acaaccatgt atttattgca atgtacatac tgtaatgtta attgtaaatt atctgttctt 5820
attaaaacat catcccatga tgggatggtg ttgatatatt tggaaactct tggtgagaga 5880
atgaatggtg tgtatacata ctctgtacat ttttcttttc tcctgtaata tagtcttgtc 5940
accttagagc ttgtttatgg aagattcaag aaaactataa aatacttaaa gatatataaa 6000
tttaaaaaaa catagctgca ggtctttggt cccagggctg tgccttaact ttaaccaata 6060
ttttcttctg ttttgctgca tttgaaaggt aacagtggag ctagggctgg gcattttaca 6120
tccaggcttt taattgatta gaattctgcc aataggtgga ttttacaaaa ccacagacaa 6180
cctctgaaag attctgagac ccttttgaga cagaagctct taagtacttc ttgccaggga 6240
gcagcactgc atgtgtgatg gttgtttgcc atctgttgat caggaactac ttcagctact 6300
tgcatttgat tatttccttt tttttttttt ttaactcgga aacacaactg gggaaatata 6360
ttctttccca gtgattataa acaatctttt tctttttttt aagtcctttt ggcttctaga 6420
gctcatagga aaatggactt gatttgaaat tggagccaga gtttactcgt gttggttatc 6480
tattcatcag cttcctgaca tgttaagaga atacattaaa gagaaaatac tgttttttaa 6540
tcctaaaatt tttcttccac taagataaac caaatgtcct tacatatatg taaacccatc 6600
tatttaaacg caaaggtggg ttgatgtcag tttacatagc agaaagcatt cactatcctc 6660
taagatttgt ttctgcaaaa ctttcattgc tttagaattt taaaatttca ccttgtacaa 6720
tggccagccc ctaaagcagg aaacatttat aatggattat atggaaacat cctcccagta 6780
cttgcccagc ccttgaatca tgtggctttt cagtgaaagg aaagattctt tttctaggaa 6840
aaatgagcct attttatttt attttatttt attttttgac acaaactgta gattttagca 6900
gccctggccc aaaggaattt gattactttt gttttaaaca gtacaaaggg gacactataa 6960
ttacaaaaac atccttaact gatttgagtt gtttttattt ctttggatat attttcagag 7020

tggtaaattg tgtgtgagaa ttacaaatga ttattctttt agtggtttct tagcctctct 7080

tacagcccac ggggatagta ctgtacatca ataccttcat atgaaatttt tatatgcaat 7140

gaaaataaaa gcatgggttg attctgccta tttatgactc aatcttttac aaataaaaga 7200

ttattcattt taaattatag ttcaatcagc atgtctctta ggatactgaa cgtggttgaa 7260

atgaaaggat agtgacatca taagttagta ctgatattca taaccaaata aagccaactt 7320

gagtaatttt gctacattaa aaattaccaa aattacttag atggcctata agattaagca 7380

tggtgttttc taagcaagct ttgaaagggg ccttccatac ttacttaatt gaatattctg 7440

ggatattgaa aattattcag atacttgaca attatttttg gttacctact ccgcaaacta 7500

caaagtttta aggactcaac aataagttaa tgagacacag tgtttgcttt catggagctt 7560

acagtctgga ggggacaaag gcttaaacaa tactcatata attatatatg tgatcagtac 7620

aatgaaggag ctcagtgggg taaataagca ggaacctgaa cttgatctgt tccggagggc 7680

cacagaaggc ttccttgagg ccttgagaaa gtgatttgca tctgagttct gaaggattgt 7740

aagaggtaac tagggaaaaa gttgacagga agaggaaggg gatccagaca agaaacattt 7800

gcaaagatct tgaggcataa atgagcttga gacatctgga gaaactgagg aaaagtgaga 7860

gagtaggcag ggcctggagc cgcagagcca ttgctaacca tcctgtgtga gatatccccc 7920

attctgtagc tttattctca taaccctgct caattttctt tataacactt ctcacagatt 7980

tatatacgtg tttgtttttg ttatctgtct ctcccaccag accacagctc catgagagca 8040

aggtctttgc ttaccaatat atcactagca cttaaaacta tgcctggtac acagtaggtt 8100

cttaatatgt gttgaatata gccatcaaat tgatattgga tataattcaa tctgataaga 8160

tattttgaga tattaaagag tttttaactt gataccataa aaa 8203

<210> 38
<211> 7783
<212> DNA
<213> Homo sapiens

<400> 38
aatacttgtt gcaataattg cccacgatag ctgctcaaac aagagagttg gaattcatct 60

gtaaaaatca ctacatgtaa cgtaggagac aagaaaaata ttaatgacag aagatctgcg 120

aacatgatgc acgtgaataa ttttcccttt agaaggcatt cctggatatg ttttgatgtg 180

gacaatggca catctgcggg acggagtccc ttggatccca tgaccagccc aggatccggg 240

ctaattctcc aagcaaattt tgtccacagt caacgacggg agtccttcct gtatcgatcc 300

gacagcgatt atgacctctc tccaaagtct atgtcccgga actcctccat tgccagtgat 360

atacacggag atgacttgat tgtgactcca tttgctcagg tcttggccag tctgcgaact 420

gtacgaaaca actttgctgc attaactaat ttgcaagatc gagcacctag caaaagatca 480

cccatgtgca accaaccatc catcaacaaa gccaccataa cagaggaggc ctaccagaaa 540
ctggccagcg agaccctgga ggagctggac tggtgtctgg accagctaga gaccctacag 600
accaggcact ccgtcagtga gatggcctcc aacaagttta aaaggatgct taatcgggag 660
ctcacccatc tctctgaaat gagtcggtct ggaaatcaag tgtcagagtt tatatcaaac 720
acattcttag ataagcaaca tgaagtggaa attccttctc caactcagaa ggaaaaggag 780
aaaaagaaaa gaccaatgtc tcagatcagt ggagtcaaga aattgatgca cagctctagt 840
ctgactaatt caagtatccc aaggtttgga gttaaaactg aacaagaaga tgtccttgcc 900
aaggaactag aagatgtgaa caaatggggt cttcatgttt tcagaatagc agagttgtct 960
ggtaaccggc ccttgactgt tatcatgcac accatttttc aggaacggga tttattaaaa 1020
acatttaaaa ttccagtaga tactttaatt acatatctta tgactctcga agaccattac 1080
catgctgatg tggcctatca caacaatatc catgctgcag atgttgtcca gtctactcat 1140
gtgctattat ctacacctgc tttggaggct gtgtttacag atttggagat tcttgcagca 1200
atttttgcca gtgcaataca tgatgtagat catcctggtg tgtccaatca atttctgatc 1260
aatacaaact ctgaacttgc cttgatgtac aatgattcct cagtcttaga gaaccatcat 1320
ttggctgtgg gctttaaatt gcttcaggaa gaaaactgtg acattttcca gaatttgacc 1380
aaaaaacaaa gacaatcttt aaggaaaatg gtcattgaca tcgtacttgc aacagatatg 1440
tcaaaacaca tgaatctact ggctgatttg aagactatgg ttgaaactaa gaaagtgaca 1500
agctctggag ttcttcttct tgataattat tccgatagga ttcaggttct tcagaatatg 1560
gtgcactgtg cagatctgag caacccaaca aagcctctcc agctgtaccg ccagtggacg 1620
gaccggataa tggaggagtt cttccgccaa ggagaccgag agagggaacg tggcatggag 1680
ataagcccca tgtgtgacaa gcacaatgct tccgtggaaa aatcacaggt gggcttcata 1740
gactatattg ttcatcccct ctgggagaca tgggcagacc tcgtccaccc tgacgcccag 1800
gatattttgg acactttgga ggacaatcgt gaatggtacc agagcacaat ccctcagagc 1860
ccctctcctg cacctgatga cccagaggag ggccggcagg gtcaaactga gaaattccag 1920
tttgaactaa ctttagagga agatggtgag tcagacacgg aaaaggacag tggcagtcaa 1980
gtggaagaag acactagctg cagtgactcc aagactcttt gtactcaaga ctcagagtct 2040
actgaaattc cccttgatga acaggttgaa gaggaggcag taggggaaga agaggaaagc 2100
cagcctgaag cctgtgtcat agatgatcgt tctcctgaca cgtaacagtg caaaaacttt 2160
catgcctttt tttttttaa gtagaaaaat tgtttccaaa gtgcatgtca catgccacaa 2220
ccacggtcac acctcactgt catctgccag gacgtttgtt gaacaaaact gaccttgact 2280
actcagtcca gcgctcagga atatcgtaac cagtttttc acctccatgt catccgagca 2340
aggtggacat cttcacgaac agcgttttta acaagatttc agcttggtag agctgacaaa 2400

gcagataaaa tctactccaa attattttca agagagtgtg actcatcagg cagcccaaaa 2460

gtttattgga cttggggttt ctattccttt ttatttgttt gcaatatttt cagaagaaag 2520

gcattgcaca gagtgaactt aatggacgaa gcaacaaata tgtcaagaac aggacatagc 2580

acgaatctgt taccagtagg aggaggatga gccacagaaa ttgcataatt ttctaatttc 2640

aagtcttcct gatacatgac tgaatagtgt ggttcagtga gctgcactga cctctacatt 2700

ttgtatgata tgtaaaacag atttttgta gagcttactt ttattattaa atgtattgag 2760

gtattatatt taaaaaaaac tatgttcaga acttcatctg ccactggtta tttttttcta 2820

aggagtaact tgcaagtttt cagtacaaat ctgtgctaca ctggataaaa atctaattta 2880

tgaattttac ttgcacctta tagttcatag caattaactg atttgtagtg attcattgtt 2940

tgttttatat accaatgact tccatatttt aaaagagaaa aacaacttta tgttgcagga 3000

aacccttttt gtaagtcttt attatttact ttgcattttg tttcactctt tccagataag 3060

cagagttgct cttcaccagt gtttttcttc atgtgcaaag tgactatttg ttctataata 3120

cttttatgtg tgttatatca aatgtgtctt aagcttcatg caaactcagt catcagttcg 3180

tgttgtctga agcaagtggg agatatataa atacccagta gctaaaatgg tcagtctttt 3240

ttagatgttt tcctacttag tatctcctaa taacgttttg ctgtgtcact agatgttcat 3300

ttcacaagtg catgtctttc taataatcca cacatttcat gctctaataa tccacacatt 3360

tcatgctcat ttttattgtt tttacagcca gttatagtaa gaaaaaggtt tttccccttg 3420

tgctgcttta taatttagcg tgtgtctgaa ccttatccat gtttgctaga tgaggtcttg 3480

tcaaatatat cactaccatt gtcaccggtg aaaagaaaca ggtagttaag ttagggttaa 3540

cattcatttc aaccacgagg ttgtatatca tgactagctt ttactcttgg tttacagaga 3600

aaagttaaac agccaactag gcagtttta agaatattaa caatatatta acaaacacca 3660

atacaactaa tcctatttgg ttttaatgat ttcaccatgg gattaagaac tatatcagga 3720

acatccctga gaaacggttt taagtgtagc aactactctt ccttaatgga cagccacata 3780

acgtgtagga agtcctttat cacttatcct cgatccataa gcatatcttg cagaggggaa 3840

ctacttcttt aaacacatgg agggaaagaa gatgatgcca ctggcaccag agggttagta 3900

ctgtgatgca tcctaaaata tttattatat tggtaaaaat tctggttaaa taaaaaatta 3960

gagatcactc ttggctgatt tcagcaccag gaactgtatt acagttttag agattaattc 4020

ctagtgttta cctgattata gcagttggca tcatggggca tttaattctg actttatccc 4080

cacgtcagcc ttaataaagt cttctttacc ttctctatga agactttaaa gcccaaataa 4140

tcatttttca cattgatatt caagaattga gatagataga agccaaagtg ggtatctgac 4200

aagtggaaaa tcaaacgttt aagaagaatt acaactctga aaagcattta tatgtggaac 4260

ttctcaagga gcctcctggg gactggaaag taagtcatca gccaggcaaa tgactcatgc 4320
tgaagagagt ccccatttca gtcccctgag atctagctga tgcttagatc ctttgaaata 4380
aaaattatgt ctttataact ctgatctttt acataaagca gaagaggaat caactagtta 4440
attgcaaggt ttctactctg tttcctctgt aaagatcaga tggtaatctt tcaaataaga 4500
aaaaaataaa gacgtatgtt tgaccaagta gtttcacaag aatatttggg aacttgtttc 4560
ttttaatttt atttgtccct gagtgaagtc tagaaagaaa ggtaaagagt ctagagttta 4620
ttcctctttc caaaacattc tcattcctct cctccctaca cttagtattt cccccacaga 4680
gtgcctagaa tcttaataat gaataaaata aaaagcagca atatgtcatt aacaaatcca 4740
gacctgaaag ggtaaagggt ttataactgc actaataaag agaggctctt tttttttctt 4800
ccagtttgtt ggtttttaat ggtaccgtgt tgtaaagata cccactaatg gacaatcaaa 4860
ttgcagaaaa ggctcaatat ccaagagaca gggactaatg cactgtacaa tctgcttatc 4920
cttgcccttc tctcttgcca aagtgtgctt cagaaatata tactgcttta aaaaagaata 4980
aaagaatatc cttttacaag tggctttaca tttcctaaaa tgccataaga aaatgcaata 5040
tctgggtact gtatggggaa aaaaatgtcc aagtttgtgt aaaaccagtg catttcagct 5100
tgcaagttac tgaacacaat aatgctgttt taattttgtt ttatatcagt taaaattcac 5160
aataatgtag atagaacaaa ttacagacaa ggaaagaaaa aacttgaatg aaatggattt 5220
tacagaaagc tttatgataa tttttgaatg cattatttat tttttgtgcc atgcattttt 5280
tttctcacca aatgacctta cctgtaatac agtcttgttt gtctgtttac aaccatgtat 5340
ttattgcaat gtacatactg taatgttaat tgtaaattat ctgttcttat taaaacatca 5400
tcccatgatg ggatggtgtt gatatatttg gaaactcttg gtgagagaat gaatggtgtg 5460
tatacatact ctgtacattt ttcttttctc ctgtaatata gtcttgtcac cttagagctt 5520
gtttatggaa gattcaagaa aactataaaa tacttaaaga tatataaatt taaaaaaaca 5580
tagctgcagg tctttggtcc cagggctgtg ccttaacttt aaccaatatt ttcttctgtt 5640
ttgctgcatt tgaaaggtaa cagtggagct agggctgggc attttacatc caggctttta 5700
attgattaga attctgccaa taggtggatt ttacaaaacc acagacaacc tctgaaagat 5760
tctgagaccc ttttgagaca gaagctctta agtacttctt gccagggagc agcactgcat 5820
gtgtgatggt tgtttgccat ctgttgatca ggaactactt cagctacttg catttgatta 5880
tttccttttt tttttttttt aactcggaaa cacaactggg gaaatatatt ctttcccagt 5940
gattataaac aatctttttc tttttttaa gtccttttgg cttctagagc tcataggaaa 6000
atggacttga tttgaaattg gagccagagt ttactcgtgt tggttatcta ttcatcagct 6060
tcctgacatg ttaagagaat acattaaaga gaaaatactg tttttaatc ctaaaatttt 6120
tcttccacta agataaacca aatgtcctta catatatgta aacccatcta tttaaacgca 6180

aaggtgggtt gatgtcagtt tacatagcag aaagcattca ctatcctcta agatttgttt 6240

ctgcaaaact ttcattgctt tagaatttta aaatttcacc ttgtacaatg gccagcccct 6300

aaagcaggaa acatttataa tggattatat ggaaacatcc tcccagtact tgcccagccc 6360

ttgaatcatg tggcttttca gtgaaaggaa agattctttt tctaggaaaa atgagcctat 6420

tttattttat tttattttat tttttgacac aaactgtaga ttttagcagc cctggcccaa 6480

aggaatttga ttacttttgt tttaaacagt acaaagggga cactataatt acaaaaacat 6540

ccttaactga tttgagttgt ttttatttct ttggatatat tttcagagtg gtaaattgtg 6600

tgtgagaatt acaaatgatt attcttttag tggtttctta gcctctctta cagcccacgg 6660

ggatagtact gtacatcaat accttcatat gaaattttta tatgcaatga aaataaaagc 6720

atgggttgat tctgcctatt tatgactcaa tcttttacaa ataaaagatt attcatttta 6780

aattatagtt caatcagcat gtctcttagg atactgaacg tggttgaaat gaaaggatag 6840

tgacatcata agttagtact gatattcata accaaataaa gccaacttga gtaattttgc 6900

tacattaaaa attaccaaaa ttacttagat ggcctataag attaagcatg gtgttttcta 6960

agcaagcttt gaaaggggcc ttccatactt acttaattga atattctggg atattgaaaa 7020

ttattcagat acttgacaat tatttttggt tacctactcc gcaaactaca aagttttaag 7080

gactcaacaa taagttaatg agacacagtg tttgctttca tggagcttac agtctggagg 7140

ggacaaaggc ttaaacaata ctcatataat tatatatgtg atcagtacaa tgaaggagct 7200

cagtggggta aataagcagg aacctgaact tgatctgttc cggagggcca cagaaggctt 7260

ccttgaggcc ttgagaaagt gatttgcatc tgagttctga aggattgtaa gaggtaacta 7320

gggaaaaagt tgacaggaag aggaagggga tccagacaag aaacatttgc aaagatcttg 7380

aggcataaat gagcttgaga catctggaga aactgaggaa aagtgagaga gtaggcaggg 7440

cctggagccg cagagccatt gctaaccatc ctgtgtgaga tatcccccat tctgtagctt 7500

tattctcata accctgctca attttcttta taacacttct cacagattta tatacgtgtt 7560

tgtttttgtt atctgtctct cccaccagac cacagctcca tgagagcaag gtctttgctt 7620

accaatatat cactagcact taaaactatg cctggtacac agtaggttct taatatgtgt 7680

tgaatatagc catcaaattg atattggata taattcaatc tgataagata ttttgagata 7740

ttaaagagtt tttaacttga taccataaaa aaaaaaaaaa aaa 7783

<210> 39
<211> 7667
<212> DNA
<213> Homo sapiens

<400> 39
acttcaagtg ccgtgcagaa ggctcggcag gcggggcggg cgtggggccg cggctccggg 60

```
ttggggaccg aggagatccg gctgtggacc agacgctcct ctgcggggcg ggcacccaag     120
cgcgctcgcc accccctcgc catccgctag agccgggctc ctggactggg actcgggccc     180
gccgcacagt tgaaaagtcg catagtggtt tttccgctcg cgtcgctgtg tgaaagttgg     240
ctcgccgctc tttgcacgcc ctccctggag gccgacccga gacgccaagc tggagagacc     300
gtgcctcccc gaggccggcc gccccgcgag cacagcctcc gcccccgttg cactgccggg     360
ctgggcaata tgaaggagca gccctcatgt gccggcaccg ggcatccgag catggcggga     420
ggaggcctac cagaaactgg ccagcgagac cctggaggag ctggactggt gtctggacca     480
gctagagacc ctacagacca ggcactccgt cagtgagatg gcctccaaca agtttaaaag     540
gatgcttaat cgggagctca cccatctctc tgaaatgagt cggtctggaa atcaagtgtc     600
agagtttata tcaaacacat tcttagataa gcaacatgaa gtggaaattc cttctccaac     660
tcagaaggaa aaggagaaaa agaaaaagacc aatgtctcag atcagtggag tcaagaaatt    720
gatgcacagc tctagtctga ctaattcaag tatcccaagg tttggagtta aaactgaaca     780
agaagatgtc cttgccaagg aactagaaga tgtgaacaaa tggggtcttc atgttttcag     840
aatagcagag ttgtctggta accggccctt gactgttatc atgcacacca tttttcagga     900
acgggattta ttaaaaacat ttaaaattcc agtagatact ttaattacat atcttatgac     960
tctcgaagac cattaccatg ctgatgtggc ctatcacaac aatatccatg ctgcagatgt    1020
tgtccagtct actcatgtgc tattatctac acctgctttg gaggctgtgt ttacagattt    1080
ggagattctt gcagcaattt ttgccagtgc aatacatgat gtagatcatc ctggtgtgtc    1140
caatcaattt ctgatcaata caaactctga acttgccttg atgtacaatg attcctcagt    1200
cttagagaac catcatttgg ctgtgggctt taaattgctt caggaagaaa actgtgacat    1260
tttccagaat ttgaccaaaa aacaaagaca atctttaagg aaaatggtca ttgacatcgt    1320
acttgcaaca gatatgtcaa aacacatgaa tctactggct gatttgaaga ctatggttga    1380
aactaagaaa gtgacaagct ctggagttct tcttcttgat aattattccg ataggattca    1440
ggttcttcag aatatggtgc actgtgcaga tctgagcaac ccaacaaagc ctctccagct    1500
gtaccgccag tggacggacc ggataatgga ggagttcttc cgccaaggag accgagagag    1560
ggaacgtggc atggagataa gccccatgtg tgacaagcac aatgcttccg tggaaaaatc    1620
acaggtgggc ttcatagact atattgttca tcccctctgg gagacatggg cagacctcgt    1680
ccaccctgac gcccaggata ttttggacac tttggaggac aatcgtgaat ggtaccagag    1740
cacaatccct cagagcccct ctcctgcacc tgatgaccca gaggagggcc ggcagggtca    1800
aactgagaaa ttccagtttg aactaacttt agaggaagat ggtgagtcag acacggaaaa    1860
ggacagtggc agtcaagtgg aagaagacac tagctgcagt gactccaaga ctctttgtac    1920
```

tcaagactca gagtctactg aaattcccct tgatgaacag gttgaagagg aggcagtagg 1980

ggaagaagag gaaagccagc ctgaagcctg tgtcatagat gatcgttctc ctgacacgta 2040

acagtgcaaa aactttcatg cctttttttt ttttaagtag aaaaattgtt tccaaagtgc 2100

atgtcacatg ccacaaccac ggtcacacct cactgtcatc tgccaggacg tttgttgaac 2160

aaaactgacc ttgactactc agtccagcgc tcaggaatat cgtaaccagt tttttcacct 2220

ccatgtcatc cgagcaaggt ggacatcttc acgaacagcg tttttaacaa gatttcagct 2280

tggtagagct gacaaagcag ataaaatcta ctccaaatta ttttcaagag agtgtgactc 2340

atcaggcagc ccaaaagttt attggacttg gggtttctat tccttttttat ttgtttgcaa 2400

tattttcaga agaaaggcat tgcacagagt gaacttaatg gacgaagcaa caaatatgtc 2460

aagaacagga catagcacga atctgttacc agtaggagga ggatgagcca cagaaattgc 2520

ataattttct aatttcaagt cttcctgata catgactgaa tagtgtggtt cagtgagctg 2580

cactgacctc tacattttgt atgatatgta aaacagattt tttgtagagc ttacttttat 2640

tattaaatgt attgaggtat tatatttaaa aaaaactatg ttcagaactt catctgccac 2700

tggttatttt tttctaagga gtaacttgca agttttcagt acaaatctgt gctacactgg 2760

ataaaaatct aatttatgaa ttttacttgc accttatagt tcatagcaat taactgattt 2820

gtagtgattc attgtttgtt ttatatacca atgacttcca tattttaaaa gagaaaaaca 2880

actttatgtt gcaggaaacc ctttttgtaa gtctttatta tttactttgc attttgtttc 2940

actctttcca gataagcaga gttgctcttc accagtgttt ttcttcatgt gcaaagtgac 3000

tatttgttct ataatacttt tatgtgtgtt atatcaaatg tgtcttaagc ttcatgcaaa 3060

ctcagtcatc agttcgtgtt gtctgaagca agtgggagat atataaatac ccagtagcta 3120

aaatggtcag tctttttttag atgttttcct acttagtatc tcctaataac gttttgctgt 3180

gtcactagat gttcatttca caagtgcatg tctttctaat aatccacaca tttcatgctc 3240

taataatcca cacatttcat gctcattttt attgttttta cagccagtta tagtaagaaa 3300

aaggtttttc cccttgtgct gctttataat ttagcgtgtg tctgaacctt atccatgttt 3360

gctagatgag gtcttgtcaa atatatcact accattgtca ccggtgaaaa gaaacaggta 3420

gttaagttag ggttaacatt catttcaacc acgaggttgt atatcatgac tagcttttac 3480

tcttggttta cagagaaaag ttaaacagcc aactaggcag tttttaagaa tattaacaat 3540

atattaacaa acaccaatac aactaatcct atttggtttt aatgatttca ccatgggatt 3600

aagaactata tcaggaacat ccctgagaaa cggttttaag tgtagcaact actcttcctt 3660

aatggacagc cacataacgt gtaggaagtc ctttatcact tatcctcgat ccataagcat 3720

atcttgcaga ggggaactac ttctttaaac acatggaggg aaagaagatg atgccactgg 3780

caccagaggg ttagtactgt gatgcatcct aaaatattta ttatattggt aaaaattctg 3840

gttaaataaa aaattagaga tcactcttgg ctgatttcag caccaggaac tgtattacag 3900
ttttagagat taattcctag tgtttacctg attatagcag ttggcatcat ggggcattta 3960
attctgactt tatccccacg tcagccttaa taaagtcttc tttaccttct ctatgaagac 4020
tttaaagccc aaataatcat ttttcacatt gatattcaag aattgagata gatagaagcc 4080
aaagtgggta tctgacaagt ggaaaatcaa acgtttaaga agaattacaa ctctgaaaag 4140
catttatatg tggaacttct caaggagcct cctggggact ggaaagtaag tcatcagcca 4200
ggcaaatgac tcatgctgaa gagagtcccc atttcagtcc cctgagatct agctgatgct 4260
tagatccttt gaaataaaaa ttatgtcttt ataactctga tcttttacat aaagcagaag 4320
aggaatcaac tagttaattg caaggtttct actctgtttc ctctgtaaag atcagatggt 4380
aatctttcaa ataagaaaaa aataaagacg tatgtttgac caagtagttt cacaagaata 4440
tttgggaact tgtttctttt aattttattt gtccctgagt gaagtctaga aagaaaggta 4500
aagagtctag agtttattcc tctttccaaa acattctcat tcctctcctc cctacactta 4560
gtatttcccc cacagagtgc ctagaatctt aataatgaat aaaataaaaa gcagcaatat 4620
gtcattaaca aatccagacc tgaaagggta aagggtttat aactgcacta ataaagagag 4680
gctctttttt tttcttccag tttgttggtt tttaatggta ccgtgttgta aagataccca 4740
ctaatggaca atcaaattgc agaaaaggct caatatccaa gagacaggga ctaatgcact 4800
gtacaatctg cttatccttg cccttctctc ttgccaaagt gtgcttcaga aatatatact 4860
gctttaaaaa agaataaaag aatatccttt tacaagtggc tttacatttc ctaaaatgcc 4920
ataagaaaat gcaatatctg ggtactgtat ggggaaaaaa atgtccaagt ttgtgtaaaa 4980
ccagtgcatt tcagcttgca agttactgaa cacaataatg ctgttttaat tttgttttat 5040
atcagttaaa attcacaata atgtagatag aacaaattac agacaaggaa agaaaaaact 5100
tgaatgaaat ggattttaca gaaagcttta tgataatttt tgaatgcatt atttattttt 5160
tgtgccatgc attttttttc tcaccaaatg accttacctg taatacagtc ttgtttgtct 5220
gtttacaacc atgtatttat tgcaatgtac atactgtaat gttaattgta aattatctgt 5280
tcttattaaa acatcatccc atgatgggat ggtgttgata tatttggaaa ctcttggtga 5340
gagaatgaat ggtgtgtata catactctgt acatttttct tttctcctgt aatatagtct 5400
tgtcacctta gagcttgttt atggaagatt caagaaaact ataaaatact taaagatata 5460
taaatttaaa aaaacatagc tgcaggtctt tggtcccagg gctgtgcctt aactttaacc 5520
aatattttct tctgttttgc tgcatttgaa aggtaacagt ggagctaggg ctgggcattt 5580
tacatccagg cttttaattg attagaattc tgccaatagg tggattttac aaaaccacag 5640
acaacctctg aaagattctg agaccctttt gagacagaag ctcttaagta cttcttgcca 5700

gggagcagca ctgcatgtgt gatggttgtt tgccatctgt tgatcaggaa ctacttcagc 5760
tacttgcatt tgattatttc cttttttttt ttttttaact cggaaacaca actggggaaa 5820
tatattcttt cccagtgatt ataaacaatc tttttctttt ttttaagtcc ttttggcttc 5880
tagagctcat aggaaaatgg acttgatttg aaattggagc cagagtttac tcgtgttggt 5940
tatctattca tcagcttcct gacatgttaa gagaatacat taaagagaaa atactgtttt 6000
ttaatcctaa aatttttctt ccactaagat aaaccaaatg tccttacata tatgtaaacc 6060
catctattta aacgcaaagg tgggttgatg tcagtttaca tagcagaaag cattcactat 6120
cctctaagat ttgtttctgc aaaactttca ttgctttaga attttaaaat ttcaccttgt 6180
acaatggcca gcccctaaag caggaaacat ttataatgga ttatatggaa acatcctccc 6240
agtacttgcc cagcccttga atcatgtggc ttttcagtga aaggaaagat tctttttcta 6300
ggaaaaatga gcctatttta ttttatttta ttttattttt tgacacaaac tgtagatttt 6360
agcagccctg gcccaaagga atttgattac ttttgtttta aacagtacaa aggggacact 6420
ataattacaa aaacatcctt aactgatttg agttgttttt atttctttgg atatattttc 6480
agagtggtaa attgtgtgtg agaattacaa atgattattc ttttagtggt ttcttagcct 6540
ctcttacagc ccacggggat agtactgtac atcaatacct tcatatgaaa tttttatatg 6600
caatgaaaat aaaagcatgg gttgattctg cctatttatg actcaatctt ttacaaataa 6660
aagattattc attttaaatt atagttcaat cagcatgtct cttaggatac tgaacgtggt 6720
tgaaatgaaa ggatagtgac atcataagtt agtactgata ttcataacca aataaagcca 6780
acttgagtaa ttttgctaca ttaaaaatta ccaaaattac ttagatggcc tataagatta 6840
agcatggtgt tttctaagca agctttgaaa ggggccttcc atacttactt aattgaatat 6900
tctgggatat tgaaaattat tcagatactt gacaattatt tttggttacc tactccgcaa 6960
actacaaagt tttaaggact caacaataag ttaatgagac acagtgtttg ctttcatgga 7020
gcttacagtc tggaggggac aaaggcttaa acaatactca tataattata tatgtgatca 7080
gtacaatgaa ggagctcagt ggggtaaata agcaggaacc tgaacttgat ctgttccgga 7140
gggccacaga aggcttcctt gaggccttga gaaagtgatt tgcatctgag ttctgaagga 7200
ttgtaagagg taactaggga aaaagttgac aggaagagga aggggatcca gacaagaaac 7260
atttgcaaag atcttgaggc ataaatgagc ttgagacatc tggagaaact gaggaaaagt 7320
gagagagtag gcagggcctg gagccgcaga gccattgcta accatcctgt gtgagatatc 7380
ccccattctg tagctttatt ctcataaccc tgctcaattt tctttataac acttctcaca 7440
gatttatata cgtgtttgtt tttgttatct gtctctccca ccagaccaca gctccatgag 7500
agcaaggtct ttgcttacca atatatcact agcacttaaa actatgcctg gtacacagta 7560
ggttcttaat atgtgttgaa tatagccatc aaattgatat tggatataat tcaatctgat 7620

aagatatttt gagatattaa agagttttta acttgatacc ataaaaa 7667

<210> 40
<211> 8379
<212> DNA
<213> Homo sapiens

<400> 40
cactgccctg cggtgttttg aactgccttc ttacagacgt catacagccc ttgaggaata 60

gtttctgcct ggtgagattg aatgatagtt ctcattcaca aaaccctgga ttctaagcag 120

ggacacacag aaattacttt cgcaggtaaa tcagcccacc cagccaaagt gtggagagat 180

ttgttccttg gctgacttct ttgctccacg gagaggagtg ttttcctgtg cttgccctga 240

aatggaactt ccttgacagc tctcccgtgt tacagtacct cccggtcatt ttctttttct 300

ctctctctac ctgcgctctt cgagtgtcag aaacctttaa agctgttact atggaattgc 360

aaaaaagaga tcaagtgact ctttcactat gctggtttcc cttgtgaccc agatgaagaa 420

tcaattcaga attcagttcc tcccttggca ttgcaagaca cagaagaaac tgtcacttcc 480

taacagccta gtactggagt aaattcagta tgaaggaaga aagcgctcct gcgtgttaga 540

accttgccca tgagctggac cgaggacagg agatggactc caggaaaatt ggatttcttc 600

aagcagcctc ccttggaaat ggaatatctt taaaatcttc tttgcagaaa gacagttaga 660

atgtattaat cagaatagtt gaagacttat tttccttttt atttttttc aaaatgagca 720

ttattatgaa gccaagatcc cgatctacaa gttccctaag gactgcagag gcagtttgtt 780

ttgatgtgga caatggcaca tctgcgggac ggagtccctt ggatcccatg accagcccag 840

gatccgggct aattctccaa gcaaattttg tccacagtca acgacgggag tccttcctgt 900

atcgatccga cagcgattat gacctctctc caaagtctat gtcccggaac tcctccattg 960

ccagtgatat acacggagat gacttgattg tgactccatt tgctcaggtc ttggccagtc 1020

tgcgaactgt acgaaacaac tttgctgcat taactaattt gcaagatcga gcacctagca 1080

aaagatcacc catgtgcaac caaccatcca tcaacaaagc caccataaca gaggaggcct 1140

accagaaact ggccagcgag accctggagg agctggactg gtgtctggac cagctagaga 1200

ccctacagac caggcactcc gtcagtgaga tggcctccaa caagtttaaa aggatgctta 1260

atcgggagct cacccatctc tctgaaatga gtcggtctgg aaatcaagtg tcagagttta 1320

tatcaaacac attcttagat aagcaacatg aagtggaaat tccttctcca actcagaagg 1380

aaaaggagaa aaagaaaaga ccaatgtctc agatcagtgg agtcaagaaa ttgatgcaca 1440

gctctagtct gactaattca agtatcccaa ggtttggagt taaaactgaa caagaagatg 1500

tccttgccaa ggaactagaa gatgtgaaca aatggggtct tcatgttttc agaatagcag 1560

agttgtctgg taaccggccc ttgactgtta tcatgcacac cattttttcag gaacgggatt 1620

tattaaaaac atttaaaatt ccagtagata ctttaattac atatcttatg actctcgaag 1680

accattacca tgctgatgtg gcctatcaca acaatatcca tgctgcagat gttgtccagt 1740

ctactcatgt gctattatct acacctgctt tggaggctgt gtttacagat ttggagattc 1800

ttgcagcaat ttttgccagt gcaatacatg atgtagatca tcctggtgtg tccaatcaat 1860

ttctgatcaa tacaaactct gaacttgcct tgatgtacaa tgattcctca gtcttagaga 1920

accatcattt ggctgtgggc tttaaattgc ttcaggaaga aaactgtgac attttccaga 1980

atttgaccaa aaaacaaaga caatctttaa ggaaaatggt cattgacatc gtacttgcaa 2040

cagatatgtc aaaacacatg aatctactgg ctgatttgaa gactatggtt gaaactaaga 2100

aagtgacaag ctctggagtt cttcttcttg ataattattc cgataggatt caggttcttc 2160

agaatatggt gcactgtgca gatctgagca acccaacaaa gcctctccag ctgtaccgcc 2220

agtggacgga ccggataatg gaggagttct tccgccaagg agaccgagag agggaacgtg 2280

gcatggagat aagccccatg tgtgacaagc acaatgcttc cgtggaaaaa tcacaggtgg 2340

gcttcataga ctatattgtt catcccctct gggagacatg ggcagacctc gtccaccctg 2400

acgcccagga tattttggac actttggagg acaatcgtga atggtaccag agcacaatcc 2460

ctcagagccc ctctcctgca cctgatgacc cagaggaggg ccggcagggt caaactgaga 2520

aattccagtt tgaactaact ttagaggaag atggtgagtc agacacggaa aaggacagtg 2580

gcagtcaagt ggaagaagac actagctgca gtgactccaa gactctttgt actcaagact 2640

cagagtctac tgaaattccc cttgatgaac aggttgaaga ggaggcagta ggggaagaag 2700

aggaaagcca gcctgaagcc tgtgtcatag atgatcgttc tcctgacacg taacagtgca 2760

aaaactttca tgcctttttt tttttaagt agaaaaattg tttccaaagt gcatgtcaca 2820

tgccacaacc acggtcacac ctcactgtca tctgccagga cgtttgttga acaaaactga 2880

ccttgactac tcagtccagc gctcaggaat atcgtaacca gtttttcac ctccatgtca 2940

tccgagcaag gtggacatct tcacgaacag cgtttttaac aagatttcag cttggtagag 3000

ctgacaaagc agataaaatc tactccaaat tattttcaag agagtgtgac tcatcaggca 3060

gcccaaaagt ttattggact tggggtttct attccttttt atttgtttgc aatattttca 3120

gaagaaaggc attgcacaga gtgaacttaa tggacgaagc aacaaatatg tcaagaacag 3180

gacatagcac gaatctgtta ccagtaggag gaggatgagc cacagaaatt gcataatttt 3240

ctaatttcaa gtcttcctga tacatgactg aatagtgtgg ttcagtgagc tgcactgacc 3300

tctacatttt gtatgatatg taaaacagat tttttgtaga gcttactttt attattaaat 3360

gtattgaggt attatattta aaaaaaacta tgttcagaac ttcatctgcc actggttatt 3420

tttttctaag gagtaacttg caagttttca gtacaaatct gtgctacact ggataaaaat 3480

ctaatttatg aattttactt gcaccttata gttcatagca attaactgat ttgtagtgat 3540

tcattgtttg ttttatatac caatgacttc catattttaa aagagaaaaa caactttatg 3600

ttgcaggaaa cccttttttgt aagtctttat tatttacttt gcattttgtt tcactctttc 3660

cagataagca gagttgctct tcaccagtgt ttttcttcat gtgcaaagtg actatttgtt 3720

ctataatact tttatgtgtg ttatatcaaa tgtgtcttaa gcttcatgca aactcagtca 3780

tcagttcgtg ttgtctgaag caagtgggag atatataaat acccagtagc taaaatggtc 3840

agtctttttt agatgttttc ctacttagta tctcctaata acgttttgct gtgtcactag 3900

atgttcattt cacaagtgca tgtctttcta ataatccaca catttcatgc tctaataatc 3960

cacacatttc atgctcattt ttattgtttt tacagccagt tatagtaaga aaaaggtttt 4020

tccccttgtg ctgctttata atttagcgtg tgtctgaacc ttatccatgt ttgctagatg 4080

aggtcttgtc aaatatatca ctaccattgt caccggtgaa aagaaacagg tagttaagtt 4140

agggttaaca ttcatttcaa ccacgaggtt gtatatcatg actagctttt actcttggtt 4200

tacagagaaa agttaaacag ccaactaggc agtttttaag aatattaaca atatattaac 4260

aaacaccaat acaactaatc ctatttggtt ttaatgattt caccatggga ttaagaacta 4320

tatcaggaac atccctgaga aacggtttta agtgtagcaa ctactcttcc ttaatggaca 4380

gccacataac gtgtaggaag tcctttatca cttatcctcg atccataagc atatcttgca 4440

gaggggaact acttctttaa acacatggag ggaaagaaga tgatgccact ggcaccagag 4500

ggttagtact gtgatgcatc ctaaaatatt tattatattg gtaaaaattc tggttaaata 4560

aaaaattaga gatcactctt ggctgatttc agcaccagga actgtattac agttttagag 4620

attaattcct agtgtttacc tgattatagc agttggcatc atggggcatt taattctgac 4680

tttatcccca cgtcagcctt aataaagtct tctttacctt ctctatgaag actttaaagc 4740

ccaaataatc atttttcaca ttgatattca agaattgaga tagatagaag ccaaagtggg 4800

tatctgacaa gtggaaaatc aaacgtttaa gaagaattac aactctgaaa agcatttata 4860

tgtggaactt ctcaaggagc ctcctgggga ctggaaagta agtcatcagc caggcaaatg 4920

actcatgctg aagagagtcc ccatttcagt cccctgagat ctagctgatg cttagatcct 4980

ttgaaataaa aattatgtct ttataactct gatcttttac ataaagcaga agaggaatca 5040

actagttaat tgcaaggttt ctactctgtt tcctctgtaa agatcagatg gtaatctttc 5100

aaataagaaa aaaataaaga cgtatgtttg accaagtagt ttcacaagaa tatttgggaa 5160

cttgtttctt ttaattttat ttgtccctga gtgaagtcta gaaagaaagg taaagagtct 5220

agagtttatt cctctttcca aaacattctc attcctctcc tccctacact tagtatttcc 5280

cccacagagt gcctagaatc ttaataatga ataaaataaa aagcagcaat atgtcattaa 5340

caaatccaga cctgaaaggg taaagggttt ataactgcac taataaagag aggctctttt 5400

tttttcttcc agtttgttgg tttttaatgg taccgtgttg taaagatacc cactaatgga 5460
caatcaaatt gcagaaaagg ctcaatatcc aagagacagg gactaatgca ctgtacaatc 5520
tgcttatcct tgcccttctc tcttgccaaa gtgtgcttca gaaatatata ctgctttaaa 5580
aaagaataaa agaatatcct tttacaagtg gctttacatt tcctaaaatg ccataagaaa 5640
atgcaatatc tgggtactgt atggggaaaa aaatgtccaa gtttgtgtaa aaccagtgca 5700
tttcagcttg caagttactg aacacaataa tgctgtttta attttgtttt atatcagtta 5760
aaattcacaa taatgtagat agaacaaatt acagacaagg aaagaaaaaa cttgaatgaa 5820
atggatttta cagaaagctt tatgataatt tttgaatgca ttatttattt tttgtgccat 5880
gcattttttt tctcaccaaa tgaccttacc tgtaatacag tcttgtttgt ctgtttacaa 5940
ccatgtattt attgcaatgt acatactgta atgttaattg taaattatct gttcttatta 6000
aaacatcatc ccatgatggg atggtgttga tatatttgga aactcttggt gagagaatga 6060
atggtgtgta tacatactct gtacattttt cttttctcct gtaatatagt cttgtcacct 6120
tagagcttgt ttatggaaga ttcaagaaaa ctataaaata cttaaagata tataaattta 6180
aaaaaacata gctgcaggtc tttggtccca gggctgtgcc ttaactttaa ccaatatttt 6240
cttctgtttt gctgcatttg aaaggtaaca gtggagctag ggctgggcat tttacatcca 6300
ggcttttaat tgattagaat tctgccaata ggtggatttt acaaaaccac agacaacctc 6360
tgaaagattc tgagaccctt ttgagacaga agctcttaag tacttcttgc cagggagcag 6420
cactgcatgt gtgatggttg tttgccatct gttgatcagg aactacttca gctacttgca 6480
tttgattatt tccttttttt ttttttttaa ctcggaaaca caactgggga aatatattct 6540
ttcccagtga ttataaacaa tctttttctt ttttttaagt ccttttggct tctagagctc 6600
ataggaaaat ggacttgatt tgaaattgga gccagagttt actcgtgttg gttatctatt 6660
catcagcttc ctgacatgtt aagagaatac attaaagaga aaatactgtt ttttaatcct 6720
aaaatttttc ttccactaag ataaaccaaa tgtccttaca tatatgtaaa cccatctatt 6780
taaacgcaaa ggtgggttga tgtcagttta catagcagaa agcattcact atcctctaag 6840
atttgtttct gcaaaacttt cattgcttta gaattttaaa atttcacctt gtacaatggc 6900
cagcccctaa agcaggaaac atttataatg gattatatgg aaacatcctc ccagtacttg 6960
cccagccctt gaatcatgtg gcttttcagt gaaaggaaag attctttttc taggaaaaat 7020
gagcctattt tattttattt tattttattt tttgacacaa actgtagatt ttagcagccc 7080
tggcccaaag gaatttgatt acttttgttt taaacagtac aaaggggaca ctataattac 7140
aaaaacatcc ttaactgatt tgagttgttt ttatttcttt ggatatattt tcagagtggt 7200
aaattgtgtg tgagaattac aaatgattat tcttttagtg gtttcttagc ctctcttaca 7260

gcccacgggg atagtactgt acatcaatac cttcatatga aatttttata tgcaatgaaa 7320

ataaaagcat gggttgattc tgcctattta tgactcaatc ttttacaaat aaaagattat 7380

tcattttaaa ttatagttca atcagcatgt ctcttaggat actgaacgtg gttgaaatga 7440

aaggatagtg acatcataag ttagtactga tattcataac caaataaagc caacttgagt 7500

aattttgcta cattaaaaat taccaaaatt acttagatgg cctataagat taagcatggt 7560

gttttctaag caagctttga aaggggcctt ccatacttac ttaattgaat attctgggat 7620

attgaaaatt attcagatac ttgacaatta tttttggtta cctactccgc aaactacaaa 7680

gttttaagga ctcaacaata agttaatgag acacagtgtt tgctttcatg gagcttacag 7740

tctggagggg acaaaggctt aaacaatact catataatta tatatgtgat cagtacaatg 7800

aaggagctca gtggggtaaa taagcaggaa cctgaacttg atctgttccg gagggccaca 7860

gaaggcttcc ttgaggcctt gagaaagtga tttgcatctg agttctgaag gattgtaaga 7920

ggtaactagg gaaaaagttg acaggaagag gaaggggatc cagacaagaa acatttgcaa 7980

agatcttgag gcataaatga gcttgagaca tctggagaaa ctgaggaaaa gtgagagagt 8040

aggcagggcc tggagccgca gagccattgc taaccatcct gtgtgagata tcccccattc 8100

tgtagcttta ttctcataac cctgctcaat ttctttata acacttctca cagatttata 8160

tacgtgtttg tttttgttat ctgtctctcc caccagacca cagctccatg agagcaaggt 8220

ctttgcttac caatatatca ctagcactta aaactatgcc tggtacacag taggttctta 8280

atatgtgttg aatatagcca tcaaattgat attggatata attcaatctg ataagatatt 8340

ttgagatatt aaagagtttt taacttgata ccataaaaa 8379

<210> 41
<211> 7545
<212> DNA
<213> Homo sapiens

<400> 41
ctttcttgca actaagcatc ttgacataca ttattcatta agccctggag ctcgggagag 60

aaagatgcag acccttagat ctttagatat tcctttatca cgtggatttt ctttattcag 120

aatagttgct gaattttgtg ccattctgga gtcttacaaa tggcatgtat tcgatgggaa 180

gacggctgga tgggatttaa tgcgaggctt tcttatgtat acttaattac caaaaatctt 240

taaaaactca tactctgcgt ggcttgtgga ggttgttaaa gtgtcgagat tttgaagcta 300

aatacatttt agagcttact atatatatac atatatatat atatacatat aatcaatcaa 360

aaatgcctga agcaaactat ttactgtcag tgtcttgggg ctacataaag tttaaaagga 420

tgcttaatcg ggagctcacc catctctctg aaatgagtcg gtctggaaat caagtgtcag 480

agtttatatc aaacacattc ttagataagc aacatgaagt ggaaattcct tctccaactc 540

```
agaaggaaaa ggagaaaaag aaaagaccaa tgtctcagat cagtggagtc aagaaattga      600
tgcacagctc tagtctgact aattcaagta tcccaaggtt tggagttaaa actgaacaag      660
aagatgtcct tgccaaggaa ctagaagatg tgaacaaatg gggtcttcat gttttcagaa      720
tagcagagtt gtctggtaac cggcccttga ctgttatcat gcacaccatt tttcaggaac      780
gggatttatt aaaaacattt aaaattccag tagatacttt aattacatat cttatgactc      840
tcgaagacca ttaccatgct gatgtggcct atcacaacaa tatccatgct gcagatgttg      900
tccagtctac tcatgtgcta ttatctacac ctgctttgga ggctgtgttt acagatttgg      960
agattcttgc agcaattttt gccagtgcaa tacatgatgt agatcatcct ggtgtgtcca     1020
atcaatttct gatcaataca aactctgaac ttgccttgat gtacaatgat tcctcagtct     1080
tagagaacca tcatttggct gtgggcttta aattgcttca ggaagaaaac tgtgacattt     1140
tccagaattt gaccaaaaaa caaagacaat ctttaaggaa aatggtcatt gacatcgtac     1200
ttgcaacaga tatgtcaaaa cacatgaatc tactggctga tttgaagact atggttgaaa     1260
ctaagaaagt gacaagctct ggagttcttc ttcttgataa ttattccgat aggattcagg     1320
ttcttcagaa tatggtgcac tgtgcagatc tgagcaaccc aacaaagcct ctccagctgt     1380
accgccagtg gacggaccgg ataatggagg agttcttccg ccaaggagac cgagagaggg     1440
aacgtggcat ggagataagc cccatgtgtg acaagcacaa tgcttccgtg gaaaaatcac     1500
aggtgggctt catagactat attgttcatc ccctctggga gacatgggca gacctcgtcc     1560
accctgacgc ccaggatatt ttggacactt tggaggacaa tcgtgaatgg taccagagca     1620
caatccctca gagcccctct cctgcacctg atgacccaga ggagggccgg cagggtcaaa     1680
ctgagaaatt ccagtttgaa ctaactttag aggaagatgg tgagtcagac acggaaaagg     1740
acagtggcag tcaagtggaa gaagacacta gctgcagtga ctccaagact ctttgtactc     1800
aagactcaga gtctactgaa attccccttg atgaacaggt tgaagaggag gcagtagggg     1860
aagaagagga aagccagcct gaagcctgtg tcatagatga tcgttctcct gacacgtaac     1920
agtgcaaaaa ctttcatgcc tttttttttt ttaagtagaa aaattgtttc caaagtgcat     1980
gtcacatgcc acaaccacgg tcacacctca ctgtcatctg ccaggacgtt tgttgaacaa     2040
aactgacctt gactactcag tccagcgctc aggaatatcg taaccagttt tttcacctcc     2100
atgtcatccg agcaaggtgg acatcttcac gaacagcgtt tttaacaaga tttcagcttg     2160
gtagagctga caaagcagat aaaatctact ccaaattatt ttcaagagag tgtgactcat     2220
caggcagccc aaaagtttat tggacttggg gtttctattc ctttttattt gtttgcaata     2280
ttttcagaag aaaggcattg cacagagtga acttaatgga cgaagcaaca aatatgtcaa     2340
gaacaggaca tagcacgaat ctgttaccag taggaggagg atgagccaca gaaattgcat     2400
aattttctaa tttcaagtct tcctgataca tgactgaata gtgtggttca gtgagctgca     2460
```

ctgacctcta cattttgtat gatatgtaaa acagattttt tgtagagctt acttttatta 2520
ttaaatgtat tgaggtatta tatttaaaaa aaactatgtt cagaacttca tctgccactg 2580
gttatttttt tctaaggagt aacttgcaag ttttcagtac aaatctgtgc tacactggat 2640
aaaaatctaa tttatgaatt ttacttgcac cttatagttc atagcaatta actgatttgt 2700
agtgattcat tgtttgtttt atataccaat gacttccata ttttaaaaga gaaaaacaac 2760
tttatgttgc aggaaaccct ttttgtaagt ctttattatt tactttgcat tttgtttcac 2820
tctttccaga taagcagagt tgctcttcac cagtgttttt cttcatgtgc aaagtgacta 2880
tttgttctat aatactttta tgtgtgttat atcaaatgtg tcttaagctt catgcaaact 2940
cagtcatcag ttcgtgttgt ctgaagcaag tgggagatat ataaataccc agtagctaaa 3000
atggtcagtc ttttttagat gttttcctac ttagtatctc ctaataacgt tttgctgtgt 3060
cactagatgt tcatttcaca agtgcatgtc tttctaataa tccacacatt tcatgctcta 3120
ataatccaca catttcatgc tcatttttat tgtttttaca gccagttata gtaagaaaaa 3180
ggtttttccc cttgtgctgc tttataattt agcgtgtgtc tgaaccttat ccatgtttgc 3240
tagatgaggt cttgtcaaat atatcactac cattgtcacc ggtgaaaaga aacaggtagt 3300
taagttaggg ttaacattca tttcaaccac gaggttgtat atcatgacta gcttttactc 3360
ttggtttaca gagaaaagtt aaacagccaa ctaggcagtt tttaagaata ttaacaatat 3420
attaacaaac accaatacaa ctaatcctat ttggttttaa tgatttcacc atgggattaa 3480
gaactatatc aggaacatcc ctgagaaacg gttttaagtg tagcaactac tcttccttaa 3540
tggacagcca cataacgtgt aggaagtcct ttatcactta tcctcgatcc ataagcatat 3600
cttgcagagg ggaactactt ctttaaacac atggagggaa agaagatgat gccactggca 3660
ccagagggtt agtactgtga tgcatcctaa aatatttatt atattggtaa aaattctggt 3720
taaataaaaa attagagatc actcttggct gatttcagca ccaggaactg tattacagtt 3780
ttagagatta attcctagtg tttacctgat tatagcagtt ggcatcatgg ggcatttaat 3840
tctgacttta tccccacgtc agccttaata aagtcttctt taccttctct atgaagactt 3900
taaagcccaa ataatcattt ttcacattga tattcaagaa ttgagataga tagaagccaa 3960
agtgggtatc tgacaagtgg aaaatcaaac gtttaagaag aattacaact ctgaaaagca 4020
tttatatgtg gaacttctca aggagcctcc tggggactgg aaagtaagtc atcagccagg 4080
caaatgactc atgctgaaga gagtccccat ttcagtcccc tgagatctag ctgatgctta 4140
gatcctttga aataaaaatt atgtctttat aactctgatc ttttacataa agcagaagag 4200
gaatcaacta gttaattgca aggtttctac tctgtttcct ctgtaaagat cagatggtaa 4260
tctttcaaat aagaaaaaaa taaagacgta tgtttgacca agtagtttca caagaatatt 4320

tgggaacttg tttcttttaa ttttatttgt ccctgagtga agtctagaaa gaaaggtaaa 4380
gagtctagag tttattcctc tttccaaaac attctcattc ctctcctccc tacacttagt 4440
atttccccca cagagtgcct agaatcttaa taatgaataa aataaaaagc agcaatatgt 4500
cattaacaaa tccagacctg aaagggtaaa gggtttataa ctgcactaat aaagagaggc 4560
tctttttttt tcttccagtt tgttggtttt taatggtacc gtgttgtaaa gatacccact 4620
aatggacaat caaattgcag aaaaggctca atatccaaga gacagggact aatgcactgt 4680
acaatctgct tatccttgcc cttctctctt gccaaagtgt gcttcagaaa tatatactgc 4740
tttaaaaaag aataaaagaa tatcctttta caagtggctt tacatttcct aaaatgccat 4800
aagaaaatgc aatatctggg tactgtatgg ggaaaaaaat gtccaagttt gtgtaaaacc 4860
agtgcatttc agcttgcaag ttactgaaca caataatgct gttttaattt tgttttatat 4920
cagttaaaat tcacaataat gtagatagaa caaattacag acaaggaaag aaaaaacttg 4980
aatgaaatgg attttacaga aagctttatg ataatttttg aatgcattat ttattttttg 5040
tgccatgcat ttttttctc accaaatgac cttacctgta atacagtctt gtttgtctgt 5100
ttacaaccat gtatttattg caatgtacat actgtaatgt taattgtaaa ttatctgttc 5160
ttattaaaac atcatcccat gatgggatgg tgttgatata tttggaaact cttggtgaga 5220
gaatgaatgg tgtgtataca tactctgtac atttttcttt tctcctgtaa tatagtcttg 5280
tcaccttaga gcttgtttat ggaagattca agaaaactat aaaatactta aagatatata 5340
aatttaaaaa aacatagctg caggtctttg gtcccagggc tgtgccttaa ctttaaccaa 5400
tattttcttc tgttttgctg catttgaaag gtaacagtgg agctagggct gggcatttta 5460
catccaggct tttaattgat tagaattctg ccaataggtg gattttacaa aaccacagac 5520
aacctctgaa agattctgag accctttga gacagaagct cttaagtact tcttgccagg 5580
gagcagcact gcatgtgtga tggttgtttg ccatctgttg atcaggaact acttcagcta 5640
cttgcatttg attatttcct tttttttttt ttttaactcg gaaacacaac tggggaaata 5700
tattctttcc cagtgattat aaacaatctt tttcttttt ttaagtcctt ttggcttcta 5760
gagctcatag gaaaatggac ttgatttgaa attggagcca gagtttactc gtgttggtta 5820
tctattcatc agcttcctga catgttaaga gaatacatta aagagaaaat actgtttttt 5880
aatcctaaaa tttttcttcc actaagataa accaaatgtc cttacatata tgtaaaccca 5940
tctatttaaa cgcaaaggtg ggttgatgtc agtttacata gcagaaagca ttcactatcc 6000
tctaagattt gtttctgcaa aactttcatt gctttagaat tttaaaattt caccttgtac 6060
aatggccagc ccctaaagca ggaaacattt ataatggatt atatggaaac atcctcccag 6120
tacttgccca gcccttgaat catgtggctt ttcagtgaaa ggaaagattc tttttctagg 6180
aaaaatgagc ctattttatt ttattttatt ttattttttg acacaaactg tagattttag 6240

cagccctggc ccaaaggaat ttgattactt ttgttttaaa cagtacaaag gggacactat 6300

aattacaaaa acatccttaa ctgatttgag ttgtttttat ttctttggat atattttcag 6360

agtggtaaat tgtgtgtgag aattacaaat gattattctt ttagtggttt cttagcctct 6420

cttacagccc acggggatag tactgtacat caataccttc atatgaaatt tttatatgca 6480

atgaaaataa aagcatgggt tgattctgcc tatttatgac tcaatctttt acaaataaaa 6540

gattattcat tttaaattat agttcaatca gcatgtctct taggatactg aacgtggttg 6600

aaatgaaagg atagtgacat cataagttag tactgatatt cataaccaaa taaagccaac 6660

ttgagtaatt ttgctacatt aaaaattacc aaaattactt agatggccta taagattaag 6720

catggtgttt tctaagcaag ctttgaaagg ggccttccat acttacttaa ttgaatattc 6780

tgggatattg aaaattattc agatacttga caattatttt tggttaccta ctccgcaaac 6840

tacaaagttt taaggactca acaataagtt aatgagacac agtgtttgct ttcatggagc 6900

ttacagtctg gaggggacaa aggcttaaac aatactcata taattatata tgtgatcagt 6960

acaatgaagg agctcagtgg ggtaaataag caggaacctg aacttgatct gttccggagg 7020

gccacagaag gcttccttga ggccttgaga aagtgatttg catctgagtt ctgaaggatt 7080

gtaagaggta actagggaaa aagttgacag gaagaggaag gggatccaga caagaaacat 7140

ttgcaaagat cttgaggcat aaatgagctt gagacatctg gagaaactga ggaaaagtga 7200

gagagtaggc agggcctgga gccgcagagc cattgctaac catcctgtgt gagatatccc 7260

ccattctgta gctttattct cataaccctg ctcaattttc tttataacac ttctcacaga 7320

tttatatacg tgtttgtttt tgttatctgt ctctcccacc agaccacagc tccatgagag 7380

caaggtcttt gcttaccaat atatcactag cacttaaaac tatgcctggt acacagtagg 7440

ttcttaatat gtgttgaata tagccatcaa attgatattg gatataattc aatctgataa 7500

gatattttga gatattaaag agtttttaac ttgataccat aaaaa 7545

<210> 42
<211> 8130
<212> DNA
<213> Homo sapiens

<400> 42
agattatagc ccagcgtacg agaagcacga gtcctatagt tggcgtaccc tgaggcctgc 60

cagttcctgc cttaatgcat atgtagtcgt aattgagttc tgacacggcc ttggatgttt 120

ctgtcctaaa tagctgacat tgcatcttca agactgtcat tccagttggc ttttgagtgg 180

atacgtgcag tgagatcatt gacactggaa acactagttc ccattttaat tacttaaaac 240

accacgatga aaagaaatac ctgtgatttg ctttctcgga gcaaaagtgc ctctgaggaa 300

acactacatt ccagtaatga agaggaagac cctttccgcg gaatggaacc ctatcttgtc 360

cggagacttt catgtcgcaa tattcagctt ccccctctcg ccttcagaca gttggaacaa 420
gctgacttga aaagtgaatc agagaacatt caacgaccaa ccagcctccc cctgaagatt 480
ctgccgctga ttgctatcac ttctgcagaa tccagtggtt ttgatgtgga caatggcaca 540
tctgcgggac ggagtccctt ggatcccatg accagcccag gatccgggct aattctccaa 600
gcaaattttg tccacagtca acgacgggag tccttcctgt atcgatccga cagcgattat 660
gacctctctc caaagtctat gtcccggaac tcctccattg ccagtgatat acacggagat 720
gacttgattg tgactccatt tgctcaggtc ttggccagtc tgcgaactgt acgaaacaac 780
tttgctgcat taactaattt gcaagatcga gcacctagca aaagatcacc catgtgcaac 840
caaccatcca tcaacaaagc caccataaca gaggaggcct accagaaact ggccagcgag 900
accctggagg agctggactg gtgtctggac cagctagaga ccctacagac caggcactcc 960
gtcagtgaga tggcctccaa caagtttaaa aggatgctta atcgggagct cacccatctc 1020
tctgaaatga gtcggtctgg aaatcaagtg tcagagttta tatcaaacac attcttagat 1080
aagcaacatg aagtggaaat tccttctcca actcagaagg aaaaggagaa aaagaaaaga 1140
ccaatgtctc agatcagtgg agtcaagaaa ttgatgcaca gctctagtct gactaattca 1200
agtatcccaa ggtttggagt taaaactgaa caagaagatg tccttgccaa ggaactagaa 1260
gatgtgaaca aatggggtct tcatgttttc agaatagcag agttgtctgg taaccggccc 1320
ttgactgtta tcatgcacac catttttcag gaacgggatt tattaaaaac atttaaaatt 1380
ccagtagata ctttaattac atatcttatg actctcgaag accattacca tgctgatgtg 1440
gcctatcaca acaatatcca tgctgcagat gttgtccagt ctactcatgt gctattatct 1500
acacctgctt tggaggctgt gtttacagat ttggagattc ttgcagcaat tttgccagt 1560
gcaatacatg atgtagatca tcctggtgtg tccaatcaat ttctgatcaa tacaaactct 1620
gaacttgcct tgatgtacaa tgattcctca gtcttagaga accatcattt ggctgtgggc 1680
tttaaattgc ttcaggaaga aaactgtgac attttccaga atttgaccaa aaaacaaaga 1740
caatctttaa ggaaaatggt cattgacatc gtacttgcaa cagatatgtc aaaacacatg 1800
aatctactgg ctgatttgaa gactatggtt gaaactaaga aagtgacaag ctctggagtt 1860
cttcttcttg ataattattc cgataggatt caggttcttc agaatatggt gcactgtgca 1920
gatctgagca acccaacaaa gcctctccag ctgtaccgcc agtggacgga ccggataatg 1980
gaggagttct tccgccaagg agaccgagag agggaacgtg gcatggagat aagccccatg 2040
tgtgacaagc acaatgcttc cgtggaaaaa tcacaggtgg gcttcataga ctatattgtt 2100
catcccctct gggagacatg ggcagacctc gtccaccctg acgcccagga tattttggac 2160
actttggagg acaatcgtga atggtaccag agcacaatcc ctcagagccc ctctcctgca 2220

cctgatgacc cagaggaggg ccggcagggt caaactgaga aattccagtt tgaactaact 2280
ttagaggaag atggtgagtc agacacggaa aaggacagtg gcagtcaagt ggaagaagac 2340
actagctgca gtgactccaa gactctttgt actcaagact cagagtctac tgaaattccc 2400
cttgatgaac aggttgaaga ggaggcagta ggggaagaag aggaaagcca gcctgaagcc 2460
tgtgtcatag atgatcgttc tcctgacacg taacagtgca aaaactttca tgcctttttt 2520
ttttttaagt agaaaaattg tttccaaagt gcatgtcaca tgccacaacc acggtcacac 2580
ctcactgtca tctgccagga cgtttgttga acaaaactga ccttgactac tcagtccagc 2640
gctcaggaat atcgtaacca gtttttcac ctccatgtca tccgagcaag gtggacatct 2700
tcacgaacag cgtttttaac aagatttcag cttggtagag ctgacaaagc agataaaatc 2760
tactccaaat tattttcaag agagtgtgac tcatcaggca gcccaaaagt ttattggact 2820
tggggtttct attccttttt atttgtttgc aatattttca gaagaaaggc attgcacaga 2880
gtgaacttaa tggacgaagc aacaaatatg tcaagaacag gacatagcac gaatctgtta 2940
ccagtaggag gaggatgagc cacagaaatt gcataatttt ctaatttcaa gtcttcctga 3000
tacatgactg aatagtgtgg ttcagtgagc tgcactgacc tctacatttt gtatgatatg 3060
taaaacagat tttttgtaga gcttactttt attattaaat gtattgaggt attatattta 3120
aaaaaaacta tgttcagaac ttcatctgcc actggttatt tttttctaag gagtaacttg 3180
caagttttca gtacaaatct gtgctacact ggataaaaat ctaatttatg aattttactt 3240
gcaccttata gttcatagca attaactgat ttgtagtgat tcattgtttg ttttatatac 3300
caatgacttc catattttaa aagagaaaaa caactttatg ttgcaggaaa cccttttttgt 3360
aagtctttat tatttacttt gcattttgtt tcactctttc cagataagca gagttgctct 3420
tcaccagtgt ttttcttcat gtgcaaagtg actatttgtt ctataatact tttatgtgtg 3480
ttatatcaaa tgtgtcttaa gcttcatgca aactcagtca tcagttcgtg ttgtctgaag 3540
caagtgggag atatataaat acccagtagc taaaatggtc agtctttttt agatgttttc 3600
ctacttagta tctcctaata acgttttgct gtgtcactag atgttcattt cacaagtgca 3660
tgtctttcta ataatccaca catttcatgc tctaataatc cacacatttc atgctcattt 3720
ttattgtttt tacagccagt tatagtaaga aaaaggtttt tccccttgtg ctgctttata 3780
atttagcgtg tgtctgaacc ttatccatgt ttgctagatg aggtcttgtc aaatatatca 3840
ctaccattgt caccggtgaa aagaaacagg tagttaagtt agggttaaca ttcatttcaa 3900
ccacgaggtt gtatatcatg actagctttt actcttggtt tacagagaaa agttaaacag 3960
ccaactaggc agtttttaag aatattaaca atatattaac aaacaccaat acaactaatc 4020
ctatttggtt ttaatgattt caccatggga ttaagaacta tatcaggaac atccctgaga 4080
aacggtttta agtgtagcaa ctactcttcc ttaatggaca gccacataac gtgtaggaag 4140

tcctttatca cttatcctcg atccataagc atatcttgca gaggggaact acttctttaa 4200
acacatggag ggaaagaaga tgatgccact ggcaccagag ggttagtact gtgatgcatc 4260
ctaaaatatt tattatattg gtaaaaattc tggttaaata aaaaattaga gatcactctt 4320
ggctgatttc agcaccagga actgtattac agttttagag attaattcct agtgtttacc 4380
tgattatagc agttggcatc atggggcatt taattctgac tttatcccca cgtcagcctt 4440
aataaagtct tctttacctt ctctatgaag actttaaagc ccaaataatc atttttcaca 4500
ttgatattca agaattgaga tagatagaag ccaaagtggg tatctgacaa gtggaaaatc 4560
aaacgtttaa gaagaattac aactctgaaa agcatttata tgtggaactt ctcaaggagc 4620
ctcctgggga ctggaaagta agtcatcagc caggcaaatg actcatgctg aagagagtcc 4680
ccatttcagt cccctgagat ctagctgatg cttagatcct ttgaaataaa aattatgtct 4740
ttataactct gatcttttac ataaagcaga agaggaatca actagttaat tgcaaggttt 4800
ctactctgtt tcctctgtaa agatcagatg gtaatctttc aaataagaaa aaaataaaga 4860
cgtatgtttg accaagtagt ttcacaagaa tatttgggaa cttgtttctt ttaattttat 4920
ttgtccctga gtgaagtcta gaaagaaagg taaagagtct agagtttatt cctctttcca 4980
aaacattctc attcctctcc tccctacact tagtatttcc cccacagagt gcctagaatc 5040
ttaataatga ataaaataaa aagcagcaat atgtcattaa caaatccaga cctgaaaggg 5100
taaagggttt ataactgcac taataaagag aggctctttt tttttcttcc agtttgttgg 5160
tttttaatgg taccgtgttg taaagatacc cactaatgga caatcaaatt gcagaaaagg 5220
ctcaatatcc aagagacagg gactaatgca ctgtacaatc tgcttatcct tgcccttctc 5280
tcttgccaaa gtgtgcttca gaaatatata ctgctttaaa aaagaataaa agaatatcct 5340
tttacaagtg gctttacatt tcctaaaatg ccataagaaa atgcaatatc tgggtactgt 5400
atggggaaaa aaatgtccaa gtttgtgtaa aaccagtgca tttcagcttg caagttactg 5460
aacacaataa tgctgtttta attttgtttt atatcagtta aaattcacaa taatgtagat 5520
agaacaaatt acagacaagg aaagaaaaaa cttgaatgaa atggatttta cagaaagctt 5580
tatgataatt tttgaatgca ttatttattt tttgtgccat gcattttttt tctcaccaaa 5640
tgaccttacc tgtaatacag tcttgtttgt ctgtttacaa ccatgtattt attgcaatgt 5700
acatactgta atgttaattg taaattatct gttcttatta aaacatcatc ccatgatggg 5760
atggtgttga tatatttgga aactcttggt gagagaatga atggtgtgta tacatactct 5820
gtacattttt cttttctcct gtaatatagt cttgtcacct tagagcttgt ttatggaaga 5880
ttcaagaaaa ctataaaata cttaaagata tataaattta aaaaaacata gctgcaggtc 5940
tttggtccca gggctgtgcc ttaactttaa ccaatatttt cttctgtttt gctgcatttg 6000

aaaggtaaca gtggagctag ggctgggcat tttacatcca ggcttttaat tgattagaat 6060

tctgccaata ggtggatttt acaaaaccac agacaacctc tgaaagattc tgagaccctt 6120

ttgagacaga agctcttaag tacttcttgc cagggagcag cactgcatgt gtgatggttg 6180

tttgccatct gttgatcagg aactacttca gctacttgca tttgattatt tccttttttt 6240

ttttttttaa ctcggaaaca caactgggga aatatattct ttcccagtga ttataaacaa 6300

tctttttctt ttttttaagt ccttttggct tctagagctc ataggaaaat ggacttgatt 6360

tgaaattgga gccagagttt actcgtgttg gttatctatt catcagcttc ctgacatgtt 6420

aagagaatac attaaagaga aaatactgtt ttttaatcct aaaatttttc ttccactaag 6480

ataaaccaaa tgtccttaca tatatgtaaa cccatctatt taaacgcaaa ggtgggttga 6540

tgtcagttta catagcagaa agcattcact atcctctaag atttgtttct gcaaaacttt 6600

cattgcttta gaattttaaa atttcacctt gtacaatggc cagcccctaa agcaggaaac 6660

atttataatg gattatatgg aaacatcctc ccagtacttg cccagccctt gaatcatgtg 6720

gcttttcagt gaaaggaaag attctttttc taggaaaaat gagcctattt tattttattt 6780

tattttattt tttgacacaa actgtagatt ttagcagccc tggcccaaag gaatttgatt 6840

acttttgttt taaacagtac aaaggggaca ctataattac aaaaacatcc ttaactgatt 6900

tgagttgttt ttatttcttt ggatatattt tcagagtggt aaattgtgtg tgagaattac 6960

aaatgattat tcttttagtg gtttcttagc ctctcttaca gcccacgggg atagtactgt 7020

acatcaatac cttcatatga aatttttata tgcaatgaaa ataaaagcat gggttgattc 7080

tgcctattta tgactcaatc ttttacaaat aaaagattat tcattttaaa ttatagttca 7140

atcagcatgt ctcttaggat actgaacgtg gttgaaatga aaggatagtg acatcataag 7200

ttagtactga tattcataac caaataaagc caacttgagt aattttgcta cattaaaaat 7260

taccaaaatt acttagatgg cctataagat taagcatggt gttttctaag caagctttga 7320

aaggggcctt ccatacttac ttaattgaat attctgggat attgaaaatt attcagatac 7380

ttgacaatta tttttggtta cctactccgc aaactacaaa gttttaagga ctcaacaata 7440

agttaatgag acacagtgtt tgctttcatg gagcttacag tctggagggg acaaaggctt 7500

aaacaatact catataatta tatatgtgat cagtacaatg aaggagctca gtggggtaaa 7560

taagcaggaa cctgaacttg atctgttccg gagggccaca gaaggcttcc ttgaggcctt 7620

gagaaagtga tttgcatctg agttctgaag gattgtaaga ggtaactagg gaaaaagttg 7680

acaggaagag gaaggggatc cagacaagaa acatttgcaa agatcttgag gcataaatga 7740

gcttgagaca tctggagaaa ctgaggaaaa gtgagagagt aggcagggcc tggagccgca 7800

gagccattgc taaccatcct gtgtgagata tccccccattc tgtagcttta ttctcataac 7860

cctgctcaat tttctttata acacttctca cagatttata tacgtgtttg tttttgttat 7920

ctgtctctcc caccagacca cagctccatg agagcaaggt ctttgcttac caatatatca 7980

ctagcactta aaactatgcc tggtacacag taggttctta atatgtgttg aatatagcca 8040

tcaaattgat attggatata attcaatctg ataagatatt ttgagatatt aaagagtttt 8100

taacttgata ccataaaaaa aaaaaaaaaa 8130

<210> 43
<211> 7814
<212> DNA
<213> Homo sapiens

<400> 43
acaaaggaaa tgccctcact cagctacaag tgcctcctgc tccgtgcggg gcctcagggc 60

cccagagccc cgcaccagct ctgacttctc gtggttcctg ggatcttgg catcgtcctt 120

aaaaatggct tttgtttggg atcctctggg agccacggtg ccaggaccat ctacaagagc 180

caaatcaaga ttgcgtttct caaagtccta cagttttgat gtggacaatg gcacatctgc 240

gggacggagt cccttggatc ccatgaccag cccaggatcc gggctaattc tccaagcaaa 300

ttttgtccac agtcaacgac gggagtcctt cctgtatcga tccgacagcg attatgacct 360

ctctccaaag tctatgtccc ggaactcctc cattgccagt gatatacacg gagatgactt 420

gattgtgact ccatttgctc aggtcttggc cagtctgcga actgtacgaa acaactttgc 480

tgcattaact aatttgcaag atcgagcacc tagcaaaaga tcacccatgt gcaaccaacc 540

atccatcaac aaagccacca taacagagga ggcctaccag aaactggcca gcgagaccct 600

ggaggagctg gactggtgtc tggaccagct agagacccta cagaccaggc actccgtcag 660

tgagatggcc tccaacaagt ttaaaaggat gcttaatcgg gagctcaccc atctctctga 720

aatgagtcgg tctggaaatc aagtgtcaga gtttatatca aacacattct tagataagca 780

acatgaagtg gaaattcctt ctccaactca gaaggaaaag gagaaaaaga aaagaccaat 840

gtctcagatc agtggagtca agaaattgat gcacagctct agtctgacta attcaagtat 900

cccaaggttt ggagttaaaa ctgaacaaga agatgtcctt gccaaggaac tagaagatgt 960

gaacaaatgg ggtcttcatg ttttcagaat agcagagttg tctggtaacc ggcccttgac 1020

tgttatcatg cacaccattt ttcaggaacg ggatttatta aaaacattta aaattccagt 1080

agatacttta attacatatc ttatgactct cgaagaccat taccatgctg atgtggccta 1140

tcacaacaat atccatgctg cagatgttgt ccagtctact catgtgctat tatctacacc 1200

tgctttggag gctgtgttta cagatttgga gattcttgca gcaatttttg ccagtgcaat 1260

acatgatgta gatcatcctg gtgtgtccaa tcaatttctg atcaatacaa actctgaact 1320

tgccttgatg tacaatgatt cctcagtctt agagaaccat catttggctg tgggctttaa 1380

attgcttcag gaagaaaact gtgacatttt ccagaatttg accaaaaaac aaagacaatc 1440

tttaaggaaa atggtcattg acatcgtact tgcaacagat atgtcaaaac acatgaatct 1500

actggctgat ttgaagacta tggttgaaac taagaaagtg acaagctctg gagttcttct 1560

tcttgataat tattccgata ggattcaggt tcttcagaat atggtgcact gtgcagatct 1620

gagcaaccca acaaagcctc tccagctgta ccgccagtgg acggaccgga taatggagga 1680

gttcttccgc caaggagacc gagagaggga acgtggcatg gagataagcc ccatgtgtga 1740

caagcacaat gcttccgtgg aaaaatcaca ggtgggcttc atagactata ttgttcatcc 1800

cctctgggag acatgggcag acctcgtcca ccctgacgcc caggatattt tggacacttt 1860

ggaggacaat cgtgaatggt accagagcac aatccctcag agcccctctc ctgcacctga 1920

tgacccagag gagggccggc agggtcaaac tgagaaattc cagtttgaac taactttaga 1980

ggaagatggt gagtcagaca cggaaaagga cagtggcagt caagtggaag aagacactag 2040

ctgcagtgac tccaagactc tttgtactca agactcagag tctactgaaa ttccccttga 2100

tgaacaggtt gaagaggagg cagtagggga agaagaggaa agccagcctg aagcctgtgt 2160

catagatgat cgttctcctg acacgtaaca gtgcaaaaac tttcatgcct tttttttttt 2220

taagtagaaa aattgtttcc aaagtgcatg tcacatgcca caaccacggt cacacctcac 2280

tgtcatctgc caggacgttt gttgaacaaa actgaccttg actactcagt ccagcgctca 2340

ggaatatcgt aaccagtttt ttcacctcca tgtcatccga gcaaggtgga catcttcacg 2400

aacagcgttt ttaacaagat ttcagcttgg tagagctgac aaagcagata aaatctactc 2460

caaattattt tcaagagagt gtgactcatc aggcagccca aaagtttatt ggacttgggg 2520

tttctattcc tttttatttg tttgcaatat tttcagaaga aaggcattgc acagagtgaa 2580

cttaatggac gaagcaacaa atatgtcaag aacaggacat agcacgaatc tgttaccagt 2640

aggaggagga tgagccacag aaattgcata attttctaat ttcaagtctt cctgatacat 2700

gactgaatag tgtggttcag tgagctgcac tgacctctac attttgtatg atatgtaaaa 2760

cagatttttt gtagagctta cttttattat taaatgtatt gaggtattat atttaaaaaa 2820

aactatgttc agaacttcat ctgccactgg ttattttttt ctaaggagta acttgcaagt 2880

tttcagtaca aatctgtgct acactggata aaaatctaat ttatgaattt tacttgcacc 2940

ttatagttca tagcaattaa ctgatttgta gtgattcatt gtttgtttta tataccaatg 3000

acttccatat tttaaaagag aaaaacaact ttatgttgca ggaaaccctt tttgtaagtc 3060

tttattattt actttgcatt ttgtttcact ctttccagat aagcagagtt gctcttcacc 3120

agtgtttttc ttcatgtgca aagtgactat ttgttctata atacttttat gtgtgttata 3180

tcaaatgtgt cttaagcttc atgcaaactc agtcatcagt tcgtgttgtc tgaagcaagt 3240

gggagatata taaataccca gtagctaaaa tggtcagtct tttttagatg ttttcctact 3300

tagtatctcc taataacgtt ttgctgtgtc actagatgtt catttcacaa gtgcatgtct 3360
ttctaataat ccacacattt catgctctaa taatccacac atttcatgct catttttatt 3420
gtttttacag ccagttatag taagaaaaag gtttttcccc ttgtgctgct ttataattta 3480
gcgtgtgtct gaaccttatc catgtttgct agatgaggtc ttgtcaaata tatcactacc 3540
attgtcaccg gtgaaaagaa acaggtagtt aagttagggt taacattcat ttcaaccacg 3600
aggttgtata tcatgactag cttttactct tggtttacag agaaaagtta aacagccaac 3660
taggcagttt ttaagaatat taacaatata ttaacaaaca ccaatacaac taatcctatt 3720
tggttttaat gatttcacca tgggattaag aactatatca ggaacatccc tgagaaacgg 3780
ttttaagtgt agcaactact cttccttaat ggacagccac ataacgtgta ggaagtcctt 3840
tatcacttat cctcgatcca taagcatatc ttgcagaggg gaactacttc tttaaacaca 3900
tggagggaaa gaagatgatg ccactggcac cagagggtta gtactgtgat gcatcctaaa 3960
atatttatta tattggtaaa aattctggtt aaataaaaaa ttagagatca ctcttggctg 4020
atttcagcac caggaactgt attacagttt tagagattaa ttcctagtgt ttacctgatt 4080
atagcagttg gcatcatggg gcatttaatt ctgactttat ccccacgtca gccttaataa 4140
agtcttcttt accttctcta tgaagacttt aaagcccaaa taatcatttt tcacattgat 4200
attcaagaat tgagatagat agaagccaaa gtgggtatct gacaagtgga aaatcaaacg 4260
tttaagaaga attacaactc tgaaaagcat ttatatgtgg aacttctcaa ggagcctcct 4320
ggggactgga aagtaagtca tcagccaggc aaatgactca tgctgaagag agtccccatt 4380
tcagtcccct gagatctagc tgatgcttag atcctttgaa ataaaaatta tgtctttata 4440
actctgatct tttacataaa gcagaagagg aatcaactag ttaattgcaa ggtttctact 4500
ctgtttcctc tgtaaagatc agatggtaat ctttcaaata agaaaaaaat aaagacgtat 4560
gtttgaccaa gtagtttcac aagaatattt gggaacttgt ttcttttaat tttatttgtc 4620
cctgagtgaa gtctagaaag aaaggtaaag agtctagagt ttattcctct ttccaaaaca 4680
ttctcattcc tctcctccct acacttagta tttcccccac agagtgccta gaatcttaat 4740
aatgaataaa ataaaaagca gcaatatgtc attaacaaat ccagacctga aagggtaaag 4800
ggtttataac tgcactaata aagagaggct cttttttttt cttccagttt gttggttttt 4860
aatggtaccg tgttgtaaag atacccacta atggacaatc aaattgcaga aaaggctcaa 4920
tatccaagag acagggacta atgcactgta caatctgctt atccttgccc ttctctcttg 4980
ccaaagtgtg cttcagaaat atatactgct ttaaaaaaga ataaaagaat atccttttac 5040
aagtggcttt acatttccta aaatgccata agaaaatgca atatctgggt actgtatggg 5100
gaaaaaaatg tccaagtttg tgtaaaacca gtgcatttca gcttgcaagt tactgaacac 5160
aataatgctg ttttaatttt gttttatatc agttaaaatt cacaataatg tagatagaac 5220

aaattacaga caaggaaaga aaaaacttga atgaaatgga ttttacagaa agctttatga 5280
taatttttga atgcattatt tattttttgt gccatgcatt ttttttctca ccaaatgacc 5340
ttacctgtaa tacagtcttg tttgtctgtt tacaaccatg tatttattgc aatgtacata 5400
ctgtaatgtt aattgtaaat tatctgttct tattaaaaca tcatcccatg atgggatggt 5460
gttgatatat ttggaaactc ttggtgagag aatgaatggt gtgtatacat actctgtaca 5520
tttttctttt ctcctgtaat atagtcttgt caccttagag cttgtttatg gaagattcaa 5580
gaaaactata aaatacttaa agatatataa atttaaaaaa acatagctgc aggtctttgg 5640
tcccagggct gtgccttaac tttaaccaat attttcttct gttttgctgc atttgaaagg 5700
taacagtgga gctagggctg ggcattttac atccaggctt ttaattgatt agaattctgc 5760
caataggtgg attttacaaa accacagaca acctctgaaa gattctgaga cccttttgag 5820
acagaagctc ttaagtactt cttgccaggg agcagcactg catgtgtgat ggttgtttgc 5880
catctgttga tcaggaacta cttcagctac ttgcatttga ttatttcctt tttttttttt 5940
tttaactcgg aaacacaact ggggaaatat attctttccc agtgattata aacaatcttt 6000
ttcttttttt taagtccttt tggcttctag agctcatagg aaaatggact tgatttgaaa 6060
ttggagccag agtttactcg tgttggttat ctattcatca gcttcctgac atgttaagag 6120
aatacattaa agagaaaata ctgtttttta atcctaaaat ttttcttcca ctaagataaa 6180
ccaaatgtcc ttacatatat gtaaacccat ctatttaaac gcaaaggtgg gttgatgtca 6240
gtttacatag cagaaagcat tcactatcct ctaagatttg tttctgcaaa actttcattg 6300
ctttagaatt ttaaaatttc accttgtaca atggccagcc cctaaagcag gaaacattta 6360
taatggatta tatggaaaca tcctcccagt acttgcccag cccttgaatc atgtggcttt 6420
tcagtgaaag gaaagattct ttttctagga aaaatgagcc tattttattt tattttattt 6480
tatttttga cacaaactgt agattttagc agccctggcc caaaggaatt tgattacttt 6540
tgttttaaac agtacaaagg ggacactata attacaaaaa catccttaac tgatttgagt 6600
tgtttttatt tctttggata tattttcaga gtggtaaatt gtgtgtgaga attacaaatg 6660
attattcttt tagtggtttc ttagcctctc ttacagccca cggggatagt actgtacatc 6720
aataccttca tatgaaattt ttatatgcaa tgaaaataaa agcatgggtt gattctgcct 6780
atttatgact caatctttta caaataaaag attattcatt ttaaattata gttcaatcag 6840
catgtctctt aggatactga acgtggttga aatgaaagga tagtgacatc ataagttagt 6900
actgatattc ataaccaaat aaagccaact tgagtaattt tgctacatta aaaattacca 6960
aaattactta gatggcctat aagattaagc atggtgtttt ctaagcaagc tttgaaaggg 7020
gccttccata cttacttaat tgaatattct gggatattga aaattattca gatacttgac 7080

aattattttt ggttacctac tccgcaaact acaaagtttt aaggactcaa caataagtta 7140

atgagacaca gtgtttgctt tcatggagct tacagtctgg aggggacaaa ggcttaaaca 7200

atactcatat aattatatat gtgatcagta caatgaagga gctcagtggg gtaaataagc 7260

aggaacctga acttgatctg ttccggaggg ccacagaagg cttccttgag gccttgagaa 7320

agtgatttgc atctgagttc tgaaggattg taagaggtaa ctagggaaaa agttgacagg 7380

aagaggaagg ggatccagac aagaaacatt tgcaaagatc ttgaggcata aatgagcttg 7440

agacatctgg agaaactgag gaaaagtgag agagtaggca gggcctggag ccgcagagcc 7500

attgctaacc atcctgtgtg agatatcccc cattctgtag ctttattctc ataaccctgc 7560

tcaattttct ttataacact tctcacagat ttatatacgt gtttgttttt gttatctgtc 7620

tctcccacca gaccacagct ccatgagagc aaggtctttg cttaccaata tatcactagc 7680

acttaaaact atgcctggta cacagtaggt tcttaatatg tgttgaatat agccatcaaa 7740

ttgatattgg atataattca atctgataag atattttgag atattaaaga gtttttaact 7800

tgataccata aaaa 7814

<210> 44
<211> 809
<212> PRT
<213> Homo sapiens

<400> 44

Met Glu Ala Glu Gly Ser Ser Ala Pro Ala Arg Ala Gly Ser Gly Glu
1 5 10 15

Gly Ser Asp Ser Ala Gly Gly Ala Thr Leu Lys Ala Pro Lys His Leu
20 25 30

Trp Arg His Glu Gln His His Gln Tyr Pro Leu Arg Gln Pro Gln Phe
35 40 45

Arg Leu Leu His Pro His His His Leu Pro Pro Pro Pro Pro Pro Ser
50 55 60

Pro Gln Pro Gln Pro Gln Cys Pro Leu Gln Pro Pro Pro Pro Pro Pro
65 70 75 80

Leu Pro Pro Pro Pro Pro Pro Pro Gly Ala Ala Arg Gly Arg Tyr Ala
85 90 95

Ser Ser Gly Ala Thr Gly Arg Val Arg His Arg Gly Tyr Ser Asp Thr
100 105 110

Glu Arg Tyr Leu Tyr Cys Arg Ala Met Asp Arg Thr Ser Tyr Ala Val

115 120 125

Glu Thr Gly His Arg Pro Gly Leu Lys Lys Ser Arg Met Ser Trp Pro
130 135 140

Ser Ser Phe Gln Gly Leu Arg Arg Phe Asp Val Asp Asn Gly Thr Ser
145 150 155 160

Ala Gly Arg Ser Pro Leu Asp Pro Met Thr Ser Pro Gly Ser Gly Leu
165 170 175

Ile Leu Gln Ala Asn Phe Val His Ser Gln Arg Arg Glu Ser Phe Leu
180 185 190

Tyr Arg Ser Asp Ser Asp Tyr Asp Leu Ser Pro Lys Ser Met Ser Arg
195 200 205

Asn Ser Ser Ile Ala Ser Asp Ile His Gly Asp Asp Leu Ile Val Thr
210 215 220

Pro Phe Ala Gln Val Leu Ala Ser Leu Arg Thr Val Arg Asn Asn Phe
225 230 235 240

Ala Ala Leu Thr Asn Leu Gln Asp Arg Ala Pro Ser Lys Arg Ser Pro
245 250 255

Met Cys Asn Gln Pro Ser Ile Asn Lys Ala Thr Ile Thr Glu Glu Ala
260 265 270

Tyr Gln Lys Leu Ala Ser Glu Thr Leu Glu Glu Leu Asp Trp Cys Leu
275 280 285

Asp Gln Leu Glu Thr Leu Gln Thr Arg His Ser Val Ser Glu Met Ala
290 295 300

Ser Asn Lys Phe Lys Arg Met Leu Asn Arg Glu Leu Thr His Leu Ser
305 310 315 320

Glu Met Ser Arg Ser Gly Asn Gln Val Ser Glu Phe Ile Ser Asn Thr
325 330 335

Phe Leu Asp Lys Gln His Glu Val Glu Ile Pro Ser Pro Thr Gln Lys
340 345 350

Glu Lys Glu Lys Lys Lys Arg Pro Met Ser Gln Ile Ser Gly Val Lys
355 360 365

```
Lys Leu Met His Ser Ser Ser Leu Thr Asn Ser Ser Ile Pro Arg Phe
    370                 375                 380

Gly Val Lys Thr Glu Gln Glu Asp Val Leu Ala Lys Glu Leu Glu Asp
385                 390                 395                 400

Val Asn Lys Trp Gly Leu His Val Phe Arg Ile Ala Glu Leu Ser Gly
                405                 410                 415

Asn Arg Pro Leu Thr Val Ile Met His Thr Ile Phe Gln Glu Arg Asp
            420                 425                 430

Leu Leu Lys Thr Phe Lys Ile Pro Val Asp Thr Leu Ile Thr Tyr Leu
        435                 440                 445

Met Thr Leu Glu Asp His Tyr His Ala Asp Val Ala Tyr His Asn Asn
    450                 455                 460

Ile His Ala Ala Asp Val Val Gln Ser Thr His Val Leu Leu Ser Thr
465                 470                 475                 480

Pro Ala Leu Glu Ala Val Phe Thr Asp Leu Glu Ile Leu Ala Ala Ile
                485                 490                 495

Phe Ala Ser Ala Ile His Asp Val Asp His Pro Gly Val Ser Asn Gln
            500                 505                 510

Phe Leu Ile Asn Thr Asn Ser Glu Leu Ala Leu Met Tyr Asn Asp Ser
        515                 520                 525

Ser Val Leu Glu Asn His His Leu Ala Val Gly Phe Lys Leu Leu Gln
    530                 535                 540

Glu Glu Asn Cys Asp Ile Phe Gln Asn Leu Thr Lys Lys Gln Arg Gln
545                 550                 555                 560

Ser Leu Arg Lys Met Val Ile Asp Ile Val Leu Ala Thr Asp Met Ser
                565                 570                 575

Lys His Met Asn Leu Leu Ala Asp Leu Lys Thr Met Val Glu Thr Lys
            580                 585                 590

Lys Val Thr Ser Ser Gly Val Leu Leu Leu Asp Asn Tyr Ser Asp Arg
        595                 600                 605

Ile Gln Val Leu Gln Asn Met Val His Cys Ala Asp Leu Ser Asn Pro
    610                 615                 620
```

Thr Lys Pro Leu Gln Leu Tyr Arg Gln Trp Thr Asp Arg Ile Met Glu
625 630 635 640

Glu Phe Phe Arg Gln Gly Asp Arg Glu Arg Glu Arg Gly Met Glu Ile
645 650 655

Ser Pro Met Cys Asp Lys His Asn Ala Ser Val Glu Lys Ser Gln Val
660 665 670

Gly Phe Ile Asp Tyr Ile Val His Pro Leu Trp Glu Thr Trp Ala Asp
675 680 685

Leu Val His Pro Asp Ala Gln Asp Ile Leu Asp Thr Leu Glu Asp Asn
690 695 700

Arg Glu Trp Tyr Gln Ser Thr Ile Pro Gln Ser Pro Ser Pro Ala Pro
705 710 715 720

Asp Asp Pro Glu Glu Gly Arg Gln Gly Gln Thr Glu Lys Phe Gln Phe
725 730 735

Glu Leu Thr Leu Glu Glu Asp Gly Glu Ser Asp Thr Glu Lys Asp Ser
740 745 750

Gly Ser Gln Val Glu Glu Asp Thr Ser Cys Ser Asp Ser Lys Thr Leu
755 760 765

Cys Thr Gln Asp Ser Glu Ser Thr Glu Ile Pro Leu Asp Glu Gln Val
770 775 780

Glu Glu Glu Ala Val Gly Glu Glu Glu Glu Ser Gln Pro Glu Ala Cys
785 790 795 800

Val Ile Asp Asp Arg Ser Pro Asp Thr
805

<210> 45
<211> 699
<212> PRT
<213> Homo sapiens

<400> 45

Met Ser Leu Pro Ser Ser Cys Glu Tyr Leu Thr Leu Gly Lys Val Ala
1 5 10 15

Arg Phe Arg Ser Ala Tyr Val His Gly Ser Pro Tyr Ala Ile Asn Met
20 25 30

```
Pro Ile Asp Ile Lys Pro Gln Arg Arg Arg Phe Asp Val Asp Asn Gly
        35                  40                  45

Thr Ser Ala Gly Arg Ser Pro Leu Asp Pro Met Thr Ser Pro Gly Ser
    50                  55                  60

Gly Leu Ile Leu Gln Ala Asn Phe Val His Ser Gln Arg Arg Glu Ser
65                  70                  75                  80

Phe Leu Tyr Arg Ser Asp Ser Asp Tyr Asp Leu Ser Pro Lys Ser Met
                85                  90                  95

Ser Arg Asn Ser Ser Ile Ala Ser Asp Ile His Gly Asp Asp Leu Ile
            100                 105                 110

Val Thr Pro Phe Ala Gln Val Leu Ala Ser Leu Arg Thr Val Arg Asn
        115                 120                 125

Asn Phe Ala Ala Leu Thr Asn Leu Gln Asp Arg Ala Pro Ser Lys Arg
    130                 135                 140

Ser Pro Met Cys Asn Gln Pro Ser Ile Asn Lys Ala Thr Ile Thr Glu
145                 150                 155                 160

Glu Ala Tyr Gln Lys Leu Ala Ser Glu Thr Leu Glu Glu Leu Asp Trp
                165                 170                 175

Cys Leu Asp Gln Leu Glu Thr Leu Gln Thr Arg His Ser Val Ser Glu
            180                 185                 190

Met Ala Ser Asn Lys Phe Lys Arg Met Leu Asn Arg Glu Leu Thr His
        195                 200                 205

Leu Ser Glu Met Ser Arg Ser Gly Asn Gln Val Ser Glu Phe Ile Ser
    210                 215                 220

Asn Thr Phe Leu Asp Lys Gln His Glu Val Glu Ile Pro Ser Pro Thr
225                 230                 235                 240

Gln Lys Glu Lys Glu Lys Lys Lys Arg Pro Met Ser Gln Ile Ser Gly
                245                 250                 255

Val Lys Lys Leu Met His Ser Ser Ser Leu Thr Asn Ser Ser Ile Pro
            260                 265                 270

Arg Phe Gly Val Lys Thr Glu Gln Glu Asp Val Leu Ala Lys Glu Leu
        275                 280                 285
```

Glu Asp Val Asn Lys Trp Gly Leu His Val Phe Arg Ile Ala Glu Leu
290 295 300

Ser Gly Asn Arg Pro Leu Thr Val Ile Met His Thr Ile Phe Gln Glu
305 310 315 320

Arg Asp Leu Leu Lys Thr Phe Lys Ile Pro Val Asp Thr Leu Ile Thr
325 330 335

Tyr Leu Met Thr Leu Glu Asp His Tyr His Ala Asp Val Ala Tyr His
340 345 350

Asn Asn Ile His Ala Ala Asp Val Val Gln Ser Thr His Val Leu Leu
355 360 365

Ser Thr Pro Ala Leu Glu Ala Val Phe Thr Asp Leu Glu Ile Leu Ala
370 375 380

Ala Ile Phe Ala Ser Ala Ile His Asp Val Asp His Pro Gly Val Ser
385 390 395 400

Asn Gln Phe Leu Ile Asn Thr Asn Ser Glu Leu Ala Leu Met Tyr Asn
405 410 415

Asp Ser Ser Val Leu Glu Asn His His Leu Ala Val Gly Phe Lys Leu
420 425 430

Leu Gln Glu Glu Asn Cys Asp Ile Phe Gln Asn Leu Thr Lys Lys Gln
435 440 445

Arg Gln Ser Leu Arg Lys Met Val Ile Asp Ile Val Leu Ala Thr Asp
450 455 460

Met Ser Lys His Met Asn Leu Leu Ala Asp Leu Lys Thr Met Val Glu
465 470 475 480

Thr Lys Lys Val Thr Ser Ser Gly Val Leu Leu Leu Asp Asn Tyr Ser
485 490 495

Asp Arg Ile Gln Val Leu Gln Asn Met Val His Cys Ala Asp Leu Ser
500 505 510

Asn Pro Thr Lys Pro Leu Gln Leu Tyr Arg Gln Trp Thr Asp Arg Ile
515 520 525

Met Glu Glu Phe Phe Arg Gln Gly Asp Arg Glu Arg Glu Arg Gly Met

530 535 540

Glu Ile Ser Pro Met Cys Asp Lys His Asn Ala Ser Val Glu Lys Ser
545 550 555 560

Gln Val Gly Phe Ile Asp Tyr Ile Val His Pro Leu Trp Glu Thr Trp
565 570 575

Ala Asp Leu Val His Pro Asp Ala Gln Asp Ile Leu Asp Thr Leu Glu
580 585 590

Asp Asn Arg Glu Trp Tyr Gln Ser Thr Ile Pro Gln Ser Pro Ser Pro
595 600 605

Ala Pro Asp Asp Pro Glu Glu Gly Arg Gln Gly Gln Thr Glu Lys Phe
610 615 620

Gln Phe Glu Leu Thr Leu Glu Glu Asp Gly Glu Ser Asp Thr Glu Lys
625 630 635 640

Asp Ser Gly Ser Gln Val Glu Glu Asp Thr Ser Cys Ser Asp Ser Lys
645 650 655

Thr Leu Cys Thr Gln Asp Ser Glu Ser Thr Glu Ile Pro Leu Asp Glu
660 665 670

Gln Val Glu Glu Glu Ala Val Gly Glu Glu Glu Glu Ser Gln Pro Glu
675 680 685

Ala Cys Val Ile Asp Asp Arg Ser Pro Asp Thr
690 695

<210> 46
<211> 745
<212> PRT
<213> Homo sapiens

<400> 46

Met Ala Gln Gln Thr Ser Pro Asp Thr Leu Thr Val Pro Glu Val Asp
1 5 10 15

Asn Pro His Cys Pro Asn Pro Trp Leu Asn Glu Asp Leu Val Lys Ser
20 25 30

Leu Arg Glu Asn Leu Leu Gln His Glu Lys Ser Lys Thr Ala Arg Lys
35 40 45

Ser Val Ser Pro Lys Leu Ser Pro Val Ile Ser Pro Arg Asn Ser Pro

50 55 60

Arg Leu Leu Arg Arg Met Leu Leu Ser Ser Asn Ile Pro Lys Gln Arg
65 70 75 80

Arg Phe Thr Val Ala His Thr Cys Phe Asp Val Asp Asn Gly Thr Ser
85 90 95

Ala Gly Arg Ser Pro Leu Asp Pro Met Thr Ser Pro Gly Ser Gly Leu
100 105 110

Ile Leu Gln Ala Asn Phe Val His Ser Gln Arg Arg Glu Ser Phe Leu
115 120 125

Tyr Arg Ser Asp Ser Asp Tyr Asp Leu Ser Pro Lys Ser Met Ser Arg
130 135 140

Asn Ser Ser Ile Ala Ser Asp Ile His Gly Asp Asp Leu Ile Val Thr
145 150 155 160

Pro Phe Ala Gln Val Leu Ala Ser Leu Arg Thr Val Arg Asn Asn Phe
165 170 175

Ala Ala Leu Thr Asn Leu Gln Asp Arg Ala Pro Ser Lys Arg Ser Pro
180 185 190

Met Cys Asn Gln Pro Ser Ile Asn Lys Ala Thr Ile Thr Glu Glu Ala
195 200 205

Tyr Gln Lys Leu Ala Ser Glu Thr Leu Glu Glu Leu Asp Trp Cys Leu
210 215 220

Asp Gln Leu Glu Thr Leu Gln Thr Arg His Ser Val Ser Glu Met Ala
225 230 235 240

Ser Asn Lys Phe Lys Arg Met Leu Asn Arg Glu Leu Thr His Leu Ser
245 250 255

Glu Met Ser Arg Ser Gly Asn Gln Val Ser Glu Phe Ile Ser Asn Thr
260 265 270

Phe Leu Asp Lys Gln His Glu Val Glu Ile Pro Ser Pro Thr Gln Lys
275 280 285

Glu Lys Glu Lys Lys Lys Arg Pro Met Ser Gln Ile Ser Gly Val Lys
290 295 300

Lys Leu Met His Ser Ser Ser Leu Thr Asn Ser Ser Ile Pro Arg Phe
305 310 315 320

Gly Val Lys Thr Glu Gln Glu Asp Val Leu Ala Lys Glu Leu Glu Asp
325 330 335

Val Asn Lys Trp Gly Leu His Val Phe Arg Ile Ala Glu Leu Ser Gly
340 345 350

Asn Arg Pro Leu Thr Val Ile Met His Thr Ile Phe Gln Glu Arg Asp
355 360 365

Leu Leu Lys Thr Phe Lys Ile Pro Val Asp Thr Leu Ile Thr Tyr Leu
370 375 380

Met Thr Leu Glu Asp His Tyr His Ala Asp Val Ala Tyr His Asn Asn
385 390 395 400

Ile His Ala Ala Asp Val Val Gln Ser Thr His Val Leu Leu Ser Thr
405 410 415

Pro Ala Leu Glu Ala Val Phe Thr Asp Leu Glu Ile Leu Ala Ala Ile
420 425 430

Phe Ala Ser Ala Ile His Asp Val Asp His Pro Gly Val Ser Asn Gln
435 440 445

Phe Leu Ile Asn Thr Asn Ser Glu Leu Ala Leu Met Tyr Asn Asp Ser
450 455 460

Ser Val Leu Glu Asn His His Leu Ala Val Gly Phe Lys Leu Leu Gln
465 470 475 480

Glu Glu Asn Cys Asp Ile Phe Gln Asn Leu Thr Lys Lys Gln Arg Gln
485 490 495

Ser Leu Arg Lys Met Val Ile Asp Ile Val Leu Ala Thr Asp Met Ser
500 505 510

Lys His Met Asn Leu Leu Ala Asp Leu Lys Thr Met Val Glu Thr Lys
515 520 525

Lys Val Thr Ser Ser Gly Val Leu Leu Leu Asp Asn Tyr Ser Asp Arg
530 535 540

Ile Gln Val Leu Gln Asn Met Val His Cys Ala Asp Leu Ser Asn Pro
545 550 555 560

Thr Lys Pro Leu Gln Leu Tyr Arg Gln Trp Thr Asp Arg Ile Met Glu
565 570 575

Glu Phe Phe Arg Gln Gly Asp Arg Glu Arg Glu Arg Gly Met Glu Ile
580 585 590

Ser Pro Met Cys Asp Lys His Asn Ala Ser Val Glu Lys Ser Gln Val
595 600 605

Gly Phe Ile Asp Tyr Ile Val His Pro Leu Trp Glu Thr Trp Ala Asp
610 615 620

Leu Val His Pro Asp Ala Gln Asp Ile Leu Asp Thr Leu Glu Asp Asn
625 630 635 640

Arg Glu Trp Tyr Gln Ser Thr Ile Pro Gln Ser Pro Ser Pro Ala Pro
645 650 655

Asp Asp Pro Glu Glu Gly Arg Gln Gly Gln Thr Glu Lys Phe Gln Phe
660 665 670

Glu Leu Thr Leu Glu Glu Asp Gly Glu Ser Asp Thr Glu Lys Asp Ser
675 680 685

Gly Ser Gln Val Glu Glu Asp Thr Ser Cys Ser Asp Ser Lys Thr Leu
690 695 700

Cys Thr Gln Asp Ser Glu Ser Thr Glu Ile Pro Leu Asp Glu Gln Val
705 710 715 720

Glu Glu Glu Ala Val Gly Glu Glu Glu Glu Ser Gln Pro Glu Ala Cys
725 730 735

Val Ile Asp Asp Arg Ser Pro Asp Thr
740 745

<210> 47
<211> 673
<212> PRT
<213> Homo sapiens

<400> 47

Met Met His Val Asn Asn Phe Pro Phe Arg Arg His Ser Trp Ile Cys
1 5 10 15

Phe Asp Val Asp Asn Gly Thr Ser Ala Gly Arg Ser Pro Leu Asp Pro
20 25 30

Met Thr Ser Pro Gly Ser Gly Leu Ile Leu Gln Ala Asn Phe Val His
35 40 45

Ser Gln Arg Arg Glu Ser Phe Leu Tyr Arg Ser Asp Ser Asp Tyr Asp
50 55 60

Leu Ser Pro Lys Ser Met Ser Arg Asn Ser Ser Ile Ala Ser Asp Ile
65 70 75 80

His Gly Asp Asp Leu Ile Val Thr Pro Phe Ala Gln Val Leu Ala Ser
85 90 95

Leu Arg Thr Val Arg Asn Asn Phe Ala Ala Leu Thr Asn Leu Gln Asp
100 105 110

Arg Ala Pro Ser Lys Arg Ser Pro Met Cys Asn Gln Pro Ser Ile Asn
115 120 125

Lys Ala Thr Ile Thr Glu Glu Ala Tyr Gln Lys Leu Ala Ser Glu Thr
130 135 140

Leu Glu Glu Leu Asp Trp Cys Leu Asp Gln Leu Glu Thr Leu Gln Thr
145 150 155 160

Arg His Ser Val Ser Glu Met Ala Ser Asn Lys Phe Lys Arg Met Leu
165 170 175

Asn Arg Glu Leu Thr His Leu Ser Glu Met Ser Arg Ser Gly Asn Gln
180 185 190

Val Ser Glu Phe Ile Ser Asn Thr Phe Leu Asp Lys Gln His Glu Val
195 200 205

Glu Ile Pro Ser Pro Thr Gln Lys Glu Lys Glu Lys Lys Lys Arg Pro
210 215 220

Met Ser Gln Ile Ser Gly Val Lys Lys Leu Met His Ser Ser Ser Leu
225 230 235 240

Thr Asn Ser Ser Ile Pro Arg Phe Gly Val Lys Thr Glu Gln Glu Asp
245 250 255

Val Leu Ala Lys Glu Leu Glu Asp Val Asn Lys Trp Gly Leu His Val
260 265 270

Phe Arg Ile Ala Glu Leu Ser Gly Asn Arg Pro Leu Thr Val Ile Met
275 280 285

His Thr Ile Phe Gln Glu Arg Asp Leu Leu Lys Thr Phe Lys Ile Pro
    290                 295                 300

Val Asp Thr Leu Ile Thr Tyr Leu Met Thr Leu Glu Asp His Tyr His
305                 310                 315                 320

Ala Asp Val Ala Tyr His Asn Asn Ile His Ala Ala Asp Val Val Gln
                325                 330                 335

Ser Thr His Val Leu Leu Ser Thr Pro Ala Leu Glu Ala Val Phe Thr
            340                 345                 350

Asp Leu Glu Ile Leu Ala Ala Ile Phe Ala Ser Ala Ile His Asp Val
        355                 360                 365

Asp His Pro Gly Val Ser Asn Gln Phe Leu Ile Asn Thr Asn Ser Glu
    370                 375                 380

Leu Ala Leu Met Tyr Asn Asp Ser Ser Val Leu Glu Asn His His Leu
385                 390                 395                 400

Ala Val Gly Phe Lys Leu Leu Gln Glu Glu Asn Cys Asp Ile Phe Gln
                405                 410                 415

Asn Leu Thr Lys Lys Gln Arg Gln Ser Leu Arg Lys Met Val Ile Asp
            420                 425                 430

Ile Val Leu Ala Thr Asp Met Ser Lys His Met Asn Leu Leu Ala Asp
        435                 440                 445

Leu Lys Thr Met Val Glu Thr Lys Lys Val Thr Ser Ser Gly Val Leu
    450                 455                 460

Leu Leu Asp Asn Tyr Ser Asp Arg Ile Gln Val Leu Gln Asn Met Val
465                 470                 475                 480

His Cys Ala Asp Leu Ser Asn Pro Thr Lys Pro Leu Gln Leu Tyr Arg
                485                 490                 495

Gln Trp Thr Asp Arg Ile Met Glu Glu Phe Phe Arg Gln Gly Asp Arg
            500                 505                 510

Glu Arg Glu Arg Gly Met Glu Ile Ser Pro Met Cys Asp Lys His Asn
        515                 520                 525

Ala Ser Val Glu Lys Ser Gln Val Gly Phe Ile Asp Tyr Ile Val His

530 535 540

Pro Leu Trp Glu Thr Trp Ala Asp Leu Val His Pro Asp Ala Gln Asp
545 550 555 560

Ile Leu Asp Thr Leu Glu Asp Asn Arg Glu Trp Tyr Gln Ser Thr Ile
565 570 575

Pro Gln Ser Pro Ser Pro Ala Pro Asp Asp Pro Glu Glu Gly Arg Gln
580 585 590

Gly Gln Thr Glu Lys Phe Gln Phe Glu Leu Thr Leu Glu Glu Asp Gly
595 600 605

Glu Ser Asp Thr Glu Lys Asp Ser Gly Ser Gln Val Glu Glu Asp Thr
610 615 620

Ser Cys Ser Asp Ser Lys Thr Leu Cys Thr Gln Asp Ser Glu Ser Thr
625 630 635 640

Glu Ile Pro Leu Asp Glu Gln Val Glu Glu Glu Ala Val Gly Glu Glu
645 650 655

Glu Glu Ser Gln Pro Glu Ala Cys Val Ile Asp Asp Arg Ser Pro Asp
660 665 670

Thr

<210> 48
<211> 507
<212> PRT
<213> Homo sapiens

<400> 48

Met Ala Ser Asn Lys Phe Lys Arg Met Leu Asn Arg Glu Leu Thr His
1 5 10 15

Leu Ser Glu Met Ser Arg Ser Gly Asn Gln Val Ser Glu Phe Ile Ser
20 25 30

Asn Thr Phe Leu Asp Lys Gln His Glu Val Glu Ile Pro Ser Pro Thr
35 40 45

Gln Lys Glu Lys Glu Lys Lys Lys Arg Pro Met Ser Gln Ile Ser Gly
50 55 60

Val Lys Lys Leu Met His Ser Ser Ser Leu Thr Asn Ser Ser Ile Pro

```
65                  70                  75                  80

Arg Phe Gly Val Lys Thr Glu Gln Glu Asp Val Leu Ala Lys Glu Leu
                85                  90                  95

Glu Asp Val Asn Lys Trp Gly Leu His Val Phe Arg Ile Ala Glu Leu
            100                 105                 110

Ser Gly Asn Arg Pro Leu Thr Val Ile Met His Thr Ile Phe Gln Glu
        115                 120                 125

Arg Asp Leu Leu Lys Thr Phe Lys Ile Pro Val Asp Thr Leu Ile Thr
    130                 135                 140

Tyr Leu Met Thr Leu Glu Asp His Tyr His Ala Asp Val Ala Tyr His
145                 150                 155                 160

Asn Asn Ile His Ala Ala Asp Val Val Gln Ser Thr His Val Leu Leu
                165                 170                 175

Ser Thr Pro Ala Leu Glu Ala Val Phe Thr Asp Leu Glu Ile Leu Ala
            180                 185                 190

Ala Ile Phe Ala Ser Ala Ile His Asp Val Asp His Pro Gly Val Ser
        195                 200                 205

Asn Gln Phe Leu Ile Asn Thr Asn Ser Glu Leu Ala Leu Met Tyr Asn
    210                 215                 220

Asp Ser Ser Val Leu Glu Asn His His Leu Ala Val Gly Phe Lys Leu
225                 230                 235                 240

Leu Gln Glu Glu Asn Cys Asp Ile Phe Gln Asn Leu Thr Lys Lys Gln
                245                 250                 255

Arg Gln Ser Leu Arg Lys Met Val Ile Asp Ile Val Leu Ala Thr Asp
            260                 265                 270

Met Ser Lys His Met Asn Leu Leu Ala Asp Leu Lys Thr Met Val Glu
        275                 280                 285

Thr Lys Lys Val Thr Ser Ser Gly Val Leu Leu Leu Asp Asn Tyr Ser
    290                 295                 300

Asp Arg Ile Gln Val Leu Gln Asn Met Val His Cys Ala Asp Leu Ser
305                 310                 315                 320
```

Asn Pro Thr Lys Pro Leu Gln Leu Tyr Arg Gln Trp Thr Asp Arg Ile
325 330 335

Met Glu Glu Phe Phe Arg Gln Gly Asp Arg Glu Arg Glu Arg Gly Met
340 345 350

Glu Ile Ser Pro Met Cys Asp Lys His Asn Ala Ser Val Glu Lys Ser
355 360 365

Gln Val Gly Phe Ile Asp Tyr Ile Val His Pro Leu Trp Glu Thr Trp
370 375 380

Ala Asp Leu Val His Pro Asp Ala Gln Asp Ile Leu Asp Thr Leu Glu
385 390 395 400

Asp Asn Arg Glu Trp Tyr Gln Ser Thr Ile Pro Gln Ser Pro Ser Pro
405 410 415

Ala Pro Asp Asp Pro Glu Glu Gly Arg Gln Gly Gln Thr Glu Lys Phe
420 425 430

Gln Phe Glu Leu Thr Leu Glu Glu Asp Gly Glu Ser Asp Thr Glu Lys
435 440 445

Asp Ser Gly Ser Gln Val Glu Glu Asp Thr Ser Cys Ser Asp Ser Lys
450 455 460

Thr Leu Cys Thr Gln Asp Ser Glu Ser Thr Glu Ile Pro Leu Asp Glu
465 470 475 480

Gln Val Glu Glu Glu Ala Val Gly Glu Glu Glu Glu Ser Gln Pro Glu
485 490 495

Ala Cys Val Ile Asp Asp Arg Ser Pro Asp Thr
500 505

<210> 49
<211> 679
<212> PRT
<213> Homo sapiens

<400> 49

Met Ser Ile Ile Met Lys Pro Arg Ser Arg Ser Thr Ser Ser Leu Arg
1 5 10 15

Thr Ala Glu Ala Val Cys Phe Asp Val Asp Asn Gly Thr Ser Ala Gly
20 25 30

Arg Ser Pro Leu Asp Pro Met Thr Ser Pro Gly Ser Gly Leu Ile Leu
35 40 45

Gln Ala Asn Phe Val His Ser Gln Arg Arg Glu Ser Phe Leu Tyr Arg
50 55 60

Ser Asp Ser Asp Tyr Asp Leu Ser Pro Lys Ser Met Ser Arg Asn Ser
65 70 75 80

Ser Ile Ala Ser Asp Ile His Gly Asp Asp Leu Ile Val Thr Pro Phe
85 90 95

Ala Gln Val Leu Ala Ser Leu Arg Thr Val Arg Asn Asn Phe Ala Ala
100 105 110

Leu Thr Asn Leu Gln Asp Arg Ala Pro Ser Lys Arg Ser Pro Met Cys
115 120 125

Asn Gln Pro Ser Ile Asn Lys Ala Thr Ile Thr Glu Glu Ala Tyr Gln
130 135 140

Lys Leu Ala Ser Glu Thr Leu Glu Glu Leu Asp Trp Cys Leu Asp Gln
145 150 155 160

Leu Glu Thr Leu Gln Thr Arg His Ser Val Ser Glu Met Ala Ser Asn
165 170 175

Lys Phe Lys Arg Met Leu Asn Arg Glu Leu Thr His Leu Ser Glu Met
180 185 190

Ser Arg Ser Gly Asn Gln Val Ser Glu Phe Ile Ser Asn Thr Phe Leu
195 200 205

Asp Lys Gln His Glu Val Glu Ile Pro Ser Pro Thr Gln Lys Glu Lys
210 215 220

Glu Lys Lys Lys Arg Pro Met Ser Gln Ile Ser Gly Val Lys Lys Leu
225 230 235 240

Met His Ser Ser Ser Leu Thr Asn Ser Ser Ile Pro Arg Phe Gly Val
245 250 255

Lys Thr Glu Gln Glu Asp Val Leu Ala Lys Glu Leu Glu Asp Val Asn
260 265 270

Lys Trp Gly Leu His Val Phe Arg Ile Ala Glu Leu Ser Gly Asn Arg
275 280 285

```
Pro Leu Thr Val Ile Met His Thr Ile Phe Gln Glu Arg Asp Leu Leu
    290                 295                 300

Lys Thr Phe Lys Ile Pro Val Asp Thr Leu Ile Thr Tyr Leu Met Thr
305                 310                 315                 320

Leu Glu Asp His Tyr His Ala Asp Val Ala Tyr His Asn Asn Ile His
                325                 330                 335

Ala Ala Asp Val Val Gln Ser Thr His Val Leu Leu Ser Thr Pro Ala
            340                 345                 350

Leu Glu Ala Val Phe Thr Asp Leu Glu Ile Leu Ala Ala Ile Phe Ala
        355                 360                 365

Ser Ala Ile His Asp Val Asp His Pro Gly Val Ser Asn Gln Phe Leu
    370                 375                 380

Ile Asn Thr Asn Ser Glu Leu Ala Leu Met Tyr Asn Asp Ser Ser Val
385                 390                 395                 400

Leu Glu Asn His His Leu Ala Val Gly Phe Lys Leu Leu Gln Glu Glu
                405                 410                 415

Asn Cys Asp Ile Phe Gln Asn Leu Thr Lys Lys Gln Arg Gln Ser Leu
            420                 425                 430

Arg Lys Met Val Ile Asp Ile Val Leu Ala Thr Asp Met Ser Lys His
        435                 440                 445

Met Asn Leu Leu Ala Asp Leu Lys Thr Met Val Glu Thr Lys Lys Val
    450                 455                 460

Thr Ser Ser Gly Val Leu Leu Leu Asp Asn Tyr Ser Asp Arg Ile Gln
465                 470                 475                 480

Val Leu Gln Asn Met Val His Cys Ala Asp Leu Ser Asn Pro Thr Lys
                485                 490                 495

Pro Leu Gln Leu Tyr Arg Gln Trp Thr Asp Arg Ile Met Glu Glu Phe
            500                 505                 510

Phe Arg Gln Gly Asp Arg Glu Arg Glu Arg Gly Met Glu Ile Ser Pro
        515                 520                 525

Met Cys Asp Lys His Asn Ala Ser Val Glu Lys Ser Gln Val Gly Phe
    530                 535                 540
```

Ile Asp Tyr Ile Val His Pro Leu Trp Glu Thr Trp Ala Asp Leu Val
545 550 555 560

His Pro Asp Ala Gln Asp Ile Leu Asp Thr Leu Glu Asp Asn Arg Glu
565 570 575

Trp Tyr Gln Ser Thr Ile Pro Gln Ser Pro Ser Pro Ala Pro Asp Asp
580 585 590

Pro Glu Glu Gly Arg Gln Gly Gln Thr Glu Lys Phe Gln Phe Glu Leu
595 600 605

Thr Leu Glu Glu Asp Gly Glu Ser Asp Thr Glu Lys Asp Ser Gly Ser
610 615 620

Gln Val Glu Glu Asp Thr Ser Cys Ser Asp Ser Lys Thr Leu Cys Thr
625 630 635 640

Gln Asp Ser Glu Ser Thr Glu Ile Pro Leu Asp Glu Gln Val Glu Glu
645 650 655

Glu Ala Val Gly Glu Glu Glu Glu Ser Gln Pro Glu Ala Cys Val Ile
660 665 670

Asp Asp Arg Ser Pro Asp Thr
675

<210> 50
<211> 518
<212> PRT
<213> Homo sapiens

<400> 50

Met Pro Glu Ala Asn Tyr Leu Leu Ser Val Ser Trp Gly Tyr Ile Lys
1 5 10 15

Phe Lys Arg Met Leu Asn Arg Glu Leu Thr His Leu Ser Glu Met Ser
20 25 30

Arg Ser Gly Asn Gln Val Ser Glu Phe Ile Ser Asn Thr Phe Leu Asp
35 40 45

Lys Gln His Glu Val Glu Ile Pro Ser Pro Thr Gln Lys Glu Lys Glu
50 55 60

Lys Lys Lys Arg Pro Met Ser Gln Ile Ser Gly Val Lys Lys Leu Met
65 70 75 80

His Ser Ser Ser Leu Thr Asn Ser Ser Ile Pro Arg Phe Gly Val Lys
85 90 95

Thr Glu Gln Glu Asp Val Leu Ala Lys Glu Leu Glu Asp Val Asn Lys
100 105 110

Trp Gly Leu His Val Phe Arg Ile Ala Glu Leu Ser Gly Asn Arg Pro
115 120 125

Leu Thr Val Ile Met His Thr Ile Phe Gln Glu Arg Asp Leu Leu Lys
130 135 140

Thr Phe Lys Ile Pro Val Asp Thr Leu Ile Thr Tyr Leu Met Thr Leu
145 150 155 160

Glu Asp His Tyr His Ala Asp Val Ala Tyr His Asn Asn Ile His Ala
165 170 175

Ala Asp Val Val Gln Ser Thr His Val Leu Leu Ser Thr Pro Ala Leu
180 185 190

Glu Ala Val Phe Thr Asp Leu Glu Ile Leu Ala Ala Ile Phe Ala Ser
195 200 205

Ala Ile His Asp Val Asp His Pro Gly Val Ser Asn Gln Phe Leu Ile
210 215 220

Asn Thr Asn Ser Glu Leu Ala Leu Met Tyr Asn Asp Ser Ser Val Leu
225 230 235 240

Glu Asn His His Leu Ala Val Gly Phe Lys Leu Leu Gln Glu Glu Asn
245 250 255

Cys Asp Ile Phe Gln Asn Leu Thr Lys Lys Gln Arg Gln Ser Leu Arg
260 265 270

Lys Met Val Ile Asp Ile Val Leu Ala Thr Asp Met Ser Lys His Met
275 280 285

Asn Leu Leu Ala Asp Leu Lys Thr Met Val Glu Thr Lys Lys Val Thr
290 295 300

Ser Ser Gly Val Leu Leu Leu Asp Asn Tyr Ser Asp Arg Ile Gln Val
305 310 315 320

Leu Gln Asn Met Val His Cys Ala Asp Leu Ser Asn Pro Thr Lys Pro

325 330 335

Leu Gln Leu Tyr Arg Gln Trp Thr Asp Arg Ile Met Glu Glu Phe Phe
340 345 350

Arg Gln Gly Asp Arg Glu Arg Glu Arg Gly Met Glu Ile Ser Pro Met
355 360 365

Cys Asp Lys His Asn Ala Ser Val Glu Lys Ser Gln Val Gly Phe Ile
370 375 380

Asp Tyr Ile Val His Pro Leu Trp Glu Thr Trp Ala Asp Leu Val His
385 390 395 400

Pro Asp Ala Gln Asp Ile Leu Asp Thr Leu Glu Asp Asn Arg Glu Trp
405 410 415

Tyr Gln Ser Thr Ile Pro Gln Ser Pro Ser Pro Ala Pro Asp Asp Pro
420 425 430

Glu Glu Gly Arg Gln Gly Gln Thr Glu Lys Phe Gln Phe Glu Leu Thr
435 440 445

Leu Glu Glu Asp Gly Glu Ser Asp Thr Glu Lys Asp Ser Gly Ser Gln
450 455 460

Val Glu Glu Asp Thr Ser Cys Ser Asp Ser Lys Thr Leu Cys Thr Gln
465 470 475 480

Asp Ser Glu Ser Thr Glu Ile Pro Leu Asp Glu Gln Val Glu Glu Glu
485 490 495

Ala Val Gly Glu Glu Glu Glu Ser Gln Pro Glu Ala Cys Val Ile Asp
500 505 510

Asp Arg Ser Pro Asp Thr
515

<210> 51
<211> 748
<212> PRT
<213> Homo sapiens

<400> 51

Met Lys Arg Asn Thr Cys Asp Leu Leu Ser Arg Ser Lys Ser Ala Ser
1 5 10 15

Glu Glu Thr Leu His Ser Ser Asn Glu Glu Glu Asp Pro Phe Arg Gly

20 25 30

Met Glu Pro Tyr Leu Val Arg Arg Leu Ser Cys Arg Asn Ile Gln Leu
35 40 45

Pro Pro Leu Ala Phe Arg Gln Leu Glu Gln Ala Asp Leu Lys Ser Glu
50 55 60

Ser Glu Asn Ile Gln Arg Pro Thr Ser Leu Pro Leu Lys Ile Leu Pro
65 70 75 80

Leu Ile Ala Ile Thr Ser Ala Glu Ser Ser Gly Phe Asp Val Asp Asn
85 90 95

Gly Thr Ser Ala Gly Arg Ser Pro Leu Asp Pro Met Thr Ser Pro Gly
100 105 110

Ser Gly Leu Ile Leu Gln Ala Asn Phe Val His Ser Gln Arg Arg Glu
115 120 125

Ser Phe Leu Tyr Arg Ser Asp Ser Asp Tyr Asp Leu Ser Pro Lys Ser
130 135 140

Met Ser Arg Asn Ser Ser Ile Ala Ser Asp Ile His Gly Asp Asp Leu
145 150 155 160

Ile Val Thr Pro Phe Ala Gln Val Leu Ala Ser Leu Arg Thr Val Arg
165 170 175

Asn Asn Phe Ala Ala Leu Thr Asn Leu Gln Asp Arg Ala Pro Ser Lys
180 185 190

Arg Ser Pro Met Cys Asn Gln Pro Ser Ile Asn Lys Ala Thr Ile Thr
195 200 205

Glu Glu Ala Tyr Gln Lys Leu Ala Ser Glu Thr Leu Glu Glu Leu Asp
210 215 220

Trp Cys Leu Asp Gln Leu Glu Thr Leu Gln Thr Arg His Ser Val Ser
225 230 235 240

Glu Met Ala Ser Asn Lys Phe Lys Arg Met Leu Asn Arg Glu Leu Thr
245 250 255

His Leu Ser Glu Met Ser Arg Ser Gly Asn Gln Val Ser Glu Phe Ile
260 265 270

Ser Asn Thr Phe Leu Asp Lys Gln His Glu Val Glu Ile Pro Ser Pro
275 280 285

Thr Gln Lys Glu Lys Glu Lys Lys Lys Arg Pro Met Ser Gln Ile Ser
290 295 300

Gly Val Lys Lys Leu Met His Ser Ser Ser Leu Thr Asn Ser Ser Ile
305 310 315 320

Pro Arg Phe Gly Val Lys Thr Glu Gln Glu Asp Val Leu Ala Lys Glu
325 330 335

Leu Glu Asp Val Asn Lys Trp Gly Leu His Val Phe Arg Ile Ala Glu
340 345 350

Leu Ser Gly Asn Arg Pro Leu Thr Val Ile Met His Thr Ile Phe Gln
355 360 365

Glu Arg Asp Leu Leu Lys Thr Phe Lys Ile Pro Val Asp Thr Leu Ile
370 375 380

Thr Tyr Leu Met Thr Leu Glu Asp His Tyr His Ala Asp Val Ala Tyr
385 390 395 400

His Asn Asn Ile His Ala Ala Asp Val Val Gln Ser Thr His Val Leu
405 410 415

Leu Ser Thr Pro Ala Leu Glu Ala Val Phe Thr Asp Leu Glu Ile Leu
420 425 430

Ala Ala Ile Phe Ala Ser Ala Ile His Asp Val Asp His Pro Gly Val
435 440 445

Ser Asn Gln Phe Leu Ile Asn Thr Asn Ser Glu Leu Ala Leu Met Tyr
450 455 460

Asn Asp Ser Ser Val Leu Glu Asn His His Leu Ala Val Gly Phe Lys
465 470 475 480

Leu Leu Gln Glu Glu Asn Cys Asp Ile Phe Gln Asn Leu Thr Lys Lys
485 490 495

Gln Arg Gln Ser Leu Arg Lys Met Val Ile Asp Ile Val Leu Ala Thr
500 505 510

Asp Met Ser Lys His Met Asn Leu Leu Ala Asp Leu Lys Thr Met Val
515 520 525

Glu Thr Lys Lys Val Thr Ser Ser Gly Val Leu Leu Leu Asp Asn Tyr
    530                 535                 540

Ser Asp Arg Ile Gln Val Leu Gln Asn Met Val His Cys Ala Asp Leu
545                 550                 555                 560

Ser Asn Pro Thr Lys Pro Leu Gln Leu Tyr Arg Gln Trp Thr Asp Arg
                565                 570                 575

Ile Met Glu Glu Phe Phe Arg Gln Gly Asp Arg Glu Arg Glu Arg Gly
            580                 585                 590

Met Glu Ile Ser Pro Met Cys Asp Lys His Asn Ala Ser Val Glu Lys
        595                 600                 605

Ser Gln Val Gly Phe Ile Asp Tyr Ile Val His Pro Leu Trp Glu Thr
    610                 615                 620

Trp Ala Asp Leu Val His Pro Asp Ala Gln Asp Ile Leu Asp Thr Leu
625                 630                 635                 640

Glu Asp Asn Arg Glu Trp Tyr Gln Ser Thr Ile Pro Gln Ser Pro Ser
                645                 650                 655

Pro Ala Pro Asp Asp Pro Glu Glu Gly Arg Gln Gly Gln Thr Glu Lys
            660                 665                 670

Phe Gln Phe Glu Leu Thr Leu Glu Glu Asp Gly Glu Ser Asp Thr Glu
        675                 680                 685

Lys Asp Ser Gly Ser Gln Val Glu Glu Asp Thr Ser Cys Ser Asp Ser
    690                 695                 700

Lys Thr Leu Cys Thr Gln Asp Ser Glu Ser Thr Glu Ile Pro Leu Asp
705                 710                 715                 720

Glu Gln Val Glu Glu Glu Ala Val Gly Glu Glu Glu Glu Ser Gln Pro
                725                 730                 735

Glu Ala Cys Val Ile Asp Asp Arg Ser Pro Asp Thr
            740                 745

<210> 52
<211> 687
<212> PRT
<213> Homo sapiens

<400> 52

```
Met Ala Phe Val Trp Asp Pro Leu Gly Ala Thr Val Pro Gly Pro Ser
1               5                   10                  15

Thr Arg Ala Lys Ser Arg Leu Arg Phe Ser Lys Ser Tyr Ser Phe Asp
            20                  25                  30

Val Asp Asn Gly Thr Ser Ala Gly Arg Ser Pro Leu Asp Pro Met Thr
        35                  40                  45

Ser Pro Gly Ser Gly Leu Ile Leu Gln Ala Asn Phe Val His Ser Gln
    50                  55                  60

Arg Arg Glu Ser Phe Leu Tyr Arg Ser Asp Ser Asp Tyr Asp Leu Ser
65                  70                  75                  80

Pro Lys Ser Met Ser Arg Asn Ser Ser Ile Ala Ser Asp Ile His Gly
                85                  90                  95

Asp Asp Leu Ile Val Thr Pro Phe Ala Gln Val Leu Ala Ser Leu Arg
            100                 105                 110

Thr Val Arg Asn Asn Phe Ala Ala Leu Thr Asn Leu Gln Asp Arg Ala
        115                 120                 125

Pro Ser Lys Arg Ser Pro Met Cys Asn Gln Pro Ser Ile Asn Lys Ala
    130                 135                 140

Thr Ile Thr Glu Glu Ala Tyr Gln Lys Leu Ala Ser Glu Thr Leu Glu
145                 150                 155                 160

Glu Leu Asp Trp Cys Leu Asp Gln Leu Glu Thr Leu Gln Thr Arg His
                165                 170                 175

Ser Val Ser Glu Met Ala Ser Asn Lys Phe Lys Arg Met Leu Asn Arg
            180                 185                 190

Glu Leu Thr His Leu Ser Glu Met Ser Arg Ser Gly Asn Gln Val Ser
        195                 200                 205

Glu Phe Ile Ser Asn Thr Phe Leu Asp Lys Gln His Glu Val Glu Ile
    210                 215                 220

Pro Ser Pro Thr Gln Lys Glu Lys Glu Lys Lys Lys Arg Pro Met Ser
225                 230                 235                 240

Gln Ile Ser Gly Val Lys Lys Leu Met His Ser Ser Ser Leu Thr Asn
                245                 250                 255
```

Ser Ser Ile Pro Arg Phe Gly Val Lys Thr Glu Gln Glu Asp Val Leu
260 265 270

Ala Lys Glu Leu Glu Asp Val Asn Lys Trp Gly Leu His Val Phe Arg
275 280 285

Ile Ala Glu Leu Ser Gly Asn Arg Pro Leu Thr Val Ile Met His Thr
290 295 300

Ile Phe Gln Glu Arg Asp Leu Leu Lys Thr Phe Lys Ile Pro Val Asp
305 310 315 320

Thr Leu Ile Thr Tyr Leu Met Thr Leu Glu Asp His Tyr His Ala Asp
325 330 335

Val Ala Tyr His Asn Asn Ile His Ala Ala Asp Val Val Gln Ser Thr
340 345 350

His Val Leu Leu Ser Thr Pro Ala Leu Glu Ala Val Phe Thr Asp Leu
355 360 365

Glu Ile Leu Ala Ala Ile Phe Ala Ser Ala Ile His Asp Val Asp His
370 375 380

Pro Gly Val Ser Asn Gln Phe Leu Ile Asn Thr Asn Ser Glu Leu Ala
385 390 395 400

Leu Met Tyr Asn Asp Ser Ser Val Leu Glu Asn His His Leu Ala Val
405 410 415

Gly Phe Lys Leu Leu Gln Glu Glu Asn Cys Asp Ile Phe Gln Asn Leu
420 425 430

Thr Lys Lys Gln Arg Gln Ser Leu Arg Lys Met Val Ile Asp Ile Val
435 440 445

Leu Ala Thr Asp Met Ser Lys His Met Asn Leu Leu Ala Asp Leu Lys
450 455 460

Thr Met Val Glu Thr Lys Lys Val Thr Ser Ser Gly Val Leu Leu Leu
465 470 475 480

Asp Asn Tyr Ser Asp Arg Ile Gln Val Leu Gln Asn Met Val His Cys
485 490 495

Ala Asp Leu Ser Asn Pro Thr Lys Pro Leu Gln Leu Tyr Arg Gln Trp

500 505 510

Thr Asp Arg Ile Met Glu Glu Phe Phe Arg Gln Gly Asp Arg Glu Arg
515 520 525

Glu Arg Gly Met Glu Ile Ser Pro Met Cys Asp Lys His Asn Ala Ser
530 535 540

Val Glu Lys Ser Gln Val Gly Phe Ile Asp Tyr Ile Val His Pro Leu
545 550 555 560

Trp Glu Thr Trp Ala Asp Leu Val His Pro Asp Ala Gln Asp Ile Leu
565 570 575

Asp Thr Leu Glu Asp Asn Arg Glu Trp Tyr Gln Ser Thr Ile Pro Gln
580 585 590

Ser Pro Ser Pro Ala Pro Asp Asp Pro Glu Glu Gly Arg Gln Gly Gln
595 600 605

Thr Glu Lys Phe Gln Phe Glu Leu Thr Leu Glu Glu Asp Gly Glu Ser
610 615 620

Asp Thr Glu Lys Asp Ser Gly Ser Gln Val Glu Glu Asp Thr Ser Cys
625 630 635 640

Ser Asp Ser Lys Thr Leu Cys Thr Gln Asp Ser Glu Ser Thr Glu Ile
645 650 655

Pro Leu Asp Glu Gln Val Glu Glu Glu Ala Val Gly Glu Glu Glu Glu
660 665 670

Ser Gln Pro Glu Ala Cys Val Ile Asp Asp Arg Ser Pro Asp Thr
675 680 685

<210> 53
<211> 2695
<212> DNA
<213> Homo sapiens

<400> 53
acagacagcg gcagagatct tgggctgagg ttcccgggcg ggcgggcgcg gagagacgcg 60

ggaagcaggg gctgggcggg ggtcgcggcg ccgcagctag cgcagccagc ccgagggccg 120

ccgccgccgc cgcccagcgc gctccggggc cgccggccgc agccagcacc cgccgcgccg 180

cagctccggg accggccccg gccgccgccg ccgcgatggg caacgccgcc gccgccaaga 240

agggcagcga gcaggagagc gtgaaagaat tcttagccaa agccaaagaa gatttctta 300

aaaaatggga aagtcccgct cagaacacag cccacttgga tcagtttgaa cgaatcaaga 360
ccctcggcac gggctccttc gggcgggtga tgctggtgaa acacaaggag accgggaacc 420
actatgccat gaagatcctc gacaaacaga aggtggtgaa actgaaacag atcgaacaca 480
ccctgaatga aaagcgcatc ctgcaagctg tcaactttcc gttcctcgtc aaactcgagt 540
tctccttcaa ggacaactca aacttataca tggtcatgga gtacgtgccc ggcggggaga 600
tgttctcaca cctacggcgg atcggaaggt tcagtgagcc ccatgcccgt ttctacgcgg 660
cccagatcgt cctgaccttt gagtatctgc actcgctgga tctcatctac agggacctga 720
agccggagaa tctgctcatt gaccagcagg gctacattca ggtgacagac ttcggtttcg 780
ccaagcgcgt gaagggccgc acttggacct tgtgcggcac ccctgagtac ctggcccctg 840
agattatcct gagcaaaggc tacaacaagg ccgtggactg gtgggccctg gggttcttta 900
tctatgaaat ggccgctggc tacccgccct tcttcgcaga ccagcccatc cagatctatg 960
agaagatcgt ctctgggaag gtgcgcttcc cttcccactt cagctctgac ttgaaggacc 1020
tgctgcggaa cctcctgcag gtagatctca ccaagcgctt tgggaacctc aagaatgggg 1080
tcaacgatat caagaaccac aagtggtttg ccacaactga ctggattgcc atctaccaga 1140
ggaaggtgga agctcccttc ataccaaagt ttaaaggccc tggggatacg agtaactttg 1200
acgactatga ggaagaagaa atccgggtct ccatcaatga gaagtgtggc aaggagtttt 1260
ctgagtttta ggggcatgcc tgtgccccca tgggttttct tttttctttt ttcttttttt 1320
tggtcggggg ggtgggaggg ttggattgaa cagccagagg gccccagagt tccttgcatc 1380
taatttcacc cccaccccac cctccagggt tagggggagc aggaagccca gataatcaga 1440
gggacagaaa caccagctgc tccccctcat ccccttcacc ctcctgcccc ctctcccact 1500
tttcccttcc tctttcccca cagcccccca gcccctcagc cctcccagcc cacttctgcc 1560
tgttttaaac gagtttctca actccagtca gaccaggtct tgctggtgta tccagggaca 1620
gggtatggaa agaggggctc acgcttaact ccagccccca cccacacccc catcccaccc 1680
aaccacaggc cccacttgct aagggcaaat gaacgaagcg ccaaccttcc tttcggagta 1740
atcctgcctg ggaaggagag atttttagtg acatgttcag tgggttgctt gctagaattt 1800
ttttaaaaaa acaacaattt aaaatcttat ttaagttcca ccagtgcctc cctccctcct 1860
tcctctactc ccacccctcc catgtccccc cattcctcaa atccatttta aagagaagca 1920
gactgacttt ggaaagggag gcgctggggt ttgaacctcc ccgctgctaa tctcccctgg 1980
gcccctcccc ggggaatcct ctctgccaat cctgcgaggg tctaggcccc tttaggaagc 2040
ctccgctctc tttttcccca acagacctgt cttcaccctt gggctttgaa agccagacaa 2100
agcagctgcc cctctccctg ccaaagagga gtcatccccc aaaaagacag agggggagcc 2160
ccaagcccaa gtctttcctc ccagcagcgt ttccccccaa ctccttaatt ttattctccg 2220

ctagatttta acgtccagcc ttccctcagc tgagtgggga gggcatccct gcaaaaggga 2280

acagaagagg ccaagtcccc ccaagccacg gcccggggtt caaggctaga gctgctgggg 2340

aggggctgcc tgttttactc acccaccagc ttccgcctcc cccatcctgg gcgcccctcc 2400

tccagcttag ctgtcagctg tccatcacct ctcccccact ttctcatttg tgctttttc 2460

tctcgtaata gaaaagtggg gagccgctgg ggagccaccc cattcatccc cgtatttccc 2520

cctctcataa cttctcccca tcccaggagg agttctcagg cctggggtgg ggccccgggt 2580

gggtgcgggg gcgattcaac ctgtgtgctg cgaaggacga gacttcctct tgaacagtgt 2640

gctgttgtaa acatatttga aaactattac caataaagtt ttgtttaaaa aaaaa 2695

<210> 54
<211> 2492
<212> DNA
<213> Homo sapiens

<400> 54
accgtagtgc cggtgccctg agaacaggac tgagtgatgg cttccaactc cagcgatgtg 60

aaagaattct tagccaaagc caaagaagat tttcttaaaa aatgggaaag tcccgctcag 120

aacacagccc acttggatca gtttgaacga atcaagaccc tcggcacggg ctccttcggg 180

cgggtgatgc tggtgaaaca caaggagacc gggaaccact atgccatgaa gatcctcgac 240

aaacagaagg tggtgaaact gaaacagatc gaacacaccc tgaatgaaaa gcgcatcctg 300

caagctgtca actttccgtt cctcgtcaaa ctcgagttct ccttcaagga caactcaaac 360

ttatacatgg tcatggagta cgtgcccggc ggggagatgt tctcacacct acggcggatc 420

ggaaggttca gtgagcccca tgcccgtttc tacgcggccc agatcgtcct gacctttgag 480

tatctgcact cgctggatct catctacagg gacctgaagc cggagaatct gctcattgac 540

cagcagggct acattcaggt gacagacttc ggtttcgcca agcgcgtgaa gggccgcact 600

tggaccttgt gcggcacccc tgagtacctg gcccctgaga ttatcctgag caaaggctac 660

aacaaggccg tggactggtg ggccctgggg gttcttatct atgaaatggc cgctggctac 720

ccgcccttct tcgcagacca gcccatccag atctatgaga agatcgtctc tgggaaggtg 780

cgcttccctt cccacttcag ctctgacttg aaggacctgc tgcggaacct cctgcaggta 840

gatctcacca agcgctttgg gaacctcaag aatggggtca acgatatcaa gaaccacaag 900

tggtttgcca caactgactg gattgccatc taccagagga aggtggaagc tcccttcata 960

ccaaagttta aaggccctgg ggatacgagt aactttgacg actatgagga agaagaaatc 1020

cgggtctcca tcaatgagaa gtgtggcaag gagttttctg agttttaggg gcatgcctgt 1080

gccccatgg gttttctttt ttcttttttc tttttttgg tcggggggt gggagggttg 1140

gattgaacag ccagagggcc ccagagttcc ttgcatctaa tttcacccc accccaccct 1200

ccagggttag ggggagcagg aagcccagat aatcagaggg acagaaacac cagctgctcc 1260

ccctcatccc cttcaccctc ctgccccctc tcccactttt cccttcctct ttccccacag 1320

ccccccagcc cctcagccct cccagcccac ttctgcctgt tttaaacgag tttctcaact 1380

ccagtcagac caggtcttgc tggtgtatcc agggacaggg tatggaaaga ggggctcacg 1440

cttaactcca gcccccaccc acacccccat cccacccaac cacaggcccc acttgctaag 1500

ggcaaatgaa cgaagcgcca accttccttt cggagtaatc ctgcctggga aggagagatt 1560

tttagtgaca tgttcagtgg gttgcttgct agaatttttt taaaaaaaca acaatttaaa 1620

atcttattta agttccacca gtgcctccct ccctccttcc tctactccca cccctcccat 1680

gtccccccat tcctcaaatc cattttaaag agaagcagac tgactttgga aagggaggcg 1740

ctggggtttg aacctccccg ctgctaatct cccctgggcc cctccccggg gaatcctctc 1800

tgccaatcct gcgagggtct aggccccttt aggaagcctc cgctctcttt ttccccaaca 1860

gacctgtctt caccccttggg ctttgaaagc cagacaaagc agctgcccct ctccctgcca 1920

aagaggagtc atcccccaaa aagacagagg gggagcccca agcccaagtc tttcctccca 1980

gcagcgtttc cccccaactc cttaatttta ttctccgcta gattttaacg tccagccttc 2040

cctcagctga gtggggaggg catccctgca aaagggaaca gaagaggcca agtcccccca 2100

agccacggcc cggggttcaa ggctagagct gctggggagg ggctgcctgt tttactcacc 2160

caccagcttc cgcctccccc atcctgggcg cccctcctcc agcttagctg tcagctgtcc 2220

atcacctctc ccccactttc tcatttgtgc ttttttctct cgtaatagaa aagtggggag 2280

ccgctgggga gccaccccat tcatccccgt atttccccct ctcataactt ctccccatcc 2340

caggaggagt tctcaggcct ggggtggggc cccgggtggg tgcgggggcg attcaacctg 2400

tgtgctgcga aggacgagac ttcctcttga acagtgtgct gttgtaaaca tatttgaaaa 2460

ctattaccaa taaagttttg tttaaaaaaa aa 2492

<210> 55
<211> 351
<212> PRT
<213> Homo sapiens

<400> 55

Met Gly Asn Ala Ala Ala Ala Lys Lys Gly Ser Glu Gln Glu Ser Val
1 5 10 15

Lys Glu Phe Leu Ala Lys Ala Lys Glu Asp Phe Leu Lys Lys Trp Glu
20 25 30

Ser Pro Ala Gln Asn Thr Ala His Leu Asp Gln Phe Glu Arg Ile Lys
35 40 45

```
Thr Leu Gly Thr Gly Ser Phe Gly Arg Val Met Leu Val Lys His Lys
    50                  55                  60

Glu Thr Gly Asn His Tyr Ala Met Lys Ile Leu Asp Lys Gln Lys Val
65                  70                  75                  80

Val Lys Leu Lys Gln Ile Glu His Thr Leu Asn Glu Lys Arg Ile Leu
                85                  90                  95

Gln Ala Val Asn Phe Pro Phe Leu Val Lys Leu Glu Phe Ser Phe Lys
            100                 105                 110

Asp Asn Ser Asn Leu Tyr Met Val Met Glu Tyr Val Pro Gly Gly Glu
        115                 120                 125

Met Phe Ser His Leu Arg Arg Ile Gly Arg Phe Ser Glu Pro His Ala
    130                 135                 140

Arg Phe Tyr Ala Ala Gln Ile Val Leu Thr Phe Glu Tyr Leu His Ser
145                 150                 155                 160

Leu Asp Leu Ile Tyr Arg Asp Leu Lys Pro Glu Asn Leu Leu Ile Asp
                165                 170                 175

Gln Gln Gly Tyr Ile Gln Val Thr Asp Phe Gly Phe Ala Lys Arg Val
            180                 185                 190

Lys Gly Arg Thr Trp Thr Leu Cys Gly Thr Pro Glu Tyr Leu Ala Pro
        195                 200                 205

Glu Ile Ile Leu Ser Lys Gly Tyr Asn Lys Ala Val Asp Trp Trp Ala
    210                 215                 220

Leu Gly Val Leu Ile Tyr Glu Met Ala Ala Gly Tyr Pro Pro Phe Phe
225                 230                 235                 240

Ala Asp Gln Pro Ile Gln Ile Tyr Glu Lys Ile Val Ser Gly Lys Val
                245                 250                 255

Arg Phe Pro Ser His Phe Ser Ser Asp Leu Lys Asp Leu Leu Arg Asn
            260                 265                 270

Leu Leu Gln Val Asp Leu Thr Lys Arg Phe Gly Asn Leu Lys Asn Gly
        275                 280                 285

Val Asn Asp Ile Lys Asn His Lys Trp Phe Ala Thr Thr Asp Trp Ile
```

290 295 300

Ala Ile Tyr Gln Arg Lys Val Glu Ala Pro Phe Ile Pro Lys Phe Lys
305 310 315 320

Gly Pro Gly Asp Thr Ser Asn Phe Asp Asp Tyr Glu Glu Glu Glu Ile
325 330 335

Arg Val Ser Ile Asn Glu Lys Cys Gly Lys Glu Phe Ser Glu Phe
340 345 350

<210> 56
<211> 343
<212> PRT
<213> Homo sapiens

<400> 56

Met Ala Ser Asn Ser Ser Asp Val Lys Glu Phe Leu Ala Lys Ala Lys
1 5 10 15

Glu Asp Phe Leu Lys Lys Trp Glu Ser Pro Ala Gln Asn Thr Ala His
20 25 30

Leu Asp Gln Phe Glu Arg Ile Lys Thr Leu Gly Thr Gly Ser Phe Gly
35 40 45

Arg Val Met Leu Val Lys His Lys Glu Thr Gly Asn His Tyr Ala Met
50 55 60

Lys Ile Leu Asp Lys Gln Lys Val Val Lys Leu Lys Gln Ile Glu His
65 70 75 80

Thr Leu Asn Glu Lys Arg Ile Leu Gln Ala Val Asn Phe Pro Phe Leu
85 90 95

Val Lys Leu Glu Phe Ser Phe Lys Asp Asn Ser Asn Leu Tyr Met Val
100 105 110

Met Glu Tyr Val Pro Gly Gly Glu Met Phe Ser His Leu Arg Arg Ile
115 120 125

Gly Arg Phe Ser Glu Pro His Ala Arg Phe Tyr Ala Ala Gln Ile Val
130 135 140

Leu Thr Phe Glu Tyr Leu His Ser Leu Asp Leu Ile Tyr Arg Asp Leu
145 150 155 160

Lys Pro Glu Asn Leu Leu Ile Asp Gln Gln Gly Tyr Ile Gln Val Thr

165 170 175

Asp Phe Gly Phe Ala Lys Arg Val Lys Gly Arg Thr Trp Thr Leu Cys
180 185 190

Gly Thr Pro Glu Tyr Leu Ala Pro Glu Ile Ile Leu Ser Lys Gly Tyr
195 200 205

Asn Lys Ala Val Asp Trp Trp Ala Leu Gly Val Leu Ile Tyr Glu Met
210 215 220

Ala Ala Gly Tyr Pro Pro Phe Phe Ala Asp Gln Pro Ile Gln Ile Tyr
225 230 235 240

Glu Lys Ile Val Ser Gly Lys Val Arg Phe Pro Ser His Phe Ser Ser
245 250 255

Asp Leu Lys Asp Leu Leu Arg Asn Leu Leu Gln Val Asp Leu Thr Lys
260 265 270

Arg Phe Gly Asn Leu Lys Asn Gly Val Asn Asp Ile Lys Asn His Lys
275 280 285

Trp Phe Ala Thr Thr Asp Trp Ile Ala Ile Tyr Gln Arg Lys Val Glu
290 295 300

Ala Pro Phe Ile Pro Lys Phe Lys Gly Pro Gly Asp Thr Ser Asn Phe
305 310 315 320

Asp Asp Tyr Glu Glu Glu Glu Ile Arg Val Ser Ile Asn Glu Lys Cys
325 330 335

Gly Lys Glu Phe Ser Glu Phe
340

<210> 57
<211> 4616
<212> DNA
<213> Homo sapiens

<400> 57
tgcgaagata cagtcgggcc agggcctggc ctgggcgcgc ggctgcccgg gggcgcgcag 60

agagggcgga ccgcgcgaag ggggagtgtc tgcccgccgc cgccactgct gctgccaccg 120

ccgtcgccgc cgccgccgcc gccgccgctg ctgctgccgg tgctaaggag ttcgctggag 180

ccctttcctc agacccggcc cggtcttcgc gcccggactc ctggcgccag cgctaggcgc 240

actcaccgct ctgacgggtg cagacgcggg agttgtccca gactgtggag tggcgggcac 300

ggccccagcc cccettccct tccctgaccc cttcttgcca tcgccccaga catggggaac 360

gcggcgaccg ccaagaaagg cagcgaggtg gagagcgtga aagagtttct agccaaagcc 420

aaagaagact ttttgaaaaa atgggagaat ccaactcaga ataatgccgg acttgaagat 480

tttgaaagga aaaaaaccct tggaacaggt tcatttggaa gagtcatgtt ggtaaaacac 540

aaagccactg aacagtatta tgccatgaag atcttagata agcagaaggt tgttaaactg 600

aagcaaatag agcatacttt gaatgagaaa agaatattac aggcagtgaa ttttcctttc 660

cttgttcgac tggagtatgc ttttaaggat aattctaatt tatacatggt tatggaatat 720

gtccctgggg gtgaaatgtt ttcacatcta agaagaattg gaaggttcag tgagccccat 780

gcacggttct atgcagctca gatagtgcta acattcgagt acctccattc actagacctc 840

atctacagag atctaaaacc tgaaaatctc ttaattgacc atcaaggcta tatccaggtc 900

acagactttg ggtttgccaa aagagttaaa ggcagaactt ggacattatg tggaactcca 960

gagtatttgg ctccagaaat aattctcagc aagggctaca ataaggcagt ggattggtgg 1020

gcattaggag tgctaatcta tgaaatggca gctggctatc cccattcttt tgcagaccaa 1080

ccaattcaga tttatgaaaa gattgtttct ggaaaggtcc gattcccatc ccacttcagt 1140

tcagatctca aggaccttct acggaacctg ctgcaggtgg atttgaccaa gagatttgga 1200

aatctaaaga atggtgtcag tgatataaaa actcacaagt ggtttgccac gacagattgg 1260

attgctattt accagaggaa ggttgaagct ccattcatac caaagtttag aggctctgga 1320

gataccagca actttgatga ctatgaagaa gaagatatcc gtgtctctat aacagaaaaa 1380

tgtgcaaaag aatttggtga attttaaaga ggaacaagat gacatctgag ctcacactca 1440

gtgtttgcac tctgttgaga gataaggtag agctgagacc gtccttgttg aagcagttac 1500

ctagttcctt cattccaacg actgagtgag gtctttattg ccatcatccc gtgtgcgcac 1560

tctgcatcca cctatgtaac aaggcaccgc taagcaagca ttgtctgtgc cataacacag 1620

tactagacca ctttcttact tctctttggg ttgtctttct cctctcctat atccatttct 1680

tccttttcca atttcattgg ttttctctaa acagtgctcc attttatttt gttggtgttt 1740

cagatgggca gtgttatggc tacgtgatat ttgaagggaa ggataagtgt tgctttcagt 1800

agttattgcc aatattgttg ttggtcaatg gcttgaagat aaactttcta ataattatta 1860

tttctttgag tagctcagac ttggttttgc caaaactctt ggtaattttt gaagatagac 1920

tgtcttatca ccaaggaaat ttatacaaat taagactaac tttcttggaa ttcactattc 1980

tggcaataaa ttttggtaga ctaatacagt acagctagac ccagaaattt ggaaggctgt 2040

agatcagagg ttctagttcc ctttccctcc ttttatatcc tcctctcctt gagtaatgaa 2100

gtgaccagcc tgtgtagtgt gacaaacgtg tctcattcag caggaaaaac taatgatatg 2160

gatcatcacc cagattctct cacttggtac cagcatttct gtaggtatta gagaagagtt 2220

ctaagttttc taaaccttaa ctgttcctta aggattttag ccagtatttt aatagaacat 2280

gattaatgaa agtgacaaat tttaaatttt ctctaatagt cctcatcata aactttttaa 2340

aggaaaataa gcaaactaaa aagaacattg gtttagataa atacttatac tttgcaaagt 2400

caaaaatggc ttgatttttg gaaacaatat agaggtattc atatttaaat gagggtttac 2460

atttgttttg ttttgtaacc gttaaaaaga agttgtttcc agctaattat tgtggtgtac 2520

tatatttgtg agcctagggt aggggcactg ctgcaacttc tgctttcatc ccatgcctca 2580

tcaatgagga aagggaacaa agtgtataaa actgccacaa ttgtatttta attttgaggt 2640

atgatatttt cagatatttc ataatttcta acctctgttc tctcagtaaa cagaatgtct 2700

gatcgatcat gcagatacaa tgttggtatt tgagaggtta gtttttttcc tacacttttt 2760

tttgccaact gacttaacaa cattgctgtc aggtggaaat ttcaagcact tttgcacatt 2820

tagttcagtg tttgttgaga atccatggct taacccactt gttttgctat ttttttcttt 2880

gcttttaatt ttccccatct gattttatct ctgcgtttca gtgacctacc ttaaaacaac 2940

acacgagaag agttaaactg ggttcatttt aatgatcaat ttacctgcat ataaaattta 3000

tttttaatca agctgatctt aatgtatata atcattctat ttgctttatt atcggtgcag 3060

gtaggtcatt aacaccactt cttttcatct gtaccacacc ctggtgaaac ctttgaagac 3120

ataaaaaaaa cctgtctgag atgttctttc taccaatcta tatgtctttc ggttatcaag 3180

tgtttctgca tggtaatgtc atgtaaatgc tgatattgat ttcactggtc catctatatt 3240

taaaacgtgc aagaaaaaaa taaaatactc tgctctagca agttttgtgt aacaaaggca 3300

tatcgtcatg ttaataaatt taaaacatca ttcgtataaa atattttaat tttcttgtat 3360

ttcatttaga cccaagaaca tgctgaccaa tgtgttctat atgtaaacta caaattctat 3420

ggtagctttg ttgtatatta ttgtaaaatt attttaataa gtcatgggga tgacaatttg 3480

attattacaa tttagttttc agtaatcaaa aagatttcta tgaattctaa aaaatatttt 3540

tttctatgaa attactagtg cccagctgta gaatctacct taggtagatg atccctagac 3600

atacgttggt tttgagggct attcagccat tccattttac tctctattta aaggccgtga 3660

gcaagcttgt catgagcaaa tatgtcaagg gagtcaattt ctgaccaatc aagtacacta 3720

aattagaata tttttaaagt atgtaacatt cccagtttca gccacaattt agccaagaat 3780

aagataaaaa cttgaataag aagtaagtag cataaatcag tatttaacct aaaattacat 3840

atttgaaaca gaagatatta tgttatgctc agtaaataat taagagatgg cattgtgtaa 3900

gaaggagccc tagactgaaa gtcaagacat ctgaatttca ggctggaaaa ctatcagtat 3960

gatctcagcc tcagttctct tgtctgtaaa atggaagaac tggattaggc agtttgtaag 4020

attcctccta actttcacag tcgatgacaa gattgtcttt ttatctgata ttttgaaggg 4080

tatattgctt tgaagtaagt ctcaataagg caatatattt tagggcatct ttcttcttat 4140

ctctgacagt gttcttaaaa ttatttgaat atcataagag ccttggtgtc tgtcctaatt 4200

cctttctcac tcaccgatgc tgaataccca gttgaatcaa actgtcaacc taccaaaaac 4260

gatattgtgg cttatgggta ttgctgtctc attcttggta tattcttgtg ttaactgccc 4320

attggcctga aatactcat tgtaagcctg aaaaaaaaaa tctttcccac tgttttttct 4380

gcttgttgta agaatcaaat gaaataatgt atgtgaaagc accttgtaaa ctgtaaccta 4440

tcaatgtaaa atgttaaggt gtgttgttat ttcattaatt acttctttgt ttagaatgga 4500

atttcctatg cactactgta gctaggaaat gctgaaaaca actgtgtttt ttaattaatc 4560

aataactgca aaattaaagt accttcaatg gataagacaa aaaaaaaaaa aaaaaa 4616

<210> 58
<211> 4481
<212> DNA
<213> Homo sapiens

<400> 58
acatgcatag ctcttagctt ctgtgtaaga agttgtgagc tccttctgga aacatttgca 60

gttacattaa gtaaagtgta aatgcacatg aatggcagct tatagagaac caccttgtaa 120

ccagtataca ggtacaacta cagctcttca gaaattggaa ggttttgcta gccggttatt 180

tcatagacac tctaaaggta ctgcacatga tcagaaaaca gctctggaaa atgacagcct 240

tcatttctct gaacatactg ccttatggga cagatcaatg aaagagtttc tagccaaagc 300

caaagaagac tttttgaaaa aatgggagaa tccaactcag aataatgccg gacttgaaga 360

ttttgaaagg aaaaaaaccc ttggaacagg ttcatttgga agagtcatgt tggtaaaaca 420

caaagccact gaacagtatt atgccatgaa gatcttagat aagcagaagg ttgttaaact 480

gaagcaaata gagcatactt tgaatgagaa aagaatatta caggcagtga attttccttt 540

ccttgttcga ctggagtatg cttttaagga taattctaat ttatacatgg ttatggaata 600

tgtccctggg ggtgaaatgt tttcacatct aagaagaatt ggaaggttca gtgagcccca 660

tgcacggttc tatgcagctc agatagtgct aacattcgag tacctccatt cactagacct 720

catctacaga gatctaaaac ctgaaaatct cttaattgac catcaaggct atatccaggt 780

cacagacttt gggtttgcca aaagagttaa aggcagaact tggacattat gtggaactcc 840

agagtatttg gctccagaaa taattctcag caagggctac aataaggcag tggattggtg 900

ggcattagga gtgctaatct atgaaatggc agctggctat cccccattct ttgcagacca 960

accaattcag atttatgaaa agattgtttc tggaaaggtc cgattcccat cccacttcag 1020

ttcagatctc aaggaccttc tacggaacct gctgcaggtg gatttgacca agagatttgg 1080

aaatctaaag aatggtgtca gtgatataaa aactcacaag tggtttgcca cgacagattg 1140

gattgctatt taccagagga aggttgaagc tccattcata ccaaagttta gaggctctgg 1200
agataccagc aactttgatg actatgaaga agaagatatc cgtgtctcta taacagaaaa 1260
atgtgcaaaa gaatttggtg aattttaaag aggaacaaga tgacatctga gctcacactc 1320
agtgtttgca ctctgttgag agataaggta gagctgagac cgtccttgtt gaagcagtta 1380
cctagttcct tcattccaac gactgagtga ggtctttatt gccatcatcc cgtgtgcgca 1440
ctctgcatcc acctatgtaa caaggcaccg ctaagcaagc attgtctgtg ccataacaca 1500
gtactagacc actttcttac ttctctttgg gttgtctttc tcctctccta tatccatttc 1560
ttccttttcc aatttcattg gttttctcta aacagtgctc cattttattt tgttggtgtt 1620
tcagatgggc agtgttatgg ctacgtgata tttgaaggga aggataagtg ttgctttcag 1680
tagttattgc caatattgtt gttggtcaat ggcttgaaga taaactttct aataattatt 1740
atttctttga gtagctcaga cttggttttg ccaaaactct tggtaatttt tgaagataga 1800
ctgtcttatc accaaggaaa tttatacaaa ttaagactaa ctttcttgga attcactatt 1860
ctggcaataa attttggtag actaatacag tacagctaga cccagaaatt tggaaggctg 1920
tagatcagag gttctagttc cctttccctc cttttatatc ctcctctcct tgagtaatga 1980
agtgaccagc ctgtgtagtg tgacaaacgt gtctcattca gcaggaaaaa ctaatgatat 2040
ggatcatcac ccagattctc tcacttggta ccagcatttc tgtaggtatt agagaagagt 2100
tctaagtttt ctaaacctta actgttcctt aaggatttta gccagtattt taatagaaca 2160
tgattaatga aagtgacaaa ttttaaattt tctctaatag tcctcatcat aaacttttta 2220
aaggaaaata agcaaactaa aaagaacatt ggtttagata aatacttata ctttgcaaag 2280
tcaaaaatgg cttgattttt ggaaacaata tagaggtatt catatttaaa tgagggttta 2340
catttgtttt gttttgtaac cgttaaaaag aagttgtttc cagctaatta ttgtggtgta 2400
ctatatttgt gagcctaggg taggggcact gctgcaactt ctgctttcat cccatgcctc 2460
atcaatgagg aaagggaaca aagtgtataa aactgccaca attgtatttt aattttgagg 2520
tatgatattt tcagatattt cataatttct aacctctgtt ctctcagtaa acagaatgtc 2580
tgatcgatca tgcagataca atgttggtat ttgagaggtt agttttttc ctacactttt 2640
ttttgccaac tgacttaaca acattgctgt caggtggaaa tttcaagcac ttttgcacat 2700
ttagttcagt gtttgttgag aatccatggc ttaacccact tgttttgcta tttttttctt 2760
tgcttttaat tttccccatc tgattttatc tctgcgtttc agtgacctac cttaaaacaa 2820
cacacgagaa gagttaaact gggttcattt taatgatcaa tttacctgca tataaaattt 2880
atttttaatc aagctgatct taatgtatat aatcattcta tttgctttat tatcggtgca 2940
ggtaggtcat taacaccact tcttttcatc tgtaccacac cctggtgaaa cctttgaaga 3000
cataaaaaaa acctgtctga gatgttcttt ctaccaatct atatgtcttt cggttatcaa 3060

gtgtttctgc atggtaatgt catgtaaatg ctgatattga tttcactggt ccatctatat 3120

ttaaaacgtg caagaaaaaa ataaaatact ctgctctagc aagttttgtg taacaaaggc 3180

atatcgtcat gttaataaat ttaaaacatc attcgtataa aatattttaa ttttcttgta 3240

tttcatttag acccaagaac atgctgacca atgtgttcta tatgtaaact acaaattcta 3300

tggtagcttt gttgtatatt attgtaaaat tattttaata agtcatgggg atgacaattt 3360

gattattaca atttagtttt cagtaatcaa aaagatttct atgaattcta aaaaatattt 3420

ttttctatga aattactagt gcccagctgt agaatctacc ttaggtagat gatccctaga 3480

catacgttgg ttttgagggc tattcagcca ttccatttta ctctctattt aaaggccgtg 3540

agcaagcttg tcatgagcaa atatgtcaag ggagtcaatt tctgaccaat caagtacact 3600

aaattagaat atttttaaag tatgtaacat tcccagtttc agccacaatt tagccaagaa 3660

taagataaaa acttgaataa gaagtaagta gcataaatca gtatttaacc taaaattaca 3720

tatttgaaac agaagatatt atgttatgct cagtaaataa ttaagagatg gcattgtgta 3780

agaaggagcc ctagactgaa agtcaagaca tctgaatttc aggctggaaa actatcagta 3840

tgatctcagc ctcagttctc ttgtctgtaa aatggaagaa ctggattagg cagtttgtaa 3900

gattcctcct aactttcaca gtcgatgaca agattgtctt tttatctgat attttgaagg 3960

gtatattgct ttgaagtaag tctcaataag gcaatatatt ttagggcatc tttcttctta 4020

tctctgacag tgttcttaaa attatttgaa tatcataaga gccttggtgt ctgtcctaat 4080

tcctttctca ctcaccgatg ctgaataccc agttgaatca aactgtcaac ctaccaaaaa 4140

cgatattgtg gcttatgggt attgctgtct cattcttggt atattcttgt gttaactgcc 4200

cattggcctg aaaatactca ttgtaagcct gaaaaaaaaa atctttccca ctgtttttc 4260

tgcttgttgt aagaatcaaa tgaaataatg tatgtgaaag caccttgtaa actgtaacct 4320

atcaatgtaa aatgttaagg tgtgttgtta tttcattaat tacttctttg tttagaatgg 4380

aatttcctat gcactactgt agctaggaaa tgctgaaaac aactgtgttt tttaattaat 4440

caataactgc aaaattaaag taccttcaat ggataagaca a 4481

<210> 59
<211> 4650
<212> DNA
<213> Homo sapiens

<400> 59
atgctgatat aattgagaac atcttataca tcctggttcg aacattttct ccctgccatt 60

ttgagttgtt ctagtggtat atgaaggagg ctgggataac tagcttgaaa gaaattcagt 120

ctagttatag acatctttgg cattaatctg atgtttacta gtgatatctc atgctaggca 180

gttatgcttt gcttctaggg gcttctcttt ttaaaacaaa agaaagctct tttcgttttc 240

tgtgtgctgc atgctccagt gtgtgtgttt acaccatcgg ttcttctccc tctagagatt 300
agcataactc cctttgctgt tggattgtta ttttgagcaa tatgttttgg aaaggttggt 360
tttcatcatg agtgcacgca aatcatcaga tgcatctgct tgctcctctt cagaaatatc 420
tgattccttt gtgaaagagt ttctagccaa agccaaagaa gactttttga aaaaatggga 480
gaatccaact cagaataatg ccggacttga agattttgaa aggaaaaaaa cccttggaac 540
aggttcattt ggaagagtca tgttggtaaa acacaaagcc actgaacagt attatgccat 600
gaagatctta gataagcaga aggttgttaa actgaagcaa atagagcata ctttgaatga 660
gaaaagaata ttacaggcag tgaattttcc tttccttgtt cgactggagt atgcttttaa 720
ggataattct aatttataca tggttatgga atatgtccct gggggtgaaa tgttttcaca 780
tctaagaaga attggaaggt tcagtgagcc ccatgcacgg ttctatgcag ctcagatagt 840
gctaacattc gagtacctcc attcactaga cctcatctac agagatctaa aacctgaaaa 900
tctcttaatt gaccatcaag gctatatcca ggtcacagac tttgggtttg ccaaaagagt 960
taaaggcaga acttggacat tatgtggaac tccagagtat ttggctccag aaataattct 1020
cagcaagggc tacaataagg cagtggattg gtgggcatta ggagtgctaa tctatgaaat 1080
ggcagctggc tatcccccat tctttgcaga ccaaccaatt cagatttatg aaaagattgt 1140
ttctggaaag gtccgattcc catcccactt cagttcagat ctcaaggacc ttctacggaa 1200
cctgctgcag gtggatttga ccaagagatt tggaaatcta aagaatggtg tcagtgatat 1260
aaaaactcac aagtggtttg ccacgacaga ttggattgct atttaccaga ggaaggttga 1320
agctccattc ataccaaagt ttagaggctc tggagatacc agcaactttg atgactatga 1380
agaagaagat atccgtgtct ctataacaga aaaatgtgca aaagaatttg gtgaatttta 1440
aagaggaaca agatgacatc tgagctcaca ctcagtgttt gcactctgtt gagagataag 1500
gtagagctga gaccgtcctt gttgaagcag ttacctagtt ccttcattcc aacgactgag 1560
tgaggtcttt attgccatca tcccgtgtgc gcactctgca tccacctatg taacaaggca 1620
ccgctaagca agcattgtct gtgccataac acagtactag accactttct tacttctctt 1680
tgggttgtct ttctcctctc ctatatccat ttcttccttt tccaatttca ttggttttct 1740
ctaaacagtg ctccatttta ttttgttggt gtttcagatg ggcagtgtta tggctacgtg 1800
atatttgaag ggaaggataa gtgttgcttt cagtagttat tgccaatatt gttgttggtc 1860
aatggcttga agataaactt tctaataatt attatttctt tgagtagctc agacttggtt 1920
ttgccaaaac tcttggtaat tttgaagat agactgtctt atcaccaagg aaatttatac 1980
aaattaagac taactttctt ggaattcact attctggcaa taaattttgg tagactaata 2040
cagtacagct agacccagaa atttggaagg ctgtagatca gaggttctag ttccctttcc 2100

ctccttttat atcctcctct ccttgagtaa tgaagtgacc agcctgtgta gtgtgacaaa 2160
cgtgtctcat tcagcaggaa aaactaatga tatggatcat cacccagatt ctctcacttg 2220
gtaccagcat ttctgtaggt attagagaag agttctaagt tttctaaacc ttaactgttc 2280
cttaaggatt ttagccagta ttttaataga acatgattaa tgaaagtgac aaattttaaa 2340
ttttctctaa tagtcctcat cataaacttt ttaaaggaaa ataagcaaac taaaaagaac 2400
attggtttag ataaatactt atactttgca aagtcaaaaa tggcttgatt tttggaaaca 2460
atatagaggt attcatattt aaatgagggt ttacatttgt tttgttttgt aaccgttaaa 2520
aagaagttgt ttccagctaa ttattgtggt gtactatatt tgtgagccta gggtaggggc 2580
actgctgcaa cttctgcttt catcccatgc ctcatcaatg aggaaaggga acaaagtgta 2640
taaaactgcc acaattgtat tttaattttg aggtatgata ttttcagata tttcataatt 2700
tctaacctct gttctctcag taaacagaat gtctgatcga tcatgcagat acaatgttgg 2760
tatttgagag gttagttttt ttcctacact ttttttttgcc aactgactta acaacattgc 2820
tgtcaggtgg aaatttcaag cacttttgca catttagttc agtgtttgtt gagaatccat 2880
ggcttaaccc acttgttttg ctattttttt ctttgctttt aattttcccc atctgatttt 2940
atctctgcgt ttcagtgacc taccttaaaa caacacacga gaagagttaa actgggttca 3000
ttttaatgat caatttacct gcatataaaa tttattttta atcaagctga tcttaatgta 3060
tataatcatt ctatttgctt tattatcggt gcaggtaggt cattaacacc acttcttttc 3120
atctgtacca caccctggtg aaacctttga agacataaaa aaaacctgtc tgagatgttc 3180
tttctaccaa tctatatgtc tttcggttat caagtgtttc tgcatggtaa tgtcatgtaa 3240
atgctgatat tgatttcact ggtccatcta tatttaaaac gtgcaagaaa aaaataaaat 3300
actctgctct agcaagtttt gtgtaacaaa ggcatatcgt catgttaata aatttaaaac 3360
atcattcgta taaaatattt taattttctt gtatttcatt tagacccaag aacatgctga 3420
ccaatgtgtt ctatatgtaa actacaaatt ctatggtagc tttgttgtat attattgtaa 3480
aattatttta ataagtcatg ggatgacaa tttgattatt acaatttagt tttcagtaat 3540
caaaaagatt tctatgaatt ctaaaaaata tttttttcta tgaaattact agtgcccagc 3600
tgtagaatct accttaggta gatgatccct agacatacgt tggttttgag ggctattcag 3660
ccattccatt ttactctcta tttaaaggcc gtgagcaagc ttgtcatgag caaatatgtc 3720
aagggagtca atttctgacc aatcaagtac actaaattag aatattttta aagtatgtaa 3780
cattcccagt ttcagccaca atttagccaa gaataagata aaaacttgaa taagaagtaa 3840
gtagcataaa tcagtattta acctaaaatt acatatttga aacagaagat attatgttat 3900
gctcagtaaa taattaagag atggcattgt gtaagaagga gccctagact gaaagtcaag 3960
acatctgaat ttcaggctgg aaaactatca gtatgatctc agcctcagtt ctcttgtctg 4020

taaaatggaa gaactggatt aggcagtttg taagattcct cctaactttc acagtcgatg 4080

acaagattgt ctttttatct gatattttga agggtatatt gctttgaagt aagtctcaat 4140

aaggcaatat attttagggc atctttcttc ttatctctga cagtgttctt aaaattattt 4200

gaatatcata agagccttgg tgtctgtcct aattcctttc tcactcaccg atgctgaata 4260

cccagttgaa tcaaactgtc aacctaccaa aaacgatatt gtggcttatg ggtattgctg 4320

tctcattctt ggtatattct tgtgttaact gcccattggc ctgaaaatac tcattgtaag 4380

cctgaaaaaa aaaatctttc ccactgtttt ttctgcttgt tgtaagaatc aaatgaaata 4440

atgtatgtga aagcaccttg taaactgtaa cctatcaatg taaaatgtta aggtgtgttg 4500

ttatttcatt aattacttct ttgtttagaa tggaatttcc tatgcactac tgtagctagg 4560

aaatgctgaa aacaactgtg ttttttaatt aatcaataac tgcaaaatta aagtaccttc 4620

aatggataag acaaaaaaaa aaaaaaaaaa 4650

<210> 60
<211> 4506
<212> DNA
<213> Homo sapiens

<400> 60
taacacagaa tgtttaaatg gttaacattt gtgctgcagt atagctttct ggctcatgaa 60

aaatgaaagc tatcagcgat ctcggcaata agattcatcg ccaatagtca ctagcaacag 120

cacacagcat tttaatatca gtgaggtcca cagctagcag taagagctgg tgtaattgaa 180

agacgtttag gtgcaatcat tctgctgttt gctccttgcc aggttcaaca tgggattgtc 240

acgcaaatca tcagatgcat ctgcttgctc ctcttcagaa atatctgtga aagagtttct 300

agccaaagcc aaagaagact ttttgaaaaa atgggagaat ccaactcaga ataatgccgg 360

acttgaagat tttgaaagga aaaaaaccct tggaacaggt tcatttggaa gagtcatgtt 420

ggtaaaacac aaagccactg aacagtatta tgccatgaag atcttagata agcagaaggt 480

tgttaaactg aagcaaatag agcatacttt gaatgagaaa agaatattac aggcagtgaa 540

ttttcctttc cttgttcgac tggagtatgc ttttaaggat aattctaatt tatacatggt 600

tatggaatat gtccctgggg gtgaaatgtt ttcacatcta agaagaattg gaaggttcag 660

tgagccccat gcacggttct atgcagctca gatagtgcta acattcgagt acctccattc 720

actagacctc atctacagag atctaaaacc tgaaaatctc ttaattgacc atcaaggcta 780

tatccaggtc acagactttg ggtttgccaa aagagttaaa ggcagaactt ggacattatg 840

tggaactcca gagtatttgg ctccagaaat aattctcagc aagggctaca ataaggcagt 900

ggattggtgg gcattaggag tgctaatcta tgaaatggca gctggctatc ccccattctt 960

tgcagaccaa ccaattcaga tttatgaaaa gattgtttct ggaaaggtcc gattcccatc 1020

ccacttcagt tcagatctca aggaccttct acggaacctg ctgcaggtgg atttgaccaa 1080
gagatttgga aatctaaaga atggtgtcag tgatataaaa actcacaagt ggtttgccac 1140
gacagattgg attgctattt accagaggaa ggttgaagct ccattcatac caaagtttag 1200
aggctctgga gataccagca actttgatga ctatgaagaa gaagatatcc gtgtctctat 1260
aacagaaaaa tgtgcaaaag aatttggtga attttaaaga ggaacaagat gacatctgag 1320
ctcacactca gtgtttgcac tctgttgaga gataaggtag agctgagacc gtccttgttg 1380
aagcagttac ctagttcctt cattccaacg actgagtgag gtctttattg ccatcatccc 1440
gtgtgcgcac tctgcatcca cctatgtaac aaggcaccgc taagcaagca ttgtctgtgc 1500
cataacacag tactagacca ctttcttact tctctttggg ttgtctttct cctctcctat 1560
atccatttct tccttttcca atttcattgg ttttctctaa acagtgctcc attttatttt 1620
gttggtgttt cagatgggca gtgttatggc tacgtgatat ttgaagggaa ggataagtgt 1680
tgctttcagt agttattgcc aatattgttg ttggtcaatg gcttgaagat aaactttcta 1740
ataattatta tttctttgag tagctcagac ttggttttgc caaaactctt ggtaattttt 1800
gaagatagac tgtcttatca ccaaggaaat ttatacaaat taagactaac tttcttggaa 1860
ttcactattc tggcaataaa ttttggtaga ctaatacagt acagctagac ccagaaattt 1920
ggaaggctgt agatcagagg ttctagttcc ctttccctcc ttttatatcc tcctctcctt 1980
gagtaatgaa gtgaccagcc tgtgtagtgt gacaaacgtg tctcattcag caggaaaaac 2040
taatgatatg gatcatcacc cagattctct cacttggtac cagcatttct gtaggtatta 2100
gagaagagtt ctaagttttc taaaccttaa ctgttcctta aggattttag ccagtatttt 2160
aatagaacat gattaatgaa agtgacaaat tttaaatttt ctctaatagt cctcatcata 2220
aactttttaa aggaaaataa gcaaactaaa aagaacattg gtttagataa atacttatac 2280
tttgcaaagt caaaaatggc ttgatttttg gaaacaatat agaggtattc atatttaaat 2340
gagggtttac atttgttttg ttttgtaacc gttaaaaaga agttgtttcc agctaattat 2400
tgtggtgtac tatatttgtg agcctagggt aggggcactg ctgcaacttc tgctttcatc 2460
ccatgcctca tcaatgagga aagggaacaa agtgtataaa actgccacaa ttgtatttta 2520
attttgaggt atgatatttt cagatatttc ataatttcta acctctgttc tctcagtaaa 2580
cagaatgtct gatcgatcat gcagatacaa tgttggtatt tgagaggtta gtttttttcc 2640
tacacttttt tttgccaact gacttaacaa cattgctgtc aggtggaaat ttcaagcact 2700
tttgcacatt tagttcagtg tttgttgaga atccatggct taacccactt gttttgctat 2760
ttttttcttt gcttttaatt ttccccatct gattttatct ctgcgtttca gtgacctacc 2820
ttaaaacaac acacgagaag agttaaactg ggttcatttt aatgatcaat ttacctgcat 2880

ataaaattta tttttaatca agctgatctt aatgtatata atcattctat ttgctttatt 2940

atcggtgcag gtaggtcatt aacaccactt cttttcatct gtaccacacc ctggtgaaac 3000

ctttgaagac ataaaaaaaa cctgtctgag atgttctttc taccaatcta tatgtctttc 3060

ggttatcaag tgtttctgca tggtaatgtc atgtaaatgc tgatattgat ttcactggtc 3120

catctatatt taaaacgtgc aagaaaaaaa taaaatactc tgctctagca agttttgtgt 3180

aacaaaggca tatcgtcatg ttaataaatt taaaacatca ttcgtataaa atattttaat 3240

tttcttgtat ttcatttaga cccaagaaca tgctgaccaa tgtgttctat atgtaaacta 3300

caaattctat ggtagctttg ttgtatatta ttgtaaaatt attttaataa gtcatgggga 3360

tgacaatttg attattacaa tttagttttc agtaatcaaa aagatttcta tgaattctaa 3420

aaaatatttt tttctatgaa attactagtg cccagctgta gaatctacct taggtagatg 3480

atccctagac atacgttggt tttgagggct attcagccat tccattttac tctctattta 3540

aaggccgtga gcaagcttgt catgagcaaa tatgtcaagg gagtcaattt ctgaccaatc 3600

aagtacacta aattagaata tttttaaagt atgtaacatt cccagtttca gccacaattt 3660

agccaagaat aagataaaaa cttgaataag aagtaagtag cataaatcag tatttaacct 3720

aaaattacat atttgaaaca gaagatatta tgttatgctc agtaaataat taagagatgg 3780

cattgtgtaa gaaggagccc tagactgaaa gtcaagacat ctgaatttca ggctggaaaa 3840

ctatcagtat gatctcagcc tcagttctct tgtctgtaaa atggaagaac tggattaggc 3900

agtttgtaag attcctccta actttcacag tcgatgacaa gattgtcttt ttatctgata 3960

ttttgaaggg tatattgctt tgaagtaagt ctcaataagg caatatattt tagggcatct 4020

ttcttcttat ctctgacagt gttcttaaaa ttatttgaat atcataagag ccttggtgtc 4080

tgtcctaatt cctttctcac tcaccgatgc tgaataccca gttgaatcaa actgtcaacc 4140

taccaaaaac gatattgtgg cttatgggta ttgctgtctc attcttggta tattcttgtg 4200

ttaactgccc attggcctga aaatactcat tgtaagcctg aaaaaaaaaa tctttcccac 4260

tgtttttttct gcttgttgta agaatcaaat gaaataatgt atgtgaaagc accttgtaaa 4320

ctgtaaccta tcaatgtaaa atgttaaggt gtgttgttat ttcattaatt acttctttgt 4380

ttagaatgga atttcctatg cactactgta gctaggaaat gctgaaaaca actgtgtttt 4440

ttaattaatc aataactgca aaattaaagt accttcaatg gataagacaa aaaaaaaaaa 4500

aaaaaa 4506

<210> 61
<211> 4458
<212> DNA
<213> Homo sapiens

<400> 61

taacacagaa tgtttaaatg gttaacattt gtgctgcagt atagctttct ggctcatgaa 60
aaatgaaagc tatcagcgat ctcggcaata agattcatcg ccaatagtca ctagcaacag 120
cacacagcat tttaatatca gtgaggtcca cagctagcag taagagctgg tgtaattgaa 180
agacgtttag gtgcaatcat tctgctgttt gctccttgcc aggttcaaca tgggattgtt 240
gaaagagttt ctagccaaag ccaaagaaga ctttttgaaa aaatgggaga atccaactca 300
gaataatgcc ggacttgaag attttgaaag gaaaaaaacc cttggaacag gttcatttgg 360
aagagtcatg ttggtaaaac acaaagccac tgaacagtat tatgccatga agatcttaga 420
taagcagaag gttgttaaac tgaagcaaat agagcatact ttgaatgaga aaagaatatt 480
acaggcagtg aattttcctt tccttgttcg actggagtat gcttttaagg ataattctaa 540
tttatacatg gttatggaat atgtccctgg gggtgaaatg ttttcacatc taagaagaat 600
tggaaggttc agtgagcccc atgcacggtt ctatgcagct cagatagtgc taacattcga 660
gtacctccat tcactagacc tcatctacag agatctaaaa cctgaaaatc tcttaattga 720
ccatcaaggc tatatccagg tcacagactt tgggtttgcc aaaagagtta aaggcagaac 780
ttggacatta tgtggaactc cagagtattt ggctccagaa ataattctca gcaagggcta 840
caataaggca gtggattggt gggcattagg agtgctaatc tatgaaatgg cagctggcta 900
tcccccattc tttgcagacc aaccaattca gatttatgaa aagattgttt ctggaaaggt 960
ccgattccca tcccacttca gttcagatct caaggacctt ctacggaacc tgctgcaggt 1020
ggatttgacc aagagatttg gaaatctaaa gaatggtgtc agtgatataa aaactcacaa 1080
gtggtttgcc acgacagatt ggattgctat ttaccagagg aaggttgaag ctccattcat 1140
accaaagttt agaggctctg gagataccag caactttgat gactatgaag aagaagatat 1200
ccgtgtctct ataacagaaa aatgtgcaaa agaatttggt gaattttaaa gaggaacaag 1260
atgacatctg agctcacact cagtgtttgc actctgttga gagataaggt agagctgaga 1320
ccgtccttgt tgaagcagtt acctagttcc ttcattccaa cgactgagtg aggtctttat 1380
tgccatcatc ccgtgtgcgc actctgcatc cacctatgta acaaggcacc gctaagcaag 1440
cattgtctgt gccataacac agtactagac cactttctta cttctctttg ggttgtcttt 1500
ctcctctcct atatccattt cttccttttc caatttcatt ggtttctct aaacagtgct 1560
ccattttatt ttgttggtgt ttcagatggg cagtgttatg gctacgtgat atttgaaggg 1620
aaggataagt gttgctttca gtagttattg ccaatattgt tgttggtcaa tggcttgaag 1680
ataaactttc taataattat tatttctttg agtagctcag acttggtttt gccaaaactc 1740
ttggtaattt ttgaagatag actgtcttat caccaaggaa atttatacaa attaagacta 1800
actttcttgg aattcactat tctggcaata aattttggta gactaataca gtacagctag 1860
acccagaaat ttggaaggct gtagatcaga ggttctagtt ccctttccct ccttttatat 1920

cctcctctcc ttgagtaatg aagtgaccag cctgtgtagt gtgacaaacg tgtctcattc 1980

agcaggaaaa actaatgata tggatcatca cccagattct ctcacttggt accagcattt 2040

ctgtaggtat tagagaagag ttctaagttt tctaaacctt aactgttcct taaggatttt 2100

agccagtatt ttaatagaac atgattaatg aaagtgacaa attttaaatt ttctctaata 2160

gtcctcatca taaacttttt aaaggaaaat aagcaaacta aaaagaacat tggtttagat 2220

aaatacttat actttgcaaa gtcaaaaatg gcttgatttt tggaaacaat atagaggtat 2280

tcatatttaa atgagggttt acatttgttt tgttttgtaa ccgttaaaaa gaagttgttt 2340

ccagctaatt attgtggtgt actatatttg tgagcctagg gtaggggcac tgctgcaact 2400

tctgctttca tcccatgcct catcaatgag gaaagggaac aaagtgtata aaactgccac 2460

aattgtattt taattttgag gtatgatatt ttcagatatt tcataatttc taacctctgt 2520

tctctcagta aacagaatgt ctgatcgatc atgcagatac aatgttggta tttgagaggt 2580

tagttttttt cctacacttt tttttgccaa ctgacttaac aacattgctg tcaggtggaa 2640

atttcaagca cttttgcaca tttagttcag tgtttgttga gaatccatgg cttaacccac 2700

ttgttttgct atttttttct ttgcttttaa ttttccccat ctgattttat ctctgcgttt 2760

cagtgaccta ccttaaaaca acacacgaga agagttaaac tgggttcatt ttaatgatca 2820

atttacctgc atataaaatt tatttttaat caagctgatc ttaatgtata taatcattct 2880

atttgcttta ttatcggtgc aggtaggtca ttaacaccac ttcttttcat ctgtaccaca 2940

ccctggtgaa acctttgaag acataaaaaa aacctgtctg agatgttctt tctaccaatc 3000

tatatgtctt tcggttatca agtgtttctg catggtaatg tcatgtaaat gctgatattg 3060

atttcactgg tccatctata tttaaaacgt gcaagaaaaa aataaaatac tctgctctag 3120

caagttttgt gtaacaaagg catatcgtca tgttaataaa tttaaaacat cattcgtata 3180

aaatatttta attttcttgt atttcattta gacccaagaa catgctgacc aatgtgttct 3240

atatgtaaac tacaaattct atggtagctt tgttgtatat tattgtaaaa ttattttaat 3300

aagtcatggg gatgacaatt tgattattac aatttagttt tcagtaatca aaaagatttc 3360

tatgaattct aaaaaatatt tttttctatg aaattactag tgcccagctg tagaatctac 3420

cttaggtaga tgatccctag acatacgttg gttttgaggg ctattcagcc attccatttt 3480

actctctatt taaaggccgt gagcaagctt gtcatgagca aatatgtcaa gggagtcaat 3540

ttctgaccaa tcaagtacac taaattagaa tatttttaaa gtatgtaaca ttcccagttt 3600

cagccacaat ttagccaaga ataagataaa aacttgaata agaagtaagt agcataaatc 3660

agtatttaac ctaaaattac atatttgaaa cagaagatat tatgttatgc tcagtaaata 3720

attaagagat ggcattgtgt aagaaggagc cctagactga aagtcaagac atctgaattt 3780

caggctggaa aactatcagt atgatctcag cctcagttct cttgtctgta aaatggaaga 3840

actggattag gcagtttgta agattcctcc taactttcac agtcgatgac aagattgtct 3900

ttttatctga tattttgaag ggtatattgc tttgaagtaa gtctcaataa ggcaatatat 3960

tttagggcat ctttcttctt atctctgaca gtgttcttaa aattatttga atatcataag 4020

agccttggtg tctgtcctaa ttcctttctc actcaccgat gctgaatacc cagttgaatc 4080

aaactgtcaa cctaccaaaa acgatattgt ggcttatggg tattgctgtc tcattcttgg 4140

tatattcttg tgttaactgc ccattggcct gaaaatactc attgtaagcc tgaaaaaaaa 4200

aatctttccc actgtttttt ctgcttgttg taagaatcaa atgaaataat gtatgtgaaa 4260

gcaccttgta aactgtaacc tatcaatgta aaatgttaag gtgtgttgtt atttcattaa 4320

ttacttcttt gtttagaatg gaatttccta tgcactactg tagctaggaa atgctgaaaa 4380

caactgtgtt ttttaattaa tcaataactg caaaattaaa gtaccttcaa tggataagac 4440

aaaaaaaaaa aaaaaaa 4458

<210> 62
<211> 4254
<212> DNA
<213> Homo sapiens

<400> 62
taaagagaga aagccactag gctctctcat gctgctacta tctagttcta taagcttata 60

taaagacaga aatgaagcaa gactgatttc ttcacagaat aatgccggac ttgaagattt 120

tgaaaggaaa aaaacccttg gaacaggttc atttggaaga gtcatgttgg taaaacacaa 180

agccactgaa cagtattatg ccatgaagat cttagataag cagaaggttg ttaaactgaa 240

gcaaatagag catactttga atgagaaaag aatattacag gcagtgaatt ttcctttcct 300

tgttcgactg gagtatgctt ttaaggataa ttctaattta tacatggtta tggaatatgt 360

ccctgggggt gaaatgtttt cacatctaag aagaattgga aggttcagtg agccccatgc 420

acggttctat gcagctcaga tagtgctaac attcgagtac ctccattcac tagacctcat 480

ctacagagat ctaaaacctg aaaatctctt aattgaccat caaggctata tccaggtcac 540

agactttggg tttgccaaaa gagttaaagg cagaacttgg acattatgtg gaactccaga 600

gtatttggct ccagaaataa ttctcagcaa gggctacaat aaggcagtgg attggtgggc 660

attaggagtg ctaatctatg aaatggcagc tggctatccc ccattctttg cagaccaacc 720

aattcagatt tatgaaaaga ttgtttctgg aaaggtccga ttcccatccc acttcagttc 780

agatctcaag gaccttctac ggaacctgct gcaggtggat ttgaccaaga gatttggaaa 840

tctaaagaat ggtgtcagtg atataaaaac tcacaagtgg tttgccacga cagattggat 900

tgctatttac cagaggaagg ttgaagctcc attcatacca aagtttagag gctctggaga 960

```
taccagcaac tttgatgact atgaagaaga agatatccgt gtctctataa cagaaaaatg     1020
tgcaaaagaa tttggtgaat tttaaagagg aacaagatga catctgagct cacactcagt     1080
gtttgcactc tgttgagaga taaggtagag ctgagaccgt ccttgttgaa gcagttacct     1140
agttccttca ttccaacgac tgagtgaggt ctttattgcc atcatcccgt gtgcgcactc     1200
tgcatccacc tatgtaacaa ggcaccgcta agcaagcatt gtctgtgcca taacacagta     1260
ctagaccact ttcttacttc tctttgggtt gtctttctcc tctcctatat ccatttcttc     1320
cttttccaat ttcattggtt ttctctaaac agtgctccat tttattttgt tggtgtttca     1380
gatgggcagt gttatggcta cgtgatattt gaagggaagg ataagtgttg ctttcagtag     1440
ttattgccaa tattgttgtt ggtcaatggc ttgaagataa actttctaat aattattatt     1500
tctttgagta gctcagactt ggttttgcca aaactcttgg taatttttga agatagactg     1560
tcttatcacc aaggaaattt atacaaatta agactaactt tcttggaatt cactattctg     1620
gcaataaatt ttggtagact aatacagtac agctagaccc agaaatttgg aaggctgtag     1680
atcagaggtt ctagttccct ttccctcctt ttatatcctc ctctccttga gtaatgaagt     1740
gaccagcctg tgtagtgtga caaacgtgtc tcattcagca ggaaaaacta atgatatgga     1800
tcatcaccca gattctctca cttggtacca gcatttctgt aggtattaga gaagagttct     1860
aagttttcta aaccttaact gttccttaag gattttagcc agtattttaa tagaacatga     1920
ttaatgaaag tgacaaattt taaattttct ctaatagtcc tcatcataaa ctttttaaag     1980
gaaaataagc aaactaaaaa gaacattggt ttagataaat acttatactt tgcaaagtca     2040
aaaatggctt gatttttgga aacaatatag aggtattcat atttaaatga gggtttacat     2100
ttgttttgtt ttgtaaccgt taaaaagaag ttgtttccag ctaattattg tggtgtacta     2160
tatttgtgag cctagggtag gggcactgct gcaacttctg ctttcatccc atgcctcatc     2220
aatgaggaaa gggaacaaag tgtataaaac tgccacaatt gtattttaat tttgaggtat     2280
gatattttca gatatttcat aatttctaac ctctgttctc tcagtaaaca gaatgtctga     2340
tcgatcatgc agatacaatg ttggtatttg agaggttagt ttttttccta cacttttttt     2400
tgccaactga cttaacaaca ttgctgtcag gtggaaattt caagcacttt tgcacattta     2460
gttcagtgtt tgttgagaat ccatggctta acccacttgt tttgctattt ttttctttgc     2520
ttttaatttt ccccatctga ttttatctct gcgtttcagt gacctacctt aaaacaacac     2580
acgagaagag ttaaactggg ttcattttaa tgatcaattt acctgcatat aaaatttatt     2640
tttaatcaag ctgatcttaa tgtatataat cattctattt gctttattat cggtgcaggt     2700
aggtcattaa caccacttct tttcatctgt accacaccct ggtgaaacct ttgaagacat     2760
aaaaaaaacc tgtctgagat gttctttcta ccaatctata tgtctttcgg ttatcaagtg     2820
tttctgcatg gtaatgtcat gtaaatgctg atattgattt cactggtcca tctatattta     2880
```

aaacgtgcaa gaaaaaaata aaatactctg ctctagcaag ttttgtgtaa caaaggcata 2940

tcgtcatgtt aataaattta aaacatcatt cgtataaaat attttaattt tcttgtattt 3000

catttagacc caagaacatg ctgaccaatg tgttctatat gtaaactaca aattctatgg 3060

tagctttgtt gtatattatt gtaaaattat tttaataagt catggggatg acaatttgat 3120

tattacaatt tagttttcag taatcaaaaa gatttctatg aattctaaaa aatatttttt 3180

tctatgaaat tactagtgcc cagctgtaga atctacctta ggtagatgat ccctagacat 3240

acgttggttt tgagggctat tcagccattc cattttactc tctatttaaa ggccgtgagc 3300

aagcttgtca tgagcaaata tgtcaaggga gtcaatttct gaccaatcaa gtacactaaa 3360

ttagaatatt tttaaagtat gtaacattcc cagtttcagc cacaatttag ccaagaataa 3420

gataaaaact tgaataagaa gtaagtagca taaatcagta tttaacctaa aattacatat 3480

ttgaaacaga agatattatg ttatgctcag taaataatta agagatggca ttgtgtaaga 3540

aggagcccta gactgaaagt caagacatct gaatttcagg ctggaaaact atcagtatga 3600

tctcagcctc agttctcttg tctgtaaaat ggaagaactg gattaggcag tttgtaagat 3660

tcctcctaac tttcacagtc gatgacaaga ttgtcttttt atctgatatt ttgaagggta 3720

tattgctttg aagtaagtct caataaggca atatatttta gggcatcttt cttcttatct 3780

ctgacagtgt tcttaaaatt atttgaatat cataagagcc ttggtgtctg tcctaattcc 3840

tttctcactc accgatgctg aatacccagt tgaatcaaac tgtcaaccta ccaaaaacga 3900

tattgtggct tatgggtatt gctgtctcat tcttggtata ttcttgtgtt aactgcccat 3960

tggcctgaaa atactcattg taagcctgaa aaaaaaaatc tttcccactg tttttttctgc 4020

ttgttgtaag aatcaaatga aataatgtat gtgaaagcac cttgtaaact gtaacctatc 4080

aatgtaaaat gttaaggtgt gttgttattt cattaattac ttctttgttt agaatggaat 4140

ttcctatgca ctactgtagc taggaaatgc tgaaaacaac tgtgtttttt aattaatcaa 4200

taactgcaaa attaaagtac cttcaatgga taagacaaaa aaaaaaaaaa aaaa 4254

<210> 63
<211> 4542
<212> DNA
<213> Homo sapiens

<400> 63
atgctgatat aattgagaac atcttataca tcctggttcg aacattttct ccctgccatt 60

ttgagttgtt ctagtggtat atgaaggagg ctgggataac tagcttgaaa gaaattcagt 120

ctagttatag acatctttgg cattaatctg atgtttacta gtgatatctc atgctaggca 180

gttatgcttt gcttctaggg gcttctcttt ttaaaacaaa agaaagctct tttcgttttc 240

tgtgtgctgc atgctccagt gtgtgtgttt acaccatcgg ttcttctccc tctagagatt 300

agcataactc cctttgctgt tggattgtta ttttgagcaa tatgttttgg aaaggttggt 360
tttcatcatg agtgcacgca aatcatcaga tgcatctgct tgctcctctt cagaaatatc 420
tgtgaaagag tttctagcca aagccaaaga agactttttg aaaaaatggg agaatccaac 480
tcagaataat gccggacttg aagattttga aaggaaaaaa acccttggaa caggttcatt 540
tggaagagtc atgttggtaa aacacaaagc cactgaacag tattatgcca tgaagatctt 600
agataagcag aaggataatt ctaatttata catggttatg gaatatgtcc ctgggggtga 660
aatgttttca catctaagaa gaattggaag gttcagtgag ccccatgcac ggttctatgc 720
agctcagata gtgctaacat tcgagtacct ccattcacta gacctcatct acagagatct 780
aaaacctgaa aatctcttaa ttgaccatca aggctatatc caggtcacag actttgggtt 840
tgccaaaaga gttaaaggca gaacttggac attatgtgga actccagagt atttggctcc 900
agaaataatt ctcagcaagg gctacaataa ggcagtggat tggtgggcat taggagtgct 960
aatctatgaa atggcagctg gctatccccc attctttgca gaccaaccaa ttcagattta 1020
tgaaaagatt gtttctggaa aggtccgatt cccatcccac ttcagttcag atctcaagga 1080
ccttctacgg aacctgctgc aggtggattt gaccaagaga tttggaaatc taaagaatgg 1140
tgtcagtgat ataaaaactc acaagtggtt tgccacgaca gattggattg ctatttacca 1200
gaggaaggtt gaagctccat tcataccaaa gtttagaggc tctggagata ccagcaactt 1260
tgatgactat gaagaagaag atatccgtgt ctctataaca gaaaaatgtg caaaagaatt 1320
tggtgaattt taaagaggaa caagatgaca tctgagctca cactcagtgt ttgcactctg 1380
ttgagagata aggtagagct gagaccgtcc ttgttgaagc agttacctag ttccttcatt 1440
ccaacgactg agtgaggtct ttattgccat catcccgtgt gcgcactctg catccaccta 1500
tgtaacaagg caccgctaag caagcattgt ctgtgccata acacagtact agaccacttt 1560
cttacttctc tttgggttgt ctttctcctc tcctatatcc atttcttcct tttccaattt 1620
cattggtttt ctctaaacag tgctccattt tattttgttg gtgtttcaga tgggcagtgt 1680
tatggctacg tgatatttga agggaaggat aagtgttgct ttcagtagtt attgccaata 1740
ttgttgttgg tcaatggctt gaagataaac tttctaataa ttattatttc tttgagtagc 1800
tcagacttgg ttttgccaaa actcttggta atttttgaag atagactgtc ttatcaccaa 1860
ggaaatttat acaaattaag actaactttc ttggaattca ctattctggc aataaatttt 1920
ggtagactaa tacagtacag ctagacccag aaatttggaa ggctgtagat cagaggttct 1980
agttcccttt ccctcctttt atatcctcct ctccttgagt aatgaagtga ccagcctgtg 2040
tagtgtgaca aacgtgtctc attcagcagg aaaaactaat gatatggatc atcacccaga 2100
ttctctcact tggtaccagc atttctgtag gtattagaga agagttctaa gttttctaaa 2160

ccttaactgt tccttaagga ttttagccag tattttaata gaacatgatt aatgaaagtg 2220
acaaatttta aattttctct aatagtcctc atcataaact ttttaaagga aaataagcaa 2280
actaaaaaga acattggttt agataaatac ttatactttg caaagtcaaa aatggcttga 2340
tttttggaaa caatatagag gtattcatat ttaaatgagg gtttacattt gttttgtttt 2400
gtaaccgtta aaaagaagtt gtttccagct aattattgtg gtgtactata tttgtgagcc 2460
tagggtaggg gcactgctgc aacttctgct ttcatcccat gcctcatcaa tgaggaaagg 2520
gaacaaagtg tataaaactg ccacaattgt attttaattt tgaggtatga tattttcaga 2580
tatttcataa tttctaacct ctgttctctc agtaaacaga atgtctgatc gatcatgcag 2640
atacaatgtt ggtatttgag aggttagttt ttttcctaca ctttttttttg ccaactgact 2700
taacaacatt gctgtcaggt ggaaatttca agcacttttg cacatttagt tcagtgtttg 2760
ttgagaatcc atggcttaac ccacttgttt tgctattttt ttctttgctt ttaattttcc 2820
ccatctgatt ttatctctgc gtttcagtga cctaccttaa aacaacacac gagaagagtt 2880
aaactgggtt cattttaatg atcaatttac ctgcatataa aatttatttt taatcaagct 2940
gatcttaatg tatataatca ttctatttgc tttattatcg gtgcaggtag gtcattaaca 3000
ccacttcttt tcatctgtac cacaccctgg tgaaaccttt gaagacataa aaaaaacctg 3060
tctgagatgt tctttctacc aatctatatg tctttcggtt atcaagtgtt tctgcatggt 3120
aatgtcatgt aaatgctgat attgatttca ctggtccatc tatatttaaa acgtgcaaga 3180
aaaaaataaa atactctgct ctagcaagtt ttgtgtaaca aaggcatatc gtcatgttaa 3240
taaatttaaa acatcattcg tataaaatat tttaattttc ttgtatttca tttagaccca 3300
agaacatgct gaccaatgtg ttctatatgt aaactacaaa ttctatggta gctttgttgt 3360
atattattgt aaaattattt taataagtca tggggatgac aatttgatta ttacaattta 3420
gttttcagta atcaaaaaga tttctatgaa ttctaaaaaa tattttttttc tatgaaatta 3480
ctagtgccca gctgtagaat ctaccttagg tagatgatcc ctagacatac gttggttttg 3540
agggctattc agccattcca ttttactctc tatttaaagg ccgtgagcaa gcttgtcatg 3600
agcaaatatg tcaagggagt caatttctga ccaatcaagt acactaaatt agaatatttt 3660
taaagtatgt aacattccca gtttcagcca caatttagcc aagaataaga taaaaacttg 3720
aataagaagt aagtagcata aatcagtatt taacctaaaa ttacatattt gaaacagaag 3780
atattatgtt atgctcagta aataattaag agatggcatt gtgtaagaag gagccctaga 3840
ctgaaagtca agacatctga atttcaggct ggaaaactat cagtatgatc tcagcctcag 3900
ttctcttgtc tgtaaaatgg aagaactgga ttaggcagtt tgtaagattc ctcctaactt 3960
tcacagtcga tgacaagatt gtctttttat ctgatatttt gaagggtata ttgctttgaa 4020
gtaagtctca ataaggcaat atattttagg gcatctttct tcttatctct gacagtgttc 4080

ttaaaattat ttgaatatca taagagcctt ggtgtctgtc ctaattcctt tctcactcac 4140

cgatgctgaa tacccagttg aatcaaactg tcaacctacc aaaaacgata ttgtggctta 4200

tgggtattgc tgtctcattc ttggtatatt cttgtgttaa ctgcccattg gcctgaaaat 4260

actcattgta agcctgaaaa aaaaaatctt tcccactgtt ttttctgctt gttgtaagaa 4320

tcaaatgaaa taatgtatgt gaaagcacct tgtaaactgt aacctatcaa tgtaaaatgt 4380

taaggtgtgt tgttatttca ttaattactt ctttgtttag aatggaattt cctatgcact 4440

actgtagcta ggaaatgctg aaaacaactg tgttttttaa ttaatcaata actgcaaaat 4500

taaagtacct tcaatggata agacaaaaaa aaaaaaaaaa aa 4542

<210> 64
<211> 2055
<212> DNA
<213> Homo sapiens

<400> 64
gccatttga gttgttctag tggtatatga aggaggctgg gataactagc ttgaaagaaa 60

ttcagtctag ttatagacat ctttggcatt aatctgatgt ttactagtga tatctcatgc 120

taggcagtta tgctttgctt ctaggggctt ctctttttaa aacaaaagaa agctcttttc 180

gttttctgtg tgctgcatgc tccagtgtgt gtgtttacac catcggttct tctccctcta 240

gagattagca taactccctt tgctgttgga ttgttatttt gagcaatatg ttttggaaag 300

gttggttttc atcatgagtg cacgcaaatc atcagatgca tctgcttgct cctcttcaga 360

aatatctgat tcctttgtga aagagtttct agccaaagcc aaagaagact ttttgaaaaa 420

atgggagaat ccaactcaga ataatgccgg acttgaagat tttgaaagga aaaaaaccct 480

tggaacaggt tcatttggaa gagtcatgtt ggtaaaacac aaagccactg aacagtatta 540

tgccatgaag atcttagata agcagaaggt tgttaaactg aagcaaatag agcatacttt 600

gaatgagaaa agaatattac aggcagtgaa ttttcctttc cttgttcgac tggagtatgc 660

ttttaaggat aattctaatt tatacatggt tatggaatat gtccctgggg gtgaaatgtt 720

ttcacatcta agaagaattg gaaggttcag tgagccccat gcacggttct atgcagctca 780

gatagtgcta acattcgagt acctccattc actagacctc atctacagag atctaaaacc 840

tgaaaatctc ttaattgacc atcaaggcta tatccaggtc acagactttg ggtttgccaa 900

aagagttaaa ggcagaactt ggacattatg tggaactcca gagtatttgg ctccagaaat 960

aattctcagc aagggctaca ataaggcagt ggattggtgg gcattaggag tgctaatcta 1020

tgaaatggca gctggctatc cccattctt tgcagaccaa ccaattcaga tttatgaaaa 1080

gattgtttct ggaaagcaga acttttgata tgaacaaaac aaaactttga gaaaaattaa 1140

cagacaaggc agtgatttat ttttgaagaa tttgagaagt gtagactctc aagaggacta 1200

aaggtcatat gaagaatgat gagagaacca aaatacatta aaatcacaaa tggaagaaga 1260

atattttact aatacaaaaa ctaagaatgt aaatgttata ataattgttt caaatcattt 1320

aattgacagt aattataaag ttcttgaatc tttactatat tacttttatt tatattcata 1380

taagaaatcc aattttctaa caaggatact gtcataacta aatttacatt tattaagaaa 1440

aactgcttta gttaaaatta atgtgtcttc atttttatgc attggcctcg atttgccaat 1500

cattctctat tggttaaatt ttatattcag ctgtttatga atatatattc attttatatc 1560

aaactttaaa attttgtatc taataatcag catatattct aaaatcataa cagtctaaat 1620

cctgggcacc ttagaagaat gacaccagaa aaccttatta tatcacaata ttctgttttc 1680

cccttcattt atttagaaat atgacaggat atttggtgta cttttgtttt ttaactaaaa 1740

gtaccagatt atctctcccc atgtgggata taaaattatc cccatctctt actcccttta 1800

ctcatctaaa gtagaagtca tgaaagtgga atttttgcca ttaaaaggct ctgtattatg 1860

tgaagttaga ttgtattaac catttcccaa taaatcatct gtttcaaaac tcaaattcaa 1920

actagaatgt gtctctattc acattgcaaa aatattattg tctctctggt tagtggctaa 1980

aagccaaatt ggaaactaac tagtttttta aattttttaa attgtgcaaa ttattaaaaa 2040

tccaatttgg tctta 2055

<210> 65
<211> 1968
<212> DNA
<213> Homo sapiens

<400> 65
actgctgctg ccaccgccgt cgccgccgcc gccgccgccg ccgctgctgc tgccggtgct 60

aaggagttcg ctggagccct ttcctcagac ccggcccggt cttcgcgccc ggactcctgg 120

cgccagcgct aggcgcactc accgctctga cgggtgcaga cgcgggagtt gtcccagact 180

gtggagtggc gggcacggcc ccagcccccc ttcccttccc tgaccccttc ttgccatcgc 240

cccagacatg gggaacgcgg cgaccgccaa gaaaggcagc gaggtggaga gcgtgaaaga 300

gtttctagcc aaagccaaag aagacttttt gaaaaaatgg gagaatccaa ctcagaataa 360

tgccggactt gaagattttg aaaggaaaaa aacccttgga acaggttcat ttggaagagt 420

catgttggta aaacacaaag ccactgaaca gtattatgcc atgaagatct tagataagca 480

gaaggttgtt aaactgaagc aaatagagca tactttgaat gagaaaagaa tattacaggc 540

agtgaatttt cctttccttg ttcgactgga gtatgctttt aaggataatt ctaatttata 600

catggttatg gaatatgtcc ctgggggtga aatgttttca catctaagaa gaattggaag 660

gttcagtgag ccccatgcac ggttctatgc agctcagata gtgctaacat tcgagtacct 720

ccattcacta gacctcatct acagagatct aaaacctgaa aatctcttaa ttgaccatca 780

aggctatatc caggtcacag actttgggtt tgccaaaaga gttaaaggca gaacttggac 840

attatgtgga actccagagt atttggctcc agaaataatt ctcagcaagg gctacaataa 900

ggcagtggat tggtgggcat taggagtgct aatctatgaa atggcagctg gctatccccc 960

attctttgca gaccaaccaa ttcagattta tgaaaagatt gtttctggaa agaacttttg 1020

atatgaacaa aacaaaactt tgagaaaaat taacagacaa ggcagtgatt tatttttgaa 1080

gaatttgaga agtgtagact ctcaagagga ctaaaggtca tatgaagaat gatgagagaa 1140

ccaaaataca ttaaaatcac aaatggaaga agaatatttt actaatacaa aaactaagaa 1200

tgtaaatgtt ataataattg tttcaaatca tttaattgac agtaattata aagttcttga 1260

atctttacta tattactttt atttatattc atataagaaa tccaattttc taacaaggat 1320

actgtcataa ctaaatttac atttattaag aaaaactgct ttagttaaaa ttaatgtgtc 1380

ttcattttta tgcattggcc tcgatttgcc aatcattctc tattggttaa attttatatt 1440

cagctgttta tgaatatata ttcattttat atcaaacttt aaaatttgt atctaataat 1500

cagcatatat tctaaaatca taacagtcta aatcctgggc accttagaag aatgacacca 1560

gaaaacctta ttatatcaca atattctgtt ttccccttca tttatttaga aatatgacag 1620

gatatttggt gtacttttgt tttttaacta aaagtaccag attatctctc cccatgtggg 1680

atataaaatt atccccatct cttactccct ttactcatct aaagtagaag tcatgaaagt 1740

ggaatttttg ccattaaaag gctctgtatt atgtgaagtt agattgtatt aaccatttcc 1800

caataaatca tctgtttcaa aactcaaatt caaactagaa tgtgtctcta ttcacattgc 1860

aaaaatatta ttgtctctct ggttagtggc taaaagccaa attggaaact aactagtttt 1920

ttaaattttt taaattgtgc aaattattaa aaatccaatt tggtctta 1968

<210> 66
<211> 4515
<212> DNA
<213> Homo sapiens

<400> 66
taacacagaa tgtttaaatg gttaacattt gtgctgcagt atagctttct ggctcatgaa 60

aaatgaaagc tatcagcgat ctcggcaata agattcatcg ccaatagtca ctagcaacag 120

cacacagcat tttaatatca gtgaggtcca cagctagcag taagagctgg tgtaattgaa 180

agacgtttag gtgcaatcat tctgctgttt gctccttgcc aggttcaaca tgggattgtc 240

acgcaaatca tcagatgcat ctgcttgctc ctcttcagaa atatctgatt cctttgtgaa 300

agagtttcta gccaaagcca aagaagactt tttgaaaaaa tgggagaatc caactcagaa 360

taatgccgga cttgaagatt ttgaaaggaa aaaaaccctt ggaacaggtt catttggaag 420

agtcatgttg gtaaaacaca aagccactga acagtattat gccatgaaga tcttagataa 480

gcagaaggtt gttaaactga agcaaataga gcatactttg aatgagaaaa gaatattaca 540
ggcagtgaat tttcctttcc ttgttcgact ggagtatgct tttaaggata attctaattt 600
atacatggtt atggaatatg tccctggggg tgaaatgttt tcacatctaa gaagaattgg 660
aaggttcagt gagccccatg cacggttcta tgcagctcag atagtgctaa cattcgagta 720
cctccattca ctagacctca tctacagaga tctaaaacct gaaaatctct taattgacca 780
tcaaggctat atccaggtca cagactttgg gtttgccaaa agagttaaag gcagaacttg 840
gacattatgt ggaactccag agtatttggc tccagaaata attctcagca agggctacaa 900
taaggcagtg gattggtggg cattaggagt gctaatctat gaaatggcag ctggctatcc 960
cccattcttt gcagaccaac caattcagat ttatgaaaag attgtttctg gaaaggtccg 1020
attcccatcc cacttcagtt cagatctcaa ggaccttcta cggaacctgc tgcaggtgga 1080
tttgaccaag agatttggaa atctaaagaa tggtgtcagt gatataaaaa ctcacaagtg 1140
gtttgccacg acagattgga ttgctattta ccagaggaag gttgaagctc cattcatacc 1200
aaagtttaga ggctctggag ataccagcaa ctttgatgac tatgaagaag aagatatccg 1260
tgtctctata acagaaaaat gtgcaaaaga atttggtgaa ttttaaagag gaacaagatg 1320
acatctgagc tcacactcag tgtttgcact ctgttgagag ataaggtaga gctgagaccg 1380
tccttgttga agcagttacc tagttccttc attccaacga ctgagtgagg tctttattgc 1440
catcatcccg tgtgcgcact ctgcatccac ctatgtaaca aggcaccgct aagcaagcat 1500
tgtctgtgcc ataacacagt actagaccac tttcttactt ctctttgggt tgtctttctc 1560
ctctcctata tccatttctt ccttttccaa tttcattggt tttctctaaa cagtgctcca 1620
ttttattttg ttggtgtttc agatgggcag tgttatggct acgtgatatt tgaagggaag 1680
gataagtgtt gctttcagta gttattgcca atattgttgt tggtcaatgg cttgaagata 1740
aactttctaa taattattat ttctttgagt agctcagact tggttttgcc aaaactcttg 1800
gtaatttttg aagatagact gtcttatcac caaggaaatt tatacaaatt aagactaact 1860
ttcttggaat tcactattct ggcaataaat tttggtagac taatacagta cagctagacc 1920
cagaaatttg gaaggctgta gatcagaggt tctagttccc tttccctcct tttatatcct 1980
cctctccttg agtaatgaag tgaccagcct gtgtagtgtg acaaacgtgt ctcattcagc 2040
aggaaaaact aatgatatgg atcatcaccc agattctctc acttggtacc agcatttctg 2100
taggtattag agaagagttc taagttttct aaaccttaac tgttccttaa ggattttagc 2160
cagtatttta atagaacatg attaatgaaa gtgacaaatt ttaaattttc tctaatagtc 2220
ctcatcataa actttttaaa ggaaaataag caaactaaaa agaacattgg tttagataaa 2280
tacttatact ttgcaaagtc aaaaatggct tgatttttgg aaacaatata gaggtattca 2340

```
tatttaaatg agggtttaca tttgttttgt tttgtaaccg ttaaaaagaa gttgtttcca     2400
gctaattatt gtggtgtact atatttgtga gcctagggta ggggcactgc tgcaacttct     2460
gctttcatcc catgcctcat caatgaggaa agggaacaaa gtgtataaaa ctgccacaat     2520
tgtattttaa ttttgaggta tgatattttc agatatttca taatttctaa cctctgttct     2580
ctcagtaaac agaatgtctg atcgatcatg cagatacaat gttggtattt gagaggttag     2640
tttttttcct acactttttt ttgccaactg acttaacaac attgctgtca ggtggaaatt     2700
tcaagcactt ttgcacattt agttcagtgt ttgttgagaa tccatggctt aacccacttg     2760
ttttgctatt tttttctttg cttttaattt tccccatctg attttatctc tgcgtttcag     2820
tgacctacct taaaacaaca cacgagaaga gttaaactgg gttcatttta atgatcaatt     2880
tacctgcata taaaatttat ttttaatcaa gctgatctta atgtatataa tcattctatt     2940
tgctttatta tcggtgcagg taggtcatta acaccacttc ttttcatctg taccacaccc     3000
tggtgaaacc tttgaagaca taaaaaaaac ctgtctgaga tgttctttct accaatctat     3060
atgtctttcg gttatcaagt gtttctgcat ggtaatgtca tgtaaatgct gatattgatt     3120
tcactggtcc atctatattt aaaacgtgca agaaaaaaat aaaatactct gctctagcaa     3180
gttttgtgta acaaaggcat atcgtcatgt taataaattt aaaacatcat tcgtataaaa     3240
tattttaatt ttcttgtatt tcatttagac ccaagaacat gctgaccaat gtgttctata     3300
tgtaaactac aaattctatg gtagctttgt tgtatattat tgtaaaatta ttttaataag     3360
tcatggggat gacaatttga ttattacaat ttagttttca gtaatcaaaa agatttctat     3420
gaattctaaa aaatattttt ttctatgaaa ttactagtgc ccagctgtag aatctacctt     3480
aggtagatga tccctagaca tacgttggtt ttgagggcta ttcagccatt ccattttact     3540
ctctatttaa aggccgtgag caagcttgtc atgagcaaat atgtcaaggg agtcaatttc     3600
tgaccaatca agtacactaa attagaatat ttttaaagta tgtaacattc ccagtttcag     3660
ccacaattta gccaagaata agataaaaac ttgaataaga agtaagtagc ataaatcagt     3720
atttaaccta aaattacata tttgaaacag aagatattat gttatgctca gtaaataatt     3780
aagagatggc attgtgtaag aaggagccct agactgaaag tcaagacatc tgaatttcag     3840
gctggaaaac tatcagtatg atctcagcct cagttctctt gtctgtaaaa tggaagaact     3900
ggattaggca gtttgtaaga ttcctcctaa ctttcacagt cgatgacaag attgtctttt     3960
tatctgatat tttgaagggt atattgcttt gaagtaagtc tcaataaggc aatatatttt     4020
agggcatctt tcttcttatc tctgacagtg ttcttaaaat tatttgaata tcataagagc     4080
cttggtgtct gtcctaattc ctttctcact caccgatgct gaatacccag ttgaatcaaa     4140
ctgtcaacct accaaaaacg atattgtggc ttatgggtat tgctgtctca ttcttggtat     4200
attcttgtgt taactgccca ttggcctgaa aatactcatt gtaagcctga aaaaaaaaat     4260
```

ctttcccact gtttttttctg cttgttgtaa gaatcaaatg aaataatgta tgtgaaagca 4320

ccttgtaaac tgtaacctat caatgtaaaa tgttaaggtg tgttgttatt tcattaatta 4380

cttctttgtt tagaatggaa tttcctatgc actactgtag ctaggaaatg ctgaaaacaa 4440

ctgtgttttt taattaatca ataactgcaa aattaaagta ccttcaatgg ataagacaaa 4500

aaaaaaaaaa aaaaa 4515

<210> 67
<211> 1793
<212> DNA
<213> Homo sapiens

<400> 67
acacagcatt ttaatatcag tgaggtccac agctagcagt aagagctggt gtaattgaaa 60

gacgtttagg tgcaatcatt ctgctgtttg ctccttgcca ggttcaacat gggattgttg 120

aaagagtttc tagccaaagc caaagaagac tttttgaaaa aatgggagaa tccaactcag 180

aataatgccg gacttgaaga ttttgaaagg aaaaaaaccc ttggaacagg ttcatttgga 240

agagtcatgt tggtaaaaca caaagccact gaacagtatt atgccatgaa gatcttagat 300

aagcagaagg ttgttaaact gaagcaaata gagcatactt tgaatgagaa aagaatatta 360

caggcagtga attttccttt ccttgttcga ctggagtatg cttttaagga taattctaat 420

ttatacatgg ttatggaata tgtccctggg ggtgaaatgt tttcacatct aagaagaatt 480

ggaaggttca gtgagcccca tgcacggttc tatgcagctc agatagtgct aacattcgag 540

tacctccatt cactagacct catctacaga gatctaaaac ctgaaaatct cttaattgac 600

catcaaggct atatccaggt cacagacttt gggtttgcca aaagagttaa aggcagaact 660

tggacattat gtggaactcc agagtatttg gctccagaaa taattctcag caagggctac 720

aataaggcag tggattggtg ggcattagga gtgctaatct atgaaatggc agctggctat 780

cccccattct ttgcagacca accaattcag atttatgaaa agattgtttc tggaaagaac 840

ttttgatatg aacaaaacaa aactttgaga aaaattaaca gacaaggcag tgatttattt 900

ttgaagaatt tgagaagtgt agactctcaa gaggactaaa ggtcatatga agaatgatga 960

gagaaccaaa atacattaaa atcacaaatg gaagaagaat attttactaa tacaaaaact 1020

aagaatgtaa atgttataat aattgtttca aatcatttaa ttgacagtaa ttataaagtt 1080

cttgaatctt tactatatta cttttattta tattcatata agaaatccaa ttttctaaca 1140

aggatactgt cataactaaa tttacattta ttaagaaaaa ctgctttagt taaaattaat 1200

gtgtcttcat ttttatgcat tggcctcgat ttgccaatca ttctctattg gttaaatttt 1260

atattcagct gtttatgaat atatattcat tttatatcaa actttaaaat tttgtatcta 1320

ataatcagca tatattctaa aatcataaca gtctaaatcc tgggcacctt agaagaatga 1380

caccagaaaa ccttattata tcacaatatt ctgttttccc cttcatttat ttagaaatat 1440

gacaggatat ttggtgtact tttgtttttt aactaaaagt accagattat ctctccccat 1500

gtgggatata aaattatccc catctcttac tccctttact catctaaagt agaagtcatg 1560

aaagtggaat ttttgccatt aaaaggctct gtattatgtg aagttagatt gtattaacca 1620

tttcccaata aatcatctgt ttcaaaactc aaattcaaac tagaatgtgt ctctattcac 1680

attgcaaaaa tattattgtc tctctggtta gtggctaaaa gccaaattgg aaactaacta 1740

gtttttttaaa tttttttaaat tgtgcaaatt attaaaaatc caatttggtc tta 1793

<210> 68
<211> 351
<212> PRT
<213> Homo sapiens

<400> 68

Met Gly Asn Ala Ala Thr Ala Lys Lys Gly Ser Glu Val Glu Ser Val
1 5 10 15

Lys Glu Phe Leu Ala Lys Ala Lys Glu Asp Phe Leu Lys Lys Trp Glu
20 25 30

Asn Pro Thr Gln Asn Asn Ala Gly Leu Glu Asp Phe Glu Arg Lys Lys
35 40 45

Thr Leu Gly Thr Gly Ser Phe Gly Arg Val Met Leu Val Lys His Lys
50 55 60

Ala Thr Glu Gln Tyr Tyr Ala Met Lys Ile Leu Asp Lys Gln Lys Val
65 70 75 80

Val Lys Leu Lys Gln Ile Glu His Thr Leu Asn Glu Lys Arg Ile Leu
85 90 95

Gln Ala Val Asn Phe Pro Phe Leu Val Arg Leu Glu Tyr Ala Phe Lys
100 105 110

Asp Asn Ser Asn Leu Tyr Met Val Met Glu Tyr Val Pro Gly Gly Glu
115 120 125

Met Phe Ser His Leu Arg Arg Ile Gly Arg Phe Ser Glu Pro His Ala
130 135 140

Arg Phe Tyr Ala Ala Gln Ile Val Leu Thr Phe Glu Tyr Leu His Ser
145 150 155 160

```
Leu Asp Leu Ile Tyr Arg Asp Leu Lys Pro Glu Asn Leu Leu Ile Asp
                165                 170                 175

His Gln Gly Tyr Ile Gln Val Thr Asp Phe Gly Phe Ala Lys Arg Val
            180                 185                 190

Lys Gly Arg Thr Trp Thr Leu Cys Gly Thr Pro Glu Tyr Leu Ala Pro
        195                 200                 205

Glu Ile Ile Leu Ser Lys Gly Tyr Asn Lys Ala Val Asp Trp Trp Ala
    210                 215                 220

Leu Gly Val Leu Ile Tyr Glu Met Ala Ala Gly Tyr Pro Pro Phe Phe
225                 230                 235                 240

Ala Asp Gln Pro Ile Gln Ile Tyr Glu Lys Ile Val Ser Gly Lys Val
                245                 250                 255

Arg Phe Pro Ser His Phe Ser Ser Asp Leu Lys Asp Leu Leu Arg Asn
            260                 265                 270

Leu Leu Gln Val Asp Leu Thr Lys Arg Phe Gly Asn Leu Lys Asn Gly
        275                 280                 285

Val Ser Asp Ile Lys Thr His Lys Trp Phe Ala Thr Thr Asp Trp Ile
    290                 295                 300

Ala Ile Tyr Gln Arg Lys Val Glu Ala Pro Phe Ile Pro Lys Phe Arg
305                 310                 315                 320

Gly Ser Gly Asp Thr Ser Asn Phe Asp Asp Tyr Glu Glu Glu Asp Ile
                325                 330                 335

Arg Val Ser Ile Thr Glu Lys Cys Ala Lys Glu Phe Gly Glu Phe
            340                 345                 350

<210>  69
<211>  398
<212>  PRT
<213>  Homo sapiens

<400>  69

Met Ala Ala Tyr Arg Glu Pro Pro Cys Asn Gln Tyr Thr Gly Thr Thr
1               5                   10                  15

Thr Ala Leu Gln Lys Leu Glu Gly Phe Ala Ser Arg Leu Phe His Arg
            20                  25                  30
```

His Ser Lys Gly Thr Ala His Asp Gln Lys Thr Ala Leu Glu Asn Asp
35 40 45

Ser Leu His Phe Ser Glu His Thr Ala Leu Trp Asp Arg Ser Met Lys
50 55 60

Glu Phe Leu Ala Lys Ala Lys Glu Asp Phe Leu Lys Lys Trp Glu Asn
65 70 75 80

Pro Thr Gln Asn Asn Ala Gly Leu Glu Asp Phe Glu Arg Lys Lys Thr
85 90 95

Leu Gly Thr Gly Ser Phe Gly Arg Val Met Leu Val Lys His Lys Ala
100 105 110

Thr Glu Gln Tyr Tyr Ala Met Lys Ile Leu Asp Lys Gln Lys Val Val
115 120 125

Lys Leu Lys Gln Ile Glu His Thr Leu Asn Glu Lys Arg Ile Leu Gln
130 135 140

Ala Val Asn Phe Pro Phe Leu Val Arg Leu Glu Tyr Ala Phe Lys Asp
145 150 155 160

Asn Ser Asn Leu Tyr Met Val Met Glu Tyr Val Pro Gly Gly Glu Met
165 170 175

Phe Ser His Leu Arg Arg Ile Gly Arg Phe Ser Glu Pro His Ala Arg
180 185 190

Phe Tyr Ala Ala Gln Ile Val Leu Thr Phe Glu Tyr Leu His Ser Leu
195 200 205

Asp Leu Ile Tyr Arg Asp Leu Lys Pro Glu Asn Leu Leu Ile Asp His
210 215 220

Gln Gly Tyr Ile Gln Val Thr Asp Phe Gly Phe Ala Lys Arg Val Lys
225 230 235 240

Gly Arg Thr Trp Thr Leu Cys Gly Thr Pro Glu Tyr Leu Ala Pro Glu
245 250 255

Ile Ile Leu Ser Lys Gly Tyr Asn Lys Ala Val Asp Trp Trp Ala Leu
260 265 270

Gly Val Leu Ile Tyr Glu Met Ala Ala Gly Tyr Pro Pro Phe Phe Ala
275 280 285

```
Asp Gln Pro Ile Gln Ile Tyr Glu Lys Ile Val Ser Gly Lys Val Arg
    290                 295                 300

Phe Pro Ser His Phe Ser Ser Asp Leu Lys Asp Leu Leu Arg Asn Leu
305                 310                 315                 320

Leu Gln Val Asp Leu Thr Lys Arg Phe Gly Asn Leu Lys Asn Gly Val
                325                 330                 335

Ser Asp Ile Lys Thr His Lys Trp Phe Ala Thr Thr Asp Trp Ile Ala
            340                 345                 350

Ile Tyr Gln Arg Lys Val Glu Ala Pro Phe Ile Pro Lys Phe Arg Gly
        355                 360                 365

Ser Gly Asp Thr Ser Asn Phe Asp Asp Tyr Glu Glu Glu Asp Ile Arg
    370                 375                 380

Val Ser Ile Thr Glu Lys Cys Ala Lys Glu Phe Gly Glu Phe
385                 390                 395


<210>  70
<211>  357
<212>  PRT
<213>  Homo sapiens

<400>  70

Met Ser Ala Arg Lys Ser Ser Asp Ala Ser Ala Cys Ser Ser Ser Glu
1               5                   10                  15

Ile Ser Asp Ser Phe Val Lys Glu Phe Leu Ala Lys Ala Lys Glu Asp
            20                  25                  30

Phe Leu Lys Lys Trp Glu Asn Pro Thr Gln Asn Asn Ala Gly Leu Glu
        35                  40                  45

Asp Phe Glu Arg Lys Lys Thr Leu Gly Thr Gly Ser Phe Gly Arg Val
    50                  55                  60

Met Leu Val Lys His Lys Ala Thr Glu Gln Tyr Tyr Ala Met Lys Ile
65                  70                  75                  80

Leu Asp Lys Gln Lys Val Val Lys Leu Lys Gln Ile Glu His Thr Leu
                85                  90                  95

Asn Glu Lys Arg Ile Leu Gln Ala Val Asn Phe Pro Phe Leu Val Arg
            100                 105                 110
```

```
Leu Glu Tyr Ala Phe Lys Asp Asn Ser Asn Leu Tyr Met Val Met Glu
        115                 120                 125

Tyr Val Pro Gly Gly Glu Met Phe Ser His Leu Arg Arg Ile Gly Arg
    130                 135                 140

Phe Ser Glu Pro His Ala Arg Phe Tyr Ala Ala Gln Ile Val Leu Thr
145                 150                 155                 160

Phe Glu Tyr Leu His Ser Leu Asp Leu Ile Tyr Arg Asp Leu Lys Pro
                165                 170                 175

Glu Asn Leu Leu Ile Asp His Gln Gly Tyr Ile Gln Val Thr Asp Phe
            180                 185                 190

Gly Phe Ala Lys Arg Val Lys Gly Arg Thr Trp Thr Leu Cys Gly Thr
        195                 200                 205

Pro Glu Tyr Leu Ala Pro Glu Ile Ile Leu Ser Lys Gly Tyr Asn Lys
    210                 215                 220

Ala Val Asp Trp Trp Ala Leu Gly Val Leu Ile Tyr Glu Met Ala Ala
225                 230                 235                 240

Gly Tyr Pro Pro Phe Phe Ala Asp Gln Pro Ile Gln Ile Tyr Glu Lys
                245                 250                 255

Ile Val Ser Gly Lys Val Arg Phe Pro Ser His Phe Ser Ser Asp Leu
            260                 265                 270

Lys Asp Leu Leu Arg Asn Leu Leu Gln Val Asp Leu Thr Lys Arg Phe
        275                 280                 285

Gly Asn Leu Lys Asn Gly Val Ser Asp Ile Lys Thr His Lys Trp Phe
    290                 295                 300

Ala Thr Thr Asp Trp Ile Ala Ile Tyr Gln Arg Lys Val Glu Ala Pro
305                 310                 315                 320

Phe Ile Pro Lys Phe Arg Gly Ser Gly Asp Thr Ser Asn Phe Asp Asp
                325                 330                 335

Tyr Glu Glu Glu Asp Ile Arg Val Ser Ile Thr Glu Lys Cys Ala Lys
            340                 345                 350

Glu Phe Gly Glu Phe
        355
```

<210> 71
<211> 355
<212> PRT
<213> Homo sapiens

<400> 71

Met Gly Leu Ser Arg Lys Ser Ser Asp Ala Ser Ala Cys Ser Ser Ser
1 5 10 15

Glu Ile Ser Val Lys Glu Phe Leu Ala Lys Ala Lys Glu Asp Phe Leu
20 25 30

Lys Lys Trp Glu Asn Pro Thr Gln Asn Asn Ala Gly Leu Glu Asp Phe
35 40 45

Glu Arg Lys Lys Thr Leu Gly Thr Gly Ser Phe Gly Arg Val Met Leu
50 55 60

Val Lys His Lys Ala Thr Glu Gln Tyr Tyr Ala Met Lys Ile Leu Asp
65 70 75 80

Lys Gln Lys Val Val Lys Leu Lys Gln Ile Glu His Thr Leu Asn Glu
85 90 95

Lys Arg Ile Leu Gln Ala Val Asn Phe Pro Phe Leu Val Arg Leu Glu
100 105 110

Tyr Ala Phe Lys Asp Asn Ser Asn Leu Tyr Met Val Met Glu Tyr Val
115 120 125

Pro Gly Gly Glu Met Phe Ser His Leu Arg Arg Ile Gly Arg Phe Ser
130 135 140

Glu Pro His Ala Arg Phe Tyr Ala Ala Gln Ile Val Leu Thr Phe Glu
145 150 155 160

Tyr Leu His Ser Leu Asp Leu Ile Tyr Arg Asp Leu Lys Pro Glu Asn
165 170 175

Leu Leu Ile Asp His Gln Gly Tyr Ile Gln Val Thr Asp Phe Gly Phe
180 185 190

Ala Lys Arg Val Lys Gly Arg Thr Trp Thr Leu Cys Gly Thr Pro Glu
195 200 205

Tyr Leu Ala Pro Glu Ile Ile Leu Ser Lys Gly Tyr Asn Lys Ala Val
210 215 220

Asp Trp Trp Ala Leu Gly Val Leu Ile Tyr Glu Met Ala Ala Gly Tyr
225                 230                 235                 240

Pro Pro Phe Phe Ala Asp Gln Pro Ile Gln Ile Tyr Glu Lys Ile Val
                245                 250                 255

Ser Gly Lys Val Arg Phe Pro Ser His Phe Ser Ser Asp Leu Lys Asp
            260                 265                 270

Leu Leu Arg Asn Leu Leu Gln Val Asp Leu Thr Lys Arg Phe Gly Asn
        275                 280                 285

Leu Lys Asn Gly Val Ser Asp Ile Lys Thr His Lys Trp Phe Ala Thr
    290                 295                 300

Thr Asp Trp Ile Ala Ile Tyr Gln Arg Lys Val Glu Ala Pro Phe Ile
305                 310                 315                 320

Pro Lys Phe Arg Gly Ser Gly Asp Thr Ser Asn Phe Asp Asp Tyr Glu
                325                 330                 335

Glu Glu Asp Ile Arg Val Ser Ile Thr Glu Lys Cys Ala Lys Glu Phe
            340                 345                 350

Gly Glu Phe
        355

<210> 72
<211> 339
<212> PRT
<213> Homo sapiens

<400> 72

Met Gly Leu Leu Lys Glu Phe Leu Ala Lys Ala Lys Glu Asp Phe Leu
1               5                   10                  15

Lys Lys Trp Glu Asn Pro Thr Gln Asn Asn Ala Gly Leu Glu Asp Phe
            20                  25                  30

Glu Arg Lys Lys Thr Leu Gly Thr Gly Ser Phe Gly Arg Val Met Leu
        35                  40                  45

Val Lys His Lys Ala Thr Glu Gln Tyr Tyr Ala Met Lys Ile Leu Asp
    50                  55                  60

Lys Gln Lys Val Val Lys Leu Lys Gln Ile Glu His Thr Leu Asn Glu
65                  70                  75                  80

```
Lys Arg Ile Leu Gln Ala Val Asn Phe Pro Phe Leu Val Arg Leu Glu
                85                  90                  95

Tyr Ala Phe Lys Asp Asn Ser Asn Leu Tyr Met Val Met Glu Tyr Val
            100                 105                 110

Pro Gly Gly Glu Met Phe Ser His Leu Arg Arg Ile Gly Arg Phe Ser
        115                 120                 125

Glu Pro His Ala Arg Phe Tyr Ala Ala Gln Ile Val Leu Thr Phe Glu
    130                 135                 140

Tyr Leu His Ser Leu Asp Leu Ile Tyr Arg Asp Leu Lys Pro Glu Asn
145                 150                 155                 160

Leu Leu Ile Asp His Gln Gly Tyr Ile Gln Val Thr Asp Phe Gly Phe
                165                 170                 175

Ala Lys Arg Val Lys Gly Arg Thr Trp Thr Leu Cys Gly Thr Pro Glu
            180                 185                 190

Tyr Leu Ala Pro Glu Ile Ile Leu Ser Lys Gly Tyr Asn Lys Ala Val
        195                 200                 205

Asp Trp Trp Ala Leu Gly Val Leu Ile Tyr Glu Met Ala Ala Gly Tyr
    210                 215                 220

Pro Pro Phe Phe Ala Asp Gln Pro Ile Gln Ile Tyr Glu Lys Ile Val
225                 230                 235                 240

Ser Gly Lys Val Arg Phe Pro Ser His Phe Ser Ser Asp Leu Lys Asp
                245                 250                 255

Leu Leu Arg Asn Leu Leu Gln Val Asp Leu Thr Lys Arg Phe Gly Asn
            260                 265                 270

Leu Lys Asn Gly Val Ser Asp Ile Lys Thr His Lys Trp Phe Ala Thr
        275                 280                 285

Thr Asp Trp Ile Ala Ile Tyr Gln Arg Lys Val Glu Ala Pro Phe Ile
    290                 295                 300

Pro Lys Phe Arg Gly Ser Gly Asp Thr Ser Asn Phe Asp Asp Tyr Glu
305                 310                 315                 320

Glu Glu Asp Ile Arg Val Ser Ile Thr Glu Lys Cys Ala Lys Glu Phe
```

325 330 335

Gly Glu Phe

<210> 73
<211> 338
<212> PRT
<213> Homo sapiens

<400> 73

Met Leu Leu Leu Ser Ser Ser Ile Ser Leu Tyr Lys Asp Arg Asn Glu
1 5 10 15

Ala Arg Leu Ile Ser Ser Gln Asn Asn Ala Gly Leu Glu Asp Phe Glu
20 25 30

Arg Lys Lys Thr Leu Gly Thr Gly Ser Phe Gly Arg Val Met Leu Val
35 40 45

Lys His Lys Ala Thr Glu Gln Tyr Tyr Ala Met Lys Ile Leu Asp Lys
50 55 60

Gln Lys Val Val Lys Leu Lys Gln Ile Glu His Thr Leu Asn Glu Lys
65 70 75 80

Arg Ile Leu Gln Ala Val Asn Phe Pro Phe Leu Val Arg Leu Glu Tyr
85 90 95

Ala Phe Lys Asp Asn Ser Asn Leu Tyr Met Val Met Glu Tyr Val Pro
100 105 110

Gly Gly Glu Met Phe Ser His Leu Arg Arg Ile Gly Arg Phe Ser Glu
115 120 125

Pro His Ala Arg Phe Tyr Ala Ala Gln Ile Val Leu Thr Phe Glu Tyr
130 135 140

Leu His Ser Leu Asp Leu Ile Tyr Arg Asp Leu Lys Pro Glu Asn Leu
145 150 155 160

Leu Ile Asp His Gln Gly Tyr Ile Gln Val Thr Asp Phe Gly Phe Ala
165 170 175

Lys Arg Val Lys Gly Arg Thr Trp Thr Leu Cys Gly Thr Pro Glu Tyr
180 185 190

Leu Ala Pro Glu Ile Ile Leu Ser Lys Gly Tyr Asn Lys Ala Val Asp

195 200 205

Trp Trp Ala Leu Gly Val Leu Ile Tyr Glu Met Ala Ala Gly Tyr Pro
210 215 220

Pro Phe Phe Ala Asp Gln Pro Ile Gln Ile Tyr Glu Lys Ile Val Ser
225 230 235 240

Gly Lys Val Arg Phe Pro Ser His Phe Ser Ser Asp Leu Lys Asp Leu
245 250 255

Leu Arg Asn Leu Leu Gln Val Asp Leu Thr Lys Arg Phe Gly Asn Leu
260 265 270

Lys Asn Gly Val Ser Asp Ile Lys Thr His Lys Trp Phe Ala Thr Thr
275 280 285

Asp Trp Ile Ala Ile Tyr Gln Arg Lys Val Glu Ala Pro Phe Ile Pro
290 295 300

Lys Phe Arg Gly Ser Gly Asp Thr Ser Asn Phe Asp Asp Tyr Glu Glu
305 310 315 320

Glu Asp Ile Arg Val Ser Ile Thr Glu Lys Cys Ala Lys Glu Phe Gly
325 330 335

Glu Phe

<210> 74
<211> 321
<212> PRT
<213> Homo sapiens

<400> 74

Met Ser Ala Arg Lys Ser Ser Asp Ala Ser Ala Cys Ser Ser Ser Glu
1 5 10 15

Ile Ser Val Lys Glu Phe Leu Ala Lys Ala Lys Glu Asp Phe Leu Lys
20 25 30

Lys Trp Glu Asn Pro Thr Gln Asn Asn Ala Gly Leu Glu Asp Phe Glu
35 40 45

Arg Lys Lys Thr Leu Gly Thr Gly Ser Phe Gly Arg Val Met Leu Val
50 55 60

Lys His Lys Ala Thr Glu Gln Tyr Tyr Ala Met Lys Ile Leu Asp Lys

```
65                  70                  75                  80

Gln Lys Asp Asn Ser Asn Leu Tyr Met Val Met Glu Tyr Val Pro Gly
                85                  90                  95

Gly Glu Met Phe Ser His Leu Arg Arg Ile Gly Arg Phe Ser Glu Pro
            100                 105                 110

His Ala Arg Phe Tyr Ala Ala Gln Ile Val Leu Thr Phe Glu Tyr Leu
        115                 120                 125

His Ser Leu Asp Leu Ile Tyr Arg Asp Leu Lys Pro Glu Asn Leu Leu
    130                 135                 140

Ile Asp His Gln Gly Tyr Ile Gln Val Thr Asp Phe Gly Phe Ala Lys
145                 150                 155                 160

Arg Val Lys Gly Arg Thr Trp Thr Leu Cys Gly Thr Pro Glu Tyr Leu
                165                 170                 175

Ala Pro Glu Ile Ile Leu Ser Lys Gly Tyr Asn Lys Ala Val Asp Trp
            180                 185                 190

Trp Ala Leu Gly Val Leu Ile Tyr Glu Met Ala Ala Gly Tyr Pro Pro
        195                 200                 205

Phe Phe Ala Asp Gln Pro Ile Gln Ile Tyr Glu Lys Ile Val Ser Gly
    210                 215                 220

Lys Val Arg Phe Pro Ser His Phe Ser Ser Asp Leu Lys Asp Leu Leu
225                 230                 235                 240

Arg Asn Leu Leu Gln Val Asp Leu Thr Lys Arg Phe Gly Asn Leu Lys
                245                 250                 255

Asn Gly Val Ser Asp Ile Lys Thr His Lys Trp Phe Ala Thr Thr Asp
            260                 265                 270

Trp Ile Ala Ile Tyr Gln Arg Lys Val Glu Ala Pro Phe Ile Pro Lys
        275                 280                 285

Phe Arg Gly Ser Gly Asp Thr Ser Asn Phe Asp Asp Tyr Glu Glu Glu
    290                 295                 300

Asp Ile Arg Val Ser Ile Thr Glu Lys Cys Ala Lys Glu Phe Gly Glu
305                 310                 315                 320
```

Phe

<210> 75
<211> 264
<212> PRT
<213> Homo sapiens

<400> 75

Met Ser Ala Arg Lys Ser Ser Asp Ala Ser Ala Cys Ser Ser Ser Glu
1 5 10 15

Ile Ser Asp Ser Phe Val Lys Glu Phe Leu Ala Lys Ala Lys Glu Asp
20 25 30

Phe Leu Lys Lys Trp Glu Asn Pro Thr Gln Asn Asn Ala Gly Leu Glu
35 40 45

Asp Phe Glu Arg Lys Lys Thr Leu Gly Thr Gly Ser Phe Gly Arg Val
50 55 60

Met Leu Val Lys His Lys Ala Thr Glu Gln Tyr Tyr Ala Met Lys Ile
65 70 75 80

Leu Asp Lys Gln Lys Val Val Lys Leu Lys Gln Ile Glu His Thr Leu
85 90 95

Asn Glu Lys Arg Ile Leu Gln Ala Val Asn Phe Pro Phe Leu Val Arg
100 105 110

Leu Glu Tyr Ala Phe Lys Asp Asn Ser Asn Leu Tyr Met Val Met Glu
115 120 125

Tyr Val Pro Gly Gly Glu Met Phe Ser His Leu Arg Arg Ile Gly Arg
130 135 140

Phe Ser Glu Pro His Ala Arg Phe Tyr Ala Ala Gln Ile Val Leu Thr
145 150 155 160

Phe Glu Tyr Leu His Ser Leu Asp Leu Ile Tyr Arg Asp Leu Lys Pro
165 170 175

Glu Asn Leu Leu Ile Asp His Gln Gly Tyr Ile Gln Val Thr Asp Phe
180 185 190

Gly Phe Ala Lys Arg Val Lys Gly Arg Thr Trp Thr Leu Cys Gly Thr
195 200 205

Pro Glu Tyr Leu Ala Pro Glu Ile Ile Leu Ser Lys Gly Tyr Asn Lys
    210                 215                 220

Ala Val Asp Trp Trp Ala Leu Gly Val Leu Ile Tyr Glu Met Ala Ala
225                 230                 235                 240

Gly Tyr Pro Pro Phe Phe Ala Asp Gln Pro Ile Gln Ile Tyr Glu Lys
                245                 250                 255

Ile Val Ser Gly Lys Gln Asn Phe
            260

<210>  76
<211>  257
<212>  PRT
<213>  Homo sapiens

<400>  76

Met Gly Asn Ala Ala Thr Ala Lys Lys Gly Ser Glu Val Glu Ser Val
1               5                   10                  15

Lys Glu Phe Leu Ala Lys Ala Lys Glu Asp Phe Leu Lys Lys Trp Glu
            20                  25                  30

Asn Pro Thr Gln Asn Asn Ala Gly Leu Glu Asp Phe Glu Arg Lys Lys
        35                  40                  45

Thr Leu Gly Thr Gly Ser Phe Gly Arg Val Met Leu Val Lys His Lys
    50                  55                  60

Ala Thr Glu Gln Tyr Tyr Ala Met Lys Ile Leu Asp Lys Gln Lys Val
65                  70                  75                  80

Val Lys Leu Lys Gln Ile Glu His Thr Leu Asn Glu Lys Arg Ile Leu
                85                  90                  95

Gln Ala Val Asn Phe Pro Phe Leu Val Arg Leu Glu Tyr Ala Phe Lys
            100                 105                 110

Asp Asn Ser Asn Leu Tyr Met Val Met Glu Tyr Val Pro Gly Gly Glu
        115                 120                 125

Met Phe Ser His Leu Arg Arg Ile Gly Arg Phe Ser Glu Pro His Ala
    130                 135                 140

Arg Phe Tyr Ala Ala Gln Ile Val Leu Thr Phe Glu Tyr Leu His Ser
145                 150                 155                 160

Leu Asp Leu Ile Tyr Arg Asp Leu Lys Pro Glu Asn Leu Leu Ile Asp
165 170 175

His Gln Gly Tyr Ile Gln Val Thr Asp Phe Gly Phe Ala Lys Arg Val
180 185 190

Lys Gly Arg Thr Trp Thr Leu Cys Gly Thr Pro Glu Tyr Leu Ala Pro
195 200 205

Glu Ile Ile Leu Ser Lys Gly Tyr Asn Lys Ala Val Asp Trp Trp Ala
210 215 220

Leu Gly Val Leu Ile Tyr Glu Met Ala Ala Gly Tyr Pro Pro Phe Phe
225 230 235 240

Ala Asp Gln Pro Ile Gln Ile Tyr Glu Lys Ile Val Ser Gly Lys Asn
245 250 255

Phe

<210> 77
<211> 358
<212> PRT
<213> Homo sapiens

<400> 77

Met Gly Leu Ser Arg Lys Ser Ser Asp Ala Ser Ala Cys Ser Ser Ser
1 5 10 15

Glu Ile Ser Asp Ser Phe Val Lys Glu Phe Leu Ala Lys Ala Lys Glu
20 25 30

Asp Phe Leu Lys Lys Trp Glu Asn Pro Thr Gln Asn Asn Ala Gly Leu
35 40 45

Glu Asp Phe Glu Arg Lys Lys Thr Leu Gly Thr Gly Ser Phe Gly Arg
50 55 60

Val Met Leu Val Lys His Lys Ala Thr Glu Gln Tyr Tyr Ala Met Lys
65 70 75 80

Ile Leu Asp Lys Gln Lys Val Val Lys Leu Lys Gln Ile Glu His Thr
85 90 95

Leu Asn Glu Lys Arg Ile Leu Gln Ala Val Asn Phe Pro Phe Leu Val
100 105 110

```
Arg Leu Glu Tyr Ala Phe Lys Asp Asn Ser Asn Leu Tyr Met Val Met
        115                 120                 125

Glu Tyr Val Pro Gly Gly Glu Met Phe Ser His Leu Arg Arg Ile Gly
    130                 135                 140

Arg Phe Ser Glu Pro His Ala Arg Phe Tyr Ala Ala Gln Ile Val Leu
145                 150                 155                 160

Thr Phe Glu Tyr Leu His Ser Leu Asp Leu Ile Tyr Arg Asp Leu Lys
                165                 170                 175

Pro Glu Asn Leu Leu Ile Asp His Gln Gly Tyr Ile Gln Val Thr Asp
            180                 185                 190

Phe Gly Phe Ala Lys Arg Val Lys Gly Arg Thr Trp Thr Leu Cys Gly
        195                 200                 205

Thr Pro Glu Tyr Leu Ala Pro Glu Ile Ile Leu Ser Lys Gly Tyr Asn
    210                 215                 220

Lys Ala Val Asp Trp Trp Ala Leu Gly Val Leu Ile Tyr Glu Met Ala
225                 230                 235                 240

Ala Gly Tyr Pro Pro Phe Phe Ala Asp Gln Pro Ile Gln Ile Tyr Glu
                245                 250                 255

Lys Ile Val Ser Gly Lys Val Arg Phe Pro Ser His Phe Ser Ser Asp
            260                 265                 270

Leu Lys Asp Leu Leu Arg Asn Leu Leu Gln Val Asp Leu Thr Lys Arg
        275                 280                 285

Phe Gly Asn Leu Lys Asn Gly Val Ser Asp Ile Lys Thr His Lys Trp
    290                 295                 300

Phe Ala Thr Thr Asp Trp Ile Ala Ile Tyr Gln Arg Lys Val Glu Ala
305                 310                 315                 320

Pro Phe Ile Pro Lys Phe Arg Gly Ser Gly Asp Thr Ser Asn Phe Asp
                325                 330                 335

Asp Tyr Glu Glu Glu Asp Ile Arg Val Ser Ile Thr Glu Lys Cys Ala
            340                 345                 350

Lys Glu Phe Gly Glu Phe
        355
```

<210> 78
<211> 245
<212> PRT
<213> Homo sapiens

<400> 78

Met Gly Leu Leu Lys Glu Phe Leu Ala Lys Ala Lys Glu Asp Phe Leu
1               5                   10                  15

Lys Lys Trp Glu Asn Pro Thr Gln Asn Asn Ala Gly Leu Glu Asp Phe
            20                  25                  30

Glu Arg Lys Lys Thr Leu Gly Thr Gly Ser Phe Gly Arg Val Met Leu
        35                  40                  45

Val Lys His Lys Ala Thr Glu Gln Tyr Tyr Ala Met Lys Ile Leu Asp
    50                  55                  60

Lys Gln Lys Val Val Lys Leu Lys Gln Ile Glu His Thr Leu Asn Glu
65                  70                  75                  80

Lys Arg Ile Leu Gln Ala Val Asn Phe Pro Phe Leu Val Arg Leu Glu
                85                  90                  95

Tyr Ala Phe Lys Asp Asn Ser Asn Leu Tyr Met Val Met Glu Tyr Val
            100                 105                 110

Pro Gly Gly Glu Met Phe Ser His Leu Arg Arg Ile Gly Arg Phe Ser
        115                 120                 125

Glu Pro His Ala Arg Phe Tyr Ala Ala Gln Ile Val Leu Thr Phe Glu
    130                 135                 140

Tyr Leu His Ser Leu Asp Leu Ile Tyr Arg Asp Leu Lys Pro Glu Asn
145                 150                 155                 160

Leu Leu Ile Asp His Gln Gly Tyr Ile Gln Val Thr Asp Phe Gly Phe
                165                 170                 175

Ala Lys Arg Val Lys Gly Arg Thr Trp Thr Leu Cys Gly Thr Pro Glu
            180                 185                 190

Tyr Leu Ala Pro Glu Ile Ile Leu Ser Lys Gly Tyr Asn Lys Ala Val
        195                 200                 205

Asp Trp Trp Ala Leu Gly Val Leu Ile Tyr Glu Met Ala Ala Gly Tyr
    210                 215                 220

Pro Pro Phe Phe Ala Asp Gln Pro Ile Gln Ile Tyr Glu Lys Ile Val
225 230 235 240

Ser Gly Lys Asn Phe
245

<210> 79
<211> 5429
<212> DNA
<213> Homo sapiens

<400> 79
agaacaactt ttttgacttc ctgcaaagag gacccttaca gtatttttgg agaagttagt 60

aaaaccgaat ctgacatcat cacctagcag ttcatgcagc tagcaagtgg tttgttctta 120

gggtaacaga ggaggaaatt gttcctcgtc tgataagaca acagtggaga aaggacgcat 180

gctgtttctt agggacacgg ctgacttcca gatatgacca tgtatttgtg gcttaaactc 240

ttggcatttg gctttgcctt tctggacaca gaagtatttg tgacagggca aagcccaaca 300

ccttccccca ctggattgac tacagcaaag atgcccagtg ttccactttc aagtgacccc 360

ttacctactc acaccactgc attctcaccc gcaagcacct ttgaaagaga aaatgacttc 420

tcagagacca caacttctct tagtccagac aatacttcca cccaagtatc cccggactct 480

ttggataatg ctagtgcttt taataccaca ggtgtttcat cagtacagac gcctcacctt 540

cccacgcacg cagactcgca gacgccctct gctggaactg acacgcagac attcagcggc 600

tccgccgcca atgcaaaact caaccctacc ccaggcagca atgctatctc agatgtccca 660

ggagagagga gtacagccag cacctttcct acagacccag tttccccatt gacaaccacc 720

ctcagccttg cacaccacag ctctgctgcc ttacctgcac gcacctccaa caccaccatc 780

acagcgaaca cctcagatgc ctaccttaat gcctctgaaa caaccactct gagcccttct 840

ggaagcgctg tcatttcaac cacaacaata gctactactc catctaagcc aacatgtgat 900

gaaaaatatg caaacatcac tgtggattac ttatataaca aggaaactaa attatttaca 960

gcaaagctaa atgttaatga gaatgtggaa tgtggaaaca atacttgcac aaacaatgag 1020

gtgcataacc ttacagaatg taaaaatgcg tctgtttcca tatctcataa ttcatgtact 1080

gctcctgata agacattaat attagatgtg ccaccagggg ttgaaaagtt tcagttacat 1140

gattgtacac aagttgaaaa agcagatact actatttgtt taaaatggaa aaatattgaa 1200

acctttactt gtgatacaca gaatattacc tacagatttc agtgtggtaa tatgatattt 1260

gataataaag aaattaaatt agaaaacctt gaacccgaac atgagtataa gtgtgactca 1320

gaaatactct ataataacca caagtttact aacgcaagta aaattattaa aacagatttt 1380

gggagtccag gagagcctca gattattttt tgtagaagtg aagctgcaca tcaaggagta 1440

attacctgga atccccctca aagatcattt cataatttta ccctctgtta tataaaagag 1500
acagaaaaag attgcctcaa tctggataaa aacctgatca aatatgattt gcaaaattta 1560
aaaccttata cgaaatatgt tttatcatta catgcctaca tcattgcaaa agtgcaacgt 1620
aatggaagtg ctgcaatgtg tcatttcaca actaaaagtg ctcctccaag ccaggtctgg 1680
aacatgactg tctccatgac atcagataat agtatgcatg tcaagtgtag gcctcccagg 1740
gaccgtaatg gcccccatga acgttaccat ttggaagttg aagctggaaa tactctggtt 1800
agaaatgagt cgcataagaa ttgcgatttc cgtgtaaaag atcttcaata ttcaacagac 1860
tacactttta aggcctattt tcacaatgga gactatcctg gagaaccctt tattttacat 1920
cattcaacat cttataattc taaggcactg atagcatttc tggcatttct gattattgtg 1980
acatcaatag ccctgcttgt tgttctctac aaaatctatg atctacataa gaaaagatcc 2040
tgcaatttag atgaacagca ggagcttgtt gaaagggatg atgaaaaaca actgatgaat 2100
gtggagccaa tccatgcaga tattttgttg gaaacttata agaggaagat tgctgatgaa 2160
ggaagacttt ttctggctga atttcagagc atcccgcggg tgttcagcaa gtttcctata 2220
aaggaagctc gaaagccctt taaccagaat aaaaaccgtt atgttgacat tcttccttat 2280
gattataacc gtgttgaact ctctgagata aacggagatg cagggtcaaa ctacataaat 2340
gccagctata ttgatggttt caaagaaccc aggaaataca ttgctgcaca aggtcccagg 2400
gatgaaactg ttgatgattt ctggaggatg atttgggaac agaaagccac agttattgtc 2460
atggtcactc gatgtgaaga aggaaacagg aacaagtgtg cagaatactg gccgtcaatg 2520
gaagagggca ctcgggcttt tggagatgtt gttgtaaaga tcaaccagca caaaagatgt 2580
ccagattaca tcattcagaa attgaacatt gtaaataaaa aagaaaaagc aactggaaga 2640
gaggtgactc acattcagtt caccagctgg ccagaccacg ggtgcctga ggatcctcac 2700
ttgctcctca aactgagaag gagagtgaat gccttcagca atttcttcag tggtcccatt 2760
gtggtgcact gcagtgctgg tgttgggcgc acaggaacct atatcggaat tgatgccatg 2820
ctagaaggcc tggaagccga gaacaaagtg gatgtttatg gttatgttgt caagctaagg 2880
cgacagagat gcctgatggt tcaagtagag gcccagtaca tcttgatcca tcaggctttg 2940
gtggaataca atcagtttgg agaaacagaa gtgaatttgt ctgaattaca tccatatcta 3000
cataacatga agaaaaggga tccacccagt gagccgtctc cactagaggc tgaattccag 3060
agacttcctt catataggag ctggaggaca cagcacattg gaaatcaaga agaaaataaa 3120
agtaaaaaca ggaattctaa tgtcatccca tatgactata acagagtgcc acttaaacat 3180
gagctggaaa tgagtaaaga gagtgagcat gattcagatg aatcctctga tgatgacagt 3240
gattcagagg aaccaagcaa atacatcaat gcatctttta taatgagcta ctggaaacct 3300
gaagtgatga ttgctgctca gggaccactg aaggagacca ttggtgactt ttggcagatg 3360

atcttccaaa gaaaagtcaa agttattgtt atgctgacag aactgaaaca tggagaccag 3420

gaaatctgtg ctcagtactg gggagaagga aagcaaacat atggagatat tgaagttgac 3480

ctgaaagaca cagacaaatc ttcaacttat acccttcgtg tctttgaact gagacattcc 3540

aagaggaaag actctcgaac tgtgtaccag taccaatata caaactggag tgtggagcag 3600

cttcctgcag aacccaagga attaatctct atgattcagg tcgtcaaaca aaaacttccc 3660

cagaagaatt cctctgaagg gaacaagcat cacaagagta cacctctact cattcactgc 3720

agggatggat ctcagcaaac gggaatattt tgtgctttgt taaatctctt agaaagtgcg 3780

gaaacagaag aggtagtgga tatttttcaa gtggtaaaag ctctacgcaa agctaggcca 3840

ggcatggttt ccacattcga gcaatatcaa ttcctatatg acgtcattgc cagcacctac 3900

cctgctcaga atggacaagt aaagaaaaac aaccatcaag aagataaaat tgaatttgat 3960

aatgaagtgg acaaagtaaa gcaggatgct aattgtgtta atccacttgg tgccccagaa 4020

aagctccctg aagcaaagga acaggctgaa ggttctgaac ccacgagtgg cactgagggg 4080

ccagaacatt ctgtcaatgg tcctgcaagt ccagctttaa atcaaggttc ataggaaaag 4140

acataaatga ggaaactcca aacctcctgt tagctgttat ttctattttt gtagaagtag 4200

gaagtgaaaa taggtataca gtggattaat taaatgcagc gaaccaatat ttgtagaagg 4260

gttatatttt actactgtgg aaaaatattt aagatagttt tgccagaaca gtttgtacag 4320

acgtatgctt attttaaaat tttatctctt attcagtaaa aaacaacttc tttgtaatcg 4380

ttatgtgtgt atatgtatgt gtgtatgggt gtgtgtttgt gtgagagaca gagaaagaga 4440

gagaattctt tcaagtgaat ctaaaagctt ttgcttttcc tttgttttta tgaagaaaaa 4500

atacatttta tattagaagt gttaacttag cttgaaggat ctgtttttaa aaatcataaa 4560

ctgtgtgcag actcaataaa atcatgtaca tttctgaaat gacctcaaga tgtcctcctt 4620

gttctactca tatatatcta tcttatatag tttactattt tacttctaga gatagtacat 4680

aaaggtggta tgtgtgtgta tgctactaca aaaaagttgt taactaaatt aacattggga 4740

aatcttatat tccatatatt agcatttagt ccaatgtctt tttaagctta tttaattaaa 4800

aaatttccag tgagcttatc atgctgtctt tacatggggt tttcaatttt gcatgctcga 4860

ttattccctg tacaatattt aaaatttatt gcttgatact tttgacaaca aattaggttt 4920

tgtacaattg aacttaaata aatgtcatta aaataaataa atgcaatatg tattaatatt 4980

cattgtataa aaatagaaga atacaaacat atttgttaaa tatttacata tgaaatttaa 5040

tatagctatt tttatggaat ttttcattga tatgaaaaat atgatattgc atatgcatag 5100

ttcccatgtt aaatcccatt cataactttc attaaagcat ttactttgaa tttctccaat 5160

gcttagaatg tttttaccag gaatggatgt cgctaatcat aataaaattc aaccattatt 5220

tttttcttgt ttataataca ttgtgttata tgttcaaata tgaaatgtgt atgcacctat 5280

tgaaatatgt ttaatgcatt tattaacatt tgcaggacac ttttacaggc cccaattatc 5340

caatagtcta ataattgttt aagatctaga aaaaaaaaat caagaatagt ggtatttttc 5400

atgaagtaat aaaaactcgt tttggtgaa 5429

<210> 80
<211> 4946
<212> DNA
<213> Homo sapiens

<400> 80
agaacaactt ttttgacttc ctgcaaagag gacccttaca gtatttttgg agaagttagt 60

aaaaccgaat ctgacatcat cacctagcag ttcatgcagc tagcaagtgg tttgttctta 120

gggtaacaga ggaggaaatt gttcctcgtc tgataagaca acagtggaga aaggacgcat 180

gctgtttctt agggacacgg ctgacttcca gatatgacca tgtatttgtg gcttaaactc 240

ttggcatttg gctttgcctt tctggacaca gaagtatttg tgacagggca aagcccaaca 300

ccttcccccca ctgatgccta ccttaatgcc tctgaaacaa ccactctgag cccttctgga 360

agcgctgtca tttcaaccac aacaatagct actactccat ctaagccaac atgtgatgaa 420

aaatatgcaa acatcactgt ggattactta tataacaagg aaactaaatt atttacagca 480

aagctaaatg ttaatgagaa tgtggaatgt ggaaacaata cttgcacaaa caatgaggtg 540

cataacctta cagaatgtaa aaatgcgtct gtttccatat ctcataattc atgtactgct 600

cctgataaga cattaatatt agatgtgcca ccaggggttg aaaagtttca gttacatgat 660

tgtacacaag ttgaaaaagc agatactact atttgtttaa aatggaaaaa tattgaaacc 720

tttacttgtg atacacagaa tattacctac agatttcagt gtggtaatat gatatttgat 780

aataaagaaa ttaaattaga aaaccttgaa cccgaacatg agtataagtg tgactcagaa 840

atactctata ataaccacaa gtttactaac gcaagtaaaa ttattaaaac agattttggg 900

agtccaggag agcctcagat tatttttgt agaagtgaag ctgcacatca aggagtaatt 960

acctggaatc cccctcaaag atcatttcat aattttaccc tctgttatat aaaagagaca 1020

gaaaaagatt gcctcaatct ggataaaaac ctgatcaaat atgatttgca aaatttaaaa 1080

ccttatacga aatatgtttt atcattacat gcctacatca ttgcaaaagt gcaacgtaat 1140

ggaagtgctg caatgtgtca tttcacaact aaaagtgctc ctccaagcca ggtctggaac 1200

atgactgtct ccatgacatc agataatagt atgcatgtca agtgtaggcc tcccagggac 1260

cgtaatggcc cccatgaacg ttaccatttg gaagttgaag ctggaaatac tctggttaga 1320

aatgagtcgc ataagaattg cgatttccgt gtaaaagatc ttcaatattc aacagactac 1380

acttttaagg cctattttca caatggagac tatcctggag aaccctttat tttacatcat 1440

tcaacatctt ataattctaa ggcactgata gcatttctgg catttctgat tattgtgaca 1500

tcaatagccc tgcttgttgt tctctacaaa atctatgatc tacataagaa aagatcctgc 1560

aatttagatg aacagcagga gcttgttgaa agggatgatg aaaaacaact gatgaatgtg 1620

gagccaatcc atgcagatat tttgttggaa acttataaga ggaagattgc tgatgaagga 1680

agactttttc tggctgaatt tcagagcatc ccgcgggtgt tcagcaagtt tcctataaag 1740

gaagctcgaa agccctttaa ccagaataaa aaccgttatg ttgacattct tccttatgat 1800

tataaccgtg ttgaactctc tgagataaac ggagatgcag ggtcaaacta cataaatgcc 1860

agctatattg atggtttcaa agaacccagg aaatacattg ctgcacaagg tcccagggat 1920

gaaactgttg atgatttctg gaggatgatt tgggaacaga aagccacagt tattgtcatg 1980

gtcactcgat gtgaagaagg aaacaggaac aagtgtgcag aatactggcc gtcaatggaa 2040

gagggcactc gggcttttgg agatgttgtt gtaaagatca accagcacaa aagatgtcca 2100

gattacatca ttcagaaatt gaacattgta aataaaaaag aaaaagcaac tggaagagag 2160

gtgactcaca ttcagttcac cagctggcca gaccacgggg tgcctgagga tcctcacttg 2220

ctcctcaaac tgagaaggag agtgaatgcc ttcagcaatt tcttcagtgg tcccattgtg 2280

gtgcactgca gtgctggtgt tgggcgcaca ggaacctata tcggaattga tgccatgcta 2340

gaaggcctgg aagccgagaa caaagtggat gtttatggtt atgttgtcaa gctaaggcga 2400

cagagatgcc tgatggttca agtagaggcc cagtacatct tgatccatca ggctttggtg 2460

gaatacaatc agtttggaga aacagaagtg aatttgtctg aattacatcc atatctacat 2520

aacatgaaga aaagggatcc acccagtgag ccgtctccac tagaggctga attccagaga 2580

cttccttcat ataggagctg gaggacacag cacattggaa atcaagaaga aaataaaagt 2640

aaaaacagga attctaatgt catcccatat gactataaca gagtgccact taaacatgag 2700

ctggaaatga gtaaagagag tgagcatgat tcagatgaat cctctgatga tgacagtgat 2760

tcagaggaac caagcaaata catcaatgca tcttttataa tgagctactg gaaacctgaa 2820

gtgatgattg ctgctcaggg accactgaag gagaccattg gtgacttttg gcagatgatc 2880

ttccaaagaa aagtcaaagt tattgttatg ctgacagaac tgaaacatgg agaccaggaa 2940

atctgtgctc agtactgggg agaaggaaag caaacatatg gagatattga agttgacctg 3000

aaagacacag acaaatcttc aacttatacc cttcgtgtct ttgaactgag acattccaag 3060

aggaaagact ctcgaactgt gtaccagtac caatatacaa actggagtgt ggagcagctt 3120

cctgcagaac ccaaggaatt aatctctatg attcaggtcg tcaaacaaaa acttccccag 3180

aagaattcct ctgaagggaa caagcatcac aagagtacac ctctactcat tcactgcagg 3240

gatggatctc agcaaacggg aatattttgt gctttgttaa atctcttaga aagtgcggaa 3300

acagaagagg tagtggatat tttttcaagtg gtaaaagctc tacgcaaagc taggccaggc 3360

atggtttcca cattcgagca atatcaattc ctatatgacg tcattgccag cacctaccct 3420

gctcagaatg gacaagtaaa gaaaaacaac catcaagaag ataaaattga atttgataat 3480

gaagtggaca aagtaaagca ggatgctaat tgtgttaatc cacttggtgc cccagaaaag 3540

ctccctgaag caaaggaaca ggctgaaggt tctgaaccca cgagtggcac tgaggggcca 3600

gaacattctg tcaatggtcc tgcaagtcca gctttaaatc aaggttcata ggaaaagaca 3660

taaatgagga aactccaaac ctcctgttag ctgttatttc tatttttgta gaagtaggaa 3720

gtgaaaatag gtatacagtg gattaattaa atgcagcgaa ccaatatttg tagaagggtt 3780

atattttact actgtggaaa aatatttaag atagttttgc cagaacagtt tgtacagacg 3840

tatgcttatt ttaaaatttt atctcttatt cagtaaaaaa caacttcttt gtaatcgtta 3900

tgtgtgtata tgtatgtgtg tatgggtgtg tgtttgtgtg agagacagag aaagagagag 3960

aattctttca agtgaatcta aaagcttttg cttttccttt gtttttatga agaaaaaata 4020

cattttatat tagaagtgtt aacttagctt gaaggatctg tttttaaaaa tcataaactg 4080

tgtgcagact caataaaatc atgtacattt ctgaaatgac ctcaagatgt cctccttgtt 4140

ctactcatat atatctatct tatatagttt actattttac ttctagagat agtacataaa 4200

ggtggtatgt gtgtgtatgc tactacaaaa aagttgttaa ctaaattaac attgggaaat 4260

cttatattcc atatattagc atttagtcca atgtcttttt aagcttattt aattaaaaaa 4320

tttccagtga gcttatcatg ctgtctttac atggggtttt caattttgca tgctcgatta 4380

ttccctgtac aatatttaaa atttattgct tgatactttt gacaacaaat taggttttgt 4440

acaattgaac ttaaataaat gtcattaaaa taaataaatg caatatgtat taatattcat 4500

tgtataaaaa tagaagaata caaacatatt tgttaaatat ttacatatga aatttaatat 4560

agctattttt atggaatttt tcattgatat gaaaaatatg atattgcata tgcatagttc 4620

ccatgttaaa tcccattcat aactttcatt aaagcattta ctttgaattt ctccaatgct 4680

tagaatgttt ttaccaggaa tggatgtcgc taatcataat aaaattcaac cattattttt 4740

ttcttgttta taatacattg tgttatatgt tcaaatatga aatgtgtatg cacctattga 4800

aatatgttta atgcatttat taacatttgc aggacacttt tacaggcccc aattatccaa 4860

tagtctaata attgtttaag atctagaaaa aaaaaatcaa gaatagtggt atttttcatg 4920

aagtaataaa aactcgtttt ggtgaa 4946

<210> 81
<211> 1306
<212> PRT
<213> Homo sapiens

<400> 81

Met Thr Met Tyr Leu Trp Leu Lys Leu Leu Ala Phe Gly Phe Ala Phe
1 5 10 15

Leu Asp Thr Glu Val Phe Val Thr Gly Gln Ser Pro Thr Pro Ser Pro
20 25 30

Thr Gly Leu Thr Thr Ala Lys Met Pro Ser Val Pro Leu Ser Ser Asp
35 40 45

Pro Leu Pro Thr His Thr Thr Ala Phe Ser Pro Ala Ser Thr Phe Glu
50 55 60

Arg Glu Asn Asp Phe Ser Glu Thr Thr Thr Ser Leu Ser Pro Asp Asn
65 70 75 80

Thr Ser Thr Gln Val Ser Pro Asp Ser Leu Asp Asn Ala Ser Ala Phe
85 90 95

Asn Thr Thr Gly Val Ser Ser Val Gln Thr Pro His Leu Pro Thr His
100 105 110

Ala Asp Ser Gln Thr Pro Ser Ala Gly Thr Asp Thr Gln Thr Phe Ser
115 120 125

Gly Ser Ala Ala Asn Ala Lys Leu Asn Pro Thr Pro Gly Ser Asn Ala
130 135 140

Ile Ser Asp Val Pro Gly Glu Arg Ser Thr Ala Ser Thr Phe Pro Thr
145 150 155 160

Asp Pro Val Ser Pro Leu Thr Thr Thr Leu Ser Leu Ala His His Ser
165 170 175

Ser Ala Ala Leu Pro Ala Arg Thr Ser Asn Thr Thr Ile Thr Ala Asn
180 185 190

Thr Ser Asp Ala Tyr Leu Asn Ala Ser Glu Thr Thr Thr Leu Ser Pro
195 200 205

Ser Gly Ser Ala Val Ile Ser Thr Thr Thr Ile Ala Thr Thr Pro Ser
210 215 220

Lys Pro Thr Cys Asp Glu Lys Tyr Ala Asn Ile Thr Val Asp Tyr Leu
225 230 235 240

Tyr Asn Lys Glu Thr Lys Leu Phe Thr Ala Lys Leu Asn Val Asn Glu
245 250 255

```
Asn Val Glu Cys Gly Asn Asn Thr Cys Thr Asn Asn Glu Val His Asn
            260                 265                 270

Leu Thr Glu Cys Lys Asn Ala Ser Val Ser Ile Ser His Asn Ser Cys
        275                 280                 285

Thr Ala Pro Asp Lys Thr Leu Ile Leu Asp Val Pro Pro Gly Val Glu
    290                 295                 300

Lys Phe Gln Leu His Asp Cys Thr Gln Val Glu Lys Ala Asp Thr Thr
305                 310                 315                 320

Ile Cys Leu Lys Trp Lys Asn Ile Glu Thr Phe Thr Cys Asp Thr Gln
                325                 330                 335

Asn Ile Thr Tyr Arg Phe Gln Cys Gly Asn Met Ile Phe Asp Asn Lys
            340                 345                 350

Glu Ile Lys Leu Glu Asn Leu Glu Pro Glu His Glu Tyr Lys Cys Asp
        355                 360                 365

Ser Glu Ile Leu Tyr Asn Asn His Lys Phe Thr Asn Ala Ser Lys Ile
    370                 375                 380

Ile Lys Thr Asp Phe Gly Ser Pro Gly Glu Pro Gln Ile Ile Phe Cys
385                 390                 395                 400

Arg Ser Glu Ala Ala His Gln Gly Val Ile Thr Trp Asn Pro Pro Gln
                405                 410                 415

Arg Ser Phe His Asn Phe Thr Leu Cys Tyr Ile Lys Glu Thr Glu Lys
            420                 425                 430

Asp Cys Leu Asn Leu Asp Lys Asn Leu Ile Lys Tyr Asp Leu Gln Asn
        435                 440                 445

Leu Lys Pro Tyr Thr Lys Tyr Val Leu Ser Leu His Ala Tyr Ile Ile
    450                 455                 460

Ala Lys Val Gln Arg Asn Gly Ser Ala Ala Met Cys His Phe Thr Thr
465                 470                 475                 480

Lys Ser Ala Pro Pro Ser Gln Val Trp Asn Met Thr Val Ser Met Thr
                485                 490                 495

Ser Asp Asn Ser Met His Val Lys Cys Arg Pro Pro Arg Asp Arg Asn
            500                 505                 510
```

Gly Pro His Glu Arg Tyr His Leu Glu Val Glu Ala Gly Asn Thr Leu
515 520 525

Val Arg Asn Glu Ser His Lys Asn Cys Asp Phe Arg Val Lys Asp Leu
530 535 540

Gln Tyr Ser Thr Asp Tyr Thr Phe Lys Ala Tyr Phe His Asn Gly Asp
545 550 555 560

Tyr Pro Gly Glu Pro Phe Ile Leu His His Ser Thr Ser Tyr Asn Ser
565 570 575

Lys Ala Leu Ile Ala Phe Leu Ala Phe Leu Ile Ile Val Thr Ser Ile
580 585 590

Ala Leu Leu Val Val Leu Tyr Lys Ile Tyr Asp Leu His Lys Lys Arg
595 600 605

Ser Cys Asn Leu Asp Glu Gln Gln Glu Leu Val Glu Arg Asp Asp Glu
610 615 620

Lys Gln Leu Met Asn Val Glu Pro Ile His Ala Asp Ile Leu Leu Glu
625 630 635 640

Thr Tyr Lys Arg Lys Ile Ala Asp Glu Gly Arg Leu Phe Leu Ala Glu
645 650 655

Phe Gln Ser Ile Pro Arg Val Phe Ser Lys Phe Pro Ile Lys Glu Ala
660 665 670

Arg Lys Pro Phe Asn Gln Asn Lys Asn Arg Tyr Val Asp Ile Leu Pro
675 680 685

Tyr Asp Tyr Asn Arg Val Glu Leu Ser Glu Ile Asn Gly Asp Ala Gly
690 695 700

Ser Asn Tyr Ile Asn Ala Ser Tyr Ile Asp Gly Phe Lys Glu Pro Arg
705 710 715 720

Lys Tyr Ile Ala Ala Gln Gly Pro Arg Asp Glu Thr Val Asp Asp Phe
725 730 735

Trp Arg Met Ile Trp Glu Gln Lys Ala Thr Val Ile Val Met Val Thr
740 745 750

Arg Cys Glu Glu Gly Asn Arg Asn Lys Cys Ala Glu Tyr Trp Pro Ser

755 760 765

Met Glu Glu Gly Thr Arg Ala Phe Gly Asp Val Val Val Lys Ile Asn
770 775 780

Gln His Lys Arg Cys Pro Asp Tyr Ile Ile Gln Lys Leu Asn Ile Val
785 790 795 800

Asn Lys Lys Glu Lys Ala Thr Gly Arg Glu Val Thr His Ile Gln Phe
805 810 815

Thr Ser Trp Pro Asp His Gly Val Pro Glu Asp Pro His Leu Leu Leu
820 825 830

Lys Leu Arg Arg Arg Val Asn Ala Phe Ser Asn Phe Phe Ser Gly Pro
835 840 845

Ile Val Val His Cys Ser Ala Gly Val Gly Arg Thr Gly Thr Tyr Ile
850 855 860

Gly Ile Asp Ala Met Leu Glu Gly Leu Glu Ala Glu Asn Lys Val Asp
865 870 875 880

Val Tyr Gly Tyr Val Val Lys Leu Arg Arg Gln Arg Cys Leu Met Val
885 890 895

Gln Val Glu Ala Gln Tyr Ile Leu Ile His Gln Ala Leu Val Glu Tyr
900 905 910

Asn Gln Phe Gly Glu Thr Glu Val Asn Leu Ser Glu Leu His Pro Tyr
915 920 925

Leu His Asn Met Lys Lys Arg Asp Pro Pro Ser Glu Pro Ser Pro Leu
930 935 940

Glu Ala Glu Phe Gln Arg Leu Pro Ser Tyr Arg Ser Trp Arg Thr Gln
945 950 955 960

His Ile Gly Asn Gln Glu Glu Asn Lys Ser Lys Asn Arg Asn Ser Asn
965 970 975

Val Ile Pro Tyr Asp Tyr Asn Arg Val Pro Leu Lys His Glu Leu Glu
980 985 990

Met Ser Lys Glu Ser Glu His Asp Ser Asp Glu Ser Ser Asp Asp Asp
995 1000 1005

```
Ser Asp  Ser Glu Glu Pro Ser  Lys Tyr Ile Asn Ala  Ser Phe Ile
    1010                 1015                 1020

Met Ser  Tyr Trp Lys Pro Glu  Val Met Ile Ala Ala  Gln Gly Pro
    1025                 1030                 1035

Leu Lys  Glu Thr Ile Gly Asp  Phe Trp Gln Met Ile  Phe Gln Arg
    1040                 1045                 1050

Lys Val  Lys Val Ile Val Met  Leu Thr Glu Leu Lys  His Gly Asp
    1055                 1060                 1065

Gln Glu  Ile Cys Ala Gln Tyr  Trp Gly Glu Gly Lys  Gln Thr Tyr
    1070                 1075                 1080

Gly Asp  Ile Glu Val Asp Leu  Lys Asp Thr Asp Lys  Ser Ser Thr
    1085                 1090                 1095

Tyr Thr  Leu Arg Val Phe Glu  Leu Arg His Ser Lys  Arg Lys Asp
    1100                 1105                 1110

Ser Arg  Thr Val Tyr Gln Tyr  Gln Tyr Thr Asn Trp  Ser Val Glu
    1115                 1120                 1125

Gln Leu  Pro Ala Glu Pro Lys  Glu Leu Ile Ser Met  Ile Gln Val
    1130                 1135                 1140

Val Lys  Gln Lys Leu Pro Gln  Lys Asn Ser Ser Glu  Gly Asn Lys
    1145                 1150                 1155

His His  Lys Ser Thr Pro Leu  Leu Ile His Cys Arg  Asp Gly Ser
    1160                 1165                 1170

Gln Gln  Thr Gly Ile Phe Cys  Ala Leu Leu Asn Leu  Leu Glu Ser
    1175                 1180                 1185

Ala Glu  Thr Glu Glu Val Val  Asp Ile Phe Gln Val  Val Lys Ala
    1190                 1195                 1200

Leu Arg  Lys Ala Arg Pro Gly  Met Val Ser Thr Phe  Glu Gln Tyr
    1205                 1210                 1215

Gln Phe  Leu Tyr Asp Val Ile  Ala Ser Thr Tyr Pro  Ala Gln Asn
    1220                 1225                 1230

Gly Gln  Val Lys Lys Asn Asn  His Gln Glu Asp Lys  Ile Glu Phe
    1235                 1240                 1245
```

Asp Asn Glu Val Asp Lys Val Lys Gln Asp Ala Asn Cys Val Asn
1250 1255 1260

Pro Leu Gly Ala Pro Glu Lys Leu Pro Glu Ala Lys Glu Gln Ala
1265 1270 1275

Glu Gly Ser Glu Pro Thr Ser Gly Thr Glu Gly Pro Glu His Ser
1280 1285 1290

Val Asn Gly Pro Ala Ser Pro Ala Leu Asn Gln Gly Ser
1295 1300 1305

<210> 82
<211> 1145
<212> PRT
<213> Homo sapiens

<400> 82

Met Thr Met Tyr Leu Trp Leu Lys Leu Leu Ala Phe Gly Phe Ala Phe
1 5 10 15

Leu Asp Thr Glu Val Phe Val Thr Gly Gln Ser Pro Thr Pro Ser Pro
20 25 30

Thr Asp Ala Tyr Leu Asn Ala Ser Glu Thr Thr Thr Leu Ser Pro Ser
35 40 45

Gly Ser Ala Val Ile Ser Thr Thr Thr Ile Ala Thr Thr Pro Ser Lys
50 55 60

Pro Thr Cys Asp Glu Lys Tyr Ala Asn Ile Thr Val Asp Tyr Leu Tyr
65 70 75 80

Asn Lys Glu Thr Lys Leu Phe Thr Ala Lys Leu Asn Val Asn Glu Asn
85 90 95

Val Glu Cys Gly Asn Asn Thr Cys Thr Asn Asn Glu Val His Asn Leu
100 105 110

Thr Glu Cys Lys Asn Ala Ser Val Ser Ile Ser His Asn Ser Cys Thr
115 120 125

Ala Pro Asp Lys Thr Leu Ile Leu Asp Val Pro Pro Gly Val Glu Lys
130 135 140

Phe Gln Leu His Asp Cys Thr Gln Val Glu Lys Ala Asp Thr Thr Ile
145 150 155 160

Cys Leu Lys Trp Lys Asn Ile Glu Thr Phe Thr Cys Asp Thr Gln Asn
165 170 175

Ile Thr Tyr Arg Phe Gln Cys Gly Asn Met Ile Phe Asp Asn Lys Glu
180 185 190

Ile Lys Leu Glu Asn Leu Glu Pro Glu His Glu Tyr Lys Cys Asp Ser
195 200 205

Glu Ile Leu Tyr Asn Asn His Lys Phe Thr Asn Ala Ser Lys Ile Ile
210 215 220

Lys Thr Asp Phe Gly Ser Pro Gly Glu Pro Gln Ile Ile Phe Cys Arg
225 230 235 240

Ser Glu Ala Ala His Gln Gly Val Ile Thr Trp Asn Pro Pro Gln Arg
245 250 255

Ser Phe His Asn Phe Thr Leu Cys Tyr Ile Lys Glu Thr Glu Lys Asp
260 265 270

Cys Leu Asn Leu Asp Lys Asn Leu Ile Lys Tyr Asp Leu Gln Asn Leu
275 280 285

Lys Pro Tyr Thr Lys Tyr Val Leu Ser Leu His Ala Tyr Ile Ile Ala
290 295 300

Lys Val Gln Arg Asn Gly Ser Ala Ala Met Cys His Phe Thr Thr Lys
305 310 315 320

Ser Ala Pro Pro Ser Gln Val Trp Asn Met Thr Val Ser Met Thr Ser
325 330 335

Asp Asn Ser Met His Val Lys Cys Arg Pro Pro Arg Asp Arg Asn Gly
340 345 350

Pro His Glu Arg Tyr His Leu Glu Val Glu Ala Gly Asn Thr Leu Val
355 360 365

Arg Asn Glu Ser His Lys Asn Cys Asp Phe Arg Val Lys Asp Leu Gln
370 375 380

Tyr Ser Thr Asp Tyr Thr Phe Lys Ala Tyr Phe His Asn Gly Asp Tyr
385 390 395 400

Pro Gly Glu Pro Phe Ile Leu His His Ser Thr Ser Tyr Asn Ser Lys
405 410 415

Ala Leu Ile Ala Phe Leu Ala Phe Leu Ile Ile Val Thr Ser Ile Ala
420 425 430

Leu Leu Val Val Leu Tyr Lys Ile Tyr Asp Leu His Lys Lys Arg Ser
435 440 445

Cys Asn Leu Asp Glu Gln Gln Glu Leu Val Glu Arg Asp Asp Glu Lys
450 455 460

Gln Leu Met Asn Val Glu Pro Ile His Ala Asp Ile Leu Leu Glu Thr
465 470 475 480

Tyr Lys Arg Lys Ile Ala Asp Glu Gly Arg Leu Phe Leu Ala Glu Phe
485 490 495

Gln Ser Ile Pro Arg Val Phe Ser Lys Phe Pro Ile Lys Glu Ala Arg
500 505 510

Lys Pro Phe Asn Gln Asn Lys Asn Arg Tyr Val Asp Ile Leu Pro Tyr
515 520 525

Asp Tyr Asn Arg Val Glu Leu Ser Glu Ile Asn Gly Asp Ala Gly Ser
530 535 540

Asn Tyr Ile Asn Ala Ser Tyr Ile Asp Gly Phe Lys Glu Pro Arg Lys
545 550 555 560

Tyr Ile Ala Ala Gln Gly Pro Arg Asp Glu Thr Val Asp Asp Phe Trp
565 570 575

Arg Met Ile Trp Glu Gln Lys Ala Thr Val Ile Val Met Val Thr Arg
580 585 590

Cys Glu Glu Gly Asn Arg Asn Lys Cys Ala Glu Tyr Trp Pro Ser Met
595 600 605

Glu Glu Gly Thr Arg Ala Phe Gly Asp Val Val Val Lys Ile Asn Gln
610 615 620

His Lys Arg Cys Pro Asp Tyr Ile Ile Gln Lys Leu Asn Ile Val Asn
625 630 635 640

Lys Lys Glu Lys Ala Thr Gly Arg Glu Val Thr His Ile Gln Phe Thr
645 650 655

Ser Trp Pro Asp His Gly Val Pro Glu Asp Pro His Leu Leu Leu Lys

660 665 670

Leu Arg Arg Arg Val Asn Ala Phe Ser Asn Phe Phe Ser Gly Pro Ile
675 680 685

Val Val His Cys Ser Ala Gly Val Gly Arg Thr Gly Thr Tyr Ile Gly
690 695 700

Ile Asp Ala Met Leu Glu Gly Leu Glu Ala Glu Asn Lys Val Asp Val
705 710 715 720

Tyr Gly Tyr Val Val Lys Leu Arg Arg Gln Arg Cys Leu Met Val Gln
725 730 735

Val Glu Ala Gln Tyr Ile Leu Ile His Gln Ala Leu Val Glu Tyr Asn
740 745 750

Gln Phe Gly Glu Thr Glu Val Asn Leu Ser Glu Leu His Pro Tyr Leu
755 760 765

His Asn Met Lys Lys Arg Asp Pro Pro Ser Glu Pro Ser Pro Leu Glu
770 775 780

Ala Glu Phe Gln Arg Leu Pro Ser Tyr Arg Ser Trp Arg Thr Gln His
785 790 795 800

Ile Gly Asn Gln Glu Glu Asn Lys Ser Lys Asn Arg Asn Ser Asn Val
805 810 815

Ile Pro Tyr Asp Tyr Asn Arg Val Pro Leu Lys His Glu Leu Glu Met
820 825 830

Ser Lys Glu Ser Glu His Asp Ser Asp Glu Ser Ser Asp Asp Asp Ser
835 840 845

Asp Ser Glu Glu Pro Ser Lys Tyr Ile Asn Ala Ser Phe Ile Met Ser
850 855 860

Tyr Trp Lys Pro Glu Val Met Ile Ala Ala Gln Gly Pro Leu Lys Glu
865 870 875 880

Thr Ile Gly Asp Phe Trp Gln Met Ile Phe Gln Arg Lys Val Lys Val
885 890 895

Ile Val Met Leu Thr Glu Leu Lys His Gly Asp Gln Glu Ile Cys Ala
900 905 910

Gln Tyr Trp Gly Glu Gly Lys Gln Thr Tyr Gly Asp Ile Glu Val Asp
915 920 925

Leu Lys Asp Thr Asp Lys Ser Ser Thr Tyr Thr Leu Arg Val Phe Glu
930 935 940

Leu Arg His Ser Lys Arg Lys Asp Ser Arg Thr Val Tyr Gln Tyr Gln
945 950 955 960

Tyr Thr Asn Trp Ser Val Glu Gln Leu Pro Ala Glu Pro Lys Glu Leu
965 970 975

Ile Ser Met Ile Gln Val Val Lys Gln Lys Leu Pro Gln Lys Asn Ser
980 985 990

Ser Glu Gly Asn Lys His His Lys Ser Thr Pro Leu Leu Ile His Cys
995 1000 1005

Arg Asp Gly Ser Gln Gln Thr Gly Ile Phe Cys Ala Leu Leu Asn
1010 1015 1020

Leu Leu Glu Ser Ala Glu Thr Glu Glu Val Val Asp Ile Phe Gln
1025 1030 1035

Val Val Lys Ala Leu Arg Lys Ala Arg Pro Gly Met Val Ser Thr
1040 1045 1050

Phe Glu Gln Tyr Gln Phe Leu Tyr Asp Val Ile Ala Ser Thr Tyr
1055 1060 1065

Pro Ala Gln Asn Gly Gln Val Lys Lys Asn Asn His Gln Glu Asp
1070 1075 1080

Lys Ile Glu Phe Asp Asn Glu Val Asp Lys Val Lys Gln Asp Ala
1085 1090 1095

Asn Cys Val Asn Pro Leu Gly Ala Pro Glu Lys Leu Pro Glu Ala
1100 1105 1110

Lys Glu Gln Ala Glu Gly Ser Glu Pro Thr Ser Gly Thr Glu Gly
1115 1120 1125

Pro Glu His Ser Val Asn Gly Pro Ala Ser Pro Ala Leu Asn Gln
1130 1135 1140

Gly Ser
1145

<210> 83
<211> 1860
<212> DNA
<213> Homo sapiens

<400> 83
atgccagacc ccgcggcgca cctgcccttc ttctacggca gcatctcgcg tgccgaggcc 60

gaggagcacc tgaagctggc gggcatggcg gacgggctct tcctgctgcg ccagtgcctg 120

cgctcgctgg gcggctatgt gctgtcgctc gtgcacgatg tgcgcttcca ccactttccc 180

atcgagcgcc agctcaacgg cacctacgcc attgccggcg gcaaagcgca ctgtggaccg 240

gcagagctct gcgagttcta ctcgcgcgac cccgacgggc tgccctgcaa cctgcgcaag 300

ccgtgcaacc ggccgtcggg cctcgagccg cagccggggg tcttcgactg cctgcgagac 360

gccatggtgc gtgactacgt gcgccagacg tggaagctgg agggcgaggc cctggagcag 420

gccatcatca gccaggcccc gcaggtggag aagctcattg ctacgacggc ccacgagcgg 480

atgccctggt accacagcag cctgacgcgt gaggaggccg agcgcaaact ttactctggg 540

gcgcagaccg acggcaagtt cctgctgagg ccgcggaagg agcagggcac atacgccctg 600

tccctcatct atgggaagac ggtgtaccac tacctcatca gccaagacaa ggcgggcaag 660

tactgcattc ccgagggcac caagtttgac acgctctggc agctggtgga gtatctgaag 720

ctgaaggcgg acgggctcat ctactgcctg aaggaggcct gccccaacag cagtgccagc 780

aacgcctcag ggctgctgc tcccacactc ccagcccacc catccacgtt gactcatcct 840

cagagacgaa tcgacaccct caactcagat ggatacaccc ctgagccagc acgcataacg 900

tccccagaca aaccgcggcc gatgcccatg gacacgagcg tgtatgagag cccctacagc 960

gacccagagg agctcaagga caagaagctc ttcctgaagc gcgataacct cctcatagct 1020

gacattgaac ttggctgcgg caactttggc tcagtgcgcc agggcgtgta ccgcatgcgc 1080

aagaagcaga tcgacgtggc catcaaggtg ctgaagcagg gcacggagaa ggcagacacg 1140

gaagagatga tgcgcgaggc gcagatcatg caccagctgg acaaccccta catcgtgcgg 1200

ctcattggcg tctgccaggc cgaggccctc atgctggtca tggagatggc tgggggcggg 1260

ccgctgcaca agttcctggt cggcaagagg gaggagatcc ctgtgagcaa tgtggccgag 1320

ctgctgcacc aggtgtccat ggggatgaag tacctggagg agaagaactt tgtgcaccgt 1380

gacctggcgg cccgcaacgt cctgctggtt aaccggcact acgccaagat cagcgacttt 1440

ggcctctcca aagcactggg tgccgacgac agctactaca ctgcccgctc agcagggaag 1500

tggccgctca agtggtacgc acccgaatgc atcaacttcc gcaagttctc cagccgcagc 1560

gatgtctgga gctatggggt caccatgtgg gaggccttgt cctacggcca gaagccctac 1620

aagaagatga aagggccgga ggtcatggcc ttcatcgagc agggcaagcg gatggagtgc 1680

ccaccagagt gtccacccga actgtacgca ctcatgagtg actgctggat ctacaagtgg 1740

gaggatcgcc ccgacttcct gaccgtggag cagcgcatgc gagcctgtta ctacagcctg 1800

gccagcaagg tggaagggcc cccaggcagc acacagaagg ctgaggctgc ctgtgcctga 1860

<210> 84
<211> 1468
<212> DNA
<213> Homo sapiens

<400> 84
tgcatgctcc agggacggca cccttgtctg gggcaggtgc ttgtggggc tgaggctgcc 60

ttgctcccca cacccctgcc cctgacctgg gagtgtaccg ctgtgtgtgc ccagcacgca 120

taacgtcccc agacaaaccg cggccgatgc ccatggacac gagcgtgtat gagagcccct 180

acagcgaccc agaggagctc aaggacaaga agctcttcct gaagcgcgat aacctcctca 240

tagctgacat tgaacttggc tgcggcaact ttggctcagt gcgccagggc gtgtaccgca 300

tgcgcaagaa gcagatcgac gtggccatca aggtgctgaa gcagggcacg gagaaggcag 360

acacggaaga gatgatgcgc gaggcgcaga tcatgcacca gctggacaac ccctacatcg 420

tgcggctcat tggcgtctgc caggccgagg ccctcatgct ggtcatggag atggctgggg 480

gcgggccgct gcacaagttc ctggtcggca agagggagga gatccctgtg agcaatgtgg 540

ccgagctgct gcaccaggtg tccatgggga tgaagtacct ggaggagaag aactttgtgc 600

accgtgacct ggcggcccgc aacgtcctgc tggttaaccg gcactacgcc aagatcagcg 660

actttggcct ctccaaagca ctgggtgccg acgacagcta ctacactgcc cgctcagcag 720

ggaagtggcc gctcaagtgg tacgcacccg aatgcatcaa cttccgcaag ttctccagcc 780

gcagcgatgt ctggagctat ggggtcacca tgtgggaggc cttgtcctac ggccagaagc 840

cctacaagaa gatgaaaggg ccggaggtca tggccttcat cgagcagggc aagcggatgg 900

agtgcccacc agagtgtcca cccgaactgt acgcactcat gagtgactgc tggatctaca 960

agtgggagga tcgccccgac ttcctgaccg tggagcagcg catgcgagcc tgttactaca 1020

gcctggccag caaggtggaa gggcccccag gcagcacaca gaaggctgag gctgcctgtg 1080

cctgagctcc cgctgcccag gggagccctc cacgccggct cttccccacc ctcagcccca 1140

ccccaggtcc tgcagtctgg ctgagccctg cttggttgtc tccacacaca gctgggctgt 1200

ggtagggggt gtctcaggcc acaccggcct tgcattgcct gcctggcccc ctgtcctctc 1260

tggctgggga gcagggaggt ccgggagggt gcggctgtgc agcctgtcct gggctggtgg 1320

ctcccggagg gccctgagct gagggcattg cttacacgga tgccttcccc tgggccctga 1380

cattggagcc tgggcatcct caggtggtca ggcgtagatc accagaataa acccagcttc 1440

cctcttgaaa aaaaaaaaaa aaaaaaa 1468

<210> 85
<211> 619
<212> PRT
<213> Homo sapiens

<400> 85

```
Met Pro Asp Pro Ala Ala His Leu Pro Phe Phe Tyr Gly Ser Ile Ser
1               5                   10                  15

Arg Ala Glu Ala Glu Glu His Leu Lys Leu Ala Gly Met Ala Asp Gly
            20                  25                  30

Leu Phe Leu Leu Arg Gln Cys Leu Arg Ser Leu Gly Gly Tyr Val Leu
        35                  40                  45

Ser Leu Val His Asp Val Arg Phe His His Phe Pro Ile Glu Arg Gln
    50                  55                  60

Leu Asn Gly Thr Tyr Ala Ile Ala Gly Gly Lys Ala His Cys Gly Pro
65                  70                  75                  80

Ala Glu Leu Cys Glu Phe Tyr Ser Arg Asp Pro Asp Gly Leu Pro Cys
                85                  90                  95

Asn Leu Arg Lys Pro Cys Asn Arg Pro Ser Gly Leu Glu Pro Gln Pro
            100                 105                 110

Gly Val Phe Asp Cys Leu Arg Asp Ala Met Val Arg Asp Tyr Val Arg
        115                 120                 125

Gln Thr Trp Lys Leu Glu Gly Glu Ala Leu Glu Gln Ala Ile Ile Ser
    130                 135                 140

Gln Ala Pro Gln Val Glu Lys Leu Ile Ala Thr Thr Ala His Glu Arg
145                 150                 155                 160

Met Pro Trp Tyr His Ser Ser Leu Thr Arg Glu Glu Ala Glu Arg Lys
                165                 170                 175

Leu Tyr Ser Gly Ala Gln Thr Asp Gly Lys Phe Leu Leu Arg Pro Arg
            180                 185                 190

Lys Glu Gln Gly Thr Tyr Ala Leu Ser Leu Ile Tyr Gly Lys Thr Val
        195                 200                 205

Tyr His Tyr Leu Ile Ser Gln Asp Lys Ala Gly Lys Tyr Cys Ile Pro
    210                 215                 220
```

Glu Gly Thr Lys Phe Asp Thr Leu Trp Gln Leu Val Glu Tyr Leu Lys
225 230 235 240

Leu Lys Ala Asp Gly Leu Ile Tyr Cys Leu Lys Glu Ala Cys Pro Asn
245 250 255

Ser Ser Ala Ser Asn Ala Ser Gly Ala Ala Ala Pro Thr Leu Pro Ala
260 265 270

His Pro Ser Thr Leu Thr His Pro Gln Arg Arg Ile Asp Thr Leu Asn
275 280 285

Ser Asp Gly Tyr Thr Pro Glu Pro Ala Arg Ile Thr Ser Pro Asp Lys
290 295 300

Pro Arg Pro Met Pro Met Asp Thr Ser Val Tyr Glu Ser Pro Tyr Ser
305 310 315 320

Asp Pro Glu Glu Leu Lys Asp Lys Lys Leu Phe Leu Lys Arg Asp Asn
325 330 335

Leu Leu Ile Ala Asp Ile Glu Leu Gly Cys Gly Asn Phe Gly Ser Val
340 345 350

Arg Gln Gly Val Tyr Arg Met Arg Lys Lys Gln Ile Asp Val Ala Ile
355 360 365

Lys Val Leu Lys Gln Gly Thr Glu Lys Ala Asp Thr Glu Glu Met Met
370 375 380

Arg Glu Ala Gln Ile Met His Gln Leu Asp Asn Pro Tyr Ile Val Arg
385 390 395 400

Leu Ile Gly Val Cys Gln Ala Glu Ala Leu Met Leu Val Met Glu Met
405 410 415

Ala Gly Gly Gly Pro Leu His Lys Phe Leu Val Gly Lys Arg Glu Glu
420 425 430

Ile Pro Val Ser Asn Val Ala Glu Leu Leu His Gln Val Ser Met Gly
435 440 445

Met Lys Tyr Leu Glu Glu Lys Asn Phe Val His Arg Asp Leu Ala Ala
450 455 460

Arg Asn Val Leu Leu Val Asn Arg His Tyr Ala Lys Ile Ser Asp Phe
465 470 475 480

```
Gly Leu Ser Lys Ala Leu Gly Ala Asp Asp Ser Tyr Tyr Thr Ala Arg
                485                 490                 495

Ser Ala Gly Lys Trp Pro Leu Lys Trp Tyr Ala Pro Glu Cys Ile Asn
            500                 505                 510

Phe Arg Lys Phe Ser Ser Arg Ser Asp Val Trp Ser Tyr Gly Val Thr
        515                 520                 525

Met Trp Glu Ala Leu Ser Tyr Gly Gln Lys Pro Tyr Lys Lys Met Lys
    530                 535                 540

Gly Pro Glu Val Met Ala Phe Ile Glu Gln Gly Lys Arg Met Glu Cys
545                 550                 555                 560

Pro Pro Glu Cys Pro Pro Glu Leu Tyr Ala Leu Met Ser Asp Cys Trp
                565                 570                 575

Ile Tyr Lys Trp Glu Asp Arg Pro Asp Phe Leu Thr Val Glu Gln Arg
            580                 585                 590

Met Arg Ala Cys Tyr Tyr Ser Leu Ala Ser Lys Val Glu Gly Pro Pro
        595                 600                 605

Gly Ser Thr Gln Lys Ala Glu Ala Ala Cys Ala
    610                 615


<210>  86
<211>  312
<212>  PRT
<213>  Homo sapiens

<400>  86

Met Pro Met Asp Thr Ser Val Tyr Glu Ser Pro Tyr Ser Asp Pro Glu
1               5                   10                  15

Glu Leu Lys Asp Lys Lys Leu Phe Leu Lys Arg Asp Asn Leu Leu Ile
            20                  25                  30

Ala Asp Ile Glu Leu Gly Cys Gly Asn Phe Gly Ser Val Arg Gln Gly
        35                  40                  45

Val Tyr Arg Met Arg Lys Lys Gln Ile Asp Val Ala Ile Lys Val Leu
    50                  55                  60

Lys Gln Gly Thr Glu Lys Ala Asp Thr Glu Glu Met Met Arg Glu Ala
65                  70                  75                  80
```

```
Gln Ile Met His Gln Leu Asp Asn Pro Tyr Ile Val Arg Leu Ile Gly
                85                  90                  95

Val Cys Gln Ala Glu Ala Leu Met Leu Val Met Glu Met Ala Gly Gly
            100                 105                 110

Gly Pro Leu His Lys Phe Leu Val Gly Lys Arg Glu Glu Ile Pro Val
        115                 120                 125

Ser Asn Val Ala Glu Leu Leu His Gln Val Ser Met Gly Met Lys Tyr
    130                 135                 140

Leu Glu Glu Lys Asn Phe Val His Arg Asp Leu Ala Ala Arg Asn Val
145                 150                 155                 160

Leu Leu Val Asn Arg His Tyr Ala Lys Ile Ser Asp Phe Gly Leu Ser
                165                 170                 175

Lys Ala Leu Gly Ala Asp Asp Ser Tyr Tyr Thr Ala Arg Ser Ala Gly
            180                 185                 190

Lys Trp Pro Leu Lys Trp Tyr Ala Pro Glu Cys Ile Asn Phe Arg Lys
        195                 200                 205

Phe Ser Ser Arg Ser Asp Val Trp Ser Tyr Gly Val Thr Met Trp Glu
    210                 215                 220

Ala Leu Ser Tyr Gly Gln Lys Pro Tyr Lys Lys Met Lys Gly Pro Glu
225                 230                 235                 240

Val Met Ala Phe Ile Glu Gln Gly Lys Arg Met Glu Cys Pro Pro Glu
                245                 250                 255

Cys Pro Pro Glu Leu Tyr Ala Leu Met Ser Asp Cys Trp Ile Tyr Lys
            260                 265                 270

Trp Glu Asp Arg Pro Asp Phe Leu Thr Val Glu Gln Arg Met Arg Ala
        275                 280                 285

Cys Tyr Tyr Ser Leu Ala Ser Lys Val Glu Gly Pro Pro Gly Ser Thr
    290                 295                 300

Gln Lys Ala Glu Ala Ala Cys Ala
305                 310
```

## Description

FIELD OF THE INVENTION

The invention relates to a method of predicting a response of a prostate cancer subject to radiotherapy. Moreover, the invention relates to a diagnostic kit, to a use of the kit in a method of predicting a response of a prostate cancer subject to radiotherapy, to a use of a gene expression profile for each of one or more T-Cell receptor signaling genes in radiotherapy prediction for a prostate cancer subject, and to a corresponding computer program product.

BACKGROUND OF THE INVENTION

Cancer is a class of diseases in which a group of cells displays uncontrolled growth, invasion and sometimes metastasis. These three malignant properties of cancers differentiate them from benign tumours, which are self-limited and do not invade or metastasize. Prostate Cancer (PCa) is the second most commonly-occurring non-skin malignancy in men, with an estimated 1.3 million new cases diagnosed and 360,000 deaths world-wide in 2018 (see Bray F. et al., "Global cancer statistics 2018: GLOBOCAN estimates of incidence and mortality worldwide for 36 cancers in 185 countries", CA Cancer J Clin, Vol. 68, No. 6, pages 394-424, 2018). In the US, about 90% of the new cases concern localized cancer, meaning that metastases have not yet been formed (see ACS (American Cancer Society), "Cancer Facts & Figures 2010", 2010).

For the treatment of primary localized prostate cancer, several radical therapies are available, of which surgery (radical prostatectomy, RP) and radiation therapy (RT) are most commonly used. RT is administered via an external beam or via the implantation of radioactive seeds into the prostate (brachytherapy) or a combination of both. It is especially preferable for patients who are not eligible for surgery or have been diagnosed with a tumour in an advanced localized or regional stage. Radical RT is provided to up to 50% of patients diagnosed with localized prostate cancer in the US (see ACS, 2010, ibid).

After treatment, prostate cancer antigen (PSA) levels in the blood are measured for disease monitoring. An increase of the blood PSA level provides a biochemical surrogate measure for cancer recurrence or progression. However, the variation in reported biochemical progression-free survival (bPFS) is large (see Grimm P. et al., "Comparative analysis of prostate-specific antigen free survival outcomes for patients with low, intermediate and high risk prostate cancer treatment by radical therapy. Results from the Prostate Cancer Results Study Group", BJU Int, Suppl. 1, pages 22-29, 2012). For many patients, the bPFS at 5 or even 10 years after radical RT may lie above 90%. Unfortunately, for the group of patients at medium and especially higher risk of recurrence, the bPFS can drop to around 40% at 5 years, depending on the type of RT used (see Grimm P. et al., 2012, ibid).

A large number of the patients with primary localized prostate cancer that are not treated with RT will undergo RP (see ACS, 2010, ibid). After RP, an average of 60% of patients in the highest risk group experience biochemical recurrence after 5 and 10 years (see Grimm P. et al., 2012, ibid). In case of biochemical progression after RP, one of the main challenges is the uncertainty whether this is due to recurring localized disease, one or more metastases or even an indolent disease that will not lead to clinical disease progression (see Dal Pra A. et al., "Contemporary role of postoperative radiotherapy for prostate cancer", Transl Androl Urol, Vo. 7, No. 3, pages 399-413, 2018, and Herrera F.G. and Berthold D.R., "Radiation therapy after radical prostatectomy: Implications for clinicians", Front Oncol, Vol. 6, No. 117, 2016). RT to eradicate remaining cancer cells in the prostate bed is one of the main treatment options to salvage survival after a PSA increase following RP. The effectiveness of salvage radiotherapy (SRT) results in 5-year bPFS for 18% to 90% of patients, depending on multiple factors (see Herrera F.G. and Berthold D.R., 2016, ibid, and Pisansky T.M. et al., "Salvage radiation therapy dose response for biochemical failure of prostate cancer after prostatectomy - A multi-institutional observational study", Int J Radiat Oncol Biol Phys, Vol. 96, No. 5, pages 1046-1053, 2016).

It is clear that for certain patient groups, radical or salvage RT is not effective. Their situation is even worsened by the serious side effects that RT can cause, such as bowel inflammation and dysfunction, urinary incontinence and erectile dysfunction (see Resnick M.J. et al., "Long-term functional outcomes after treatment for localized prostate cancer", N Engl J Med, Vol. 368, No. 5, pages 436-445, 2013, and Hegarty S.E. et al., "Radiation therapy after radical prostatectomy for prostate cancer: Evaluation of complications and influence of radiation timing on outcomes in a large, population-based cohort", PLoS One, Vol. 10, No. 2, 2015). In addition, the median cost of one course of RT based on Medicare reimbursement is $ 18,000, with a wide variation up to about $ 40,000 (see Paravati A.J. et al., "Variation in the cost of radiation therapy among medicare patients with cancer", J Oncol Pract, Vol. 11, No. 5, pages 403-409, 2015). These figures do not include the considerable longitudinal costs of follow-up care after radical and salvage RT.

An improved prediction of effectiveness of RT for each patient, be it in the radical or the salvage setting, would improve therapy selection and potentially survival. This can be achieved by 1) optimizing RT for those patients where RT is predicted to be effective (e.g., by dose escalation or a different starting time) and 2) guiding patients where RT is predicted not to be effective to an alternative, potentially more effective form of treatment. Further, this would reduce suffering for those patients who would be spared ineffective therapy and would reduce costs spent on ineffective therapies.

Numerous investigations have been conducted into measures for response prediction of radical RT (see Hall W.A. et al., "Biomarkers of outcome in patients with localized prostate cancer treated with radiotherapy", Semin Radiat Oncol, Vol. 27, pages 11-20, 2016, and Raymond E. et al., "An appraisal of analytical tools used in predicting clinical outcomes following radiation therapy treatment of men with prostate cancer: A systematic review", Radiat Oncol, Vol. 12, No. 1, page 56, 2017) and SRT (see Herrera F.G. and Berthold D.R., 2016, ibid). Many of these measures depend on the concentration of the blood-based biomarker PSA. Metrics investigated for prediction of response before start of RT (radical as well as salvage) include the absolute value of the PSA concentration, its absolute value relative to the prostate volume, the absolute increase over a certain time and the doubling time. Other frequently considered factors are the Gleason score and the clinical tumour stage. For the SRT setting, additional factors are relevant, e.g., surgical margin status, time to recurrence after RP, pre-/peri-surgical PSA values and clinico-pathological parameters.

Although these clinical variables provide limited improvements in patient stratification in various risk groups, there is a need for better predictive tools.

A wide range of biomarker candidates in tissue and bodily fluids has been investigated, but validation is often limited and generally demonstrates prognostic information and not a predictive (therapy-specific) value (see Hall W.A. et al., 2016, ibid). A small number of gene expression panels is currently being validated by commercial organizations. One or a few of these may show predictive value for RT in future (see Dal Pra A. et al., 2018, ibid).

In conclusion, a strong need for better prediction of response to RT remains, for primary prostate cancer as well as for the post-surgery setting.

## SUMMARY OF THE INVENTION

It is an objective of the invention to provide a method of predicting a response of a prostate cancer subject to radiotherapy, which allows to make better treatment decisions. It is a further objective of the invention to provide a diagnostic kit, a use of the kit in a method of predicting a response of a prostate cancer subject to radiotherapy, a use of a gene expression profile for each of one or more T-Cell receptor signaling genes in radiotherapy prediction for a prostate cancer subject, and a corresponding computer program product.

In a first aspect of the present invention, a method of predicting a response of a prostate cancer subject to radiotherapy is presented, comprising:

- determining or receiving the result of a determination of a gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said gene expression profile(s) being determined in a biological sample obtained from the subject,
- determining the prediction of the radiotherapy response based on the gene expression profile(s) for the one or more T-Cell receptor signaling genes, and
- optionally, providing the prediction or a therapy recommendation based on the prediction to a medical caregiver or the subject.

In recent years, the importance of the immune system in cancer inhibition as well as in cancer initiation, promotion and metastasis has become very evident (see Mantovani A. et al., "Cancer-related inflammation", Nature, Vol. 454, No. 7203, pages 436-444, 2008, and Giraldo N.A. et al., "The clinical role of the TME in solid cancer", Br J Cancer, Vol. 120, No. 1, pages 45-53, 2019). The immune cells and the molecules they secrete form a crucial part of the tumour microenvironment and most immune cells can infiltrate the tumour tissue. The immune system and the tumour affect and shape one another. Thus, anti-tumour immunity can prevent tumour formation while an inflammatory tumour environment may promote cancer initiation and proliferation. At the same time, tumour cells that may have originated in an immune system-independent manner will shape the immune microenvironment by recruiting immune cells and can have a pro-inflammatory effect while also suppressing anti-cancer immunity.

Some of the immune cells in the tumour microenvironment will have either a general tumour-promoting or a general tumour-inhibiting effect, while other immune cells exhibit plasticity and show both tumour-promoting and tumour-inhibiting potential. Thus, the overall immune microenvironment of the tumour is a mixture of the various immune cells present, the cytokines they produce and their interactions with tumour cells and with other cells in the tumour microenvironment (see Giraldo N.A. et al., 2019, ibid).

The principles described above with regard to the role of the immune system in cancer in general also apply to prostate cancer. Chronic inflammation has been linked to the formation of benign as well as malignant prostate tissue (see Hall W.A. et al., 2016, ibid) and most prostate cancer tissue samples show immune cell infiltrates. The presence of specific immune cells with a pro-tumour effect has been correlated with worse prognosis, while tumours in which natural killer cells were more activated showed better response to therapy and longer recurrence-free periods (see Shiao S.L. et al., "Regulation of prostate cancer progression by tumor microenvironment", Cancer Lett, Vol. 380, No. 1, pages 340-348, 2016).

While a therapy will be influenced by the immune components of the tumour microenvironment, RT itself extensively affects the make-up of these components (see Barker H.E. et al., "The tumor microenvironment after radiotherapy: Mechanisms of resistance or recurrence", Nat Rev Cancer, Vol. 15, No. 7, pages 409-425, 2015). Because suppressive cell types are comparably radiation-insensitive, their relative numbers will increase. Counteractively, the inflicted radiation damage activates cell survival pathways and stimulates the immune system, triggering inflammatory responses and immune cell recruitment. Whether the net effect will be tumour-promoting or tumour-suppressing is as yet uncertain, but its potential for enhancement of cancer immunotherapies is being investigated. The present invention is based on the idea that, since the status of the immune system and of the immune microenvironment have an impact on therapy effectiveness, the ability to identify markers predictive for this effect might help to be better able to predict overall RT response.

An immune response against pathogens can be elicited at different levels: there are physical barriers, such as the skin, to keep invaders out. If breached, innate immunity comes into play; a first and fast non-specific response. If this is not sufficient, the adaptive immune response is elicited. This is much more specific and needs time to develop when encountering a pathogen for the first time. Lymphocytes are activated by interacting with activated antigen presenting cells from the innate immune system, and are also responsible for maintenance of memory for faster responses upon next encounters with the same pathogen.

As lymphocytes are highly specific and effective when activated, they are subject to negative selection for their ability to recognize self, a process known as central tolerance. As not all self-antigens are expressed at selection sites, peripheral tolerance mechanisms evolved as well, such as ligation of the TCR in absence of co-stimulation, expression of inhibitory co-receptors, and suppression by Tregs. A disturbed balance between activation and suppression may lead to autoimmune disorders, or immune deficiencies and cancer, respectively.

T-cell activation can have different functional consequences, depending on the location the type of T-cell involved. CD8+ T-cells differentiate into cytotoxic effector cells, whereas CD4+ T-cells can differentiate into Th1 (IFNγ secretion and promotion of cell mediated immunity) or Th2 (IL4/5/13 secretion and promotion of B cell and humoral immunity). Differentiation towards other, more recently identified T-cell subsets is also possible, for example the Tregs, which have a suppressive effect on immune activation (see Mosenden R. and Tasken K., "Cyclic AMP-mediated immune regulation - Overview of mechanisms of action in T-cells", Cell Signal, Vol. 23, No. 6, pages 1009-1016, 2011, in particular, Fig. 4, which T-cell activation and its modulation by PKA, and Tasken K. and Ruppelt A., "Negative regulation of T-cell receptor activation by the cAMP-PKA-Csk signaling pathway in T-cell lipid rafts", Front Biosci, Vol. 11, pages 2929-2939, 2006).

Both PKA and PDE4 regulated signaling intersect with TCR induced T-cell activation to fine-tune its regulation, with opposing effects (see Abrahamsen H. et al., "TCR- and CD28-mediated recruitment of phosphodiesterase 4 to lipid rafts potentiates TCR signaling", J Immunol, Vol. 173, pages 4847-4848, 2004, in particular, Fig. 6, which shows opposing effects of PKA and PDE4 on TCR activation). The molecule that connects these effectors is cyclic AMP (cAMP), an intracellular second messenger of extracellular ligand action. In T-cells, it mediates effects of prostaglandins, adenosine, histamine, beta-adrenergic agonists, neuropeptide hormones and beta-endorphin. Binding of these extracellular molecules to GPCRs leads to their conformational change, release of stimulatory subunits and subsequent activation of adenylate cyclases (AC), which hydrolyze ATP to cAMP (see Fig. 6 of Abrahamsen H. et al., 2004, ibid). Although not the only one, PKA is the principal effector of cAMP signaling (see Mosenden R. and Tasken K., 2011, ibid, and Tasken K. and Ruppelt A., 2006, ibid). At a functional level, increased levels of cAMP lead to reduced IFNγ and IL-2 production in T-cells (see Abrahamsen H. et al., 2004, ibid). Aside from interfering with TCR activation, PKA has many more effector (see Fig. 15 of Torheim E.A., "Immunity Leashed - Mechanisms of Regulation in the Human Immune System", Thesis for the degree of Philosophiae Doctor (PhD), The Biotechnology Centre of Ola, University of Oslo, Norway, 2009).

In naive T-cells, hyperphosphorylated PAG targets Csk to lipid rafts. Via the Ezrin-EBP50-PAG scaffold complex PKA is targeted to Csk. Through specific phosphorylation by PKA, Csk can negatively regulate Lck and Fyn to dampen their activity and downregulate T-cell activation (see Fig. 6 of Abrahamsen H. et al., 2004, ibid). Upon TCR activation, PAG is dephosphorylated and Csk is released from the rafts. Dissociation of Csk is needed for T-cell activation to proceed. Within the same time course, a Csk-G3BP complex is formed and seems to sequester Csk outside lipid rafts (see Mosenden R. and Tasken K., 2011, ibid, and Tasken K. and Ruppelt A., 2006, ibid).

In contrast, combined TCR and CD28 stimulation mediates recruitment of the cyclic nucleotide phosphodiesterase PDE4 to lipid rafts, which enhances cAMP degradation (see Fig. 6 of Abrahamsen H. et al., 2004, ibid). As such, TCR induced production of cAMP is countered, and the T-cell immune response potentiated. Upon TCR stimulation alone, PDE4 recruitment may be too low to fully reduce the cAMP levels and therefore maximal T-cell activation cannot occur (see Abrahamsen H. et al., 2004, ibid).

Thus, by active suppression of proximal TCR signaling, signaling via cAMP-PKA-Csk is thought to set the threshold for T-cell activation. Recruitment of PDEs can counter this suppression. Tissue or cell-type specific regulation is accomplished through expression of multiple isoforms of AC, PKA, and PDEs. As mentioned above, the balance between activation and suppression needs to be tightly regulated to prevent development of autoimmune disorders, immune deficiencies and cancer.

The identified T-Cell receptor signaling genes CD2, CD247, CD28, CD3E, CD3G, CD4, CSK, EZR, FYN, LAT, LCK, PAG1, PDE4D, PRKACA, PRKACB, PTPRC, and ZAP70 were identified as follows: A group of 538 prostate cancer patients were treated with RP and the prostate cancer tissue was stored together with clinical (e.g., pathological Gleason grade group (pGGG), pathology state (pT stage)) as well as relevant outcome parameters (e.g., biochemical recurrence (BCR), metastatic recurrence, prostate cancer specific death (PCa death), salvage radiation treatment (SRT), salvage androgen deprivation treatment (SADT), chemotherapy (CTX)). For each of these patients, a PDE4D7 score was calculated and categorized into four PDE4D7 score classes as described in Alves de Inda M. et al., "Validation of Cyclic Adenosine Monophosphate Phosphodiesterase-4D7 for its Independent Contribution to Risk Stratification in a Prostate Cancer Patient Cohort with Longitudinal Biological Outcomes", Eur Urol Focus, Vol. 4, No. 3, pages 376-384, 2018. PDE4D7 score class 1 represents patient samples with lowest expression levels of PDE4D7, whereas PDE4D7 score class 4 represents patient samples with highest levels of PDE4D7 expression. RNASeq expression data (TPM - Transcripts Per Million) of the 538 prostate cancer subjects was then investigated for differential gene expression between the PDE4D7 score classes 1 and 4. In particular, it was determined for around 20,000 protein coding transcripts whether the mean expression level of the PDE4D7 score class 1 patients was more than twice as high as the mean expression level of the PDE4D7 score class 4 patients. This analysis resulted in 637 genes with a ratio PDE4D7 score class 1 / PDE4D7 score class 4 of > 2 with a minimum mean expression of 1 TPM in each of the four PDE4D7 score classes. These 637 genes were then further subjected to molecular pathway analysis (www.david.ncifcrf.gov), which resulted in a range of enriched annotation clusters. The annotation cluster #6 demonstrated enrichment (enrichment score: 5.9) in 17 genes with a function in primary immune deficiency and activation of T-Cell receptor signaling. A further heat map analysis confirmed that these T-Cell receptor signaling genes were generally higher expressed in samples from patients in PDE4D7 score class 1 than from patients in PDE4D7 score class 4.

The term "CD2" refers to the Cluster Of Differentiation 2 gene (Ensembl: ENSG00000116824), for example, to the sequence as defined in NCBI Reference Sequence NM_001767, specifically, to the nucleotide sequence as set forth in SEQ ID NO:1, which corresponds to the sequence of the above indicated NCBI Reference Sequence of the CD2 transcript, and also relates to the corresponding amino acid sequence for example as set forth in SEQ ID NO:2, which corresponds to the protein sequence defined in NCBI Protein Accession Reference Sequence NP_001758 encoding the CD2 polypeptide.

The term "CD2" also comprises nucleotide sequences showing a high degree of homology to CD2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:1 or amino acid sequences being at least 75%, 80%, 85%, 90%, 91%, 92%, 93%, 94%, 95%, 96%, 97%, 98% or 99% identical to the sequence as set forth in SEQ ID NO:2 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:2 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 1.

The term "CD247" refers to the Cluster Of Differentiation 247 gene (Ensembl: ENSG00000198821), for example, to the sequence as defined in NCBI Reference Sequence NM_000734 or in NCBI Reference Sequence NM_198053, specifically, to the nucleotide sequence as set forth in SEQ ID NO:3 or in SEQ ID NO:4, which correspond to the sequences of the above indicated NCBI Reference Sequences of the CD247 transcript, and also relates to the corresponding amino acid sequence for example as set forth in SEQ ID NO:5 or in SEQ ID NO:6, which correspond to the protein sequences defined in NCBI Protein Accession Reference Sequence NP_000725 and in NCBI Protein Accession Reference Sequence NP_932170 encoding the CD247 polypeptide.

The term "CD247" also comprises nucleotide sequences showing a high degree of homology to CD247, e.g., nucleic acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or amino acid sequences being at least 75%, 80%, 85%, 90%, 91%, 92%, 93%, 94%, 95%, 96%, 97%, 98% or 99% identical to the sequence as set forth in SEQ ID NO:5 or in SEQ ID NO:6 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:5 or in SEQ ID NO:6 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4.

The term "CD28" refers to the Cluster Of Differentiation 28 gene (Ensembl: ENSG00000178562), for example, to the sequence as defined in NCBI Reference Sequence NM_006139 or in NCBI Reference Sequence NM_001243078, specifically, to the nucleotide sequence as set forth in SEQ ID NO:7 or in SEQ ID NO:8, which correspond to the sequences of the above indicated NCBI Reference Sequences of the CD28 transcript, and also relates to the corresponding amino acid sequence for example as set forth in SEQ ID NO:9 or in SEQ ID NO: 10, which correspond to the protein sequences defined in NCBI Protein Accession Reference Sequence NP_006130 and in NCBI Protein Accession Reference Sequence NP_001230007 encoding the CD28 polypeptide.

The term "CD28" also comprises nucleotide sequences showing a high degree of homology to CD28, e.g., nucleic acid sequences being at least 75%, 80%, 85%, 90%, 91%, 92%, 93%, 94%, 95%, 96%, 97%, 98% or 99% identical to the sequence as set forth in SEQ ID NO:7 or in SEQ ID NO:8 or amino acid sequences being at least 75%, 80%, 85%, 90%, 91%, 92%, 93%, 94%, 95%, 96%, 97%, 98% or 99% identical to the sequence as set forth in SEQ ID NO:9 or in SEQ ID NO:10 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:9 or in SEQ ID NO:10 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:7 or in SEQ ID NO:8.

The term "CD3E" refers to the Cluster Of Differentiation 3E gene (Ensembl: ENSG00000198851), for example, to the sequence as defined in NCBI Reference Sequence NM_000733, specifically, to the nucleotide sequence as set forth in SEQ ID NO:11, which corresponds to the sequence of the above indicated NCBI Reference Sequence of the CD3E transcript, and also relates to the corresponding amino acid sequence for example as set forth in SEQ ID NO:12, which corresponds to the protein sequence defined in NCBI Protein Accession Reference Sequence NP_000724 encoding the CD3E polypeptide.

The term "CD3E" also comprises nucleotide sequences showing a high degree of homology to CD3E, e.g., nucleic acid sequences being at least 75%, 80%, 85%, 90%, 91%, 92%, 93%, 94%, 95%, 96%, 97%, 98% or 99% identical to the sequence as set forth in SEQ ID NO:11 or amino acid sequences being at least 75%, 80%, 85%, 90%, 91%, 92%, 93%, 94%, 95%, 96%, 97%, 98% or 99% identical to the sequence as set forth in SEQ ID NO:12 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:12 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 11.

The term "CD3G" refers to the Cluster Of Differentiation 3G gene (Ensembl: ENSG00000160654), for example, to the sequence as defined in NCBI Reference Sequence NM_000073, specifically, to the nucleotide sequence as set forth in SEQ ID NO:13, which corresponds to the sequence of the above indicated NCBI Reference Sequence of the CD3G transcript, and also relates to the corresponding amino acid sequence for example as set forth in SEQ ID NO:14, which corresponds to the protein sequence defined in NCBI Protein Accession Reference Sequence NP_000064 encoding the CD3G polypeptide.

The term "CD3G" also comprises nucleotide sequences showing a high degree of homology to CD3G, e.g., nucleic acid sequences being at least 75%, 80%, 85%, 90%, 91%, 92%, 93%, 94%, 95%, 96%, 97%, 98% or 99% identical to the sequence as set forth in SEQ ID NO:13 or amino acid sequences being at least 75%, 80%, 85%, 90%, 91%, 92%, 93%, 94%, 95%, 96%, 97%, 98% or 99% identical to the sequence as set forth in SEQ ID NO: 14 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 14 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 13.

The term "CD4" refers to the Cluster Of Differentiation 4 gene (Ensembl: ENSG00000010610), for example, to the sequence as defined in NCBI Reference Sequence NM_000616, specifically, to the nucleotide sequence as set forth in SEQ ID NO: 15, which corresponds to the sequence of the above indicated NCBI Reference Sequence of the CD4 transcript, and also relates to the corresponding amino acid sequence for example as set forth in SEQ ID NO:16, which corresponds to the protein sequence defined in NCBI Protein Accession Reference Sequence NP_000607 encoding the CD4 polypeptide.

The term "CD4" also comprises nucleotide sequences showing a high degree of homology to CD4, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 15 or amino acid sequences being at least 75%, 80%, 85%, 90%, 91%, 92%, 93%, 94%, 95%, 96%, 97%, 98% or 99% identical to the sequence as set forth in SEQ ID NO:16 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 16 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:15.

The term "CSK" refers to the C-Terminal Src Kinase gene (Ensembl: ENSG00000103653), for example, to the sequence as defined in NCBI Reference Sequence NM_004383, specifically, to the nucleotide sequence as set forth in SEQ ID NO:17, which corresponds to the sequence of the above indicated NCBI Reference Sequence of the CSK transcript, and also relates to the corresponding amino acid sequence for example as set forth in SEQ ID NO: 18, which corresponds to the protein sequence defined in NCBI Protein Accession Reference Sequence NP_004374 encoding the CSK polypeptide.

The term "CSK" also comprises nucleotide sequences showing a high degree of homology to CSK, e.g., nucleic acid sequences being at least 75%, 80%, 85%, 90%, 91%, 92%, 93%, 94%, 95%, 96%, 97%, 98% or 99% identical to the sequence as set forth in SEQ ID NO:17 or amino acid sequences being at least 75%, 80%, 85%, 90%, 91%, 92%, 93%, 94%, 95%, 96%, 97%, 98% or 99% identical to the sequence as set forth in SEQ ID NO: 18 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 18 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 17.

The term "EZR" refers to the Ezrin gene (Ensembl: ENSG00000092820), for example, to the sequence as defined in NCBI Reference Sequence NM_003379, specifically, to the nucleotide sequence as set forth in SEQ ID NO: 19, which corresponds to the sequence of the above indicated NCBI Reference Sequence of the EZR transcript, and also relates to the corresponding amino acid sequence for example as set forth in SEQ ID NO:20, which corresponds to the protein sequence defined in NCBI Protein Accession Reference Sequence NP_003370 encoding the EZR polypeptide.

The term "EZR" also comprises nucleotide sequences showing a high degree of homology to EZR, e.g., nucleic acid sequences being at least 75%, 80%, 85%, 90%, 91%, 92%, 93%, 94%, 95%, 96%, 97%, 98% or 99% identical to the sequence as set forth in SEQ ID NO:19 or amino acid sequences being at least 75%, 80%, 85%, 90%, 91%, 92%, 93%, 94%, 95%, 96%, 97%, 98% or 99% identical to the sequence as set forth in SEQ ID NO:20 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:20 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 19.

The term "FYN" refers to the FYN Proto-Oncogene gene (Ensembl: ENSG00000010810), for example, to the sequence as defined in NCBI Reference Sequence NM_002037 or in NCBI Reference Sequence NM_153047 or in NCBI Reference Sequence NM_153048, specifically, to the nucleotide sequence as set forth in SEQ ID NO:21 or in SEQ ID NO:22 or in SEQ ID NO:23, which correspond to the sequences of the above indicated NCBI Reference Sequences of the FYN transcript, and also relates to the corresponding amino acid sequence for example as set forth in SEQ ID NO:24 or in SEQ ID NO:25 or in SEQ ID NO:26, which correspond to the protein sequences defined in NCBI Protein Accession Reference Sequence NP_002028 and in NCBI Protein Accession Reference Sequence NP_694592 and in NCBI Protein Accession Reference Sequence XP_005266949 encoding the FYN polypeptide.

The term "FYN" also comprises nucleotide sequences showing a high degree of homology to FYN, e.g., nucleic acid sequences being at least 75%, 80%, 85%, 90%, 91%, 92%, 93%, 94%, 95%, 96%, 97%, 98% or 99% identical to the sequence as set forth in SEQ ID NO:21 or in SEQ ID NO:22 or in SEQ ID NO:23 or amino acid sequences being at least 75%, 80%, 85%, 90%, 91%, 92%, 93%, 94%, 95%, 96%, 97%, 98% or 99% identical to the sequence as set forth in SEQ ID NO:24 or in SEQ ID NO:25 or in SEQ ID NO:26 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:24 or in SEQ ID NO:25 or in SEQ ID NO:26 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:21 or in SEQ ID NO:22 or in SEQ ID NO:23.

The term "LAT" refers to the Linker For Activation Of T-Cells gene (Ensembl: ENSG00000213658), for example, to the sequence as defined in NCBI Reference Sequence NM_001014987 or in NCBI Reference Sequence NM_014387, specifically, to the nucleotide sequence as set forth in SEQ ID NO:27 or in SEQ ID NO:28, which correspond to the sequences of the above indicated NCBI Reference Sequences of the LAT transcript, and also relates to the corresponding amino acid sequence for example as set forth in SEQ ID NO:29 or in SEQ ID NO:30, which corresponds to the protein sequences defined in NCBI Protein Accession Reference Sequence NP_001014987 and in NCBI Protein Accession Reference Sequence NP_055202 encoding the LAT polypeptide.

The term "LAT" also comprises nucleotide sequences showing a high degree of homology to LAT, e.g., nucleic acid sequences being at least 75%, 80%, 85%, 90%, 91%, 92%, 93%, 94%, 95%, 96%, 97%, 98% or 99% identical to the sequence as set forth in SEQ ID NO:27 or in SEQ ID NO:28 or amino acid sequences being at least 75%, 80%, 85%, 90%, 91%, 92%, 93%, 94%, 95%, 96%, 97%, 98% or 99% identical to the sequence as set forth in SEQ ID NO:29 or in SEQ ID NO:30 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:29 or in SEQ ID NO:30 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:27 or in SEQ ID NO:28.

The term "LCK" refers to the LCK Proto-Oncogene gene (Ensembl: ENSG00000182866), for example, to the sequence as defined in NCBI Reference Sequence NM_005356, specifically, to the nucleotide sequence as set forth in SEQ ID NO:31, which corresponds to the sequence of the above indicated NCBI Reference Sequence of the LCK transcript, and also relates to the corresponding amino acid sequence for example as set forth in SEQ ID NO:32, which corresponds to the protein sequence defined in NCBI Protein Accession Reference Sequence NP_005347 encoding the LCK polypeptide.

The term "LCK" also comprises nucleotide sequences showing a high degree of homology to LCK, e.g., nucleic acid sequences being at least 75%, 80%, 85%, 90%, 91%, 92%, 93%, 94%, 95%, 96%, 97%, 98% or 99% identical to the sequence as set forth in SEQ ID NO:31 or amino acid sequences being at least 75%, 80%, 85%, 90%, 91%, 92%, 93%, 94%, 95%, 96%, 97%, 98% or 99% identical to the sequence as set forth in SEQ ID NO:32 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:32 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:31.

The term "PAG1" refers to the Phosphoprotein Membrane Anchor With Glycosphingolipid Microdomains 1 gene (Ensembl: ENSG00000076641), for example, to the sequence as defined in NCBI Reference Sequence NM_018440, specifically, to the nucleotide sequence as set forth in SEQ ID NO:33, which corresponds to the sequence of the above indicated NCBI Reference Sequence of the PAG1 transcript, and also relates to the corresponding amino acid sequence for example as set forth in SEQ ID NO:34, which corresponds to the protein sequence defined in NCBI Protein Accession Reference Sequence NP_060910 encoding the PAG1 polypeptide.

The term "PAG1" also comprises nucleotide sequences showing a high degree of homology to PAG1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:33 or amino acid sequences being at least 75%, 80%, 85%, 90%, 91%, 92%, 93%, 94%, 95%, 96%, 97%, 98% or 99% identical to the sequence as set forth in SEQ ID NO:34 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:34 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:33.

The term "PDE4D" refers to the Phosphodiesterase 4D gene (Ensembl: ENSG00000113448), for example, to the sequence as defined in NCBI Reference Sequence NM_001104631 or in NCBI Reference Sequence NM_001349242 or in NCBI Reference Sequence NM_001197218 or in NCBI Reference Sequence NM_006203 or in NCBI Reference Sequence NM_001197221 or in NCBI Reference Sequence NM_001197220 or in NCBI Reference Sequence NM_001197223 or in NCBI Reference Sequence NM_001165899 or in NCBI Reference Sequence NM_001197219, specifically, to the nucleotide sequences as set forth in SEQ ID NO:35 or in SEQ ID NO:36 or in SEQ ID NO:37 or in SEQ ID NO:38 or in SEQ ID NO:39 or in SEQ ID NO:40 or in SEQ ID NO:41 or in SEQ ID NO:42 or in SEQ ID NO:43, which correspond to the sequences of the above indicated NCBI Reference Sequences of the PDE4D transcript, and also relates to the corresponding amino acid sequence for example as set forth in SEQ ID NO:44 or in SEQ ID NO:45 or in SEQ ID NO:46 or in SEQ ID NO:47 or in SEQ ID NO:48 or in SEQ ID NO:49 or in SEQ ID NO:50 or in SEQ ID NO:51 or in SEQ ID NO:52, which correspond to the protein sequences defined in NCBI Protein Accession Reference Sequence NP_001098101 and in NCBI Protein Accession Reference Sequence NP_001336171 and in NCBI Protein Accession Reference Sequence NP_001184147 and in NCBI Protein Accession Reference Sequence NP_006194 and in NCBI Protein Accession Reference Sequence NP_001184150 and in NCBI Protein Accession Reference Sequence NP_001184149 and in NCBI Protein Accession Reference Sequence NP_001184152 and in NCBI Protein Accession Reference Sequence NP_001159371 and in NCBI Protein Accession Reference Sequence NP_001184148 encoding the PDE4D polypeptide.

The term "PDE4D" also comprises nucleotide sequences showing a high degree of homology to PDE4D, e.g., nucleic acid sequences being at least 75%, 80%, 85%, 90%, 91%, 92%, 93%, 94%, 95%, 96%, 97%, 98% or 99% identical to the sequence as set forth in in SEQ ID NO:35 or in SEQ ID NO:36 or in SEQ ID NO:37 or in SEQ ID NO:38 or in SEQ ID NO:39 or in SEQ ID NO:40 or in SEQ ID NO:41 or in SEQ ID NO:42 or in SEQ ID NO:43 or amino acid sequences being at least 75%, 80%, 85%, 90%, 91%, 92%, 93%, 94%, 95%, 96%, 97%, 98% or 99% identical to the sequence as set forth in SEQ ID NO:44 or in SEQ ID NO:45 or in SEQ ID NO:46 or in SEQ ID NO:47 or in SEQ ID NO:48 or in SEQ ID NO:49 or in SEQ ID NO:50 or in SEQ ID NO:51 or in SEQ ID NO:52 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:44 or in SEQ ID NO:45 or in SEQ ID NO:46 or in SEQ ID NO:47 or in SEQ ID NO:48 or in SEQ ID NO:49 or in SEQ ID NO:50 or in SEQ ID NO:51 or in SEQ ID NO:52 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:35 or in SEQ ID NO:36 or in SEQ ID NO:37 or in SEQ ID or in SEQ ID NO:39 or in SEQ ID NO:40 or in SEQ ID NO:41 or in SEQ ID NO:42 or in SEQ ID NO:43.

The term "PRKACA" refers to the Protein Kinase cAMP-Activated Catalytic Subunit Alpha gene (Ensembl: ENSG00000072062), for example, to the sequence as defined in NCBI Reference Sequence NM_002730 or in NCBI Reference Sequence NM_207518, specifically, to the nucleotide sequences as set forth in SEQ ID NO:53 or in SEQ ID NO:54, which correspond to the sequences of the above indicated NCBI Reference Sequences of the PRKACA transcript, and also relates to the corresponding amino acid sequence for example as set forth in SEQ ID NO:55 or in SEQ ID NO:56, which correspond to the protein sequences defined in NCBI Protein Accession Reference Sequence NP_002721 and in NCBI Protein Accession Reference Sequence NP_997401 encoding the PRKACA polypeptide.

The term "PRKACA" also comprises nucleotide sequences showing a high degree of homology to PRKACA, e.g., nucleic acid sequences being at least 75%, 80%, 85%, 90%, 91%, 92%, 93%, 94%, 95%, 96%, 97%, 98% or 99% identical to the sequence as set forth in SEQ ID NO:53 or in SEQ ID NO:54 or amino acid sequences being at least 75%, 80%, 85%, 90%, 91%, 92%, 93%, 94%, 95%, 96%, 97%, 98% or 99% identical to the sequence as set forth in SEQ ID NO:55 or in SEQ ID NO:56 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:55 or in SEQ ID NO:56 or amino acid sequences being encoded by nucleic acid sequences being at least 80%, 85%, 90%, 91%, 92%, 93%, 94%, 95%, 96%, 97%, 98% or 99% identical to the sequence as set forth in SEQ ID NO:53 or in SEQ ID NO:54.

The term "PRKACB" refers to the Protein Kinase cAMP-Activated Catalytic Subunit Beta gene (Ensembl: ENSG00000142875), for example, to the sequence as defined in NCBI Reference Sequence NM_002731 or in NCBI Reference Sequence NM_182948 or in NCBI Reference Sequence NM_001242860 or in NCBI Reference Sequence NM_001242859 or in NCBI Reference Sequence NM_001242858 or in NCBI Reference Sequence NM_001242862 or in NCBI Reference Sequence NM_001242861 or in NCBI Reference Sequence NM_001300915 or in NCBI Reference Sequence NM_207578 or in NCBI Reference Sequence NM_001242857 or in NCBI Reference Sequence NM_001300917, specifically, to the nucleotide sequence as set forth in SEQ ID NO:57 or in SEQ ID NO:58 or in SEQ ID NO:59 or in SEQ ID NO:60 or in SEQ ID NO:61 or in SEQ ID NO:62 or in SEQ ID NO:63 or in SEQ ID NO:64 or in SEQ ID NO:65 or in SEQ ID NO:66 or in SEQ ID NO:67, which correspond to the sequences of the above indicated NCBI Reference Sequences of the PRKACB transcript, and also relates to the corresponding amino acid sequence for example as set forth in SEQ ID NO:68 or in SEQ ID NO:69 or in SEQ ID NO:70 or in SEQ ID NO:71 or in SEQ ID NO:72 or in SEQ ID NO:73 or in SEQ ID NO:74 or in SEQ ID NO:75 or in SEQ ID NO:76 or in SEQ ID NO:77 or in SEQ ID NO:78, which correspond to the protein sequences defined in NCBI Protein Accession Reference Sequence NP_002722 and in NCBI Protein Accession Reference Sequence NP_891993 and in NCBI Protein Accession Reference Sequence NP_001229789 and in NCBI Protein Accession Reference Sequence NP_001229788 and in NCBI Protein Accession Reference Sequence NP_001229787 and in NCBI Protein Accession Reference Sequence NP_001229791 and in NCBI Protein Accession Reference Sequence NP_001229790 and in NCBI Protein Accession Reference Sequence NP_001287844 and in NCBI Protein Accession Reference Sequence NP_997461 and in NCBI Protein Accession Reference Sequence NP_001229786 and in NCBI Protein Accession Reference Sequence NP_001287846 encoding the PRKACB polypeptide.

The term "PRKACB" also comprises nucleotide sequences showing a high degree of homology to PRKACB, e.g., nucleic acid sequences being at least 75%, 80%, 85%, 90%, 91%, 92%, 93%, 94%, 95%, 96%, 97%, 98% or 99% identical to the sequence as set forth in SEQ ID NO:57 or in SEQ ID NO:58 or in SEQ ID NO:59 or in SEQ ID NO:60 or in SEQ ID NO:61 or in SEQ ID NO:62 or in SEQ ID NO:63 or in SEQ ID NO:64 or in SEQ ID NO:65 or in SEQ ID NO:66 or in SEQ ID NO:67 or amino acid sequences being at least 80%, 85%, 90%, 91%, 92%, 93%, 94%, 95%, 96%, 97%, 98% or 99% identical to the sequence as set forth in SEQ ID NO:68 or in SEQ ID NO:69 or in SEQ ID NO:70 or in SEQ ID NO:71 or in SEQ ID NO:72 or in SEQ ID NO:73 or in SEQ ID NO:74 or in SEQ ID NO:75 or in SEQ ID NO:76 or in SEQ ID NO:77 or in SEQ ID NO:78 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:68 or in SEQ ID NO:69 or in SEQ ID NO:70 or in SEQ ID NO:71 or in SEQ ID NO:72 or in SEQ ID NO:73 or in SEQ ID NO:74 or in SEQ ID NO:75 or in SEQ ID NO:76 or in SEQ ID NO:77 or in SEQ ID NO:78 or amino acid sequences being encoded by nucleic acid sequences being at least 80%, 85%, 90%, 91%, 92%, 93%, 94%, 95%, 96%, 97%, 98% or 99% identical to the sequence as set forth in SEQ ID NO:57 or in SEQ ID NO:58 or in SEQ ID NO:59 or in SEQ ID NO:60 or in SEQ ID NO:61 or in SEQ ID NO:62 or in SEQ ID NO:63 or in SEQ ID NO:64 or in SEQ ID NO:65 or in SEQ ID NO:66 or in SEQ ID NO:67.

The term "PTPRC" refers to the Protein Tyrosine Phosphatase Receptor Type C gene (Ensembl: ENSG00000081237), for example, to the sequence as defined in NCBI Reference Sequence NM_002838 or in NCBI Reference Sequence NM_080921, specifically, to the nucleotide sequence as set forth in SEQ ID NO:79 or in SEQ ID NO:80, which correspond to the sequences of the above indicated NCBI Reference Sequences of the PTPRC transcript, and also relates to the corresponding amino acid sequence for example as set forth in SEQ ID NO:81 or in SEQ ID NO:82, which correspond to the protein sequences defined in NCBI Protein Accession Reference Sequence NP_002829 encoding the PTPRC polypeptide and in NCBI Protein Accession Reference Sequence NP_563578.

The term "PTPRC" also comprises nucleotide sequences showing a high degree of homology to PTPRC, e.g., nucleic acid sequences being at least 75%, 80%, 85%, 90%, 91%, 92%, 93%, 94%, 95%, 96%, 97%, 98% or 99% identical to the sequence as set forth in SEQ ID NO:79 or in SEQ ID NO:80 or amino acid sequences being at least 75%, 80%, 85%, 90%, 91%, 92%, 93%, 94%, 95%, 96%, 97%, 98% or 99% identical to the sequence as set forth in SEQ ID NO:81 or in SEQ ID NO:82 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:81 or in SEQ ID NO:82 or amino acid sequences being encoded by nucleic acid sequences being at least 80%, 85%, 90%, 91%, 92%, 93%, 94%, 95%, 96%, 97%, 98% or 99% identical to the sequence as set forth in SEQ ID NO:79 or in SEQ ID NO:80.

The term "ZAP70" refers to the Zeta Chain Of T-Cell Receptor Associated Protein Kinase 70 gene (Ensembl: ENSG00000115085), for example, to the sequence as defined in NCBI Reference Sequence NM_001079 or in NCBI Reference Sequence NM_207519, specifically, to the nucleotide sequences as set forth in SEQ ID NO:83 or in SEQ ID NO:84, which correspond to the sequences of the above indicated NCBI Reference Sequences of the ZAP70 transcript, and also relates to the corresponding amino acid sequence for example as set forth in SEQ ID NO:85 or in SEQ ID NO:86, which corresponds to the protein sequences defined in NCBI Protein Accession Reference Sequence NP_001070 and in NCBI Protein Accession Reference Sequence NP_997402 encoding the ZAP70 polypeptide.

The term "ZAP70" also comprises nucleotide sequences showing a high degree of homology to ZAP70, e.g., nucleic acid sequences being at least 75%, 80%, 85%, 90%, 91%, 92%, 93%, 94%, 95%, 96%, 97%, 98% or 99% identical to the sequence as set forth in SEQ ID NO:83 or in SEQ ID NO:84 or amino acid sequences being at least 75%, 80%, 85%, 90%, 91%, 92%, 93%, 94%, 95%, 96%, 97%, 98% or 99% identical to the sequence as set forth in SEQ ID NO:85 or in SEQ ID NO:86 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:85 or in SEQ ID NO:86 or amino acid sequences being encoded by nucleic acid sequences being at least 80%, 85%, 90%, 91%, 92%, 93%, 94%, 95%, 96%, 97%, 98% or 99% identical to the sequence as set forth in SEQ ID NO:83 or in SEQ ID NO:84.

The term "biological sample" or "sample obtained from a subject" refers to any biological material obtained via suitable methods known to the person skilled in the art from a subject, e.g., a prostate cancer patient. The biological sample used may be collected in a clinically acceptable manner, e.g., in a way that nucleic acids (in particular RNA) or proteins are preserved.

The biological sample(s) may include body tissue and/or a fluid, such as, but not limited to, blood, sweat, saliva, and urine. Furthermore, the biological sample may contain a cell extract derived from or a cell population including an epithelial cell, such as a cancerous epithelial cell or an epithelial cell derived from tissue suspected to be cancerous. The biological sample may contain a cell population derived from a glandular tissue, e.g., the sample may be derived from the prostate of a male subject. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumour cells, extracellular vesicles, a sample containing prostate secreted exosomes, or cell lines or cancer cell line.

In one particular realization, biopsy or resections samples may be obtained and/or used. Such samples may include cells or cell lysates.

It is also conceivable that the content of a biological sample is submitted to an enrichment step. For instance, a sample may be contacted with ligands specific for the cell membrane or organelles of certain cell types, e.g., prostate cells, functionalized for example with magnetic particles. The material concentrated by the magnetic particles may subsequently be used for detection and analysis steps as described herein above or below.

Furthermore, cells, e.g., tumour cells, may be enriched via filtration processes of fluid or liquid samples, e.g., blood, urine, etc. Such filtration processes may also be combined with enrichment steps based on ligand specific interactions as described herein above.

The term "prostate cancer" refers to a cancer of the prostate gland in the male reproductive system, which occurs when cells of the prostate mutate and begin to multiply out of control. Typically, prostate cancer is linked to an elevated level of prostate-specific antigen (PSA). In one embodiment of the present invention the term "prostate cancer" relates to a cancer showing PSA levels above 3.0. In another embodiment the term relates to cancer showing PSA levels above 2.0. The term "PSA level" refers to the concentration of PSA in the blood in ng/ml.

The term "non-progressive prostate cancer state" means that a sample of an individual does not show parameter values indicating "biochemical recurrence" and/or "clinical recurrence" and/or "metastases" and/or "castration-resistant disease" and/or "prostate cancer or disease specific death".

The term "progressive prostate cancer state" means that a sample of an individual shows parameter values indicating "biochemical recurrence" and/or "clinical recurrence" and/or "metastases" and/or "castration-resistant disease" and/or "prostate cancer or disease specific death".

The term "biochemical recurrence" generally refers to recurrent biological values of increased PSA indicating the presence of prostate cancer cells in a sample. However, it is also possible to use other markers that can be used in the detection of the presence or that rise suspicion of such presence.

The term "clinical recurrence" refers to the presence of clinical signs indicating the presence of tumour cells as measured, for example using in vivo imaging.

The term "metastases" refers to the presence of metastatic disease in organs other than the prostate.

The term "castration-resistant disease" refers to the presence of hormone-insensitive prostate cancer; i.e., a cancer in the prostate that does not any longer respond to androgen deprivation therapy (ADT).

The term "prostate cancer specific death or disease specific death" refers to death of a patient from his prostate cancer.

It is preferred that the one or more T-Cell receptor signaling genes comprise three or more of the T-Cell receptor signaling genes.

It is further preferred that the one or more T-Cell receptor signaling genes comprise six or more of the T-Cell receptor signaling genes.

It is yet further preferred that the one or more T-Cell receptor signaling genes comprise nine or more, preferably, all of the T-Cell receptor signaling genes.

It is preferred that the determining of the prediction of the radiotherapy response comprises combining the gene expression profiles for two or more, for example, 2, 3, 4, 5, 6, 7, 8, 9, 10, 11, 12, 13, 14, 15, 16 or all, of the T-Cell receptor signaling genes with a regression function that had been derived from a population of prostate cancer subjects.

Cox proportional-hazards regression allows analyzing the effect of several risk factors on time to a tested event like survival. Thereby, the risk factors maybe dichotomous or discrete variables like a risk score or a clinical stage but may also be a continuous variable like a biomarker measurement or gene expression values. The probability of the endpoint (e.g., death or disease recurrence) is called the hazard. Next to the information on whether or not the tested endpoint was reached by e.g. subject in a patient cohort (e.g., patient did die or not) also the time to the endpoint is considered in the regression analysis. The hazard is modeled as: $H(t)=H_0(t) \cdot \exp(w_1 \cdot V_1 + w_2 \cdot V_2 + w_3 \cdot V_3 + ...)$, where $V_1, V_2, V_3$ ... are predictor variables and $H_0(t)$ is the baseline hazard while $H(t)$ is the hazard at any time t. The hazard ratio (or the risk to reach the event) is represented by $Ln[H(t)/ H_0(t)]= w_1 \cdot V_1 + w_2 \cdot V_2 + w_3 \cdot V_3 + ...$, where the coefficients or weights $w_1, w_2, w_3$ ... are estimated by the Cox regression analysis and can be interpreted in a similar manner as for logistic regression analysis.

In one particular realization, the prediction of the radiotherapy response is determined as follows:

$$(w_1 \cdot C2) + (w_2 \cdot CD247) + (w_3 \cdot CD28) + (w_4 \cdot CD3E) + (w_5 \cdot CD3G) + (w_6 \cdot CD4) + (w_7 \cdot CSK) + (w_8 \cdot EZR) + (w_9 \cdot FYN) + (w_{10} \cdot LAT) + (w_{11} \cdot LCK) + (w_{12} \cdot PAG1) + (w_{13} \cdot PDE4D) + (w_{14} \cdot PRKACA) + (w_{15} \cdot PRKACB) + (w_{16} \cdot PTPRC) + (w_{17} \cdot ZAP70) \quad (1)$$

where $w_1$ to $w_{17}$ are weights and CD2, CD247, CD28, CD3E, CD3G, CD4, CSK, EZR, FYN, LAT, LCK, PAG1, PDE4D, PRKACA, PRKACB, PTPRC, and ZAP70 are the expression levels of the T-Cell receptor signaling genes.

In one example, $w_1$ may be about -0.5 to 0.5, such as -0.07388, $w_2$ may be about -3.5 to -2.5, such as -3.1496, $w_3$ may be about -1.0 to 0.0, such as -0.4443, $w_4$ may be about -0.5 to 0.5, such as -0.005986, $w_5$ may be about -1.0 to 0.0, such as -0.2943, $w_6$ may be about 0.0 to 1.0, such as 0.5738, $w_7$ may be about -0.5 to 0.5, such as 0.1856, $w_8$ may be about 0.0 to 1.0, such as 0.3914, $w_9$ may be about -0.5 to 0.5, such as 0.02173, $w_{10}$ may be about 0.5 to 1.5, such as 0.7811, $w_{11}$ may be about -0.5 to 0.5, such as 0.2116, $w_{12}$ may be about -0.5 to 0.5, such as 0.2432, $w_{13}$ may be about -5.0 to -3.0, such as -4.0934, $w_{14}$ may be about 0.5 to 1.5, such as 1.176, $w_{15}$ may be about -1.0 to 0.0, such as -0.4655, $w_{16}$ may be about -1.5 to -0.5, such as -1.153, and $w_{17}$ may be about -1.0 to 0.0, such as -0.2942.

The prediction of the radiotherapy response may also be classified or categorized into one of at least two risk groups, based on the value of the prediction of the radiotherapy response. For example, there may be two risk groups, or three risk groups, or four risk groups, or more than four predefined risk groups. Each risk group covers a respective range of (non-overlapping) values of the prediction of the radiotherapy response. For example, a risk group may indicate a probability of occurrence of a specific clinical event from 0 to <0.1 or from 0.1 to <0.25 or from 0.25 to <0.5 or from 0.5 to 1.0 or the like.

It is preferred that the biological sample is obtained from the subject before the start of the radiotherapy. The gene expression profile(s) may be determined in the form of mRNA or protein in tissue of prostate cancer. Alternatively, if the T-Cell signaling genes are present in a soluble form, the gene expression profile(s) may be determined in blood.

It is further preferred that the radiotherapy is radical radiotherapy or salvage radiotherapy.

It is preferred that the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy. The degree to which the prediction is negative may determine the degree to which the recommended therapy deviates from the standard form of radiotherapy.

In a further aspect of the present invention, an apparatus for predicting a response of a prostate cancer subject to radiotherapy is presented, comprising:

- an input adapted to receive data indicative of a gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said gene expression profile(s) being determined in a biological sample obtained from the subject,
- a processor adapted to determine the prediction of the radiotherapy response based on the gene expression profile(s) for the one or more T-Cell receptor signaling genes, and
- optionally, a providing unit adapted to provide the prediction or a therapy recommendation based on the prediction to a medical caregiver or the subject.

In a further aspect of the present invention, a computer program product is presented comprising instructions which, when the program is executed by a computer, cause the computer to carry out a method comprising:

- receiving data indicative of a gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said gene expression profile(s) being determined in a biological sample obtained from a prostate cancer subject,
- determining the prediction of the radiotherapy response based on the gene expression profile(s) for the one or more T-Cell receptor signaling genes, and
- optionally, providing the prediction or a therapy recommendation based on the prediction to a medical caregiver or the subject.

In a further aspect of the present invention, a diagnostic kit is presented, comprising:
at least one primer and/or probe for determining the gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, in a biological sample obtained from the subject, and

- optionally, an apparatus as defined in claim 9 or a computer program product as defined in claim 10.

In a further aspect of the present invention, a use of the kit as defined in claim 11 is presented.

It is preferred that the use as defined in claim 12 is in a method of predicting a response of a prostate cancer subject to radiotherapy.
In a further aspect of the present invention, a method is presented, comprising:

- receiving a biological sample obtained from a prostate cancer subject,
- using the kit as defined in claim 11 to determine a gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, in the biological sample obtained from the subject.

In a further aspect of the present invention, a use of a gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70 in a method of predicting a response of a prostate cancer subject to radiotherapy is presented, comprising:

- determining the prediction of the radiotherapy response based on the gene expression profile(s) for the one or more T-Cell receptor signaling genes, and

- optionally, providing the prediction or a therapy recommendation based on the prediction to a medical caregiver or the subject.

It shall be understood that the method of claim 1, the apparatus of claim 9, the computer program product of claim 10, the diagnostic kit of claim 11, the use of the diagnostic kit of claim 12, the method of claim 14, and the use of a gene expression profile(s) of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplarily a flowchart of an embodiment of a method of predicting a response of a prostate cancer subject to radiotherapy.

Fig. 2 shows a ROC curve analysis of two predictive models.

Fig. 3 shows a Kaplan-Meier curve analysis of the T-Cell receptor signaling model (TCR_signaling_model). The clinical endpoint that was tested was the time to prostate cancer specific death (PCa Death) after the start of the salvage radiation therapy (SRT) due to post-surgical disease recurrence.

DETAILED DESCRIPTION OF EMBODIMENTS

**Overview Of Radiotherapy Response Prediction**

Fig. 1 shows schematically and exemplarily a flowchart of an embodiment of a method of predicting a response of a prostate cancer subject to radiotherapy. The method begins at step S100.

At step S102, a biological sample is obtained from each of a first set of patients (subjects) diagnosed with prostate cancer. Preferably, monitoring prostate cancer has been performed for these prostate cancer patients over a period of time, such as at least one year, or at least two years, or about five years, after obtaining the biological sample.

At step S104, a gene expression profile for each of two or more, for example, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, is obtained for each of the biological samples obtained from the first set of patients, e.g., by performing RT-qPCR (real-time quantitative PCR) on RNA extracted from each biological sample. The exemplary gene expression profiles include an expression level (e.g., value) for each of the one or more T-Cell receptor signaling genes.

At step S106, a regression function for assigning a prediction of the radiotherapy response is determined based on the gene expression profiles for the two or more T-Cell receptor signaling genes, CD2, CD247, CD28, CD3E, CD3G, CD4, CSK, EZR, FYN, LAT, LCK, PAG1, PDE4D, PRKACA, PRKACB, PTPRC, and/or ZAP70, obtained for at least some of the biological samples obtained for the first set of patients and respective results obtained from the monitoring. In one particular realization, the regression function is determined as specified in Eq. (1) above.

At step S108, a biological sample is obtained from a patient (subject or individual). The patient can be a new patient or one of the first set.

At step S110, a gene expression profile is obtained for each of the two or more, for example, 2, 3, 4, 5, 6, 7, 8, 9, 10, 11, 12, 13, 14, 15, 16 or all, T-Cell receptor signaling genes, e.g., by performing PCR on the biological sample.

At step S112, a prediction of the radiotherapy response based on the gene expression profiles for the two or more T-Cell receptor signaling genes is determined for the patient using the regression function. This will be described in more detail later in the description.

At S114, a therapy recommendation may be provided, e.g., to the patient or his or her guardian, to a doctor, or to another healthcare worker, based on the prediction. To this end, the prediction may be categorized into one of a predefined set of risk groups, based on the value of the prediction. In one particular realization, the prediction of the radiotherapy response may be negative or positive for the effectiveness of the radiotherapy. If the prediction is negative, the recommended therapy may comprise one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

The method ends at S116.

In one embodiment, the gene expression profiles at steps S104 and S110 are determined by detecting mRNA expression using two or more primers and/or probes and/or two or more sets thereof.

The immune system interacts in a strong manner with prostate cancer, both on a systemic level and in the tumour microenvironment. T-Cell receptor signaling genes play a central role in the regulation of immune activity. T-Cell receptor signaling genes may therefore provide information on the effectiveness of RT. However, which T-Cell receptor signaling genes may have predictive value in this application is extremely difficult to deduce from existing literature due to the many factors that influence the exact function of T-Cell receptor signaling genes.

We investigated the extent to which the expression of T-Cell receptor signaling genes in prostate cancer tissue correlates with the recurrence of disease after radical RT or SRT.

We have identified 17 T-Cell receptor signaling genes for which the degree of expression in prostate cancer tissue significantly correlates with mortality after SRT, in a cohort of 151 prostate cancer patients.

Based on the significant correlation with outcome after RT, we expect that the identified molecules will provide predictive value with regard to the effectiveness of radical RT and/or SRT.

RESULTS

**Cox Regression Analysis**

We then set out to test whether the combination of these 17 T-Cell receptor signaling genes will exhibit more prognostic value. With Cox regression we modelled the expression levels of the 17 T-Cell receptor signaling genes to prostate cancer specific death after post-surgical salvage RT (TCR_signaling_model). We tested the model in ROC curve analysis as well as in Kaplan-Meier survival analysis.

The investigated patient group consisting of 538 subjects is a surgical cohort. All patients were undergoing prostatectomy to remove their primary prostate tumors. Approximately 30% of all patients experienced PSA relapse (or biochemical recurrence) within 10 years after prostate surgery. Therefore, many of those patients (#151) with PSA recurrence have undergone salvage radiation therapy (SRT) either alone or in combination with anti-androgen treatments as a secondary therapy to treat the recurring cancer cells. Despite this treatment, some 40 to 50% of these patients developed regional or distant metastases and some 25% died from prostate cancer within 5 to 10 years after the start of SRT.

The Cox regression function was derived as follows: TCR_signaling_model:

$$(w_1 \cdot C2) + (w_2 \cdot CD247) + (w_3 \cdot CD28) + (w_4 \cdot CD3E) + (w_5 \cdot CD3G) + (w_6 \cdot CD4) + (w_7 \cdot CSK) + (w_8 \cdot EZR) + (w_9 \cdot FYN) + (w_{10} \cdot LAT) + (w_{11} \cdot LCK) + (w_{12} \cdot PAG1) + (w_{13} \cdot PDE4D) + (w_{14} \cdot PRKACA) + (w_{15} \cdot PRKACB) + (w_{16} \cdot PTPRC) + (w_{17} \cdot ZAP70)$$

The details for the weights $w_1$ to $w_{17}$ are shown in the following TABLE 1.

TABLE 1: Variables and weights for the Cox regression model, i.e., the T-Cell receptor signaling model (TCR_ signaling_model).

| **Variable** | **Weights** | |
|---|---|---|
| **CD2** | $w_1$ | -0,07388 |
| **CD247** | $w_2$ | -3,1496 |
| **CD28** | $w_3$ | -0,4443 |
| **CD3E** | $w_4$ | -0,005986 |
| **CD3G** | $w_5$ | -0,2943 |
| **CD4** | $w_6$ | 0,5738 |
| **CSK** | $w_7$ | 0,1856 |
| **EZR** | $w_8$ | 0,3914 |
| **FYN** | $w_9$ | 0,02173 |

(continued)

| Variable | Weights | |
|---|---|---|
| **LAT** | $w_{10}$ | 0,7811 |
| **LCK** | $w_{11}$ | 0,2116 |
| **PAG1** | $w_{12}$ | 0,2432 |
| **PDE4D** | $w_{13}$ | -4,0934 |
| **PRKACA** | $w_{14}$ | 1,176 |
| **PRKACB** | $w_{15}$ | -0,4655 |
| **PTPRC** | $w_{16}$ | -1,153 |
| **ZAP70** | $w_{17}$ | -0,2942 |

**ROC Curve Analysis**

Next, we tested the logistic regression model as outlined above for their power to predict 5-year prostate cancer specific death (PCa Death) after the start of salvage radiation therapy (SRT) due to post-surgical disease recurrence. The performance of the model was compared to the EAU BCR risk groups (see Tilki D. et al., "External validation of the European Association of Urology Biochemical Recurrence Risk groups to predict metastasis and mortality after radical prostatectomy in a European cohort", Eur Urol, Vol. 75, No. 6, pages 896-900, 2019).

Fig. 2 shows a ROC curve analysis of two predictive models. The TCR_signaling_model (AUC=0,88) is the Cox regression model based on the 17 T-Cell receptor signaling genes. The EAU_BCR_Risk (AUC=0,77) is the EAU_BCR_Risk groups (European Association of Urology Biochemical Recurrence Risk groups).

**Kaplan-Meier Survival Analysis**

For Kaplan-Meier curve analysis, the Cox function of the risk model (TCR_signaling_model) was categorized into two sub-cohorts based on a cut-off (see description of figure below). The goal was to create patient classes by separating the classes with the median risk score as calculated from the IDR model for each patient with to some extent similar number of patients within the individual group.

The patient classes represent an increasing risk to experience the tested clinical endpoints of time to prostate cancer specific death (Fig. 3) since the start of salvage radiation therapy (SRT) for the created risk model (TCR_signaling_model).

Fig. 3 shows a Kaplan-Meier curve of the TCR_signaling_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the TCR regression model using the value -6.5 as cut-off (logrank p<0.0001; HR=6,4; CI=2,9-14,1). The following supplementary list indicate the number of patients at risk for the TCR_signaling_model classes analyzed, i.e., the patients at risk at any time interval +20 months after surgery are shown: Low risk: 76, 76, 73, 72, 61, 48, 45, 24, 3, 2, 0; High risk: 75, 68, 57, 42, 34, 28, 26, 13, 3, 1, 0.

The Kaplan-Meier curve analysis as shown in Fig. 3 demonstrates the presence of different patient risk groups. The risk group of a patient is determined by the probability to suffer from the respective clinical endpoint (e.g., prostate cancer specific death) as calculated by the risk model TCR_signaling_model. Depending on the predicted risk of a patient (i.e., depending on in which risk group 1 or 2 the patient may belong) different types of interventions might be indicated.

**Discussion**

The effectiveness of both radical RT and SRT for localized prostate cancer is limited, resulting in disease progression and ultimately death of patients, especially for those at high risk of recurrence. The prediction of the therapy outcome is very complicated as many factors play a role in therapy effectiveness and disease recurrence. It is likely that important factors have not yet been identified, while the effect of others cannot be determined precisely. Multiple clinico-pathological measures are currently investigated and applied in a clinical setting to improve response prediction and therapy selection, providing some degree of improvement. Nevertheless, a strong need remains for better prediction of the response to radical RT and to SRT, in order to increase the success rate of these therapies.

We have identified molecules of which expression shows a significant relation to mortality after radical RT and SRT and therefore are expected to improve the prediction of the effectiveness of these treatments. An improved prediction of effectiveness of RT for each patient be it in the radical or the salvage setting, will improve therapy selection and potentially survival. This can be achieved by 1) optimizing RT for those patients where RT is predicted to be effective (e.g. by dose escalation or a different starting time) and 2) guiding patients where RT is predicted not to be effective to an alternative, potentially more effective form of treatment. Further, this would reduce suffering for those patients who would be spared ineffective therapy and would reduce cost spent on ineffective therapies.

Other variations to the disclosed realizations can be understood and effected by those skilled in the art in practicing the claimed invention, from a study of the drawings, the disclosure, and the appended claims.

In a preferred embodiment, the one or more T-Cell receptor signaling genes are selected from the group consisting of: CD2, CD247, CD28, CD3E, CD3G, CD4, CSK, EZR, FYN, LAT, LCK, PAG1, PRKACA, PRKACB, PTPRC, and ZAP70. For example, 1, 2, 3, 4, 5, 6, 7, 8, 9, 10, 11, 12, 13, 14, 15 or all T-Cell receptor signaling genes from this group may be selected and the prediction of the radiotherapy response may be determined based on the gene expression profile(s) for the one or more T-Cell receptor signaling genes. Likewise, the gene expression profiles for two or more, for example, 2, 3, 4, 5, 6, 7, 8, 9, 10, 11, 12, 13, 14, 15 or all, of the T-Cell receptor signaling genes selected from this group may be combined with a regression function that had been derived from a population of prostate cancer subjects.

In the claims, the word "comprising" does not exclude other elements or steps, and the indefinite article "a" or "an" does not exclude a plurality.

One or more steps of the method illustrated in Fig. 1 may be implemented in a computer program product that may be executed on a computer. The computer program product may comprise a non-transitory computer-readable recording medium on which a control program is recorded (stored), such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other non-transitory medium from which a computer can read and use.

Alternatively, the one or more steps of the method may be implemented in transitory media, such as a transmittable carrier wave in which the control program is embodied as a data signal using transmission media, such as acoustic or light waves, such as those generated during radio wave and infrared data communications, and the like.

The exemplary method may be implemented on one or more general purpose computers, special purpose computer(s), a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLA, FPGA, Graphical card CPU (GPU), or PAL, or the like. In general, any device, capable of implementing a finite state machine that is in turn capable of implementing the flowchart shown in Fig. 1, can be used to implement one or more steps of the method of risk stratification for therapy selection in a patient with prostate cancer is illustrated. As will be appreciated, while the steps of the method may all be computer implemented, in some embodiments one or more of the steps may be at least partially performed manually.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Any reference signs in the claims should not be construed as limiting the scope.

The invention relates to a method of predicting a response of a prostate cancer subject to radiotherapy, comprising determining or receiving the result of a determination of a gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said gene expression profile(s) being determined in a biological sample obtained from the subject, determining the prediction of the radiotherapy response based on the gene expression profile(s) for the one or more T-Cell receptor signaling genes, and optionally, providing the prediction or a therapy recommendation based on the prediction to a medical caregiver or the subject. Since the status of the immune system and of the immune microenvironment have an impact on therapy effectiveness, the ability to identify markers predictive for this effect might help to be better able to predict overall RT response. T-Cell receptor signaling genes play a central role in the regulation of immune activity. The identified T-Cell receptor signaling genes were found to exhibit a significant correlation with outcome after RT, wherefore we expect that they will provide predictive value with regard to the effectiveness of radical RT and/or SRT.

**The attached Sequence Listing, entitled 2019PF00712_Sequence Listing_ST25 is incorporated herein by reference, in its entirety.**

## Claims

1. A method of predicting a response of a prostate cancer subject to radiotherapy, comprising:

    - determining or receiving the result of a determination of a gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said gene expression profile(s) being determined in a biological sample obtained from the subject,
    - determining the prediction of the radiotherapy response based on the gene expression profile(s) for the one or more T-Cell receptor signaling genes, and
    - optionally, providing the prediction or a therapy recommendation based on the prediction to a medical caregiver or the subject.

2. The method as defined in claim 1, wherein the one or more T-Cell receptor signaling genes comprise three or more of the T-Cell receptor signaling genes.

3. The method as defined in claim 1 or 2, wherein the one or more T-Cell receptor signaling genes comprise six or more of the T-Cell receptor signaling genes.

4. The method as defined in any of claims 1 to 3, wherein the one or more T-Cell receptor signaling genes comprise nine or more, preferably, all of the T-Cell receptor signaling genes.

5. The method as defined in any of claims 1 to 4, wherein the determining of the prediction of the radiotherapy response comprises combining the gene expression profiles for two or more, for example, 2, 3, 4, 5, 6, 7, 8, 9, 10, 11, 12, 13, 14, 15, 16 or all, of the T-Cell receptor signaling genes with a regression function that had been derived from a population of prostate cancer subjects.

6. The method as defined in any of claims 1 to 5, wherein the biological sample is obtained from the subject before the start of the radiotherapy.

7. The method as defined in any of claims 1 to 6, wherein the radiotherapy is radical radiotherapy or salvage radiotherapy.

8. The method as defined in any of claims 1 to 7, wherein the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

9. An apparatus for predicting a response of a prostate cancer subject to radiotherapy, comprising:

    - an input adapted to receive data indicative of a gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said gene expression profile(s) being determined in a biological sample obtained from the subject,
    - a processor adapted to determine the prediction of the radiotherapy response based on the gene expression profile(s) for the one or more T-Cell receptor signaling genes, and
    - optionally, a providing unit adapted to provide the prediction or a therapy recommendation based on the prediction to a medical caregiver or the subject.

10. A computer program product comprising instructions which, when the program is executed by a computer, cause the computer to carry out a method comprising:

    - receiving data indicative of a gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said gene expression profile(s) being determined in a biological sample obtained from a prostate cancer subject,
- determining the prediction of the radiotherapy response based on the gene expression profile(s) for the one or more T-Cell receptor signaling genes, and
- optionally, providing the prediction or a therapy recommendation based on the prediction to a medical caregiver or the subject.

**11.** A diagnostic kit, comprising:

- at least one primer and/or probe for determining the gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, in a biological sample obtained from the subject, and
- optionally, an apparatus as defined in claim 9 or a computer program product as defined in claim 10.

**12.** Use of the kit as defined in claim 11.

**13.** The use of the kit as defined in claim 12 in a method of predicting a response of a prostate cancer subject to radiotherapy.

**14.** A method, comprising:

- receiving a biological sample obtained from a prostate cancer subject,
- using the kit as defined in claim 11 to determine a gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, in the biological sample obtained from the subject.

**15.** Use of a gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70 in a method of predicting a response of a prostate cancer subject to radiotherapy, comprising:

- determining the prediction of the radiotherapy response based on the gene expression profile(s) for the one or more T-Cell receptor signaling genes, and
- optionally, providing the prediction or a therapy recommendation based on the prediction to a medical caregiver or the subject.

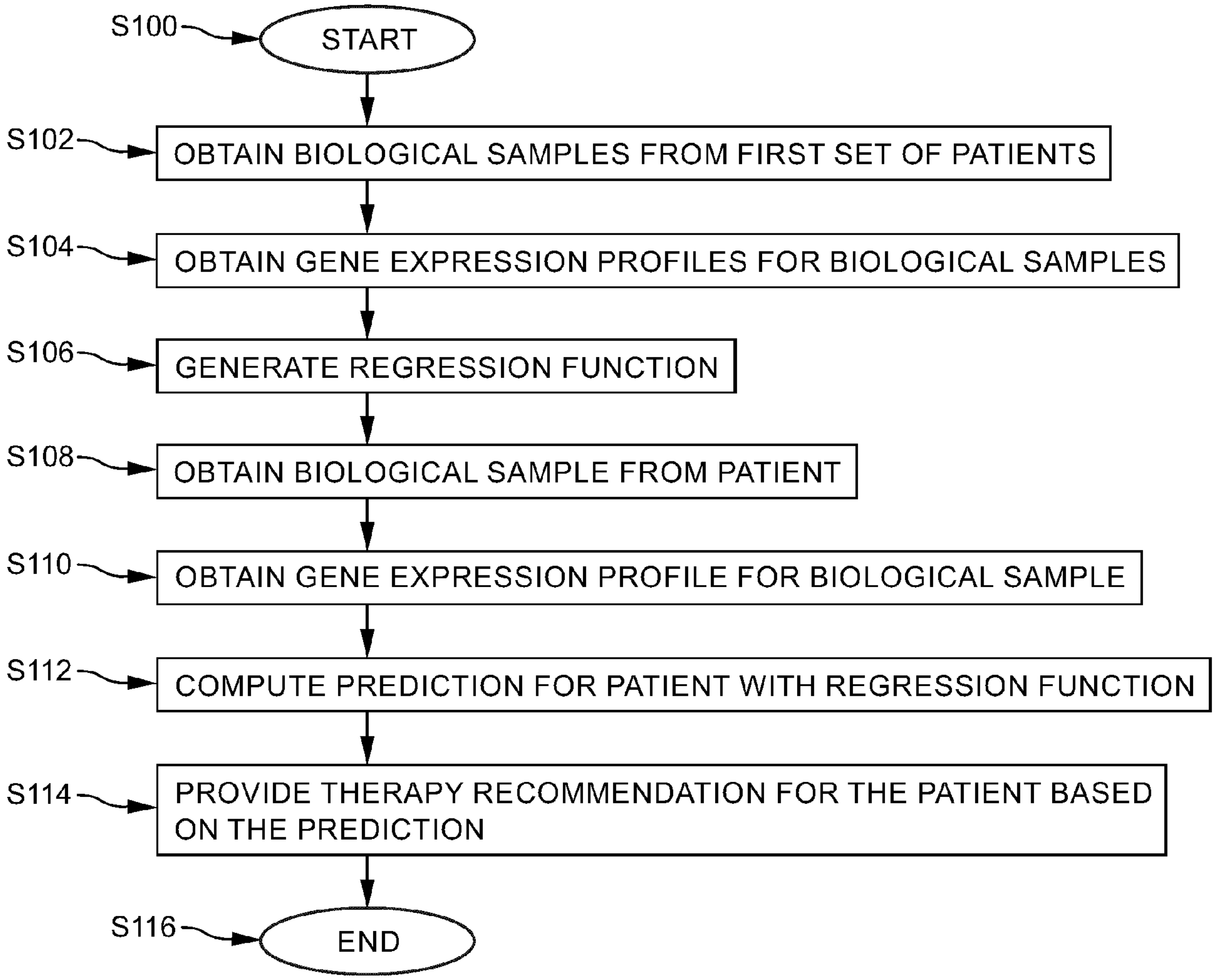
S100
START
S102
OBTAIN BIOLOGICAL SAMPLES FROM FIRST SET OF PATIENTS
S104
OBTAIN GENE EXPRESSION PROFILES FOR BIOLOGICAL SAMPLES
S106
GENERATE REGRESSION FUNCTION
S108
OBTAIN BIOLOGICAL SAMPLE FROM PATIENT
S110
OBTAIN GENE EXPRESSION PROFILE FOR BIOLOGICAL SAMPLE
S112
COMPUTE PREDICTION FOR PATIENT WITH REGRESSION FUNCTION
S114
PROVIDE THERAPY RECOMMENDATION FOR THE PATIENT BASED ON THE PREDICTION
S116
END

FIG. 1

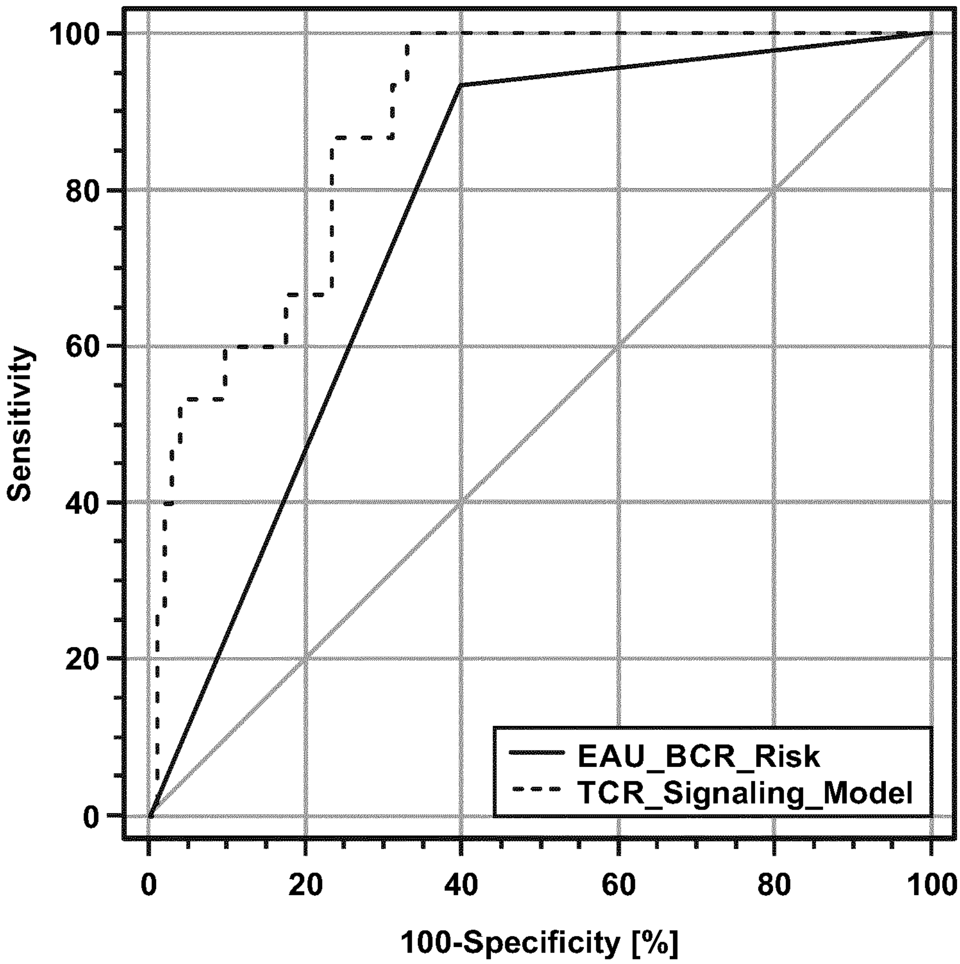
100
80
60
40
20
0
Sensitivity
0
20
40
60
80
100
100-Specificity [%]
EAU_BCR_Risk
TCR_Signaling_Model

FIG. 2

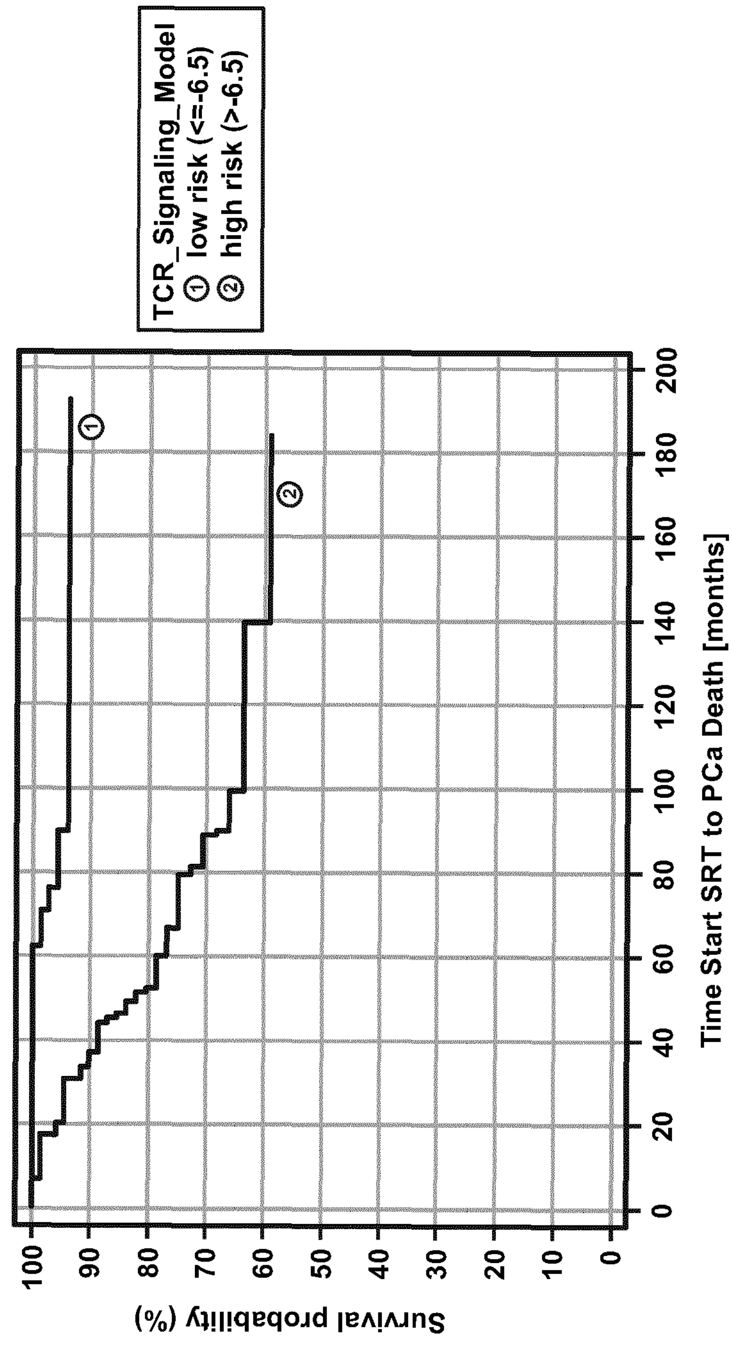
TCR_Signaling_Model
① low risk (<=-6.5)
② high risk (>-6.5)
Survival probability (%)
100
90
80
70
60
50
40
30
20
10
0
0
20
40
60
80
100
120
140
160
180
200
Time Start SRT to PCa Death [months]
①
②

FIG. 3

Europäisches Patentamt
European Patent Office
Office européen des brevets

# EUROPEAN SEARCH REPORT

Application Number
EP 20 16 1181

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/028285 A2 (GENOMEDX INC [US]; UNIV MICHIGAN REGENTS [US]) 7 February 2019 (2019-02-07) * page 3 - page 4; claim 40 * ----- | 1-15 | INV. C12Q1/6886 |
| X | WO 2017/216559 A1 (ALMAC DIAGNOSTICS LTD [GB]) 21 December 2017 (2017-12-21) * page 6 - page 7; claims 1,16,22 * ----- | 1,5-15 | |
| A | WO 2018/039490 A1 (GENOMEDX BIOSCIENCES INC [CA]; UNIV MICHIGAN REGENTS [US]) 1 March 2018 (2018-03-01) * claim 1 * ----- | 1-15 | |
| A | CHEN W S ET AL: "Associations of Stromal Infiltration with Prognosis and Response to Postoperative Radiotherapy in Prostate Cancer", INTERNATIONAL JOURNAL OF RADIATION: ONCOLOGY BIOLOGY PHYSICS, PERGAMON PRESS, USA, vol. 105, no. 1, 1 September 2019 (2019-09-01), XP085822476, ISSN: 0360-3016, DOI: 10.1016/J.IJROBP.2019.06.458 [retrieved on 2019-09-14] * the whole document * ----- | 1 | TECHNICAL FIELDS SEARCHED (IPC) C12Q |

The present search report has been drawn up for all claims

1

| Place of search | Date of completion of the search | Examiner |
|---|---|---|
| The Hague | 3 September 2020 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 16 1181

This annex lists the patent family members relating to the patent documents cited in the above-mentioned European search report. The members are as contained in the European Patent Office EDP file on
The European Patent Office is in no way liable for these particulars which are merely given for the purpose of information.

03-09-2020

| Patent document cited in search report | Publication date | Patent family member(s) | Publication date |
|---|---|---|---|
| WO 2019028285 A2 | 07-02-2019 | AU 2018310987 A1 | 27-02-2020 |
| | | CA 3072061 A1 | 07-02-2019 |
| | | EP 3662082 A2 | 10-06-2020 |
| | | WO 2019028285 A2 | 07-02-2019 |
| WO 2017216559 A1 | 21-12-2017 | NONE | |
| WO 2018039490 A1 | 01-03-2018 | AU 2017315425 A1 | 18-04-2019 |
| | | CN 110506127 A | 26-11-2019 |
| | | EP 3504348 A1 | 03-07-2019 |
| | | US 2019218621 A1 | 18-07-2019 |
| | | WO 2018039490 A1 | 01-03-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BRAY F. et al.** Global cancer statistics 2018: GLOBOCAN estimates of incidence and mortality worldwide for 36 cancers in 185 countries. *CA Cancer J Clin,* 2018, vol. 68 (6), 394-424
- Cancer Facts & Figures 2010. ACS (American Cancer Society), 2010
- CANCER FACTS & FIGURES 2010. ACS, 2010
- Comparative analysis of prostate-specific antigen free survival outcomes for patients with low, intermediate and high risk prostate cancer treatment by radical therapy. **GRIMM P. et al.** BJU Int. Prostate Cancer Results Study Group, 2012, 22-29
- **GRIMM P. et al.** *BJU INT,* 2012
- BJU INT. ACS, 2010
- **DAL PRA A. et al.** Contemporary role of postoperative radiotherapy for prostate cancer. *Transl Androl Urol,* 2018, vol. 7 (3), 399-413
- **HERRERA F.G. ; BERTHOLD D.R.** Radiation therapy after radical prostatectomy: Implications for clinicians. *Front Oncol,* 2016, vol. 6 (117
- **HERRERA F.G. ; BERTHOLD D.R.** *FRONT ONCOL,* 2016
- **PISANSKY T.M. et al.** Salvage radiation therapy dose response for biochemical failure of prostate cancer after prostatectomy - A multi-institutional observational study. *Int J Radiat Oncol Biol Phys,* 2016, vol. 96 (5), 1046-1053
- **RESNICK M.J. et al.** Long-term functional outcomes after treatment for localized prostate cancer. *N Engl J Med,* 2013, vol. 368 (5), 436-445
- **HEGARTY S.E. et al.** Radiation therapy after radical prostatectomy for prostate cancer: Evaluation of complications and influence of radiation timing on outcomes in a large, population-based cohort. *PLoS One,* 2015, vol. 10 (2
- **PARAVATI A.J. et al.** Variation in the cost of radiation therapy among medicare patients with cancer. *J Oncol Pract,* 2015, vol. 11 (5), 403-409
- **HALL W.A. et al.** Biomarkers of outcome in patients with localized prostate cancer treated with radiotherapy. *Semin Radiat Oncol,* 20 November 2016, vol. 27
- **RAYMOND E. et al.** An appraisal of analytical tools used in predicting clinical outcomes following radiation therapy treatment of men with prostate cancer: A systematic review. *Radiat Oncol,* 2017, vol. 12 (1), 56
- **BERTHOLD D.R.** *RADIAT ONCOL,* 2016
- **HALL W.A. et al.** *RADIAT ONCOL,* 2016
- **DAL PRA A. et al.** *RADIAT ONCOL,* 2018
- **MANTOVANI A. et al.** Cancer-related inflammation. *Nature,* 2008, vol. 454 (7203), 436-444
- **GIRALDO N.A. et al.** The clinical role of the TME in solid cancer. *Br J Cancer,* 2019, vol. 120 (1), 45-53
- **GIRALDO N.A. et al.** *BR J CANCER,* 2019
- **HALL W.A. et al.** *BR J CANCER,* 2016
- **SHIAO S.L. et al.** Regulation of prostate cancer progression by tumor microenvironment. *Cancer Lett,* 2016, vol. 380 (1), 340-348
- **BARKER H.E. et al.** The tumor microenvironment after radiotherapy: Mechanisms of resistance or recurrence. *Nat Rev Cancer,* 2015, vol. 15 (7), 409-425
- **MOSENDEN R. ; TASKEN K.** Cyclic AMP-mediated immune regulation - Overview of mechanisms of action in T-cells. *Cell Signal,* 2011, vol. 23 (6), 1009-1016
- **TASKEN K. ; RUPPELT A.** Negative regulation of T-cell receptor activation by the cAMP-PKA-Csk signaling pathway in T-cell lipid rafts. *Front Biosci,* 2006, vol. 11, 2929-2939
- **ABRAHAMSEN H. et al.** TCR- and CD28-mediated recruitment of phosphodiesterase 4 to lipid rafts potentiates TCR signaling. *J Immunol,* 2004, vol. 173, 4847-4848
- **ABRAHAMSEN H. et al.** *J IMMUNOL,* 2004
- **MOSENDEN R. ; TASKEN K.** *J IMMUNOL,* 2011
- **TASKEN K. ; RUPPELT A.** *J IMMUNOL,* 2006
- Immunity Leashed - Mechanisms of Regulation in the Human Immune System. **TORHEIM E.A.** Thesis for the degree of Philosophiae Doctor (PhD). The Biotechnology Centre of Ola, 2009
- **ABRAHAMSEN H. et al.** *THESIS FOR THE DEGREE OF PHILOSOPHIAE DOCTOR (PHD),* 2004
- **MOSENDEN R. ; TASKEN K.** *THESIS FOR THE DEGREE OF PHILOSOPHIAE DOCTOR (PHD),* 2011
- **TASKEN K. ; RUPPELT A.** *THESIS FOR THE DEGREE OF PHILOSOPHIAE DOCTOR (PHD),* 2006

- **ALVES DE INDA M. et al.** Validation of Cyclic Adenosine Monophosphate Phosphodiesterase-4D7 for its Independent Contribution to Risk Stratification in a Prostate Cancer Patient Cohort with Longitudinal Biological Outcomes. *Eur Urol Focus,* 2018, vol. 4 (3), 376-384
- **TILKI D. et al.** External validation of the European Association of Urology Biochemical Recurrence Risk groups to predict metastasis and mortality after radical prostatectomy in a European cohort. *Eur Urol,* 2019, vol. 75 (6), 896-900